# EUROPEAN PATENT SPECIFICATION

(11) **EP 0 569 527 B1**
(45) Date of publication and mention of the grant of the patent: **14.03.2001**
(21) Application number: 92906080.4
(22) Date of filing: 16.01.1992
(51) Int. Cl.: C12Q 1/68, C12N 15/12

(54) **INHERITED AND SOMATIC MUTATIONS OF APC GENE IN COLORECTAL CANCER OF HUMANS**
GEERBTE UND SOMATISCHE MUTATIONEN DES APC-GENS BEI MENSCHLICHEM COLORECTAL-KREBS
MUTATIONS HEREDITAIRES ET SOMATIQUES DU GENE APC DANS LES CANCERS RECTO-COLIQUES CHEZ L'HOMME

(30) Priority: 16.01.1991 GB 91009639; 08.08.1991 US 741940
(43) Date of publication of application: 18.11.1993
(73) Proprietor: THE JOHNS HOPKINS UNIVERSITY, Baltimore, MD 21205 (US); ZENECA LIMITED, London W1Y 6LN (GB); THE UNIVERSITY OF UTAH, Salt Lake City, UT 84108 (US); JAPANESE FOUNDATION FOR CANCER RESEARCH, Tokyo 170 (JP)
(72) Inventor: KINZLER, Kenneth, W., Baltimore, MD 21234 (US); VOGELSTEIN, Bert, Baltimore, MD 21208 (US); ANAND, Rakesh, Sandbach Cheshire CW11 9ES (GB); HEDGE, Philip, John, Winsford Cheshire CW7 3EA (GB); MARKHAM, Alexander, Fred, Goostrey Crewe Cheshire (GB); ALBERTSEN, Hans, Salt Lake City, UT 84103 (US); CARLSON, Mary, L., Salt Lake City, UT 84103 (US); GRODEN, Joanna, L., Salt Lake City, UT 84103 (US); JOSLYN, Geoff, Salt Lake City, UT 84103 (US); THLIVERIS, Andrew, Salt Lake City, UT 84109 (US); WHITE, Raymond, L., Salt Lake City, UT 84103 (US); NAKAMURA, Yusuke, Tokyo 204 (JP)
(74) Representative: Dost, Wolfgang, Dr.rer.nat., Dipl.-Chem.
(86) International application number: US9200376
(87) International publication number: WO9213103

(56) References cited:
- WO-A-89/01481
- WO-A-90/05180
- BIOCHEMICAL AND BIOPHYSICAL RESEARCH COMMUNICATIONS. vol. 174, no. 1, 15 January 1991, DULUTH, MINNESOTA US pages 298 - 304; Y.HOSHINO ET AL.: 'Normal human chromosome 5, on which a familial adenomatous polyposis gene is located, has tumor suppressive activity'
- SCIENCE. vol. 253, 9 August 1991, LANCASTER, PA US pages 661 - 665; K.W.KINZLER ET AL.: 'Identification of FAP locus genes from chromosome 5q21'
- SCIENCE. vol. 253, 9 August 1991, LANCASTER, PA US pages 665 - 669; I.NISHISHO ET AL.: 'Mutations of chromosome 5q21 genes in FAP and colorectal cancer patients'
- SCIENCE. vol. 253, 9 August 1991, LANCASTER, PA US page 616; J.MARX: 'Gene identified for inherited cancer susceptibility'

## Description

### TECHNICAL AREA OF THE INVENTION

The invention relates to the area of cancer diagnostics and therapeutics. More particularly, the invention relates to detection of the germline and somatic alterations of wild-type APC genes. In addition, it relates to therapeutic intervention to restore the function of APC gene product.

### BACKGROUND OF THE INVENTION

According to the model of Knudson for tumorigenesis (Cancer Research, Vol. 45, p. 1482, 1985), there are tumor suppressor genes in all normal cells which, when they become non-functional due to mutation, cause neoplastic development. Evidence for this model has been found in the cases of retinoblastoma and colorectal tumors. The implicated suppressor genes in those tumors, RB, p53, DCC and MCC, were found to be deleted or altered in many cases of the tumors studied. (Hansen and Cavenee, Cancer Research, Vol.. 47, pp. 5518-5527 (1987); Baker et al., Science, Vol.. 244, p. 217 (1989); Fearon et al., Science, Vol. 247, p. 49 (1990); Kinzler et al. Science Vol. 251. p. 1366 (1991).)

In order to fully understand the pathogenesis of tumors, it will be necessary to identify the other suppressor genes that play a role in the tumorigenesis process. Prominent among these is the one(s) presumptively located at 5q21. Cytogenetic (Herrera et al., Am J. Med. Genet., Vol. 25, p. 473 (1986) and linkage (Leppert et al., Science, Vol. 238, p. 1411 (1987); Bodmer et al., Nature, Vol. 328, p. 614 (1987)) studies have shown that this chromosome region harbors the gene responsible for familial adenomatous polyposis (FAP) and Gardner's Syndrome (GS). FAP is an autosomal-dominant, inherited disease in which affected individuals develop hundreds to thousands of adenomatous polyps, some of which progress to malignancy. GS is a variant of FAP in which desmoid tumors, osteomas and other soft tissue tumors occur together with multiple adenomas of the colon and rectum. A less severe form of polyposis has been identified in which only a few (2-40) polyps develop. This condition also is familial and is linked to the same chromosomal markers as FAP and GS (Leppert et al., New England Journal of Medicine, Vol. 322, pp. 904-908, 1990.) Additionally, this chromosomal region is often deleted from the adenomas (Vogelstein et al., N. Engl. J. Med., Vol. 319, p. 525 (1988)) and carcinomas (Vogelstein et al., N. Engl. J. Med., Vol. 319, p. 525 (1988); Solomon et al., Nature, Vol. 328, p. 616 (1987); Sasaki et al., Cancer Research, Vol. 49, p. 4402 (1989); Delattre et al., Lancet, Vol. 2, p. 353 (1989); and Ashton-Rickardt et al., Oncogene, Vol. 4, p. 1169 (1989)) of patients without FAP (sporadic tumors). Thus, a putative suppressor gene on chromosome 5q21 appears to play a role in the early stages of colorectal neoplasia in both sporadic and familial tumors.

Although the MCC gene has been identified on 5q21 as a candidate suppressor gene, it does not appear to be altered in FAP or GS patients. Thus there is a need in the art for investigations of this chromosomal region to identify genes and to determine if any of such genes are associated with FAP and/or GS and the process of tumorigenesis.

### SUMMARY OF THE INVENTION

It is an object of the present invention to provide an in vitro method for diagnosing and prognosing a neoplastic tissue of a human.

It is another object of the invention to provide an in vitro method of detecting genetic predisposition to cancer.

It is another object of the invention to provide a wild-type APC gene for use in a method of supplying wild-type APC gene function to a cell which has lost said gene function.

It is yet another object of the invention to provide a kit for determination of the nucleotide sequence of APC alleles by the polymerase chain reaction.

It is still another object of the invention to provide nucleic acid probes for detection of mutations in the human APC gene.

It is still another object of the invention to provide a cDNA molecule encoding the APC gene product.

It is yet another object of the invention to provide a preparation of the human APC protein.

It is another object of the invention to provide an in vitro method of screening for genetic predisposition to cancer.

It is an object of the invention to provide methods of testing therapeutic agents for the ability to suppress neoplasia.

It is still another object of the invention to provide animals carrying mutant APC alleles.

These and other objects of the invention are provided by one or more of the embodiments which are described below. In one embodiment of the present invention a method of diagnosing or prognosing a neoplastic tissue of a human is provided comprising: detecting somatic alteration of wild-type APC genes or their expression products in a sporadic colorectal cancer tissue, said alteration indicating neoplasia of the tissue.

In yet another embodiment an in vitro method is provided of detecting genetic predisposition to cancer in a human including familial adenomatous polyposis (FAP) and Gardner's Syndrome (GS), comprising: using an isolated human sample selected from the group consisting of blood and fetal tissue; detecting alteration of wild-type APC gene coding sequences or their expression products from the sample, said alteration indicating genetic predisposition to cancer.

In another embodiment of the present invention a wild-type APC gene for use in a method is provided for supplying wild-type APC gene function to a cell which has lost said gene function by virtue of a mutation in the APC gene, said method comprising: introducing a wild-type APC gene into a cell which has lost said gene function such that said wild-type gene is expressed in the cell.

In another embodiment a wild-type APC gene for use in a method of supplying wild-type APC gene function to a cell is provided said method comprising: introducing a portion of a wild-type APC gene into a cell which has lost said gene function such that said portion is expressed in the cell, said portion encoding a part of the APC protein which is required for non-neoplastic growth of said cell. APC protein can also be applied to cells or administered to animals to remediate for mutant APC genes. Synthetic peptides or drugs can also be used to mimic APC function in cells which have altered APC expression.

In yet another embodiment a pair of single stranded primers is provided for determination of the nucleotide sequence of the APC gene by polymerase chain reaction. The sequence of said pair of single stranded DNA primers is derived from chromosome 5q band 21, said pair of primers allowing synthesis of APC gene coding sequences.

In still another embodiment of the invention a nucleic acid probe is provided which is complementary to human wild-type APC gene coding sequences and which can form mismatches with mutant APC genes, thereby allowing their detection by enzymatic or chemical cleavage or by shifts in electrophoretic mobility.

In another embodiment of the invention a method is provided for detecting the presence of a neoplastic tissue in a human. The method comprises the use of an isolated body sample from a human; detecting in said sample alteration of a wild-type APC gene sequence or wild-type APC expression product, said alteration indicating the presence of a neoplastic tissue in the human.

In still another embodiment a cDNA molecule is provided which comprises the coding sequence of the APC gene.

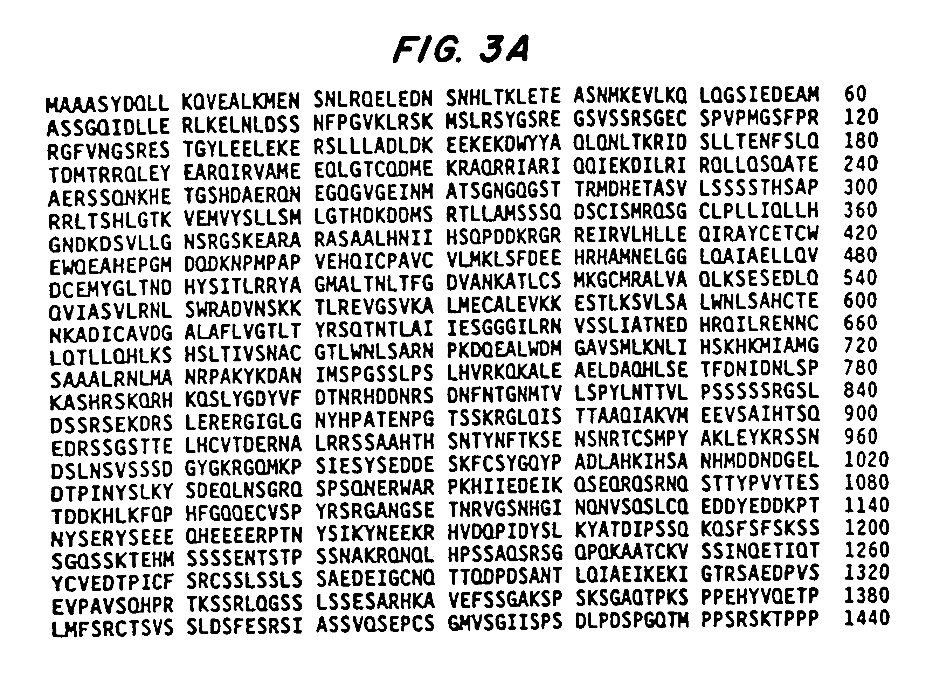

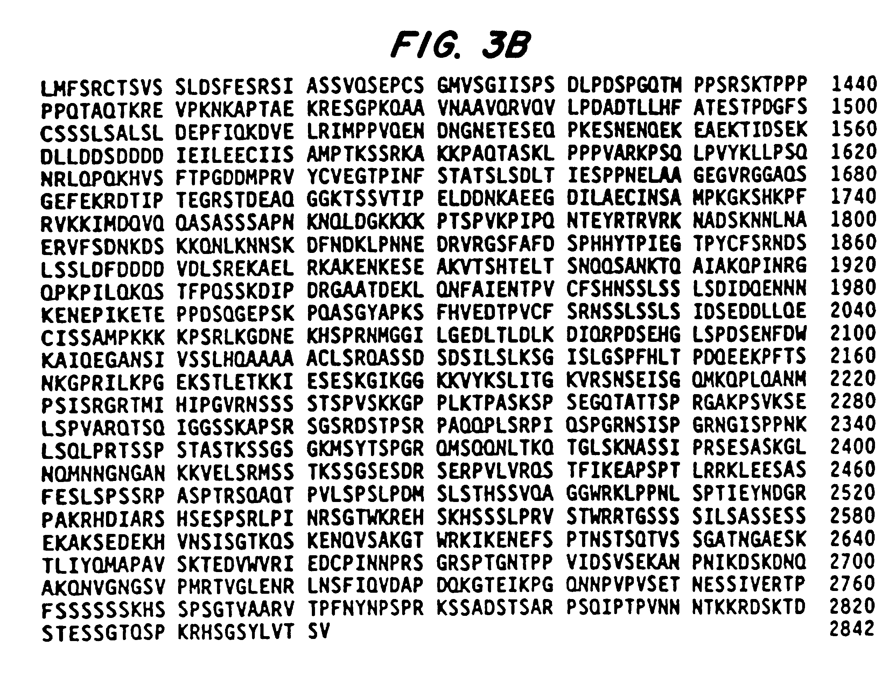

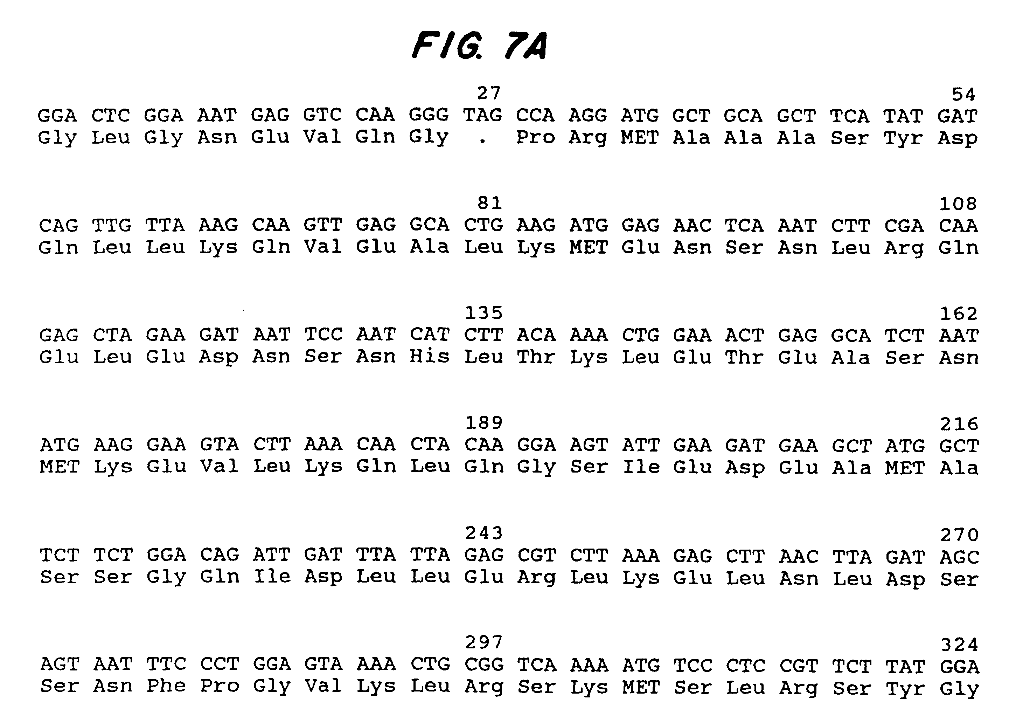

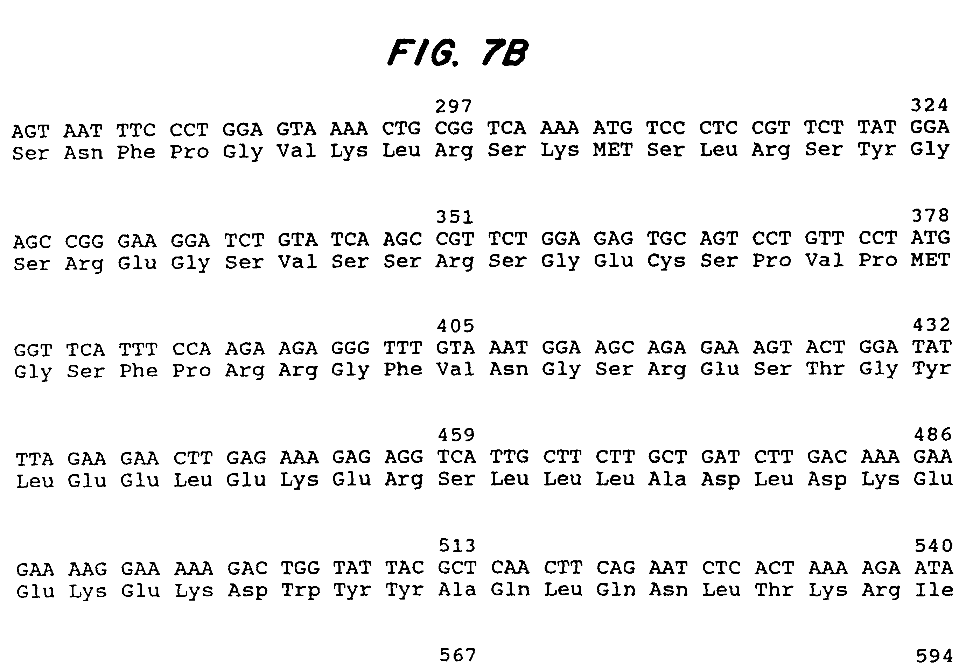

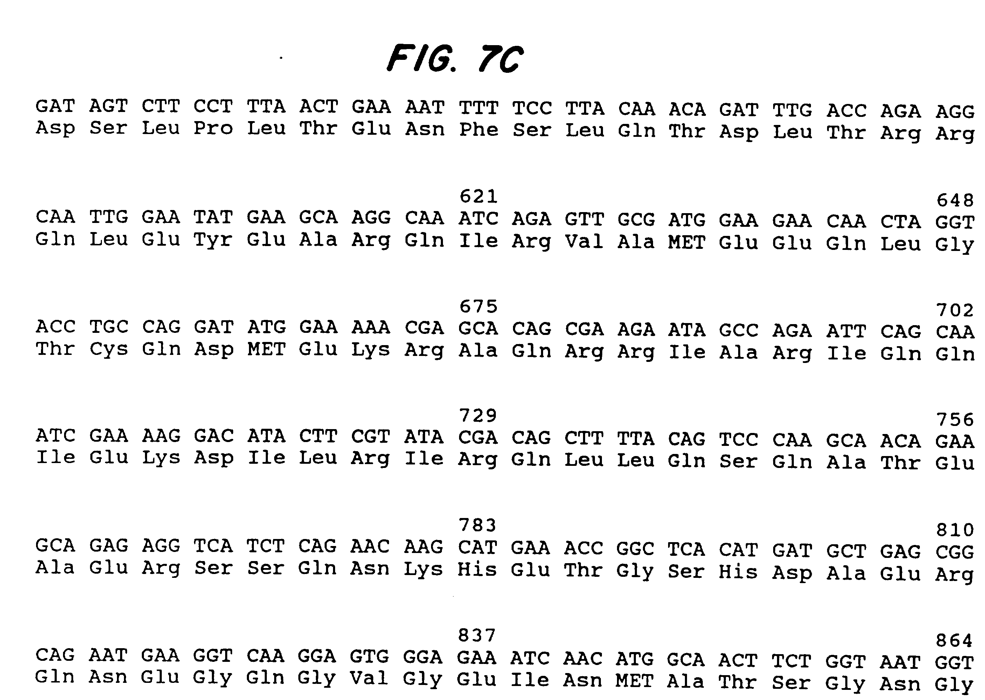

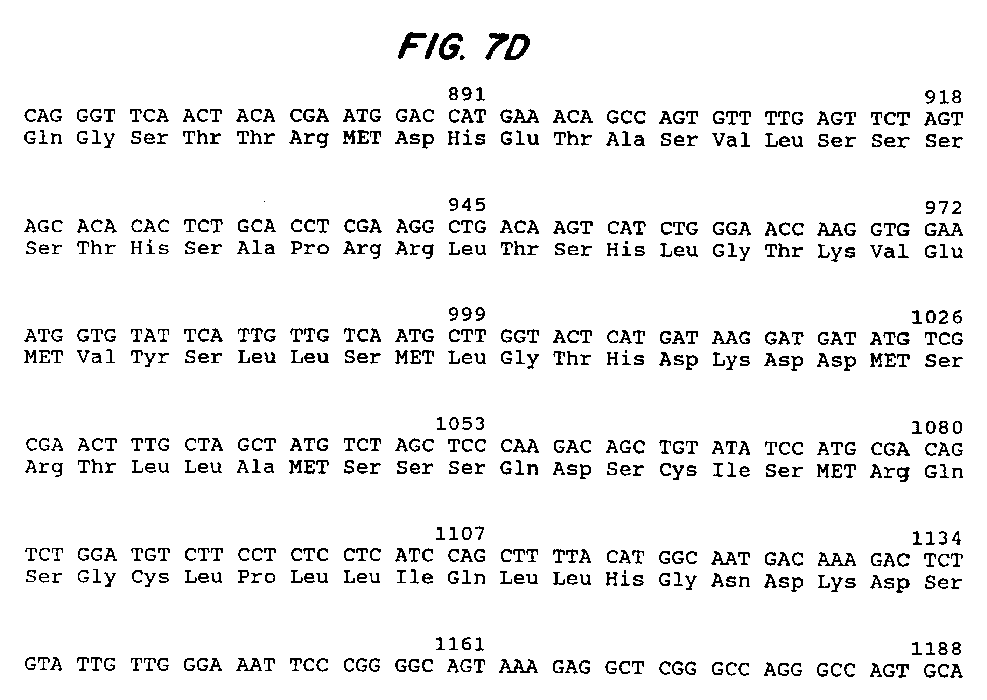

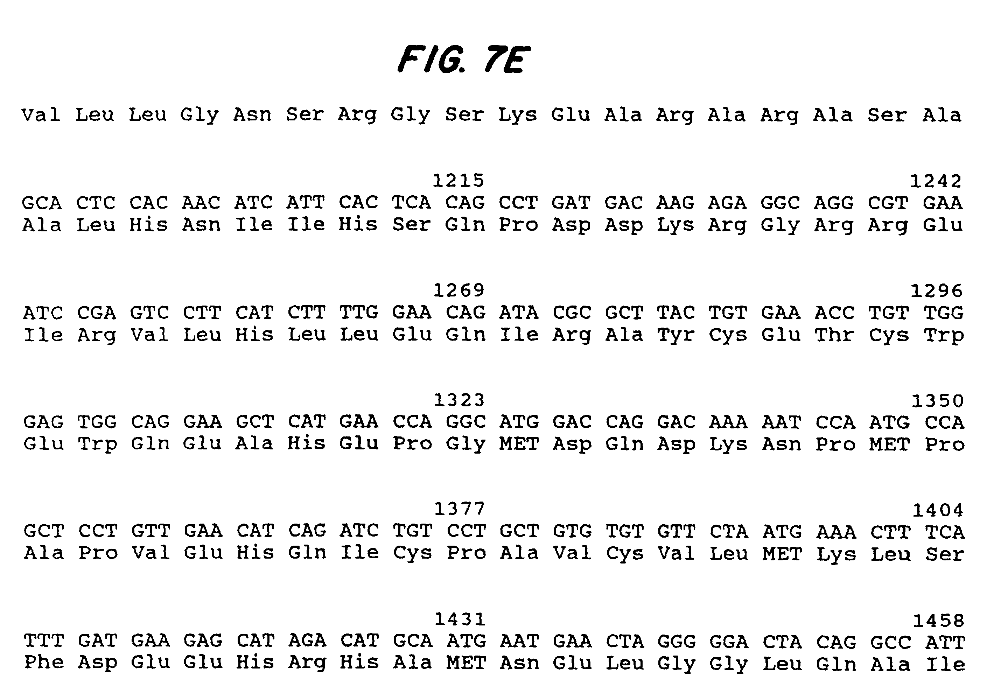

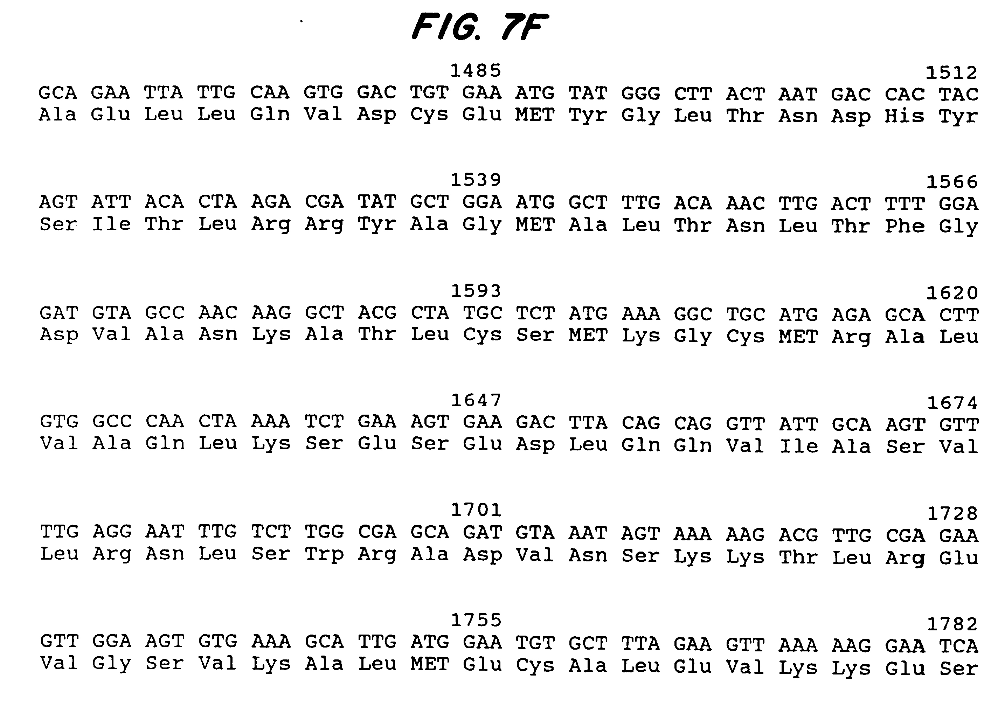

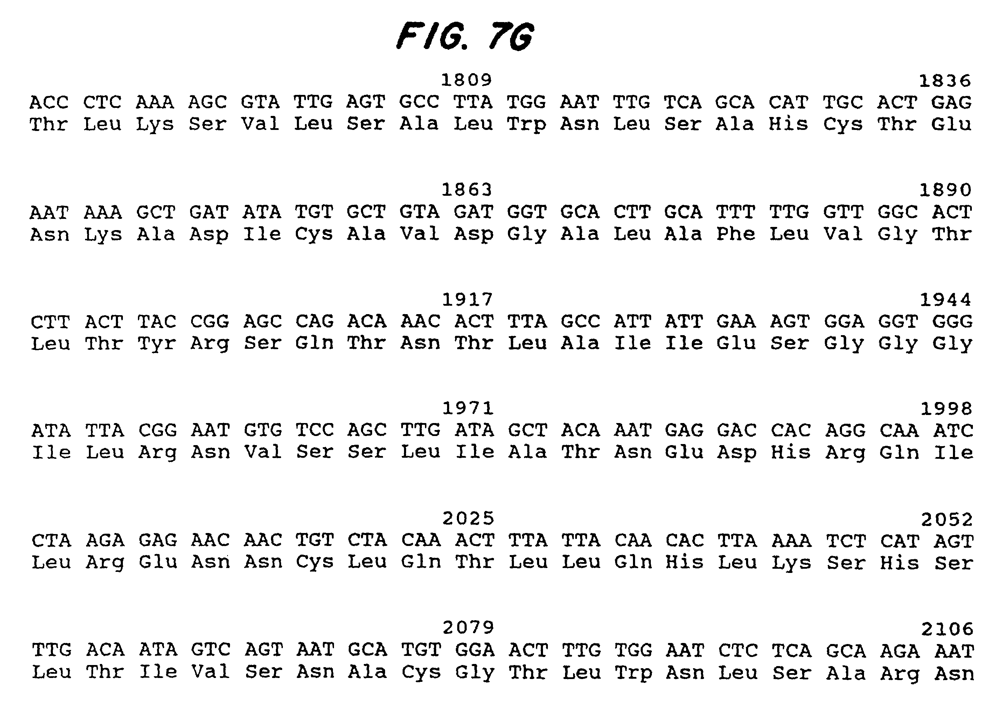

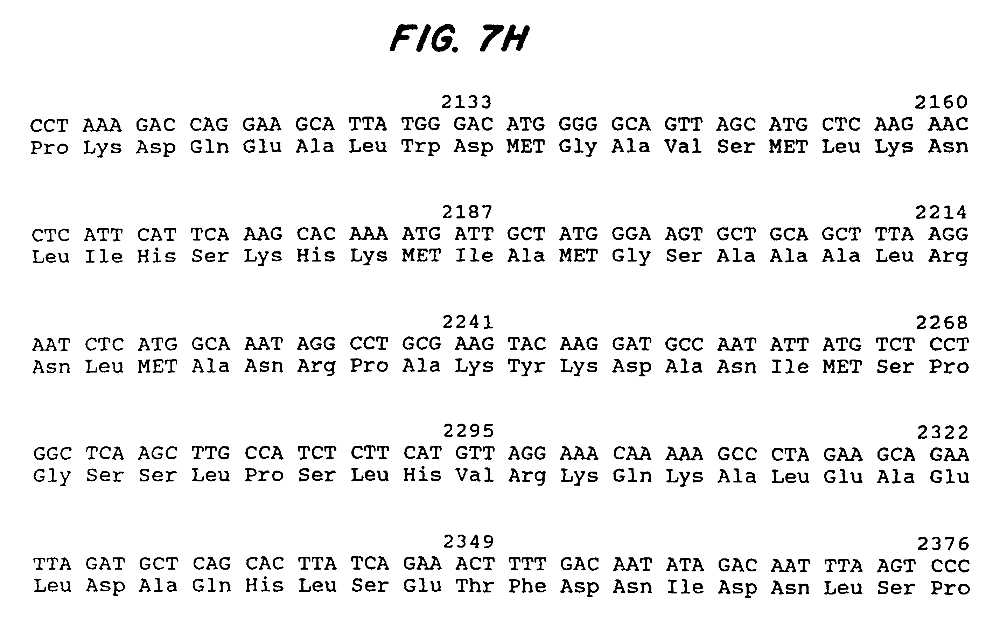

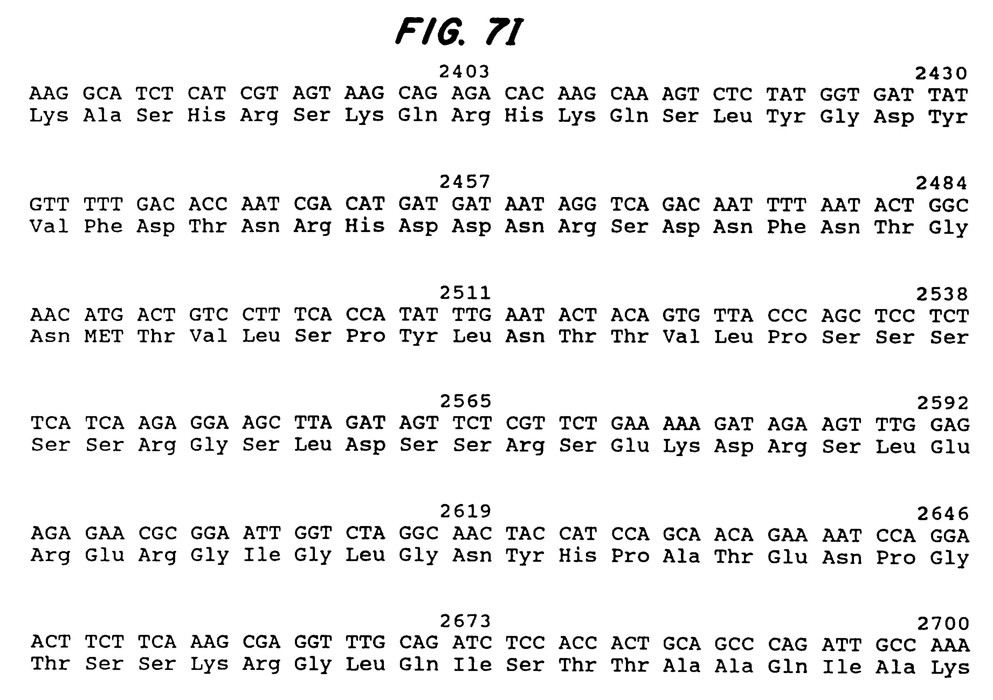

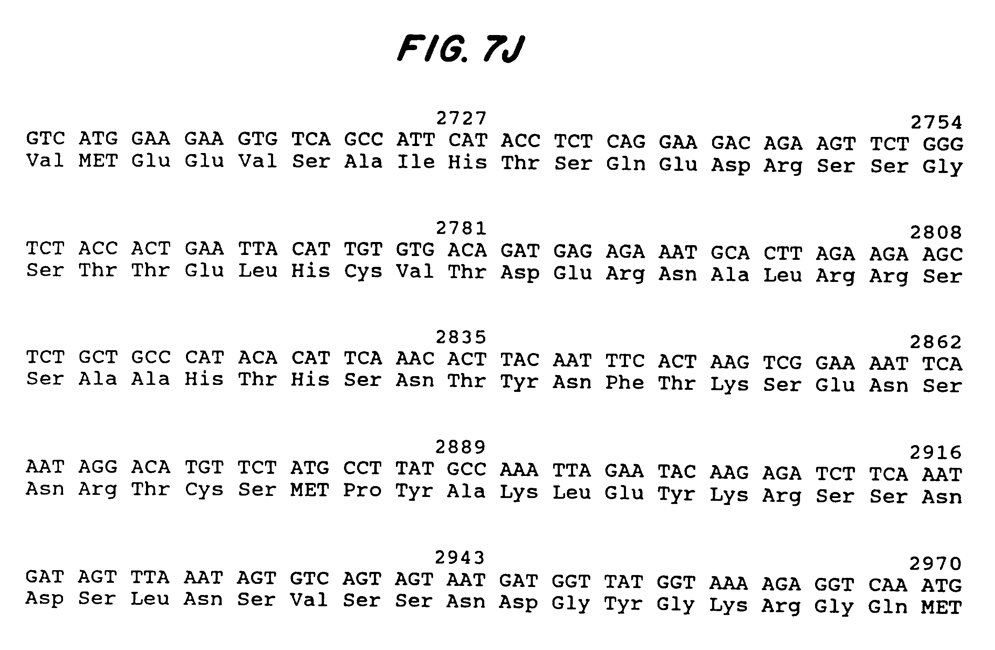

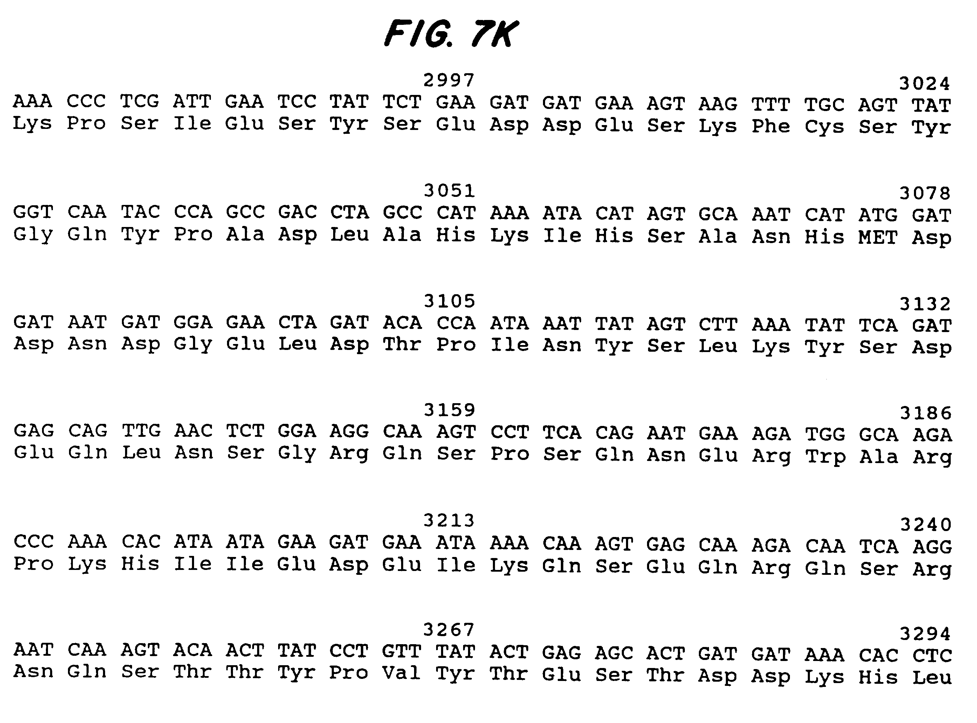

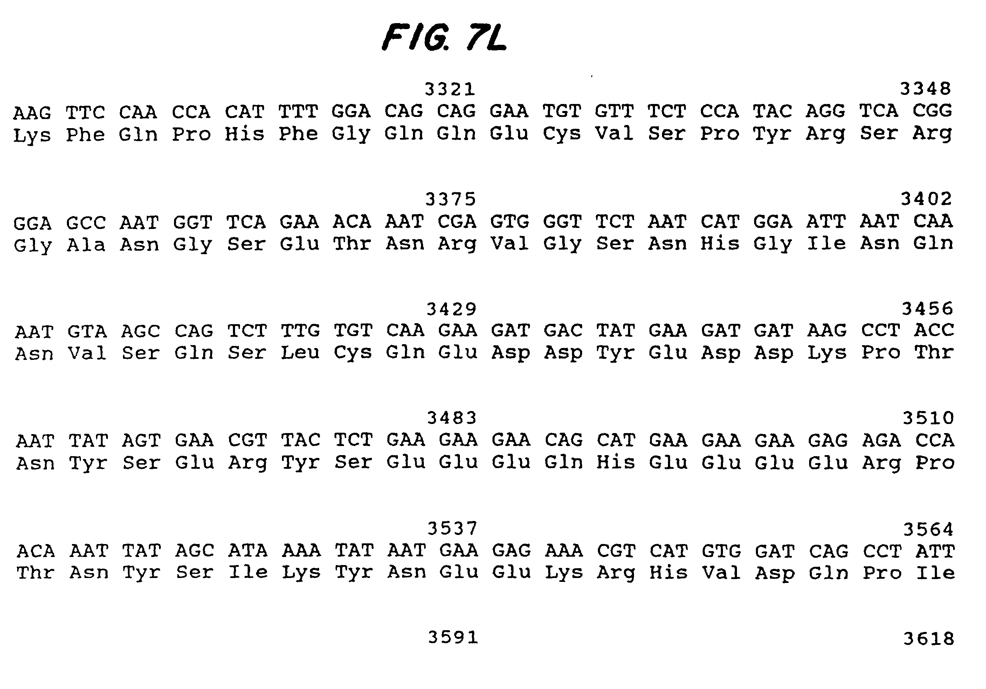

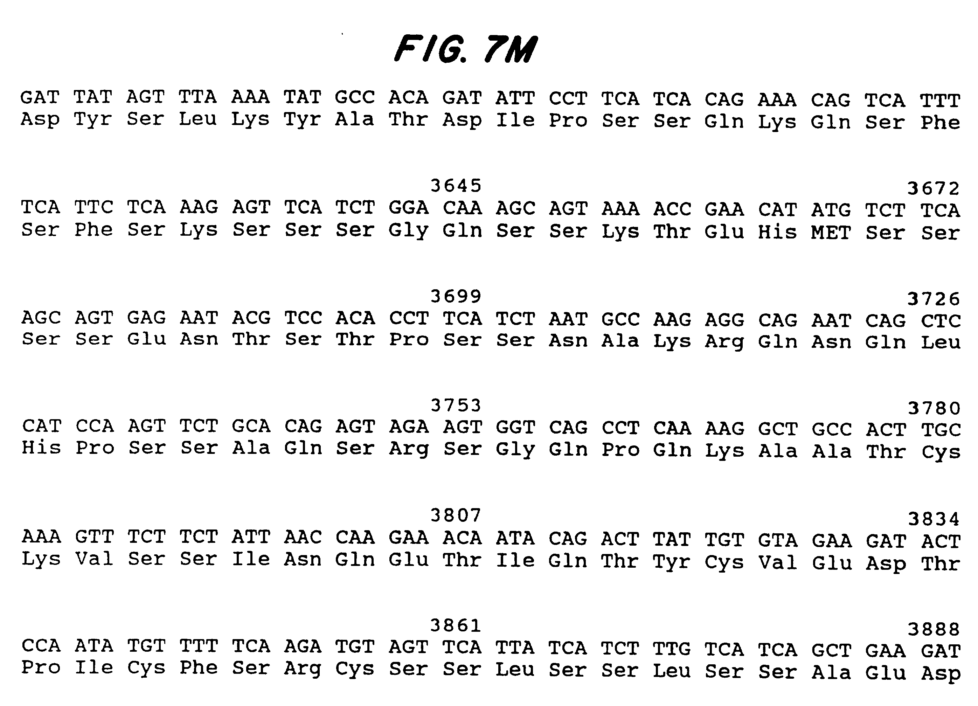

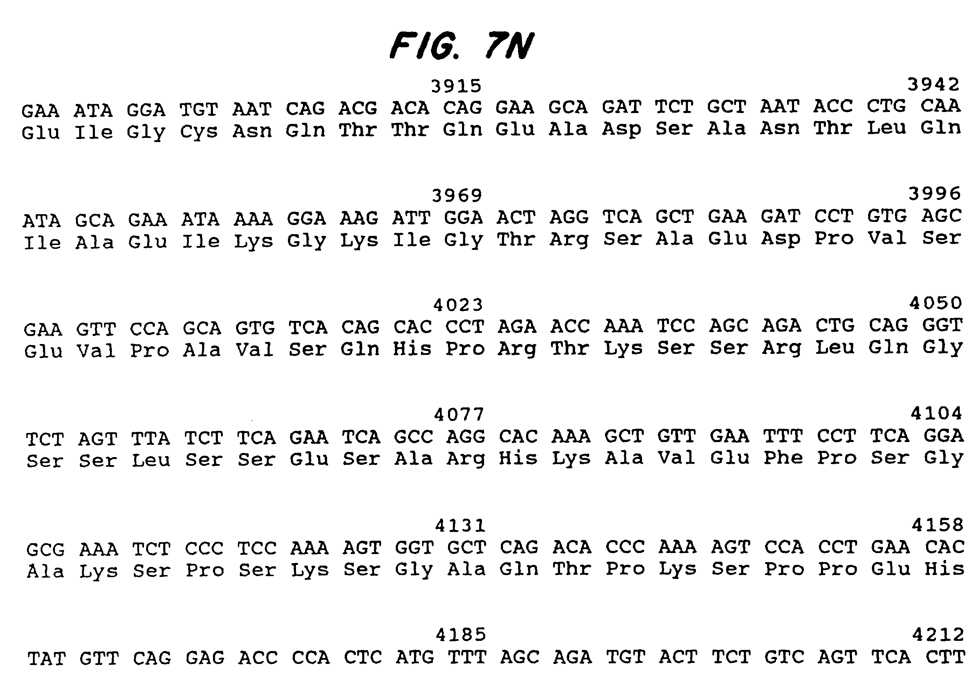

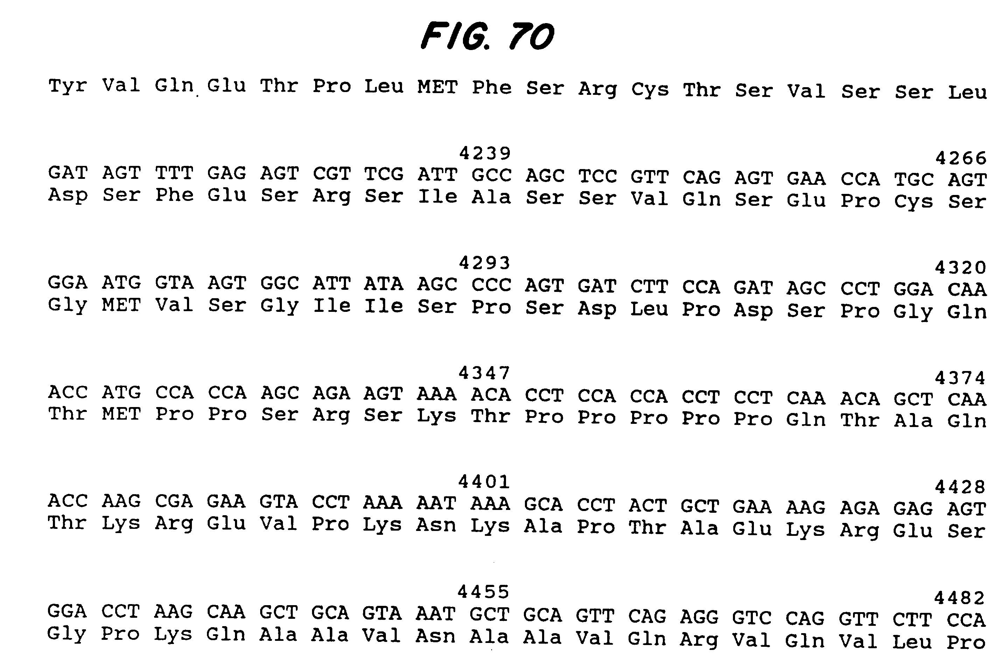

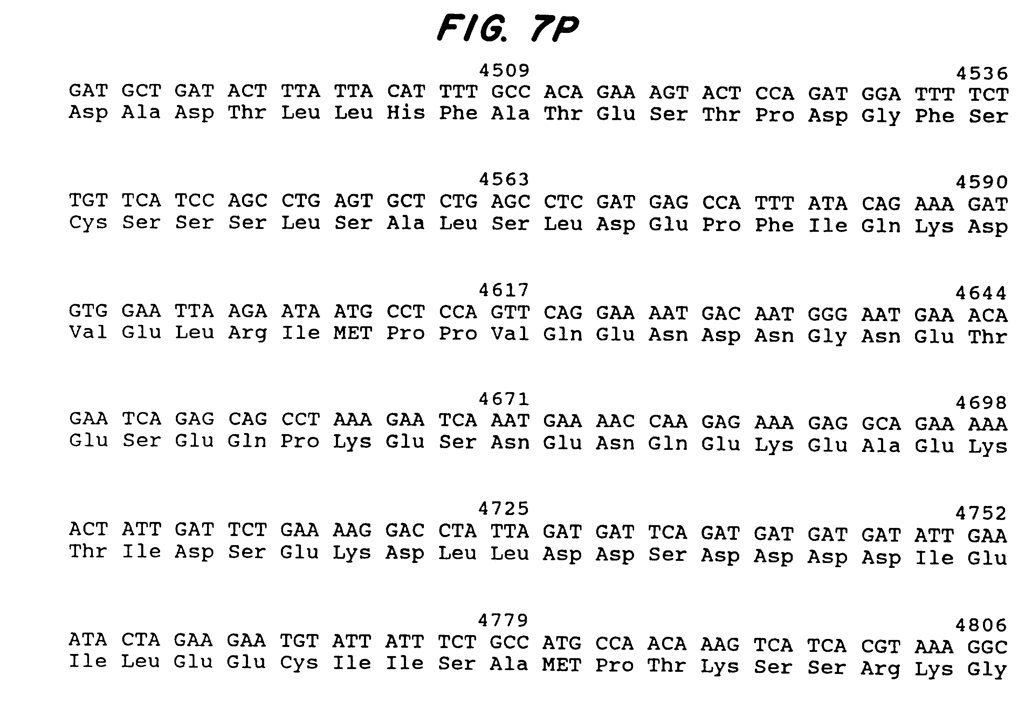

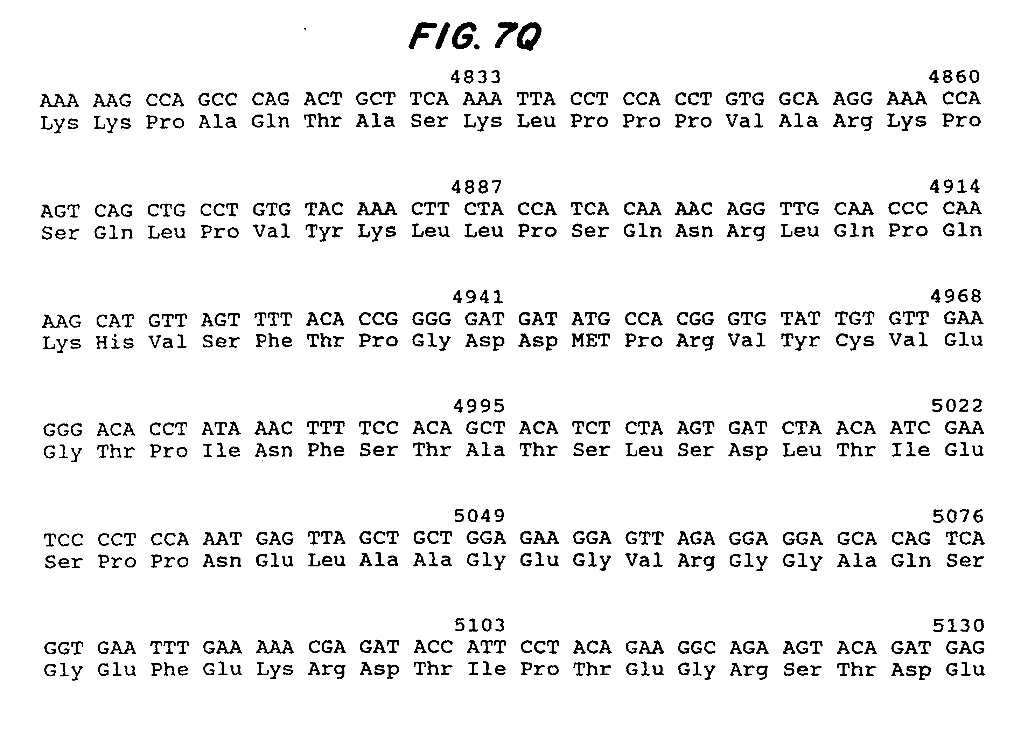

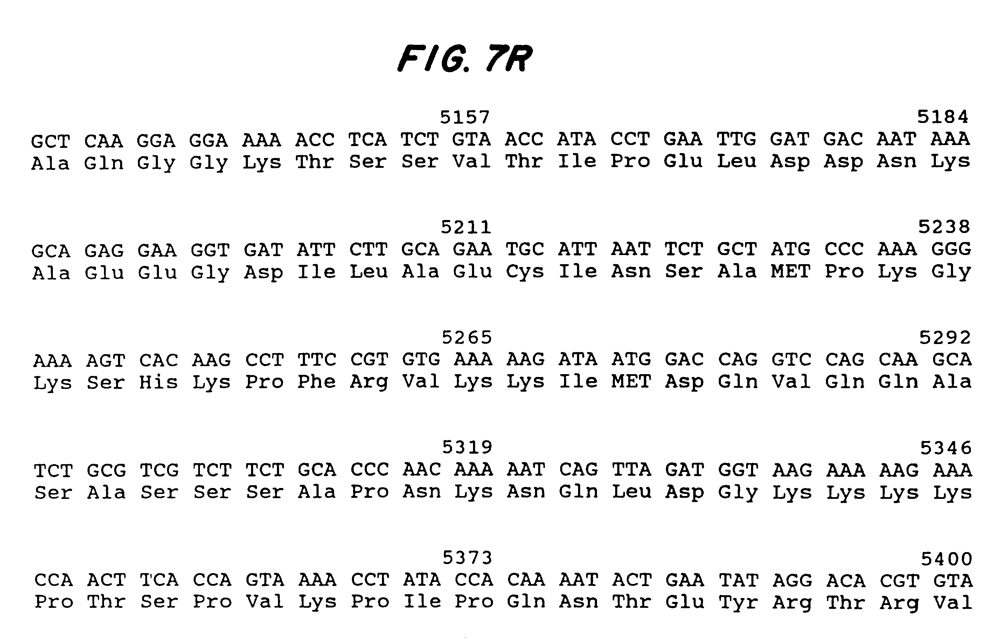

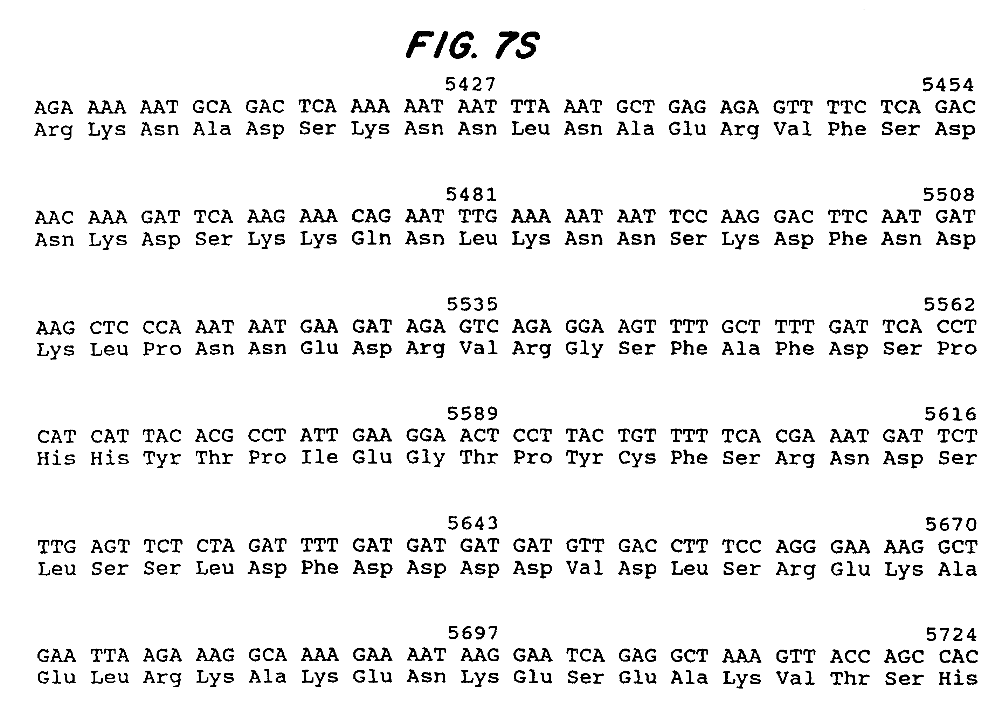

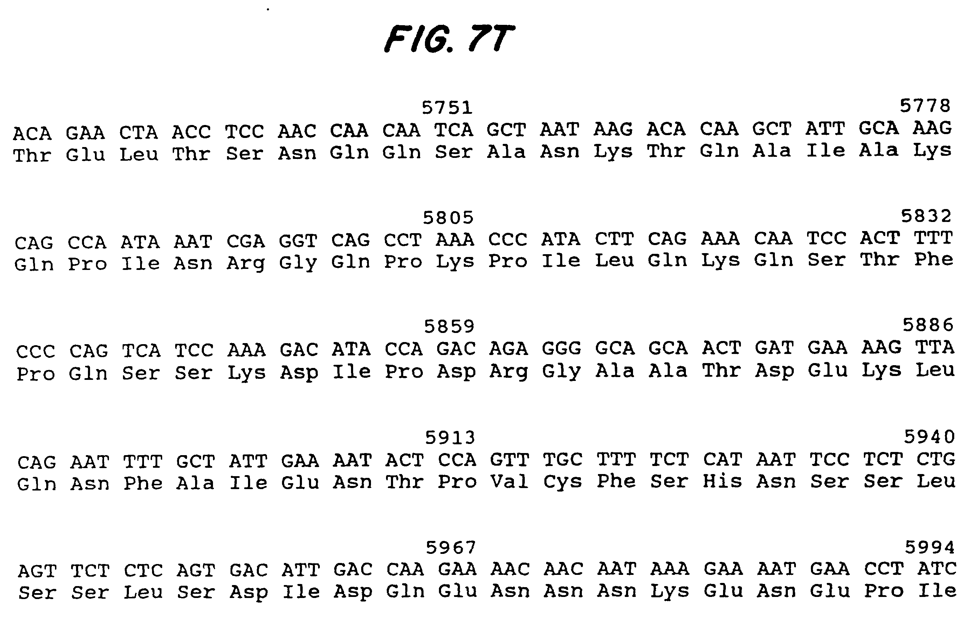

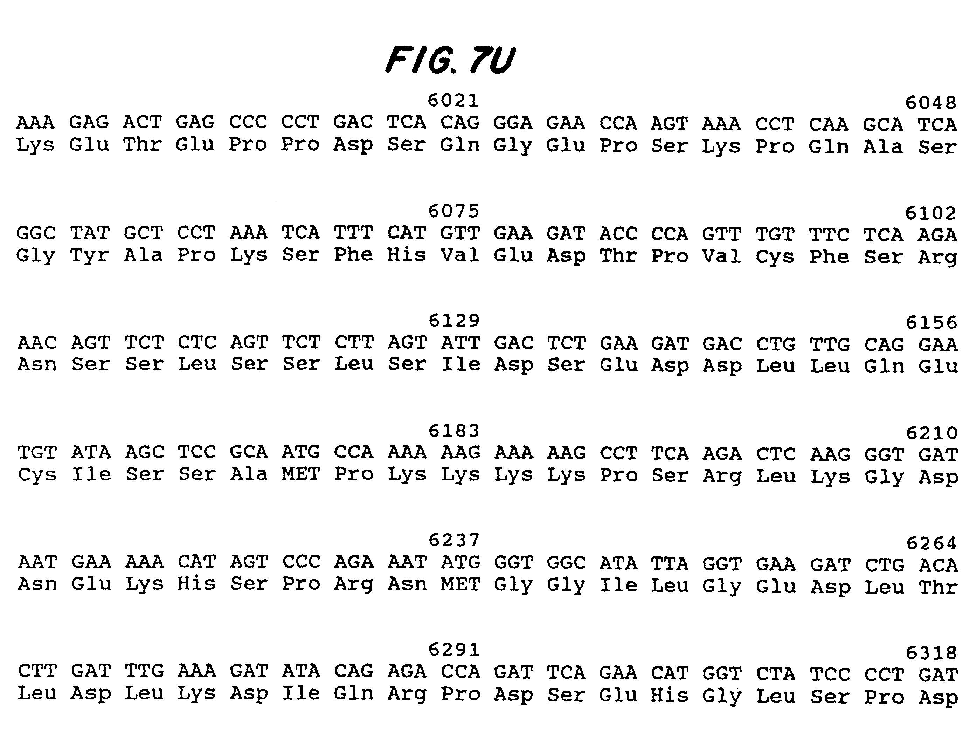

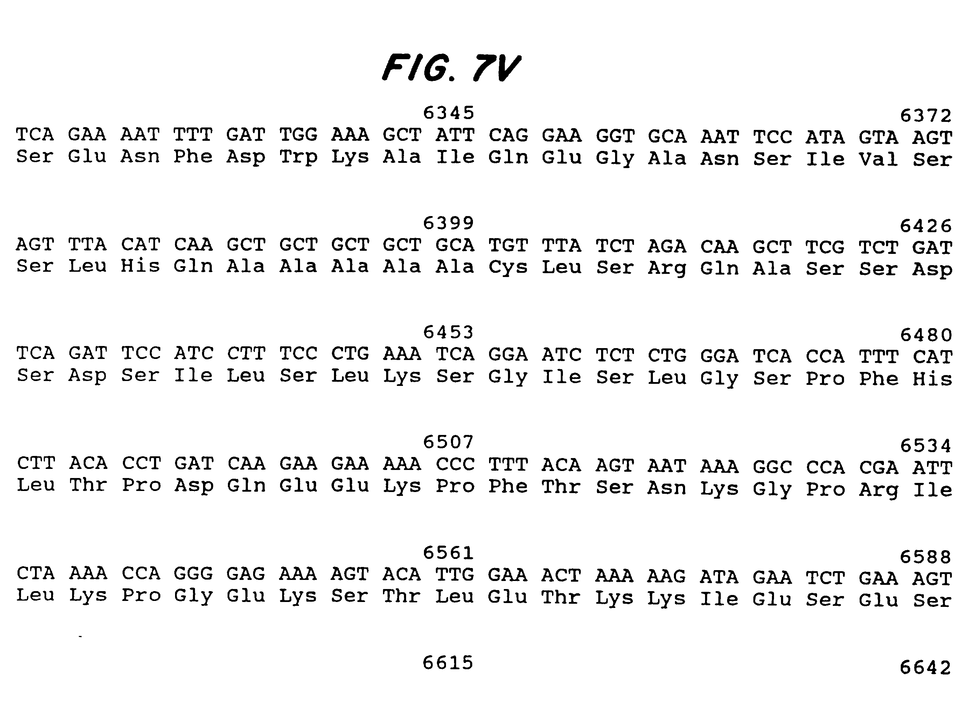

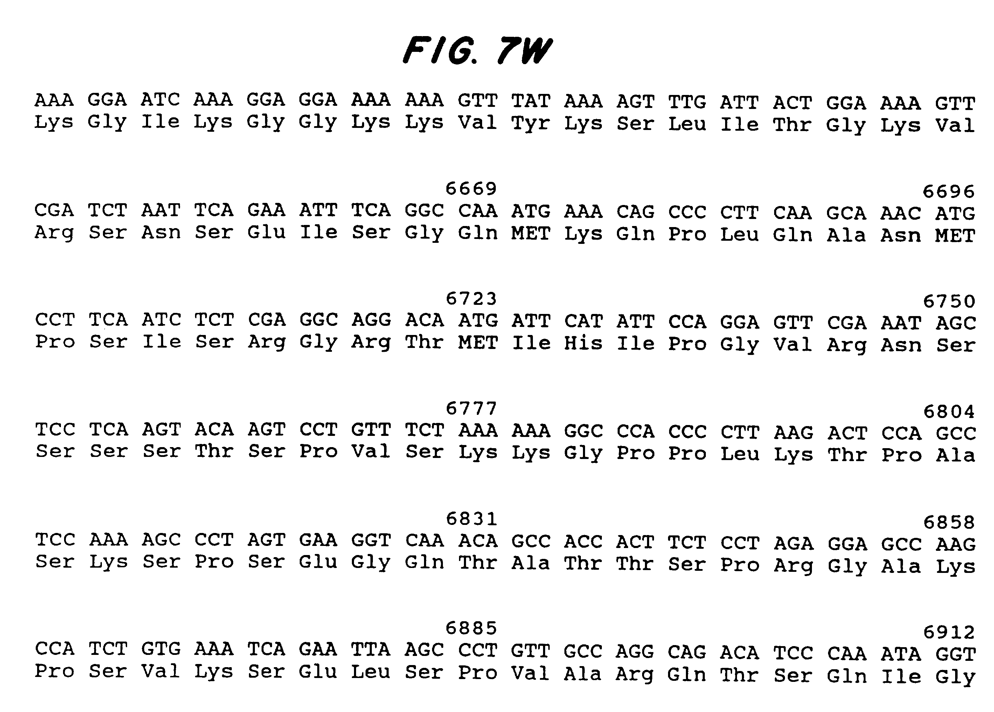

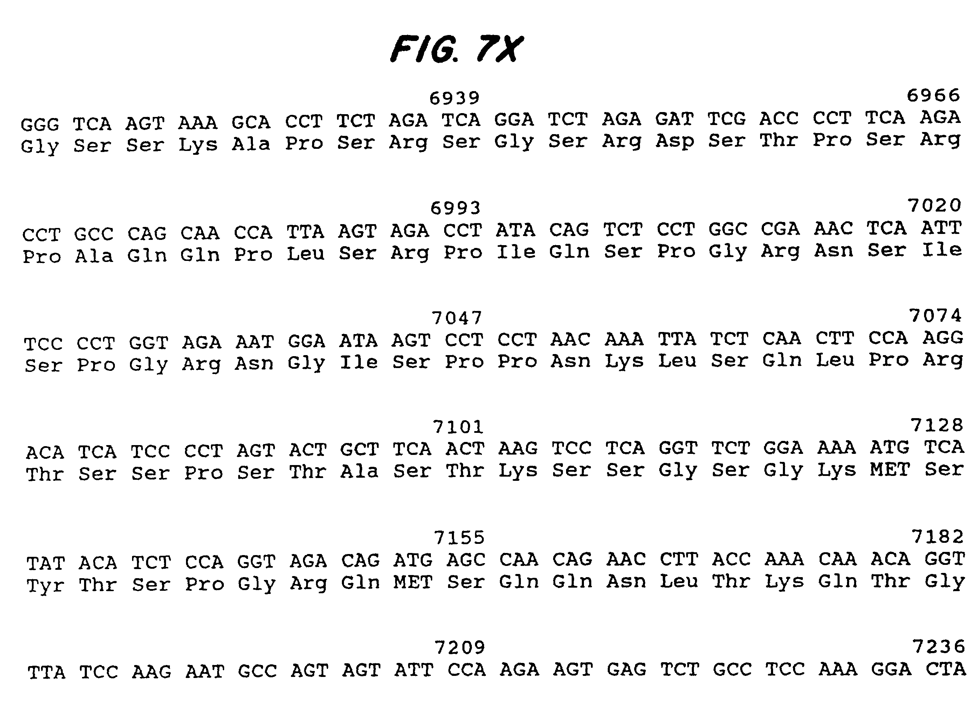

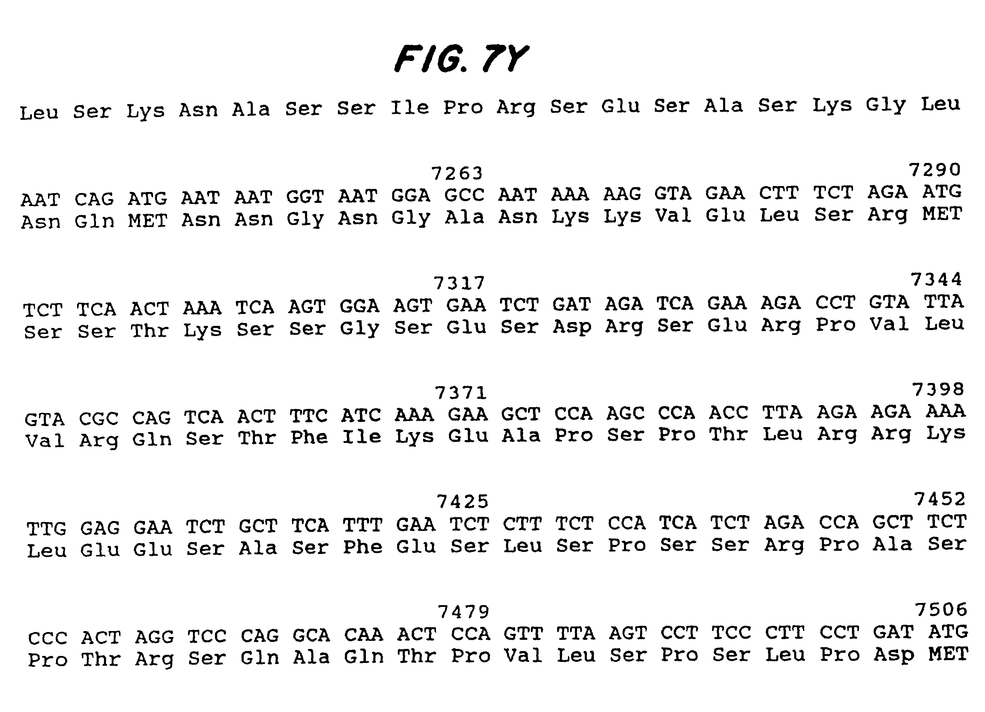

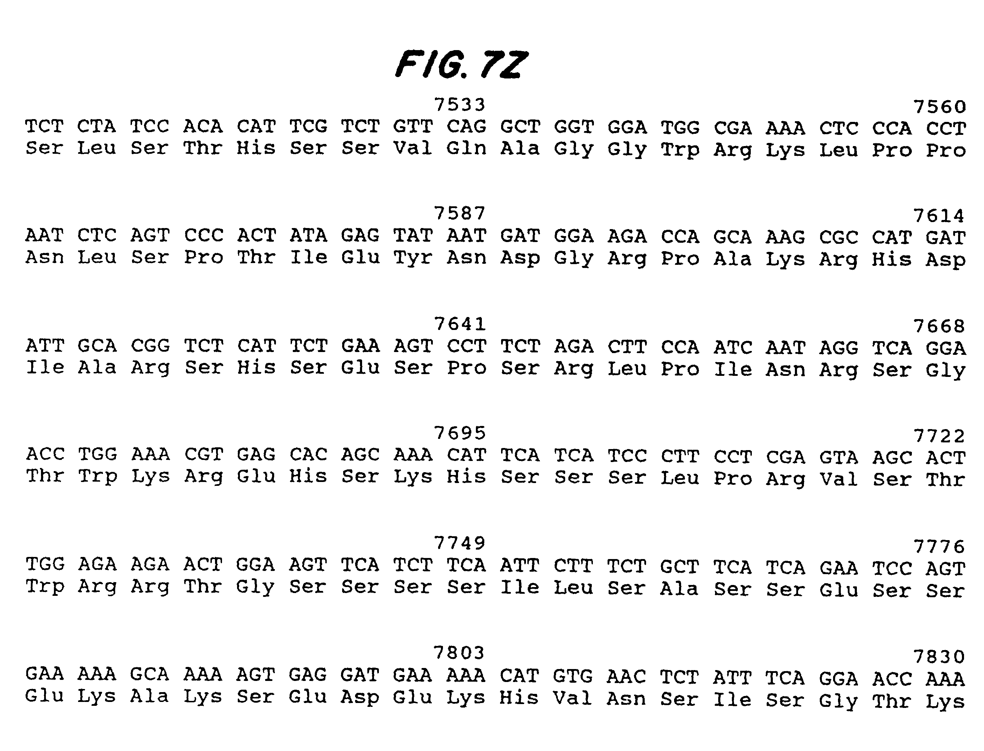

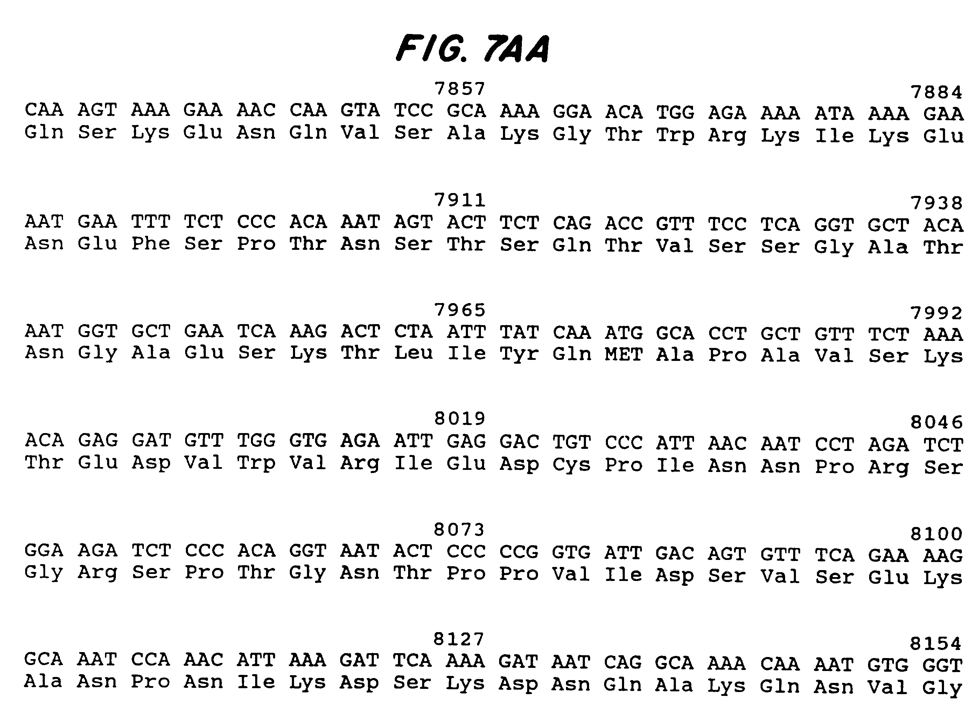

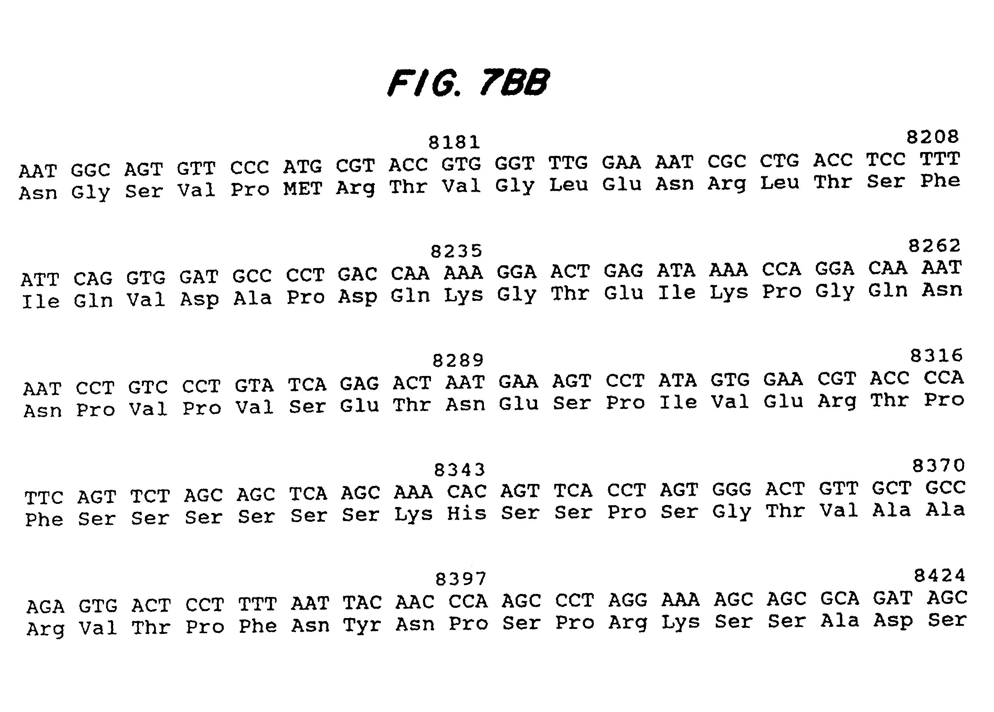

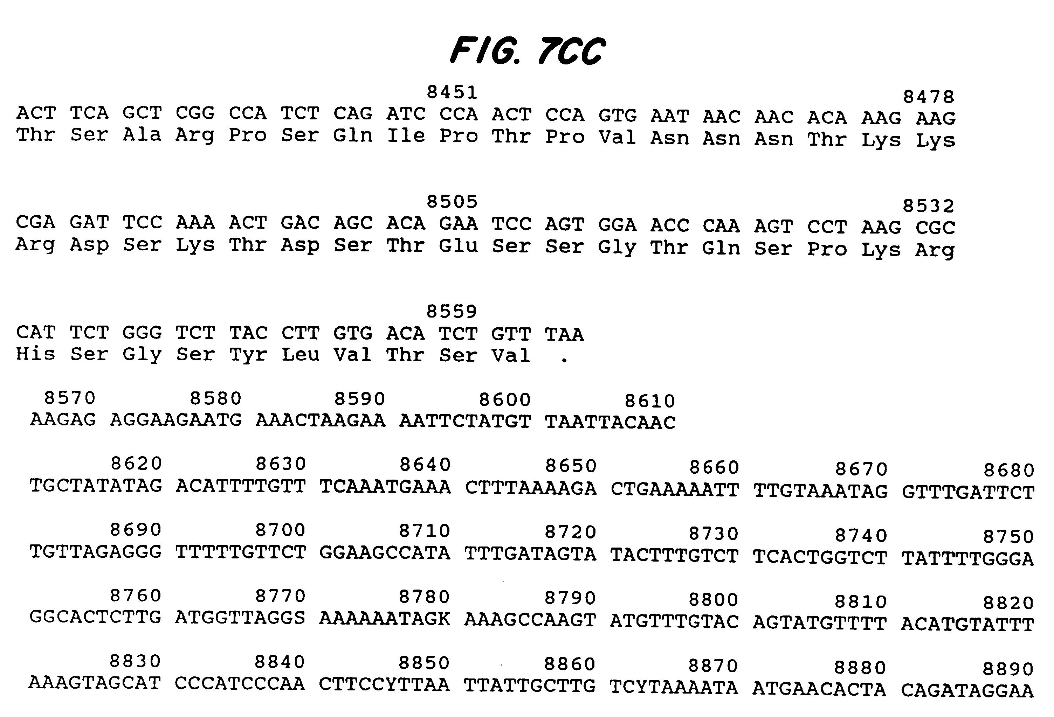

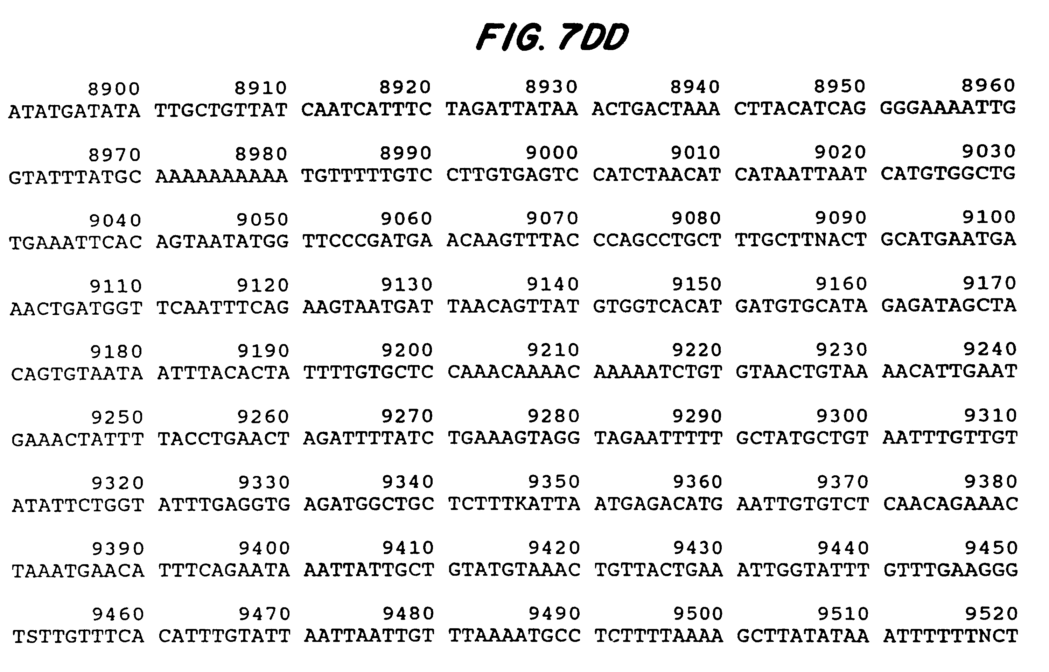

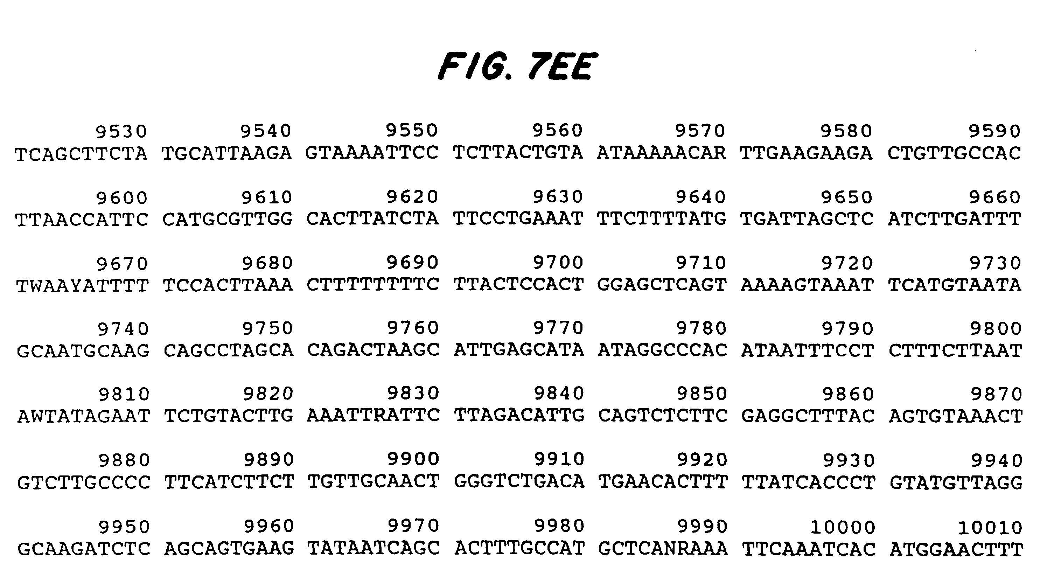

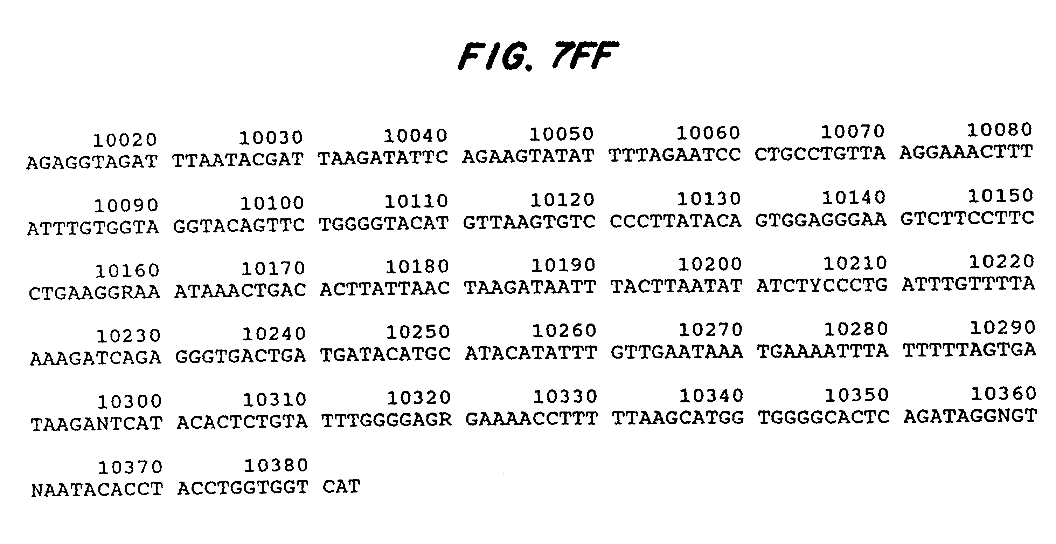

In even another embodiment a preparation of the human APC protein is provided which is substantially free of other human proteins. The amino acid sequence of the protein is shown in Figure 3 or 7.

In yet another embodiment of the invention an in vitro method is provided for screening for genetic predisposition to cancer, including familial adenomatous polyposis (FAP) and Gardner's Syndrome (GS), in a human. The method comprises: detecting among kindred persons the presence of a DNA polymorphism which is linked to a mutant APC allele in an individual having a genetic predisposition to cancer, said kindred being genetically related to the individual, the presence of said polymorphism suggesting a predisposition to cancer.

In another embodiment of the invention a method of testing therapeutic agents for the ability to suppress a neoplastically transformed phenotype is provided. The method comprises: applying a test substance to a cultured epithelial cell which carries a mutation in an APC allele; and determining whether said test substance suppresses the neoplastically transformed phenotype of the cell.

In another embodiment of the invention a method of testing therapeutic agents for the ability to suppress a neoplastically transformed phenotype is provided. The method comprises: administering a test substance to an animal to the exclusion of humans which carries a mutant APC allele; and determining whether said test substance prevents or suppresses the growth of tumors.

In still other embodiments of the invention transgenic animals are provided. The animals carry a mutant APC allele from a second animal species or have been genetically engineered to contain an insertion mutation which disrupts an APC allele.

The present invention provides the art with the information that the APC gene, a heretofore unknown gene is, in fact, a target of mutational alterations on chromosome 5q21 and that these alterations are associated with the process of tumorigenesis. This information allows highly specific assays to be performed to assess the neoplastic status of a particular tissue or the predisposition to cancer of an individual. This invention has applicability to Familial Adenomatous Polyposis, sporadic colorectal cancers, Gardner's Syndrome, as well as the less severe familial polyposis discusses above.

### BRIEF DESCRIPTION OF THE DRAWINGS

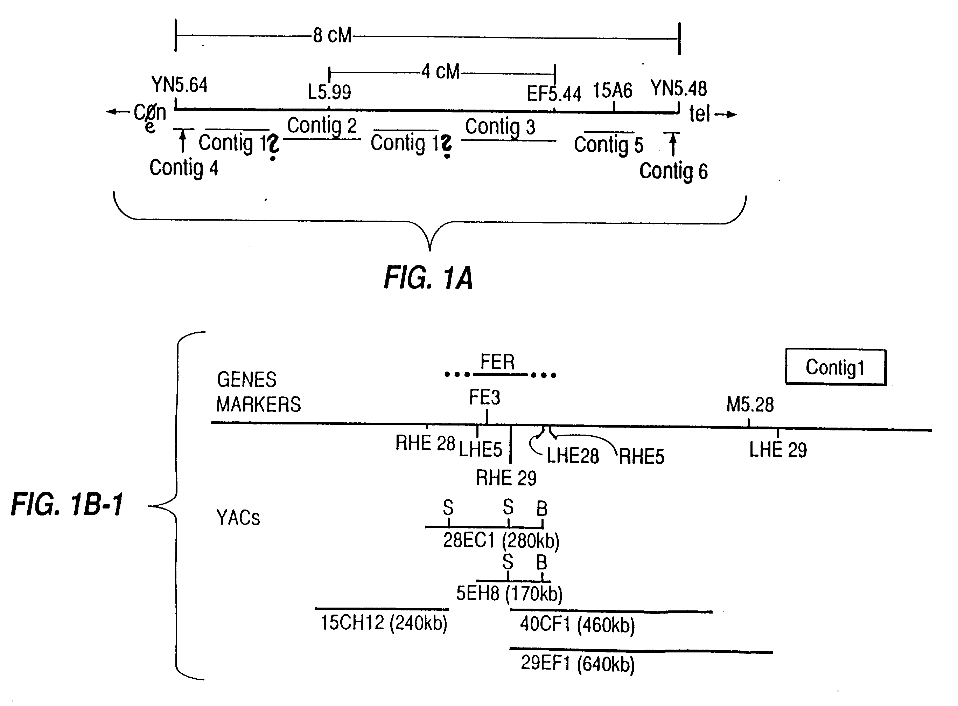

Figure 1A shows an overview of yeast artificial chromosome (YAC) contigs. Genetic distances between selected RFLP markers from within the contigs are shown in centiMorgans.

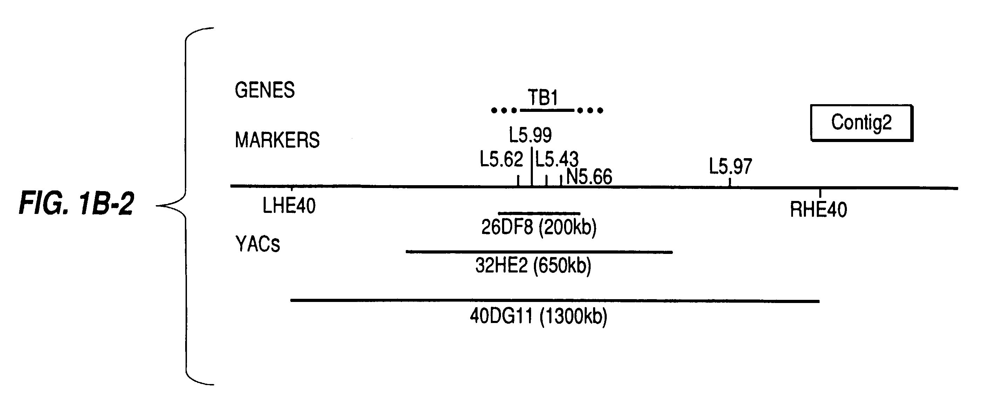

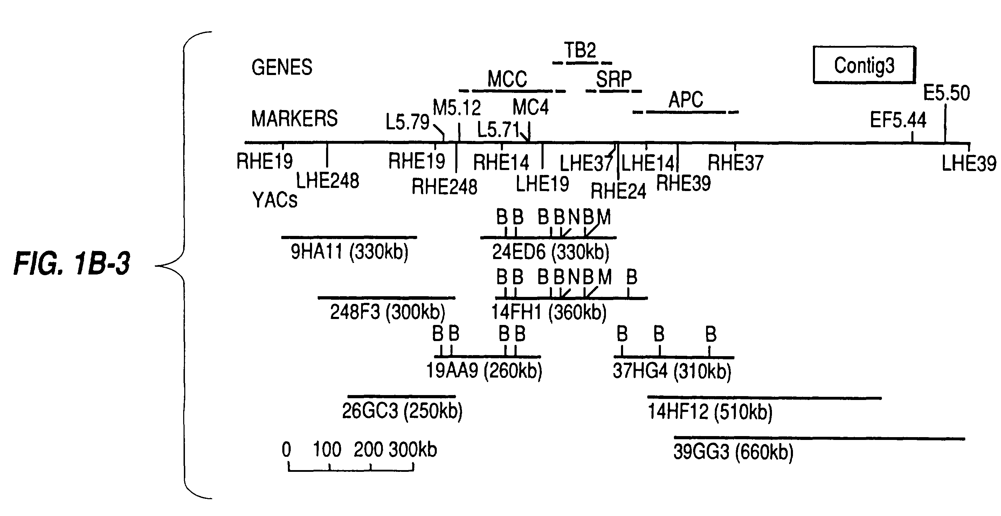

Figure 1B shows a detailed map of the three central contigs. The position of the six identified genes from within the FAP region is shown; the 5' and 3' ends of the transcripts from these genes have in general not yet been isolated, as indicated by the string of dots surrounding the bars denoting the genes' positions. Selected restriction endonuclease recognition sites are indicated. B, BssH2; S, SstII; M, MluI; N, NruI.

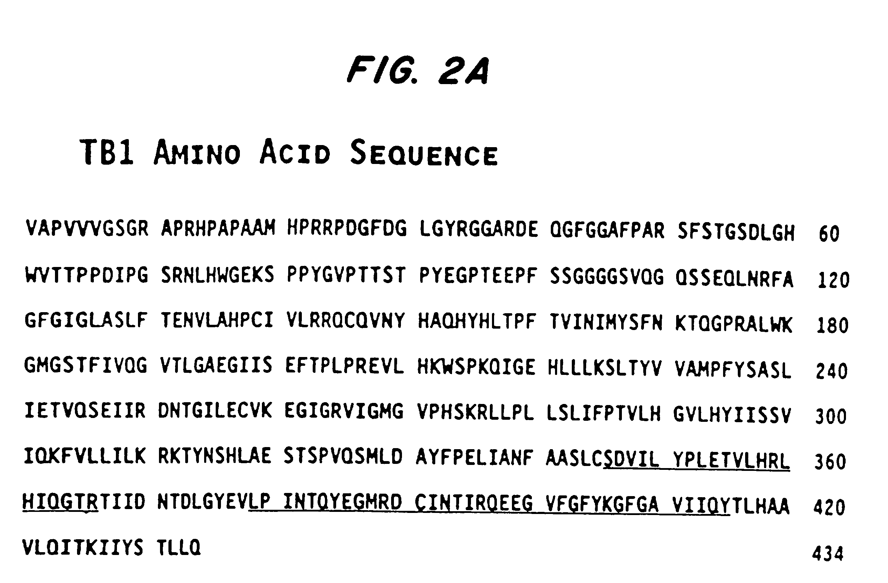

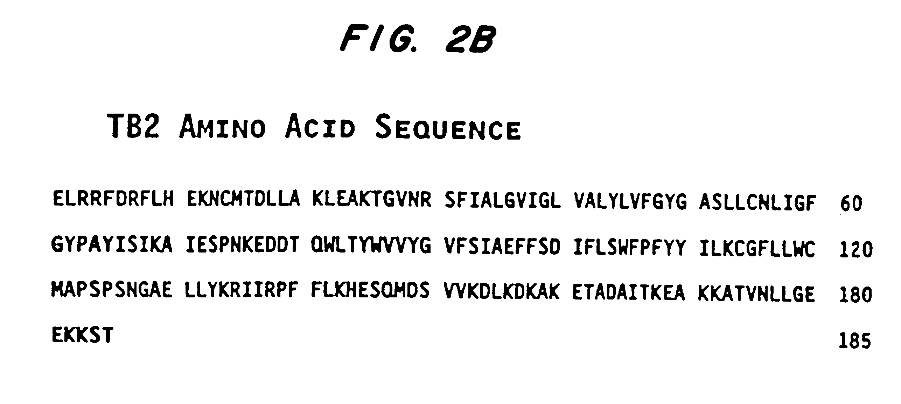

Figure 2 shows the sequence of TB1 and TB2 genes. The cDNA sequence of the TB1 gene was determined from the analysis of 11 cDNA clones derived from normal colon and liver, as described in the text. A total of 2314 bp were contained within the overlapping cDNA clones, defining an ORF of 424 amino acids beginning at nucleotide 1. Only the predicted amino acids from the ORF are shown. The carboxy-terminal end of the ORF has apparently been identified, but the 5' end of the TB1 transcript has not yet been precisely determined.

The cDNA sequence of the TB2 gene was determined from the YS-39 clone derived as described in the text. This clone consisted of 2300 bp and defined an ORF of 185 amino acids beginning at nucleotide 1. Only the predicted amino acids are shown. The carboxy terminal end of the ORF has apparently been identified, but the 5' end of the TB2 transcript has not been precisely determined.

Figure 3 shows the sequence of the APC gene product. The cDNA sequence was determined through the analysis of 87 cDNA clones derived from normal colon, liver, and brain. A total of 8973 bp were contained within overlapping cDNA clones, defining an ORF of 2842 amino acids. In frame stop codons surrounded this ORF, as described in the text, suggesting that the entire APC gene product was represented in the ORF illustrated. Only the predicted amino acids are shown.

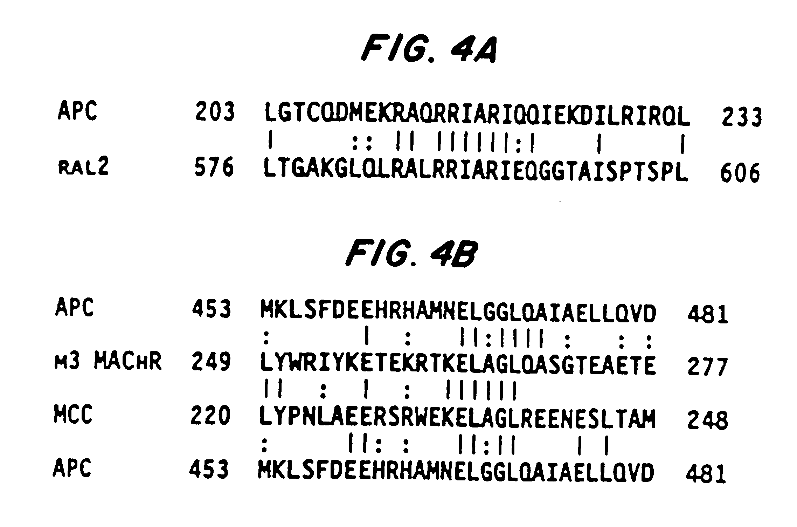

Figure 4 shows the local similarity between human APC and ral2 of yeast. Local similarity among the APC and MCC genes and the m3 muscarinic acetylcholine receptor is shown. The region of the mAChR shown corresponds to that responsible for coupling the receptor to G proteins. The connecting lines indicate identities; dots indicate related amino acids residues.

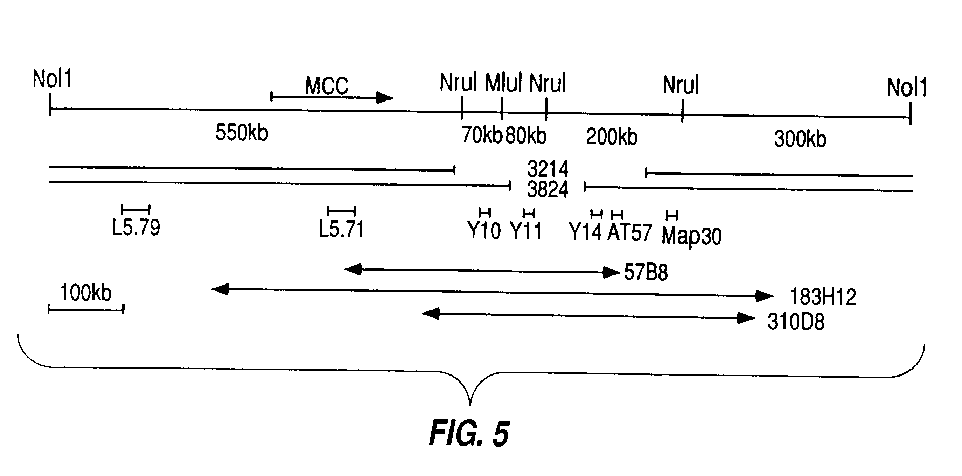

Figure 5 shows the genomic map of the 1200 kb NotI fragment at the FAP locus. The NotI fragment is shown as a bold line. Relevant parts of the deletion chromosomes from patients 3214 and 3824 are shown as stippled lines. Probes used to characterize the NotI fragment and the deletions, and three YACs from which subclones were obtained, are shown below the restriction map. The chimeric end of YAC 183H12 is indicated by a dotted line. The orientation and approximate position of MCC are indicated above the map.

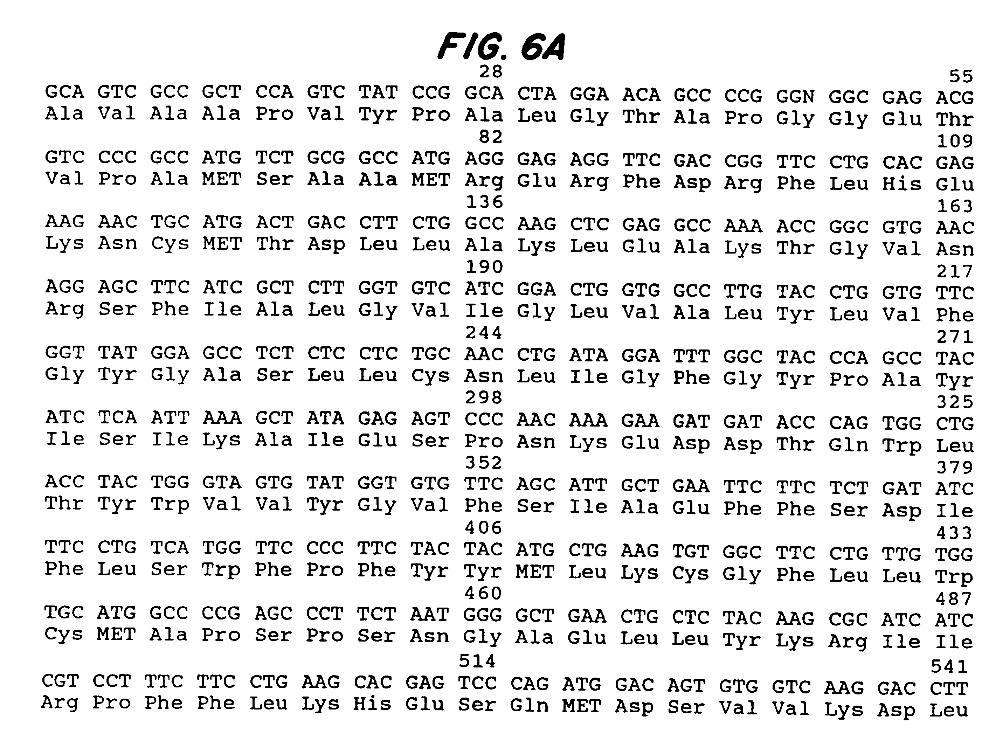

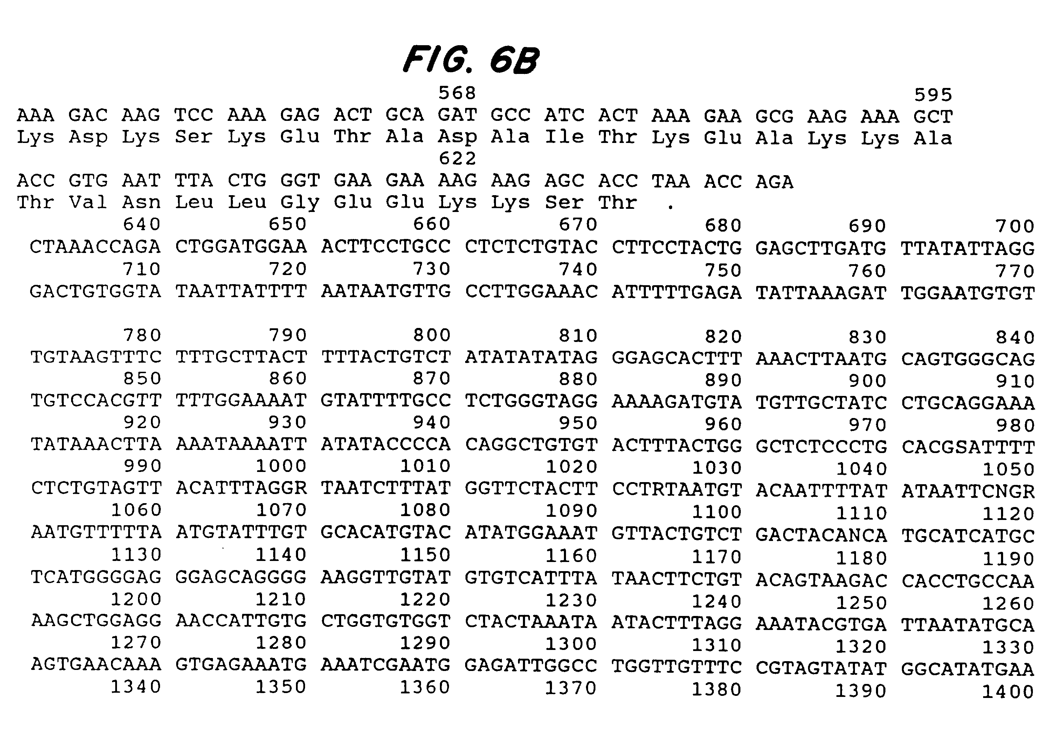

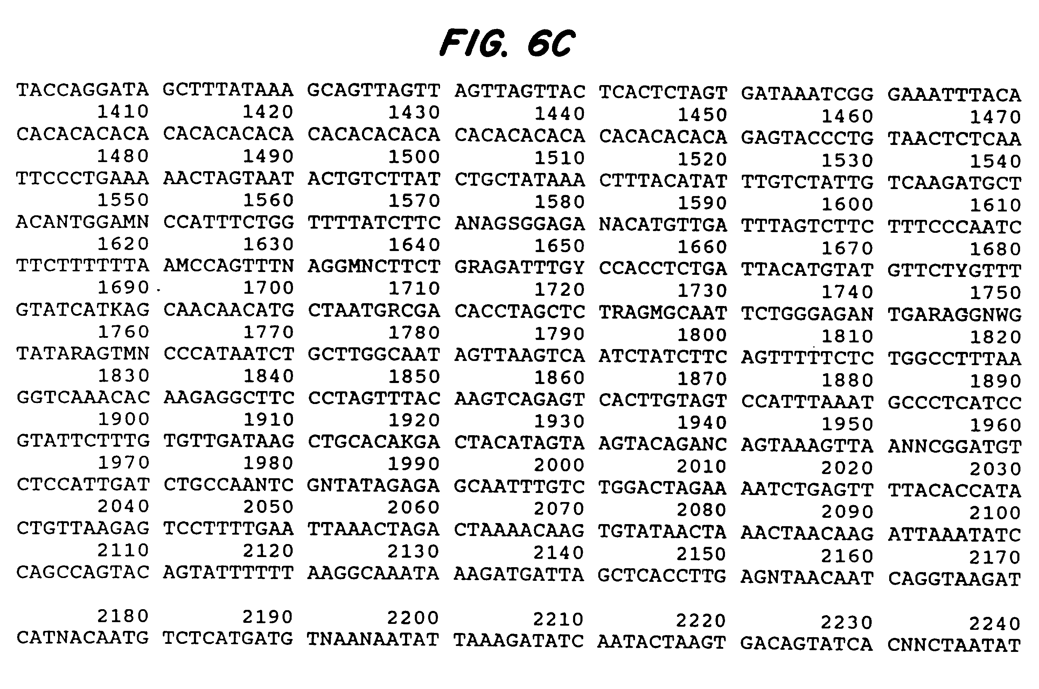

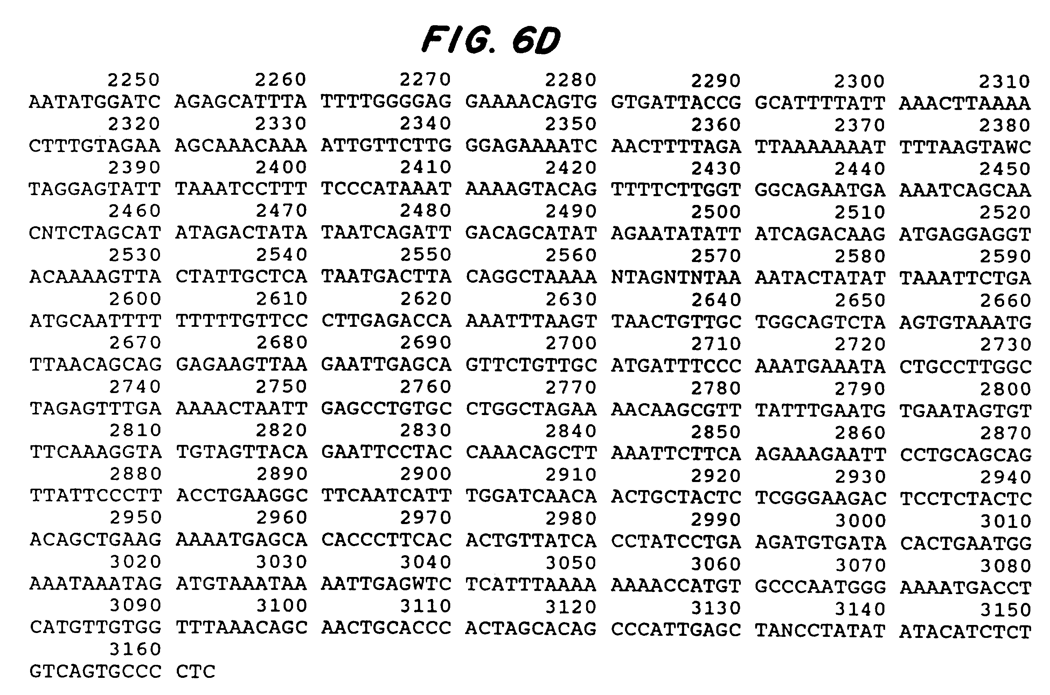

Figure 6 shows the DNA sequence and predicted amino acid sequence of DPI (TB2). The nucleotide numbering begins at the most 5' nucleotide isolated. A proposed initiation methionine (base 77) is indicated in bold type. The entire coding sequence is presented.

Figure 7 shows the cDNA and predicted amino acid sequence of DP2.5 (APC). The nucleotide numbering begins at the proposed initiation methionine. The nucleotides and amino acids of the alternatively spliced exon (exon 9) are at nucleotide positions 934-1236. At the 3'end, a poly(A) addition signal occurs at 9530, and one cDNA clone has a poly(A) at 9563. Other cDNA clones extend beyond 9563, however, and their consensus sequence is included here.

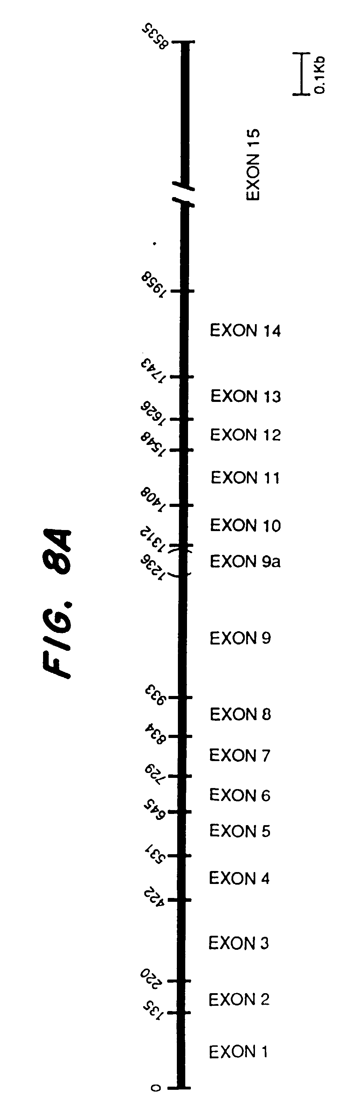

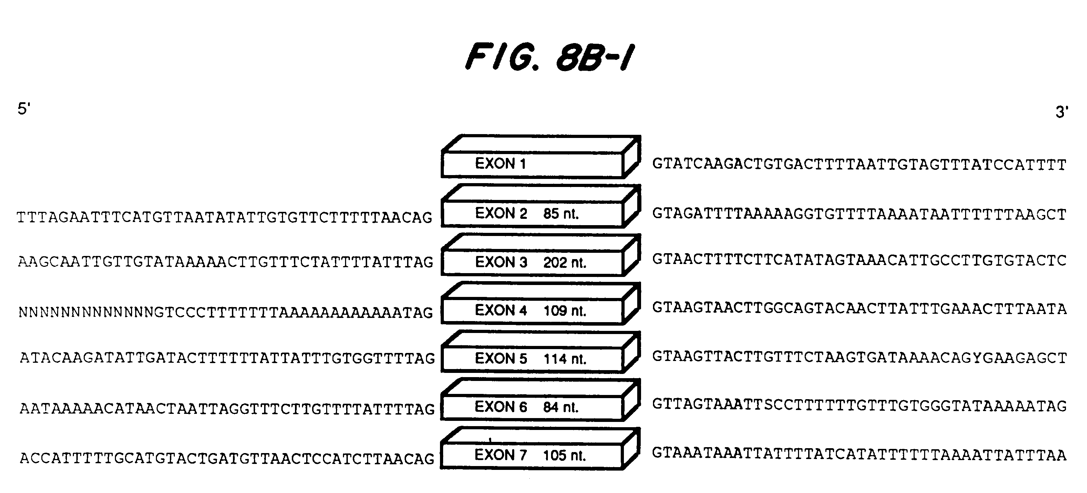

Figure 8 shows the arrangement of exons in DP2.5 (APC). (A) Exon 9 corresponds to nucleotides 933-1312; exon 9a corresponds to nucleotides 1236-1312. The stop codon in the cDNA is at nucleotide 8535. (B) Partial intronic sequence surrounding each exon is shown.

### DETAILED DESCRIPTION

It is a discovery of the present invention that mutational events associated with tumorigenesis occur in a previously unknown gene on chromosome 5q named here the APC (Adenomatous Polyposis Coli) gene. Although it was previously known that deletion of alleles on chromosome 5q were common in certain types of cancers, it was not known that a target gene of these deletions was the APC gene. Further it was not known that other types of mutational events in the APC gene are also associated with cancers. The mutations of the APC gene can involve gross rearrangements, such as insertions and deletions. Point mutations have also been observed.

According to the diagnostic and prognostic method of the present invention, alteration of the wild-type APC gene is detected. "Alteration of a wild-type gene" according to the present invention encompasses all forms of mutations - including deletions. The alteration may be due to either rearrangements such as insertions, inversions, and deletions, or to point mutations. Deletions may be of the entire gene or only a portion of the gene. Somatic mutations are those which occur only in certain tissues, e.g., in the tumor tissue, and are not inherited in the germline. Germline mutations can be found in any of a body's tissues. If only a single allele is somatically mutated, an early neoplastic state is indicated. However, if both alleles are mutated then a late neoplastic state is indicated. The finding of APC mutations thus provides both diagnostic and prognostic information. An APC allele which is not deleted (e.g., that on the sister chromosome to a chromosome carrying an APC deletion) can be screened for other mutations, such as insertions, small deletions, and point mutations. It is believed that many mutations found in tumor tissues will be those leading to decreased expression of the APC gene product. However, mutations leading to non-functional gene products would also lead to a cancerous state. Point mutational events may occur in regulatory regions, such as in the promoter of the gene, leading to loss or diminution of expression of the mRNA. Point mutations may also abolish proper RNA processing, leading to loss of expression of the APC gene product.

In order to detect the alteration of the wild-type APC gene in a tissue, it is helpful to isolate the tissue free from surrounding normal tissues. Means for enriching a tissue preparation for tumor cells are known in the art. For example, the tissue may be isolated from paraffin or cryostat sections. Cancer cells may also be separated from normal cells by flow cytometry. These as well as other techniques for separating tumor from normal cells are well known in the art. If the tumor tissue is highly contaminated with normal cells, detection of mutations is more difficult.

Detection of point mutations may be accomplished by molecular cloning of the APC allele (or alleles) and sequencing that allele(s) using techniques well known in the art. Alternatively, the polymerase chain reaction (PCR) can be used to amplify gene sequences directly from a genomic DNA preparation from the tumor tissue. The DNA sequence of the amplified sequences can then be determined. The polymerase chain reaction itself is well known in the art. See, e.g., Saiki et al., Science, Vol. 239, p. 487, 1988; U.S. 4,683,203; and U.S. 4,683,195. Specific primers which can be used in order to amplify the gene will be discussed in more detail below. The ligase chain reaction, which is known in the art, can also be used to amplify APC sequences. See Wu et al., Genomics, Vol. 4, pp. 560-569 (1989). In addition, a technique known as allele specific PCR can be used. (See Ruano and Kidd, Nucleic Acids Research, Vol. 17, p. 8392, 1989.) According to this technique, primers are used which hybridize at their 3' ends to a particular APC mutation. If the particular APC mutation is not present, an amplification product is not observed. Amplification Refractory Mutation System (ARMS) can also be used as disclosed in European Patent Application Publication No. 0332435 and in Newton et al., Nucleic Acids Research, Vol. 17, p.7, 1989. Insertions and deletions of genes can also be detected by cloning, sequencing and amplification. In addition, restriction fragment length polymorphism (RFLP) probes for the gene or surrounding marker genes can be used to score alteration of an allele or an insertion in a polymorphic fragment. Such a method is particularly useful for screening among kindred persons of an affected individual for the presence of the APC mutation found in that individual. Single stranded conformation polymorphism (SSCP) analysis can also be used to detect base change variants of an allele. (Orita et al., Proc. Natl. Acad. Sci. USA Vol. 86, pp. 2766-2770, 1989, and Genomics, Vol. 5, pp. 874-879, 1989.) Other techniques for detecting insertions and deletions as are known in the art can be used.

Alteration of wild-type genes can also be detected on the basis of the alteration of a wild-type expression product of the gene. Such expression products include both the APC mRNA as well as the APC protein product. The sequences of these products are shown in Figures 3 and 7. Point mutations may be detected by amplifying and sequencing the mRNA or via molecular cloning of cDNA made from the mRNA. The sequence of the cloned cDNA can be determined using DNA sequencing techniques which are well known in the art. The cDNA can also be sequenced via the polymerase chain reaction (PCR) which will be discussed in more detail below.

Mismatches, according to the present invention are hybridized nucleic acid duplexes which are not 100% homologous. The lack of total homology may be due to deletions, insertions, inversions, substitutions or frameshift mutations. Mismatch detection can be used to detect point mutations in the gene or its mRNA product. While these techniques are less sensitive than sequencing, they are simpler to perform on a large number of tumor samples. An example of a mismatch cleavage technique is the RNase protection method, which is described in detail in Winter et al., Proc. Natl. Acad. Sci. USA, Vol. 82, p. 7575, 1985 and Meyers et al., Science, Vol. 230, p. 1242, 1985. In the practice of the present invention the method involves the use of a labeled riboprobe which is complementary to the human wild-type APC gene coding sequence. The riboprobe and either mRNA or DNA isolated from the tumor tissue are annealed (hybridized) together and subsequently digested with the enzyme RNase A which is able to detect some mismatches in a duplex RNA structure. If a mismatch is detected by RNase A, it cleaves at the site of the mismatch. Thus, when the annealed RNA preparation is separated on an electrophoretic gel matrix, if a mismatch has been detected and cleaved by RNase A, an RNA product will be seen which is smaller than the full-length duplex RNA for the riboprobe and the mRNA or DNA. The riboprobe need not be the full length of the APC mRNA or gene but can be a segment of either. If the riboprobe comprises only a segment of the APC mRNA or gene it will be desirable to use a number of these probes to screen the whole mRNA sequence for mismatches.

In similar fashion, DNA probes can be used to detect mismatches, through enzymatic or chemical cleavage. See, e.g., Cotton et al., Proc. Natl. Acad. Sci. USA, Vol. 85, 4397, 1988; and Shenk et al., Proc. Natl. Acad. Sci. USA, Vol. 72, p. 989, 1975. Alternatively, mismatches can be detected by shifts in the electrophoretic mobility of mismatched duplexes relative to matched duplexes. See, e.g., Cariello, Human Genetics, Vol. 42, p. 726, 1988. With either riboprobes or DNA probes, the cellular mRNA or DNA which might contain a mutation can be amplified using PCR (see below) before hybridization. Changes in DNA of the APC gene can also be detected using Southern hybridization, especially if the changes are gross rearrangements, such as deletions and insertions.

DNA sequences of the APC gene which have been amplified by use of polymerase chain reaction may also be screened using allele-specific probes. These probes are nucleic acid oligomers, each of which contains a region of the APC gene sequence harboring a known mutation. For example, one oligomer may be about 30 nucleotides in length, corresponding to a portion of the APC gene sequence. By use of a battery of such allele-specific probes, PCR amplification products can be screened to identify the presence of a previously identified mutation in the APC gene. Hybridization of allele-specific probes with amplified APC sequences can be performed, for example, on a nylon filter. Hybridization to a particular probe under stringent hybridization conditions indicates the presence of the same mutation in the tumor tissue as in the allele-specific probe.

Alteration of APC mRNA expression can be detected by any technique known in the art. These include Northern blot analysis, PCR amplification and RNase protection. Diminished mRNA expression indicates an alteration of the wild-type APC gene.

Alteration of wild-type APC genes can also be detected by screening for alteration of wild-type APC protein. For example, monoclonal antibodies immunoreactive with APC can be used to screen a tissue. Lack of cognate antigen would indicate an APC mutation. Antibodies specific for products of mutant alleles could also be used to detect mutant APC gene product. Such immunological assays can be done in any convenient format known in the art. These include Western blots, immunohistochemical assays and ELISA assays. Any means for detecting an altered APC protein can be used to detect alteration of wild-type APC genes. Functional assays can be used, such as protein binding determinations. For example, it is believed that APC protein oligomerizes to itself and/or MCC protein or binds to a G protein. Thus, an assay for the ability to bind to wild type APC or MCC protein or that G protein can be employed. In addition, assays can be used which detect APC biochemical function. It is believed that APC is involved in phospholipid metabolism. Thus, assaying the enzymatic products of the involved phospholipid metabolic pathway can be used to determine APC activity. Finding a mutant APC gene product indicates alteration of a wild-type APC gene.

Mutant APC genes or gene products can also be detected in other human body samples, such as, serum, stool, urine and sputum. The same techniques discussed above for detection of mutant APC genes or gene products in tissues can be applied to other body samples. Cancer cells are sloughed off from tumors and appear in such body samples. In addition, the APC gene product itself may be secreted into the extracellular space and found in these body samples even in the absence of cancer cells. By screening such body samples, a simple early diagnosis can be achieved for many types of cancers. In addition, the progress of chemotherapy or radiotherapy can be monitored more easily by testing such body samples for mutant APC genes or gene products.

The methods of diagnosis of the present invention are applicable to any tumor in which APC has a role in tumorigenesis. Deletions of chromosome arm 5q have been observed in tumors of lung, breast, colon, rectum, bladder, liver, sarcomas, stomach and prostate, as well as in leukemias and lymphomas. Thus these are likely to be tumors in which APC has a role. The diagnostic method of the present invention is useful for clinicians so that they can decide upon an appropriate course of treatment. For example, a tumor displaying alteration of both APC alleles might suggest a more aggressive therapeutic regimen than a tumor displaying alteration of only one APC allele.

The primer pairs of the present invention are useful for determination of the nucleotide sequence of a particular APC allele using the polymerase chain reaction. The pairs of single stranded DNA primers can be annealed to sequences within or surrounding the APC gene on chromosome 5q in order to prime amplifying DNA synthesis of the APC gene itself. A complete set of these primers allows synthesis of all of the nucleotides of the APC gene coding sequences, i.e., the exons. The set of primers preferably allows synthesis of both intron and exon sequences. Allele specific primers can also be used. Such primers anneal only to particular APC mutant alleles, and thus will only amplify a product in the presence of the mutant allele as a template.

In order to facilitate subsequent cloning of amplified sequences, primers may have restriction enzyme site sequences appended to their 5' ends. Thus, all nucleotides of the primers are derived from APC sequences or sequences adjacent to APC except the few nucleotides necessary to form a restriction enzyme site. Such enzymes and sites are well known in the art. The primers themselves can be synthesized using techniques which are well known in the art. Generally, the primers can be made using oligonucleotide synthesizing machines which are commercially available. Given the sequence of the APC open reading frame shown in Figure 7, design of particular primers is well within the skill of the art.

The nucleic acid probes provided by the present invention are useful for a number of purposes. They can be used in Southern hybridization to genomic DNA and in the RNase protection method for detecting point mutations already discussed above. The probes can be used to detect PCR amplification products. They may also be used to detect mismatches with the APC gene or mRNA using other techniques. Mismatches can be detected using either enzymes (e.g., S1 nuclease), chemicals (e.g., hydroxylamine or osmium tetroxide and piperidine), or changes in electrophoretic mobility of mismatched hybrids as compared to totally matched hybrids. These techniques are known in the art. See, Cotton, supra, Shenk, supra, Myers, supra, Winter, supra, and Novack et al., Proc. Natl. Acad. Sci. USA, Vol. 83, p. 586, 1986. Generally, the probes are complementary to APC gene coding sequences, although probes to certain introns are also contemplated. An entire battery of nucleic acid probes is used to compose a kit for detecting alteration of wild-type APC genes. The kit allows for hybridization to the entire APC gene. The probes may overlap with each other or be contiguous.

If a riboprobe is used to detect mismatches with mRNA, it is complementary to the mRNA of the human wild-type APC gene. The riboprobe thus is an anti-sense probe in that it does not code for the APC protein because it is of the opposite polarity to the sense strand. The riboprobe generally will be labeled with a radioactive, colorimetric, or fluorometric material, which can be accomplished by any means known in the art. If the riboprobe is used to detect mismatches with DNA it can be of either polarity, sense or anti-sense. Similarly, DNA probes also may be used to detect mismatches.

Nucleic acid probes may also be complementary to mutant alleles of the APC gene. These are useful to detect similar mutations in other patients on the basis of hybridization rather than mismatches. These are discussed above and referred to as allele-specific probes. As mentioned above, the APC probes can also be used in Southern hybridizations to genomic DNA to detect gross chromosomal changes such as deletions and insertions. The probes can also be used to select cDNA clones of APC genes from tumor and normal tissues. In addition, the probes can be used to detect APC mRNA in tissues to determine if expression is diminished as a result of alteration of wild-type APC genes. Provided with the APC coding sequence shown in Figure 7 (SEQ ID NO: 1), design of particular probes is well within the skill of the ordinary artisan.

According to the present invention a method is also provided of supplying wild-type APC function to a cell which carries mutant APC alleles. Supplying such function should suppress neoplastic growth of the recipient cells. The wild-type APC gene or a part of the gene may be introduced into the cell in a vector such that the gene remains extrachromosomal. In such a situation the gene will be expressed by the cell from the extrachromosomal location. If a gene portion is introduced and expressed in a cell carrying a mutant APC allele, the gene portion should encode a part of the APC protein which is required for non-neoplastic growth of the cell. More preferred is the situation where the wild-type APC gene or a part of it is introduced into the mutant cell in such a way that it recombines with the endogenous mutant APC gene present in the cell. Such recombination requires a double recombination event which results in the correction of the APC gene mutation. Vectors for introduction of genes both for recombination and for extrachromosomal maintenance are known in the art and any suitable vector may be used. Methods for introducing DNA into cells such as electroporation, calcium phosphate co-precipitation and viral transduction are known in the art and the choice of method is within the competence of the routineer. Cells transformed with the wild-type APC gene can be used as model systems to study cancer remission and drug treatments which promote such remission.

Similarly, cells and animals which carry a mutant APC allele can be used as model systems to study and test for substances which have potential as therapeutic agents. The cells are typically cultured epithelial cells. These may be isolated from individuals with APC mutations, either somatic or germline. Alternatively, the cell line can be engineered to carry the mutation in the APC allele. After a test substance is applied to the cells, the neoplastically transformed phenotype of the cell will be determined. Any trait of neoplastically transformed cells can be assessed, including anchorage-independent growth, tumorigenicity in nude mice, invasiveness of cells, and growth factor dependence. Assays for each of these traits are known in the art.

Animals for testing therapeutic agents can be selected after mutagenesis of whole animals or after treatment of germline cells or zygotes. Such treatments include insertion of mutant APC alleles, usually from a second animal species, as well as insertion of disrupted homologous genes. Alternatively, the endogenous APC gene(s) of the animals may be disrupted by insertion or deletion mutation. After test substances have been administered to the animals, the growth of tumors must be assessed. If the test substance prevents or suppresses the growth of tumors, then the test substance is a candidate therapeutic agent for the treatment of FAP and/or sporadic cancers.

Polypeptides which have APC activity can be supplied to cells which carry mutant or missing APC alleles. The sequence of the APC protein is disclosed in Figure 3 or 7 (SEQ ID NO: 7 or 1). These two sequences differ slightly and appear to be indicate the existence of two different forms of the APC protein. Protein can be produced by expression of the cDNA sequence in bacteria, for example, using known expression vectors. Alternatively, APC can be extracted from APC-producing mammalian cells such as brain cells. In addition, the techniques of synthetic chemistry can be employed to synthesize APC protein. Any of such techniques can provide the preparation of the present invention which comprises the APC protein. The preparation is substantially free of other human proteins. This is most readily accomplished by synthesis in a microorganism or in vitro.

Active APC molecules can be introduced into cells by microinjection or by use of liposomes, for example. Alternatively, some such active molecules may be taken up by cells, actively or by diffusion. Extracellular application of APC gene product may be sufficient to affect tumor growth. Supply of molecules with APC activity should lead to a partial reversal of the neoplastic state. Other molecules with APC activity may also be used to effect such a reversal, for example peptides, drugs, or organic compounds.

The present invention also provides a preparation of antibodies immunoreactive with a human APC protein. The antibodies may be polyclonal or monoclonal and may be raised against native APC protein, APC fusion proteins, or mutant APC proteins. The antibodies should be immunoreactive with APC epitopes, preferably epitopes not present on other human proteins. In a preferred embodiment of the invention the antibodies will immunoprecipitate APC proteins from solution as well as react with APC protein on Western or immunoblots of polyacrylamide gels. In another preferred embodiment, the antibodies will detect APC proteins in paraffin or frozen tissue sections, using immunocytochemical techniques. Techniques for raising and purifying antibodies are well known in the art and any such techniques may be chosen to achieve the preparation of the invention.

Predisposition to cancers as in FAP and GS can be ascertained by testing any tissue of a human for mutations of the APC gene. For example, a person who has inherited a germline APC mutation would be prone to develop cancers. This can be determined by testing DNA from any tissue of the person's body. Most simply, blood can be drawn and DNA extracted from the cells of the blood. In addition, prenatal diagnosis can be accomplished by testing fetal cells, placental cells, or amniotic fluid for mutations of the APC gene. Alteration of a wild-type APC allele, whether for example, by point mutation or by deletion, can be detected by any of the means discussed above.

Molecules of cDNA according to the present invention are intron-free, APC gene coding molecules. They can be made by reverse transcriptase using the APC mRNA as a template. These molecules can be propagated in vectors and cell lines as is known in the art. Such molecules have the sequence shown in SEQ ID NO: 7. The cDNA can also be made using the techniques of synthetic chemistry given the sequence disclosed herein.

A short region of homology has been identified between APC and the human m3 muscarinic acetylcholine receptor (mAChR). This homology was largely confined to 29 residues in which 6 out of 7 amino acids (EL(GorA)GLQA) were identical (See Figure 4). Initially, it was not known whether this homology was significant, because many other proteins had higher levels of global homology (though few had six out of seven contiguous amino acids in common). However, a study on the sequence elements controlling G protein activation by mAChR subtypes (Lechleiter et al., EMBO J., p. 4381 (1990)) has shown that a 21 amino acid region from the m3 mAChR completely mediated G protein specificity when substituted for the 21 amino acids of m2 mAChR at the analogous protein position. These 21 residues overlap the 19 amino acid homology between APC and m3 mAChR.

This connection between APC and the G protein activating region of mAChR is intriguing in light of previous investigations relating G proteins to cancer. For example, the RAS oncogenes, which are often mutated in colorectal cancers (Vogelstein, et al., N. Engl. J. Med., Vol. 319, p. 525 (1988); Bos et al., Nature Vol. 327, p. 293 (1987)), are members of the G protein family (Bourne, et al., Nature, Vol. 348, p. 125 (1990)) as is an in vitro transformation suppressor (Noda et al., Proc. Natl. Acad. Sci. USA, Vol. 86, p. 162 (1989)) and genes mutated in hormone producing tumors (Candis et al., Nature, Vol. 340, p. 692 (1989); Lyons et al., Science, Vol. 249, p. 655 (1990)). Additionally, the gene responsible for neurofibromatosis (presumably a tumor suppressor gene) has been shown to activate the GTPase activity of RAS (Xu et al., Cell, Vol. 63, p. 835 (1990); Martin et al., Cell, Vol. 63, p. 843 (1990); Ballester et al., Cell, Vol. 63, p. 851 (1990)). Another interesting link between G proteins and colon cancer involves the drug sulindac. This agent has been shown to inhibit the growth of benign colon tumors in patients with FAP, presumably by virtue of its activity as a cyclooxygenase inhibitor (Waddell et al., J. Surg. Oncology 24(1), 83 (1983); Wadell, et al., Am. J. Surg., 157(1), 175 (1989); Charneau et al., Gastroenterologie Clinique at Biologique 14(2), 153 (1990)). Cyclooxygenase is required to convert arachidonic acid to prostaglandins and other biologically active molecules. G proteins are known to regulate phospholipase A2 activity, which generates arachidonic acid from phospholipids (Role et al., Proc. Natl. Acad. Sci. USA, Vol. 84, p. 3623 (1987); Kurachi et al., Nature, Vol. 337, 12 555 (1989)). Therefore we propose that wild-type APC protein functions by interacting with a G protein and is involved in phospholipid metabolism.

The following are provided for exemplification purposes only and are not intended to limit the scope of the invention which has been described in broad terms above.

### Example 1:

This example demonstrates the isolation of a 5.5 Mb region of human DNA linked to the FAP locus. Six genes are identified in this region, all of which are expressed in normal colon cells and in colorectal, lung, ad bladder tumors.

The cosmid markers YN5.64 and YN5.48 have previously been shown to delimit an 8 cM region containing the locus for FAP (Nakamura et al., Am. J. Hum. Genet. Vol. 43, p. 638 (1988)). Further linkage and pulse-field gel electrophoresis (PFGE) analysis with additional markers has shown that the FAP locus is contained within a 4 cM region bordered by cosmids EF5.44 and L5.99. In order to isolate clones representing a significant portion of this locus, a yeast artificial chromosome (YAC) library was screened with various 5q21 markers. Twenty-one YAC clones, distributed within six contigs and including 5.5 Mb from the region between YN5.64 and YN5.48, were obtained (Figure 1A).

Three contigs encompassing approximately 4Mb were contained within the central portion of this region. The YAC's constituting these contigs, together with the markers used for their isolation and orientations, are shown in Figure 1. These YAC contigs were obtained in the following way. To initiate each contig, the sequence of a genomic marker cloned from chromosome 5q21 was determined and used to design primers for PCR. PCR was then carried out on pools of YAC clones distributed in microtiter trays as previously described (Anand et al., Nucleic Acids Research, Vol. 18, p. 1951 (1980)). Individual YAC clones from the positive pools were identified by further PCR or hybridization based assays, and the YAC sizes were determined by PFGE.

To extend the areas covered by the original YAC clones, "chromosomal walking" was performed. For this purpose, YAC termini were isolated by a PCR based method and sequenced (Riley et al., Nucleic Acids Research, Vol. 18, p. 2887 (1990)). PCR primers based on these sequences were then used to rescreen the YAC library. For example, the sequence from an intron of the FER gene (Hao et al., Mol. Cell. Biol., Vol. 9, p. 1587 (1989)) was used to design PCR primers for isolation of the 28EC1 and 5EH8 YACs. The termini of the 28EC1 YAC were sequenced to derive markers RHE28 and LHE28, respectively. The sequences of these two markers were then used to isolate YAC clones 15CH12 (from RHE28) and 40CF1 and 29EF1 (from LHE28). These five YAC's formed a contig encompassing 1200 kb (contig 1, Figure 1B).

Similarly, contig 2 was initiated using cosmid N5.66 sequences, and contig 3 was initiated using sequences both from the MCC gene and from cosmid EF5.44. A walk in the telomeric direction from YAC 14FH1 and a walk in the opposite direction from YAC 39GG3 allowed connection of the initial contig 3 clones through YAC 37HG4 (Figure 1B).

Multipoint linkage analysis with the various markers used to define the contigs, combined with PFGE analysis, showed that contigs 1 and 2 were centromeric to contig 3. These contigs were used as tools to orient and/or identify genes which might be responsible for FAP. Six genes were found to lie within this cluster of YAC's, as follows:

Contig #1: FER - The FER gene was discovered through its homology to the viral oncogene ABL (Hao et al., supra). It has an intrinsic tyrosine kinase activity, and in situ hybridization with an FER probe showed that the gene was located at 5q11-23 (Morris et al., Cytogenet. Cell. Genet., Vol. 53, p. 4, (1990)). Because of the potential role of this oncogene-related gene in neoplasia, we decided to evaluate it further with regards to the FAP locus. A human genomic clone from FER was isolated (MF 2.3) and used to define a restriction fragment length polymorphism (RFLP), and the RFLP in turn used to map FER by linkage analysis using a panel of three generation families. This showed that FER was very tightly linked to previously defined polymorphic markers for the FAP locus. The genetic mapping of FER was complemented by physical mapping using the YAC clones derived from FER sequences (Figure 1B). Analysis of YAC contig 1 showed that FER was within 600 kb of cosmid marker M5.28, which maps to within 1.5 Mb of cosmid L5.99 by PFGE of human genomic DNA. Thus, the YAC mapping results were consistent with the FER linkage data and PFGE analyses.

Contig 2: TB1 - TB1 was identified through a cross-hybridization approach. Exons of genes are often evolutionarily conserved while introns and intergenic regions are much less conserved. Thus, if a human probe cross-hybridizes strongly to the DNA from non-primate species, there is a reasonable chance that it contains exon sequences. Subclones of the cosmids shown in Figure 1 were used to screen Southern blots containing rodent DNA samples. A subclone of cosmid N5.66 (p 5.66-4) was shown to strongly hybridize to rodent DNA, and this clone was used to screen cDNA libraries derived from normal adult colon and fetal liver. The ends of the initial cDNA clones obtained in this screen were then used to extend the cDNA sequence. Eventually, 11 cDNA clones were isolated, covering 2314 bp. The gene detected by these clones was named TB1. Sequence analysis of the overlapping clones revealed an open reading frame (ORF) that extended for 1302 bp starting from the most 5' sequence data obtained (Figure 2A). If this entire open reading frame were translated, it would encode 434 amino acids. The product of this gene was not globally homologous to any other sequence in the current database but showed two significant local similarities to a family of ADP, ATP carrier/translocator proteins and mitochondrial brown fat uncoupling proteins which are widely distributed from yeast to mammals. These conserved regions of TB1 (underlined in Figure 2A) may define a predictive motif for this sequence family. In addition, TB1 appeared to contain a signal peptide (or mitochondrial targeting sequence) as well as at least 7 transmembrane domains.

Contig 3: MCC, TB2, SRP and APC - The MCC gene was also discovered through a cross-hybridization approach, as described previously (Kinzler et al., Science Vol. 251, p. 1366 (1991)). The MCC gene was considered a candidate for causing FAP by virtue of its tight genetic linkage to FAP susceptibility and its somatic mutation in sporadic colorectal carcinomas. However, mapping experiments suggested that the coding region of MCC was approximately 50 kb proximal to the centromeric end of a 200 kb deletion found in an FAP patient. MCC cDNA probes detected a 10 kb mRNA transcript on Northern blot analysis of which 4151 bp, including the entire open reading frame, have been cloned. Although the 3' non-translated portion or an alternatively spliced form of MCC might have extended into this deletion, it was possible that the deletion did not affect the MCC gene product. We therefore used MCC sequences to initiate a YAC contig, and subsequently used the YAC clones to identify genes 50 to 250 kb distal to MCC that might be contained within the deletion.

In a first approach, the insert from YAC24ED6 (Figure 1B) was radiolabelled and hybridized to a cDNA library from normal colon. One of the cDNA clones (YS39) identified in this manner detected a 3.1 kb mRNA transcript when used as a probe for Northern blot hybridization. Sequence analysis of the YS39 clone revealed that it encompassed 2283 nucleotides and contained an ORF that extended for 555 bp from the most 5' sequence data obtained. If all of this ORF were translated, it would encode 185 amino acids (Figure 2B). The gene detected by YS39 was named TB2. Searches of nucleotide and protein databases revealed that the TB2 gene was not identical to any previously reported sequences nor were there any striking similarities.

Another clone (YS11) identified through the YAC 24ED6 screen appeared to contain portions of two distinct genes. Sequences from one end of YS11 were identical to at least 180 bp of the signal recognition particle protein SRP19 (Lingelbach et al. Nucleic Acids Research, Vol. 16, p. 9431 (1988). A second ORF, from the opposite end of clone YS11, proved to be identical to 78 bp of a novel gene which was independently identified through a second YAC-based approach. For the latter, DNA from yeast cells containing YAC 14FH1 (Figure 1B) was digested with EcoRI and subcloned into a plasmid vector. Plasmids that contained human DNA fragments were selected by colony hybridization using total human DNA as a probe. These clones were then used to search for cross-hybridizing sequences as described above for TB1, and the cross-hybridizing clones were subsequently used to screen cDNA libraries. One of the cDNA clones discovered in this way (FH38) contained a long ORF (2496 bp), 78 bp of which were identical to the above-noted sequences in YS11. The ends of the FH38 cDNA clone were then used to initiate cDNA walking to extend the sequence. Eventually, 85 cDNA clones were isolated from normal colon, brain and liver cDNA libraries and found to encompass 8973 nucleotides of contiguous transcript. The gene corresponding to this transcript was named APC. When used as probes for Northern blot analysis, APC cDNA clones hybridized to a single transcript of approximately 9.5 kb, suggesting that the great majority of the gene product was represented in the cDNA clones obtained. Sequences from the 5' end of the APC gene were found in YAC 37HG4 but not in YAC 14FH1. However, the 3' end of the APC gene was found in 14FH1 as well as 37HG4. The yeast artificial chromosome of the present invention designated YAC 37HG4 has been deposited with the National Collection of Industrial and Marine Bacteria (NCIMB), P.O. Box 31, 135 Abbey Road, Aberdeen AB9 8DG, Scotland, prior to the filing of this patent application. The NCIMB Accession Number of YAC clone YAC 37HG4 is 40353. Analogously, the 5' end of the MCC coding region was found in YAC clones 19AA9 and 26GC3 but not 24ED6 or 14FH1, while the 3' end displayed the opposite pattern. Thus, MCC and APC transcription units pointed in opposite directions, with the direction of transcription going from centromeric to telomeric in the case of MCC, and telomeric to centromeric in the case of APC. PFGE analysis of YAC DNA digested with various restriction endonucleases showed that TB2 and SRP were between MCC and APC, and that the 3' ends of the coding regions of MCC and APC were separated by approximately 150 kb (Figure 1B).

Sequence analysis of the APC cDNA clones revealed an open reading frame of 8,535 nucleotides. The 5' end of the ORF contained a methionine codon (codon 1) that was preceded by an in-frame stop codon 9 bp upstream, and the 3' end was followed by several in-frame stop codons. The protein produced by initiation at codon 1 would contain 2,842 amino acids (Figure 3). The results of database searching with the APC gene product were quite complex due to the presence of large segments with locally biased amino acid compositions. In spite of this, APC could be roughly divided into two domains. The N-terminal 25% of the protein had a high content of leucine residues (12%) and showed local sequence similarities to myosins, various intermediate filament proteins (e.g., desmin, vimentin, neurofilaments) and Drosophila armadillo/human plakoglobin. The latter protein is a component of adhesive junctions (desmosomes) joining epithelial cells (Franke et al., Proc. Natl. Acad. Sci. U.S.A., Vol. 86, p. 4027 (1989); Perfer et al., Cell, Vol. 63, p. 1167 (1990)) The C-terminal 75% of APC (residues 731-2832) is 17% serine by composition with serine residues more or less uniformly distributed. This large domain also contains local concentrations of charged (mostly acidic) and proline residues. There was no indication of potential signal peptides, transmembrane regions, or nuclear targeting signals in APC, suggesting a cytoplasmic localization.

To detect short similarities to APC, a database search was performed using the PAM-40 matrix (Altschul. J. Mol. Bio., Vol. 219, p. 555 (1991). Potentially interesting matches to several proteins were found. The most suggestive of these involved the ral2 gene product of yeast, which is implicated in the regulation of ras activity (Fukul et al., Mol. Cell. Biol., Vol. 9, p. 5617 (1989)). Little is known about how ral2 might interact with ras but it is interesting to note the positively-charged character of this region in the context of the negatively-charged GAP interaction region of ras. A specific electrostatic interaction between ras and GAP-related proteins has been proposed.

Because of the proximity of the MCC and APC genes, and the fact that both are implicated in colorectal tumorigenesis, we searched for similarities between the two predicted proteins. Bourne has previously noted that MCC has the potential to form alpha helical coiled coils (Nature, Vol. 351, p. 188 (1991). Lupas and colleagues have recently developed a program for predicting coiled coil potential from primary sequence data (Science, Vol. 252, p. 1162 (1991) and we have used their program to analyze both MCC and APC. Analysis of MCC indicated a discontinuous pattern of coiled-coil domains separated by putative "hinge" or "spacer" regions similar to those seen in laminin and other intermediate filament proteins. Analysis of the APC sequence revealed two regions in the N-terminal domain which had strong coiled coil-forming potential, and these regions corresponded to those that showed local similarities with myosin and IF proteins on database searching. In addition, one other putative coiled coil region was identified in the central region of APC. The potential for both APC and MCC to form coiled coils is interesting in that such structures often mediate homo- and hetero-oligomerization.

Finally, it had previously been noted that MCC shared a short similarity with the region of the m3 muscarinic acetylcholine receptor (mAChR) known to regulate specificity of G-protein coupling. The APC gene also contained a local similarity to the region of the m3 mAChR that overlapped with the MCC similarity (Figure 4B). Although the similarities to ral2 (Figure 4A) and m3 mAChR (Figure 4B) were not statistically significant, they were intriguing in light of previous observations relating G-proteins to neoplasia.

Each of the six genes described above was expressed in normal colon mucosa, as indicated by their representation in colon cDNA libraries. To study expression of the genes in neoplastic colorectal epithelium, we employed reverse transcription-polymerase chain reaction (PCR) assays. Primers based on the sequences of FER, TB1, TB2, MCC, and APC were each used to design primers for PCR performed with cDNA templates. Each of these genes was found to be expressed in normal colon, in each of ten cell lines derived from colorectal cancers, and in tumor cell lines derived from lung and bladder tumors. The ten colorectal cancer cell lines included eight from patients with sporadic CRC and two from patients with FAP.

### Example 2

This example demonstrates a genetic analysis of the role of the FER gene in FAP and sporadic colorectal cancers.

We considered FER as a candidate because of its proximity to the FAP locus as judged by physical and genetic criteria (see Example 1), and its homology to known tyrosine kinases with oncogenic potential. Primers were designed to PCR-amplify the complete coding sequence of FER from the RNA of two colorectal cancer cell lines derived from FAP patients. cDNA was generated from RNA and used as a template for PCR. The primers used were 5'-AGAAGGATCCCTTGTGCAGTGTGGA-3' and 5'-GACAGGATCCTGAAGCTGAGTTTG-3'. The underlined nucleotides were altered from the true FER sequence to create BamHI sites. The cell lines used were JW and Difi, both derived from colorectal cancers of FAP patients. (C. Paraskeva, B.G. Buckle, D. Sheer, C.B. Wigley, Int. J. Cancer 34, 49 (1984); M.E. Gross et al., Cancer Res. 51, 1452 (1991). The resultant 2554 basepair fragments were cloned and sequenced in their entirety. The PCR products were cloned in the BamHI site of Bluescript SK (Stratagene) and pools of at least 50 clones were sequenced en masse using T7 polymerase, as described in Nigro et al., Nature 342, 705 (1989).

Only a single conservative amino acid change (GTG->CTG, creating a val to leu substitution at codon 439) was observed. The region surrounding this codon was then amplified from the DNA of individuals without FAP and this substitution was found to be a common polymorphism, not specifically associated with FAP. Based on these results, we considered it unlikely (though still possible) the FER gene was responsible for FAP. To amplify the regions surrounding codon 439, the following primers were used: 5'-TCAGAAAGTGCTGAAGAG-3' and 5'-GGAATAATTAGGTCTCCAA-3'. PCR products were digested with PstI, which yields a 50 bp fragment if codon 439 is leucine, but 26 and 24 bp fragments if it is valine. The primers used for sequencing were chosen from the FER cDNA sequence in Hao et al., supra.

### Example 3

This example demonstrates the genetic analysis of MCC, TB2, SRP and APC in FAP and sporadic colorectal tumors. Each of these genes is linked and encompassed by contig 3 (see Figure 1).

Several lines of evidence suggested that this contig was of particular interest. First, at least three of the four genes in this contig were within the deleted region identified in two FAP patients. (See Example 5 infra.) Second, allelic deletions of chromosome 5q21 in sporadic cancers appeared to be centered in this region. (Ashton-Rickardt et al., Oncogene, in press; and Miki et al., Japn. J. Cancer Res., in press.) Some tumors exhibited loss of proximal RFLP markers (up to and potentially including the 5' end of MCC), but no loss of markers distal to MCC. Other tumors exhibited loss of markers distal to and perhaps including the 3' end of MCC, but no loss of sequences proximal to MCC. This suggested either that different ends of MCC were affected by loss in all such cases, or alternatively, that two genes (one proximal to and perhaps including MCC, the other distal to MCC) were separate targets of deletion. Third, clones from each of the six FAP region genes were used as probes on Southern blots containing tumor DNA from patients with sporadic CRC. Only two examples of somatic changes were observed in over 200 tumors studied: a rearrangement/deletion whose centromeric end was located within the MCC gene (Kinzler et al., supra) and an 800 bp insertion within the APC gene between nucleotides 4424 and 5584. Fourth, point mutations of MCC were observed in two tumors (Kinzler et al.) supra strongly suggesting that MCC was a target of mutation in at least some sporadic colorectal cancers.

Based on these results, we attempted to search for subtle alterations of contig 3 genes in patients with FAP. We chose to examine MCC and APC, rather than TB2 or SRP, because of the somatic mutations in MCC and APC noted above. To facilitate the identification of subtle alterations, the genomic sequences of MCC and APC exons were determined (see Table I). These sequences were used to design primers for PCR analysis of constitutional DNA from FAP patients.

We first amplified eight exons and surrounding introns of the MCC gene in affected individuals from 90 different FAP kindreds. The PCR products were analyzed by a ribonuclease (RNase) protein assay. In brief, the PCR products were hybridized to in vitro transcribed RNA probes representing the normal genomic sequences. The hybrids were digested with RNase A, which can cleave at single base pair mismatches within DNA-RNA hybrids, and the cleavage products were visualized following denaturing gel electrophoresis. Two separate RNase protection analyses were performed for each exon, one with the sense and one with the antisense strand. Under these conditions, approximately 40% of all mismatches are detectable. Although some amino acid variants of MCC were observed in FAP patients, all such variants were found in a small percentage of normal individuals. These variants were thus unlikely to be responsible for the inheritance of FAP.

We next examined three exons of the APC gene. The three exons examined included those containing nt 822-930, 931-1309, and the first 300 nt of the most distal exon (nt 1956-2256). PCR and RNase protection analysis were performed as described in Kinzler et al. supra, using the primers underlined in Table I. The primers for nt 1956-2256 were 5'-GCAAATCCTAAGAGAGAACAA-3' and 5'-GATGGCAAGCTTGAGCCAG-3'.

In 90 kindreds, the RNase protection method was used to screen for mutations and in an additional 13 kindreds, the PCR products were cloned and sequenced to search for mutations not detectable by RNase protection. PCR products were cloned into a Bluescript vector modified as described in T.A. Holton and M.W. Graham, Nucleic Acids Res. 19, 1156 (1991). A minimum of 100 clones were pooled and sequenced. Five variants were detected among the 103 kindreds analyzed. Cloning and subsequent DNA sequencing of the PCR product of patient P21 indicated a C to T transition in codon 413 that resulted in a change from arginine to cysteine. This amino acid variant was not observed in any of 200 DNA samples from individuals without FAP. Cloning and sequencing of the PCR product from patients P24 and P34, who demonstrated the same abnormal RNase protection pattern indicated that both had a C to T transition at codon 301 that resulted in a change from arginine (CGA) to a stop codon (TGA). This change was not present in 200 individuals without FAP. As this point mutation resulted in the predicted loss of the recognition site for the enzyme Taq I, appropriate PCR products could be digested with Taq I to detect the mutation. This allowed us to determine that the stop codon co-segregated with disease phenotype in members of the family of P24. The inheritance of this change in affected members of the pedigree provides additional evidence for the importance of the mutation.

Cloning and sequencing of the PCR product from FAP patient P93 indicated a C to G transversion at codon 279, also resulting in a stop codon (change from TCA to TGA). This mutation was not present in 200 individuals without FAP. Finally, one additional mutation resulting in a serine (TCA) to stop codon (TGA) at codon 712 was detected in a single patient with FAP (patient P60).

The five germline mutations identified are summarized in Table IIA, as well as four others discussed in Example 9. In addition to these germline mutations, we identified several somatic mutations of MCC and APC in sporadic CRC's. Seventeen MCC exons were examined in 90 sporadic colorectal cancers by RNase protection analysis. In each case where an abnormal RNase protection pattern was observed, the corresponding PCR products were cloned and sequenced. This led to the identification of six point mutations (two described previously) (Kinzler et al., supra), each of which was not found in the germline of these patients (Table IIB). Four of the mutations resulted in amino acid substitutions and two resulted in the alteration of splice site consensus elements. Mutations at analogous splice site positions in other genes have been shown to alter RNA processing in vivo and in vitro.

Three exons of APC were also evaluated in sporadic tumors. Sixty tumors were screened by RNase protection, and an additional 98 tumors were evaluated by sequencing. The exons examined included nt 822-930, 931-1309, and 1406-1545 (Table I). A total of three mutations were identified, each of which proved to be somatic. Tumor T27 contained a somatic mutation of CGA (arginine) to TGA (stop codon) at codon 33. Tumor T135 contained a GT to GC change at a splice donor site. Tumor T34 contained a 5 bp insertion (CAGCC between codons 289 and 290) resulting in a stop at codon 292 due to a frameshift.

We serendipitously discovered one additional somatic mutation in a colorectal cancer. During our attempt to define the sequences and splice patterns of the MCC and APC gene products in colorectal epithelial cells, we cloned cDNA from the colorectal cancer cell line SW480. The amino acid sequence of the MCC gene from SW480 was identical to that previously found in clones from human brain. The sequence of APC in SW480 cells, however, differed significantly, in that a transition at codon 1338 resulted in a change from glutamine (CAG) to a stop codon (TAG). To determine if this mutation was somatic, we recovered DNA from archival paraffin blocks of the original surgical specimen (T201) from which the tumor cell line was derived 28 years ago.

DNA was purified from paraffin sections as described in S.E. Goelz, S.R. Hamilton, and B. Vogelstein. Biochem. Blophys. Res. Comm. 130, 118 (1985). PCR was performed as described in reference 24, using the primers 5'-GTTCCAGCAGTGTCACAG-3' and 5'-GGGAGATTTCGCTCCTGA-3'. A PCR product containing codon 1338 was amplified from the archival DNA and used to show that the stop codon represented a somatic mutation present in the original primary tumor and in cell lines derived from the primary and metastatic tumor sites, but not from normal tissue of the patient.

The ten point mutations in the MCC and APC genes so far discovered in sporadic CRCs are summarized in Table IIB. Analysis of the number of mutant and wild-type PCR clones obtained from each of these tumors showed that in eight of the ten cases, the wild-type sequence was present in approximately equal proportions to the mutant. This was confirmed by RFLP analysis using flanking markers from chromosome 5q which demonstrated that only two of the ten tumors (T135 and T201) exhibited an allelic deletion on chromosome 5q. These results are consistent with previous observations showing that 20-40% of sporadic colorectal tumors had allelic deletions of chromosome 5q. Moreover, these data suggest that mutations of 5q21 genes are not limited to those colorectal tumors which contain allelic deletions of this chromosome.

### Example 4

This example characterizes small, nested deletions in DNA from two unrelated FAP patients.

DNA from 40 FAP patients was screened with cosmids that had been mapped into a region near the APC locus to identify small deletions or rearrangements. Two of these cosmids, L5.71 and L5.79, hybridized with a 1200 kb NotI fragment in DNAs from most of the FAP patients screened.

The DNA of one FAP patient, 3214, showed only a 940 kb NotI fragment instead of the expected 1200 kb fragment. DNA was analyzed from four other members of the patient's immediate family; the 940 kb fragment was present in her affected mother (4711), but not in the other, unaffected family members. The mother also carried a normal 1200 kb NotI fragment that was transmitted to her two unaffected offspring. These observations indicated that the mutant polyposis allele is on the same chromosome as the 940 kb NotI fragment. A simple interpretation is that APC patients 3214 and 4711 each carry a 260 kb deletion within the APC locus.

If a deletion were present, then other enzymes might also be expected to produce fragments with altered mobilities. Hybridization of L5.79 to NruI-digested DNAs from both affected members of the family revealed a novel NruI fragment of 1300 kb, in addition to the normal 1200 kb NruI fragment. Furthermore, MluI fragments in patients 3214 and 4711 also showed an increase in size consistent with the deletion of an MluI site. The two chromosome 5 homologs of patient 3214 were segregated in somatic cell hybrid lines; HHW1155 (deletion hybrid) carried the abnormal homolog and HHW1159 (normal hybrid) carried the normal homolog.

Because patient 3214 showed only a 940 kb NotI fragment, she had not inherited the 1200 kb fragment present in the unaffected father's DNA. This observation suggests that he must be heterozygous for, and have transmitted, either a deletion of the L5.79 probe region or a variant NotI fragment too large to resolve on the gel system. As expected, the hybrid cell line HHW1159, which carries the paternal homolog, revealed no resolved Not fragment when probed with L5.79. However, probing of HHW1159 DNA with L5.79 following digestion with other enzymes did reveal restriction fragments, demonstrating the presence of DNA homologous to the probe. The father is, therefore, interpreted as heterozygous for a polymorphism at the NotI site, with one chromosome 5 having a 1200 kb NotI fragment and the other having a fragment too large to resolve consistently on the gel. The latter was transmitted to patient 3214.

When double digests were used to order restriction sites within the 1200 kb NotI fragment, L5.71 and L5.79 were both found to lie on a 550 kb NotI-NruI fragment and, therefore, on the same side of an NruI site in the 1200 kb NotI fragment. To obtain genomic representation of sequences present over the entire 1200 kb NotI fragment, we constructed a library of small-fragment inserts enriched for sequences from this fragment. DNA from the somatic cell hybrid HHW141, which contains about 40% of chromosome 5, was digested with NotI and electrophoresed under pulsed-field gel (PFG) conditions; EcoRI fragments from the 1200 kb region of this gel were cloned into a phage vector. Probe Map30 was isolated from this library. In normal individuals probe Map30 hybridizes to the 1200 kb NotI fragment and to a 200 kb NruI fragment. This latter hybridization places Map30 distal, with respect to the locations of L5.71 and L5.79, to the NruI site of the 550 kb NotI-NruI fragment.

Because Map30 hybridized to the abnormal, 1300 kb NruI fragment of patient 3214, the locus defined by Map30 lies outside the hypothesized deletion. Furthermore, in normal chromosomes Map30 identified a 200 kb NruI fragment and L5.79 identified a 1200 kb Nrul fragment; the hypothesized deletion must, therefore, be removing an NruI site, or sites, lying between Map30 and L5.79, and these two probes must flank the hypothesized deletion. A restriction map of the genomic region, showing placement of these probes, is shown in Figure 5.

A NotI digest of DNA from another FAP patient, 3824, was probed with L5.79. In addition to the 1200 kb normal NotI fragment, a fragment of approximately 1100 kb was observed, consistent with the presence of a 100 kb deletion in one chromosome 5. In this case, however, digestion with NruI and MluI did not reveal abnormal bands, indicating that if a deletion were present, its boundaries must lie distal to the NruI and MluI sites of the fragments identified by L5.79. Consistent with this expectation, hybridization of Map30 to DNA from patient 3824 identified a 760 kb MluI fragment in addition to the expected 860 kb fragment, supporting the interpretation of a 100 kb deletion in this patient. The two chromosome 5 homologs of patient 3824 were segregated in somatic cell hybrid lines; HHW1291 was found to carry only the abnormal homolog and HHW1290 only the normal homolog.

That the 860 kb MluI fragment identified by Map30 is distinct from the 830 kb MluI fragment identified previously by L5.79 was demonstrated by hybridization of Map30 and L5.79 to a NotI-MluI double digest of DNA from the hybrid cell (HHW1159) containing the nondeleted chromosome 5 homolog of patient 3214. As previously indicated, this hybrid is interpreted as missing one of the NotI sites that define the 1200 kb fragment. A 620 kb NotI-MluI fragment was seen with probe L5.79, and an 860 kb fragment was seen with Map30. Therefore, the 830 kb MluI fragment recognized by probe L5.79 must contain a NotI site in HHW1159 DNA; because the 860 kb MluI fragment remains intact, it does not carry this NotI site and must be distinct from the 830 kb MluI fragment.

### Example 5

This example demonstrates the isolation of human sequences which span the region deleted in the two unrelated FAP patients characterized in Example 4.

A strong prediction of the hypothesis that patients 3214 and 3824 carry deletions is that some sequences present on normal chromosome 5 homologs would be missing from the hypothesized deletion homologs. Therefore, to develop genomic probes that might confirm the deletions, as well as to identify genes from the region, YAC clones from a contig seeded by cosmid L5.79 were localized from a library containing seven haploid human genome equivalents (Albertsen et al., Proc. Natl. Acad. Sci. U.S.A., Vol. 87, pp. 4256-4260 (1990)) with respect to the hypothesized deletions. Three clones, YACs 57B8, 310D8, and 183H12, were found to overlap the deleted region.

Importantly, one end of YAC 57B8 (clone AT57) was found to lie within the patient 3214 deletion. Inverse polymerase chain reaction (PCR) defined the end sequences of the insert of YAC 57B8. PCR primers based on one of these end sequences repeatedly failed to amplify DNA from the somatic cell hybrid (HHW1155) carrying the deleted homolog of patient 3214, but did amplify a product of the expected size from the somatic cell hybrid (HHW1159) carrying the normal chromosome 5 homolog. This result supported the interpretation that the abnormal restriction fragments found in the DNA of patient 3214 result from a deletion.

Additional support for the hypothesis of deletion in DNA from patient 3214 came from subcloned fragments of YAC 183H12, which spans the region in question. Yll, an EcoRI fragment cloned from YAC 183H12, hybridized to the normal, 1200 kb NotI fragment of patient 4711, but failed to hybridize to the abnormal, 940 kb NotI fragment of 4711 or to DNA from deletion cell line HHW1155. This result confirmed the deletion in patient 3214.

Two additional EcoR1 fragments from YAC 183H12, Y10 and Y14, were localized within the patient 3214 deletion by their failure to hybridizie to DNA from HHW1155. Probe Y10 hybridizes to a 150 kb NruI fragment in normal chromosome 5 homologs. Because the 3214 deletion creates the 1300 kb NruI fragment seen with the probes L5.79 and Map30 that flank the deletion, these NruI sites and the 150 kb NruI fragment lying between must be deleted in patient 3214. Furthermore, probe Y10 hybridizes to the same 620 kb NotI-MluI fragment seen with probe L5.79 in normal DNA, indicating its location as L5.79-proximal to the deleted Mlul site and placing it between the MluI site and the L5.79-proximal NruI site. The MluI site must, therefore, lie between the NruI sites that define the 150 kb NruI fragment (see Figure 5).

Probe Y11 also hybridized to the 150 kb NruI fragment in the normal chromosome 5 homolog, but failed to hybridize to the 620 kb NotI-MluI fragment, placing it L5.79-distal to the MluI site, but proximal to the second NruI site. Hybridization to the same (860 kb) MluI fragment as Map30 confirmed the localization of probe Y11 L5.79-distal to the MluI site.

Probe Y14 was shown to be L5.79-distal to both deleted NruI sites by virtue of its hybridization to the same 200 kb NruI fragment of the normal chromosome 5 seen with Map30. Therefore, the order of these EcoRI fragments derived from YAC 183H12 and deleted in patient 3214, with respect to L5.79 and Map30, is L5.79-Y10-Y11-Y14-Map30.

The 100 kb deletion of patient 3824 was confirmed by the failure of aberrant restriction fragments in this DNA to hybridize with probe Y11, combined with positive hybridizations to probes Y10 and/or Y14. Y10 and Y14 each hybridized to the 1100 kb NotI fragment of patient 3824 as well as to the normal 1200 kb NotI fragment, but Y11 hybridized to the 1200 kb fragment only. In the MluI digest, probe Y14 hybridized to the 860 kb and 760 kb fragments of patient 3824 DNA, but probe Y11 hybridized only to the 860 kb fragment. We conclude that the basis for the alteration in fragment size in DNA from patient 3824 is, indeed, a deletion. Furthermore, because probes Y10 and Y14 are missing from the deleted 3214 chromosome, but present on the deleted 3824 chromosome, and they have been shown to flank probe Y11, the deletion in patient 3824 must be nested within the patient 3214 deletion.

Probes Y10, Y11, Y14 and Map30 each hybridized to YAC 310D8, indicating that this YAC spanned the patient 3824 deletion and at a minimum, most of the 3214 deletion. The YAC characterizations, therefore, confirmed the presence of deletions in the patients and provided physical representation of the deleted region.

### Example 6

This example demonstrates that the MCC coding sequence maps outside of the region deleted in the two FAP patients characterized in Example 4.

An intriguing FAP candidate gene, MCC, recently was ascertained with cosmid L5.71 and was shown to have undergone mutation in colon carcinomas (Kinzler et al., supra). It was therefore of interest to map this gene with respect to the deletions in APC patients. Hybridization of MCC probes with an overlapping series of YAC clones extending in either direction from L5.71 showed that the 3' end of MCC must be oriented toward the region of the two APC deletions.

Therefore, two 3' cDNA clones from MCC were mapped with respect to the deletions: clone 1CI (bp 2378-4181) and clone 7 (bp 2890-3560). Clone 1CI contains sequences from the C-terminal end of the open reading frame, which stops at nucleotide 2708, as well as 3' untranslated sequence. Clone 7 contains sequence that is entirely 3' to the open reading frame. Importantly, the entire 3' untranslated sequence contained in the cDNA clones consists of a single 2.5 kb exon. These two clones were hybridized to DNAs from the YACs spanning the FAP region. Clone 7 fails to hybridize to YAC 310D8, although it does hybridize to YACs 183H12 and 57B8; the same result was obtained with the cDNA 1CI. Furthermore, these probes did show hybridization to DNAs from both hybrid cell lines (HWW1159 and HWW1155) and the lymphoblastoid cell line from patient 3214, confirming their locations outside the deleted region. Additional mapping experiments suggested that the 3' end of the MCC cDNA clone contig is likely to be located more than 45 kb from the deletion of patient 3214 and, therefore, more than 100 kb from the deletion of patient 3824.

### Example 7

This example identifies three genes within the deleted region of chromosome 5 in the two unrelated FAP patients characterized in Example 4.

Genomic clones were used to screen cDNA libraries in three separate experiments. One screening was done with a phage clone derived from YAC 310D8 known to span the 260 kb deletion of patient 3214. A large-insert phage library was constructed from this YAC; screening with Y11 identified λ205, which mapped within both deletions. When clone λ205 was used to probe a random-, plus oligo(dT)-, primed fetal brain cDNA library (approximately 300,000 phage), six cDNA clones were isolated and each of them mapped entirely within both deletions. Sequence analysis of these six clones formed a single cDNA contig, but did not reveal an extended open reading frame. One of the six cDNAs was used to isolate more cDNA clones, some of which crossed the L5.71-proximal breakpoint of the 3824 deletion, as indicated by hybridization to both chromosome of this patient. These clones also contained an open reading frame, indicating a transcriptional orientation proximal to distal with respect to L5.71. This gene was named DPI (deleted in polyposis 1). This gene is identical to TB2 described above.

cDNA walks yielded a cDNA contig of 3.0-3.5 kb, and included two clones containing terminal poly(A) sequences. This size corresponds to the 3.5 kb band seen by Northern analysis. Sequencing of the first 3163 bp of the cDNA contig revealed an open reading frame extending from the first base to nucleotide 631, followed by a 2.5 kb 3' untranslated region. The sequence surrounding the methionine codon at base 77 conforms to the Kozak consensus of an initiation methionine (Kozak, 1984). Failed attempts to walk farther, coupled with the similarity of the lengths of isolated cDNA and mRNA, suggested that the NH₂-terminus of the DPI protein had been reached. Hybridization to a combination of genomic and YAC DNAs cut with various enzymes indicated the genomic coverage of DPI to be approximately 30 kb.

Two additional probes for the locus, YS-11 and YS-39, which had been ascertained by screening of a cDNA library with an independent YAC probe identified with MCC sequences adjacent to L5.71, were mapped into the deletion region. YS-39 was shown to be a cDNA identical in sequence to DPI. Partial characterization of YS-11 had shown that 200 bp of DNA sequence at one end was identical to sequence coding for the 19 kd protein of the ribosomal signal recognition particle, SRP19 (Lingelbach et al., supra). Hybridization experiments mapped YS-11 within both deletions. The sequence of this clone, however, was found to be complex. Although 454 bp of the 1032 bp sequence of YS-11 were identical to the GenBank entry for the SRP19 gene, another 578 bp appended 5' to the SRP19 sequence was found to consist of previously unreported sequence containing no extended open reading frames. This suggested that YS-11 was either a chimeric clone containing two independent inserts or a clone of an incompletely processed or aberrant message. If YS-11 were a conventional chimeric clone, the independent segments would not be expected to map to the same physical region. The segments resulting from anomalous processing of a continuous transcript, however, would map to a single chromosomal region.

Inverse PCR with primers specific to the two ends of YS-11, the SRP19 end and the unidentified region, verified that both sequences map within the YAC 310D8; therefore, YS-11 is most likely a clone of an immature or anomalous mRNA species. Subsequently, both ends were shown to lie with the deleted region of patient 3824, and YS-11 was used to screen for additional cDNA clones.

Of the 14 cDNA clones selected from the fetal brain library, one clone, V5, was of particular interest in that it contained an open reading frame throughout, although it included only a short identity to the first 78 5' bases of the YS-11 sequence. Following the 78 bp of identical sequence, the two cDNA sequences diverged at an AG. Furthermore, divergence from genomic sequence was also seen after these 78 bp, suggesting the presence of a splice junction, and supporting the view that YS-11 represents an irregular message.

Starting with V5, successive 5' and 3' walks were performed; the resulting cDNA contig consisted of more than 100 clones, which defined a new transcript, DP2. Clones walking in the 5' direction crossed the 3824 deletion breakpoint farthest from L5.71; since its 3' end is closer to this cosmid than its 5' end, the transcriptional orientation of DP2 is opposite to that of MCC and DP1.

The third screening approach relied on hybridization with a 120 kb MluI fragment from YAC 57B8. This fragment hybridizes with probe Y11 and completely spans the 100 kb deletion in patient 3824. the fragment was purified on two preparative PFGs, labeled, and used to screen a fetal brain cDNA library. A number of cDNA clones previously identified in the development of the DPI and DP2 contigs were reascertained. However, 19 new cDNA clones mapped into the patient 3824 deletion. Analysis indicated that these 19 formed a new contig, DP3, containing a large open reading frame.

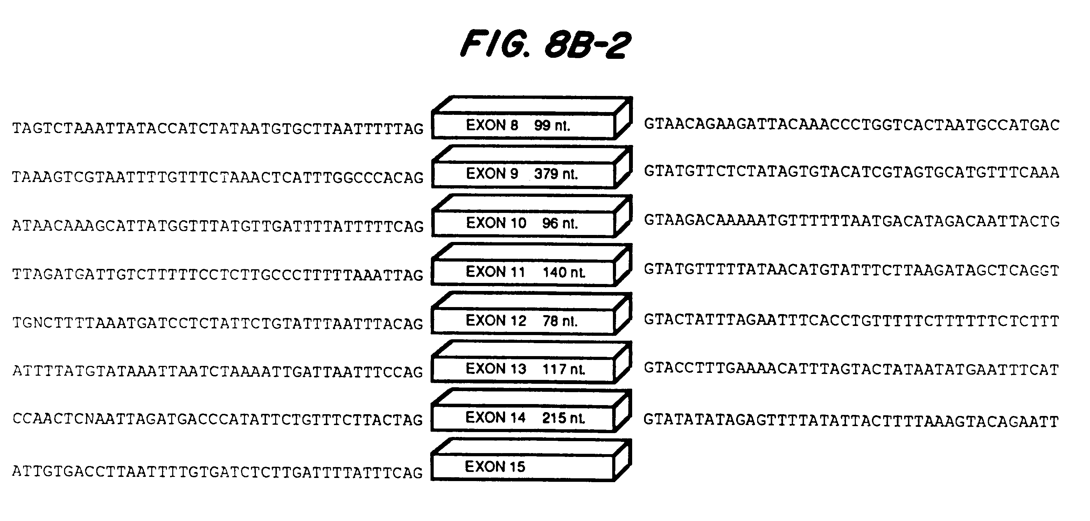

A clone from the 5' end of this new cDNA contig hybridized to the same EcoRI fragment as the 3' end of DP2. Subsequently, the DP2 and DP3 contigs were connected by a single 5' walking step from DP3, to form the single contig DP2.5. The complete nucleotide sequence of DP2.5 is shown in Figure 9.

The consensus cDNA sequence of DP2.5 suggests that the entire coding sequence of DP2.5 has been obtained and is 8532 bp long. The most 5' ATG codon occurs two codons from an in-frame stop and conforms to the Kozak initiation consensus (Kozak, Nucl. Acids. Res., Vol. 12, p. 857-872 1984). The 3' open reading frame breaks down over the final 1.8 kb, giving multiple stops in all frames. A poly(A) sequence was found in one clone approximately 1 kb into the 3' untranslated region, associated with a polyadenylation signal 33 bp upstream (position 9530). The open reading frame is almost identical to that identified as APC above.

An alternatively spliced exon at nucleotide 934 of the DP2.5 transcript is of potential interest. it was first discovered by noting that two classes of cDNA had been isolated. The more abundant cDNA class contains a 303 bp exon not included in the other. The presence in vivo of the two transcripts was verified by an exon connection experiment. Primers flanking the alternatively spliced exon were used to amplify, by PCR, cDNA prepared from various adult tissues. Two PCR products that differed in size by approximately 300 bases were amplified from all the tissues tested; the larger product was always more abundant than the smaller.

### Example 8

This example demonstrates the primers used to identify subtle mutations in DP1, SRP19, and DP25.

To obtain DNA sequence adjacent to the exons of the genes DP1, DP2.5, and SRP19, sequencing substrate was obtained by inverse PCR amplification of DNAs from two YACs, 310D8 and 183H12, that span the deletions. Ligation at low concentration cyclized the restriction enzyme-digested YAC DNAs. Oligonucleotides with sequencing tails, designed in inverse orientation at intervals along the cDNAs, primed PCR amplification from the cyclized templates. Comparison of these DNA sequences with the cDNA sequences placed exon boundaries at the divergence points. SRP19 and DPI were each shown to have five exons. DP2.5 consisted of 15 exons. The sequences of the oligonucleotides synthesized to provide PCR amplification primers for the exons of each of these genes are listed in Table III. With the exception of exons 1, 3, 4, 9, and 15 of DP2.5 (see below), the primer sequences were located in intron sequences flanking the exons. The 5' primer of exon 1 is complementary to the cDNA sequence, but extends just into the 5' Kozak consensus sequence for the initiator methionine, allowing a survey of the translated sequences. The 5' primer of exon 3 is actually in the 5' coding sequences of this exon, as three separate intronic primers simply would not amplify. The 5' primer of exon 4 just overlaps the 5' end of this exon, and we thus fail to survey the 19 most 5' bases of this exon. For exon 9, two overlapping primer sets were used, such that each had one end within the exon. For exon 15, the large 3' exon of DP2.5, overlapping primer pairs were placed along the length of the exon; each pair amplified a product of 250-400 bases.

### Example 9

This example demonstrates the use of single stranded conformation polymorphism (SSCP) analysis as described by Orita et al. Proc. Natl. Acad. Sci. U.S.A., Vol. 86, pp. 2766-70 (1989) and Genomics, Vol. 5, pp. 874-879 (1989) as applied to DP1, SRP19 and DP2.5.

SSCP analysis identifies most single- or multiple-base changes in DNA fragments up to 400 bases in length. Sequence alterations are detected as shifts in electrophoretic mobility of single-stranded DNA on nondenaturing acrylamide gels; the two complementary strands of a DNA segment usually resolve as two SSCP conformers of distinct mobilities. However, if the sample is from an individual heterozygous for a base-pair variant within the amplified segment, often three or more bands are seen. In some cases, even the sample from a homozygous individual will show multiple bands. Base-pair-change variants are identified by differences in pattern among the DNAs of the sample set.

Exons of the candidate genes were amplified by PCR from the DNAs of 61 unrelated FAP patients and a control set of 12 normal individuals. The five exons from DPI revealed no unique conformers in the FAP patients, although common conformers were observed with exons 2 and 3 in some individuals of both affected and control sets, indicating the presence of DNA sequence polymorphisms. Likewise, none of the five exons of SRP19 revealed unique conformers in DNA from FAP patients in the test panel.

Testing of exons 1 through 14 and primer sets A through N of exon 15 of the DP2.5 gene, however, revealed variant conformers specific to FAP patients in exons 7, 8, 10, 11, and 15. These variants were in the unrelated patients 3746, 3460, 3827, 3712, and 3751, respectively. The PCR-SSCP procedure was repeated for each of these exons in the five affected individuals and in an expanded set of 48 normal controls. The variant bands were reproducible in the FAP patients but were not observed in any of the control DNA samples. Additional variant conformers in exons 11 and 15 of the DP2.5 gene were seen; however, each of these was found in both the affected and control DNA sets. The five sets of conformers unique to the FAP patients were sequenced to determine the nucleotide changes responsible for their altered mobilities. The normal conformers from the host individuals were sequenced also. Bands were cut from the dried acrylamide gels, and the DNA was eluted. PCR amplification of these DNAs provided template for sequencing.

The sequences of the unique conformers from exons 7, 8, 10, and 11 of DP2.5 revealed dramatic mutations in the DP2.5 gene. The sequence of the new mutation creating the exon 7 conformer in patient 3746 was shown to contain a deletion of two adjacent nucleotides, at positions 730 and 731 in the cDNA sequence (Figure 7). The normal sequence at this splice junction is CAGGGTCA (intronic sequence underlined), with the intron-exon boundary between the two repetitions of AG. The mutant allele in this patient has the sequence CAGGTCA. Although this change is at the 5' splice site, comparison with known consensus sequences of splice junctions would suggest that a functional splice junction is maintained. If this new splice junction were functional, the mutation would introduce a frameshift that creates a stop codon 15 nucleotides downstream. If the new splice junction were not functional, messenger processing would be significantly altered.

To confirm the 2-base deletion, the PCR product from FAP patient 3746 and a control DNA were electrophoresed on an acrylamide-urea denaturing gel, along with the products of a sequencing reaction. The sample from patient 3746 showed two bands differing in size by 2 nucleotides, with the larger band identical in mobility to the control sample; this result was independent confirmation that patient 3746 is heterozygous for a 2 bp deletion.

The unique conformer found in exon 8 of patient 3460 was found to carry a C-T transition, at position 904 in the cDNA sequence of DP2.5 (shown in Figure 7), which replaced the normal sequence of CGA with TGA. This point mutation, when read in frame, results in a stop codon replacing the normal arginine codon. This single-base change had occurred within the context of a CG dimer, a potential hot spot for mutation (Barker et al., 1984).

The conformer unique to FAP patient 3827 in exon 10 was found to contain a deletion of one nucleotide (1367, 1368, or 1369) when compared to the normal sequence found in the other bands on the SSCP gel. This deletion, occurring within a set of three T's, changed the sequence from CTTTCA to CTTCA; this 1 base frameshift creates a downstream stop within 30 bases. The PCR product amplified from this patient's DNA also was electrophoresed on an acrylamide-urea denaturing gel, along with the PCR product from a control DNA and products from a sequencing reaction. The patient's PCR product showed two bands differing by 1 bp in length, with the larger identical in mobility to the PCR product from the normal DNA; this result confirmed the presence of a 1 bp deletion in patient 3827.

Sequence analysis of the variant conformer of exon 11 from patient 3712 revealed the substitution of a T by a G at position 1500, changing the normal tyrosine codon to a stop codon.

The pair of conformers observed in exon 15 of the DP2.5 gene for FAP patient 3751 also was sequenced. These conformers were found to carry a nucleotide substitution of C to G at position 5253, the third base of a valine codon. No amino acid change resulted from this substitution, suggesting that this conformer reflects a genetically silent polymorphism.

The observation of distinct inactivating mutations in the DP2.5 gene in four unrelated patients strongly suggested that DP2.5 is the gene involved in FAP. These mutations are summarized in Table IIA.

### Example 10

This example demonstrates that the mutations identified in the DP2.5 (APC) gene segregate with the FAP phenotype.

Patient 3746, described above as carrying an APC allele with a frameshift mutation, is an affected offspring of two normal parents. Colonoscopy revealed no polyps in either parent nor among the patient's three siblings.

DNA samples from both parents, from the patient's wife, and from their three children were examined. SSCP analysis of DNA from both of the patient's parents displayed the normal pattern of conformers for exon 7, as did DNA from the patients's wife and one of his offspring. The two other children, however, displayed the same new conformers as their affected father. Testing of the patient and his parents with highly polymorphic VNTR (variable number of tandem repeat) markers showed a 99.98% likelihood that they are his biological parents.

These observations confirmed that this novel conformer, known to reflect a 2 bp deletion mutation in the DP2.5 gene, appeared spontaneously with FAP in this pedigree and was transmitted to two of the children of the affected individual.

### Example 11

This example demonstrates polymorphisms in the APC gene which appear to be unrelated to disease (FAP).

Sequencing of variant conformers found among controls as well as individuals with APC has revealed the following polymorphisms in the APC gene: first, in exon 11, at position 1458, a substitution of T to C creating an RsaI restriction site but no amino acid change; and second, in exon 15, at positions 5037 and 5271, substitutions of A to G and G to T, respectively, neither resulting in amino acid substitutions. These nucleotide polymorphisms in the APC gene sequence may be useful for diagnostic purposes.

### Example 12

This example shows the structure of the APC gene.

The structure of the APC gene is schematically shown in Figure 8, with flanking intron sequences indicated.

The continuity of the very large (6.5 kb), most 3' exon in DP2.5 was shown in two ways. First, inverse PCR with primers spanning the entire length of this exon revealed no divergence of the cDNA sequence from the genomic sequence. Second, PCR amplification with converging primers placed at intervals along the exon generated products of the same size whether amplified from the originally isolated cDNA, cDNA from various tissues, or genomic template. Two forms of exon 9 were found in DP2.5: one is the complete exon; and the other, labeled exon 9A, is the result of a splice into the interior of the exon that deletes bases 934 to 1236 in the mRNA and removes 101 amino acids from the predicted protein (see Figure 7).

### Example 13

This example demonstrates the mapping of the FAP deletions with respect to the APC exons.

Somatic cell hybrids carrying the segregated chromosomes 5 from the 100 kb (HHW1291) and 260 kb (HHW1155) deletion patients were used to determine the distribution of the APC genes exons across the deletions. DNAs from these cell lines were used as template, along with genomic DNA from a normal control, for PCR-based amplification of the APC exons.

PCR analysis of the hybrids from the 260 kb deletion of patient 3214 showed that all but one (exon 1) of the APC exons are removed by this deletion. PCR analysis of the somatic cell hybrid HHW1291, carrying the chromosome 5 homolog with the 100 kb deletion from patient 3824, revealed that exons 1 through 9 are present but exons 10 through 15 are missing. This result placed the deletion breakpoint either between exons 9 and 10 or within exon 10.

### Example 14

This example demonstrates the expression of alternately spliced APC messenger in normal tissues and in cancer cell lines.

Tissues that express the APC gene were identified by PCR amplification of cDNA made to mRNA with primers located within adjacent APC exons. In addition, PCR primers that flank the alternatively spliced exon 9 were chosen so that the expression pattern of both splice forms could be assessed. All tissue types tested (brain, lung, aorta, spleen, heart, kidney, liver, stomach, placenta, and colonic mucosa) and cultured cell lines (lymphoblasts, HL60, and choriocarcinoma) expressed both splice forms of the APC gene. We note, however, that expression by lymphocytes normally residing in some tissues, including colon, prevents unequivocal assessment of expression. The large mRNA, containing the complete exon 9 rather than only exon 9A, appears to be the more abundant message.

Northern analysis of poly(A)-selected RNA from lymphoblasts revealed a single band of approximately 10 kb, consistent with the size of the sequenced cDNA.

### Example 15

This example discusses structural features of the APC protein predicted from the sequence.

The cDNA consensus sequence of APC predicts that the longer, more abundant form of the message codes for a 2842 or 28444 amino acid peptide with a mass of 311.8 kd. This predicted APC peptide was compared with the current data bases of protein and DNA sequences using both Intelligenetics and GCG software packages. No genes with a high degree of amino acid sequence similarity were found. Although many short (approximately 20 amino acid) regions of sequence similarity were uncovered, none was sufficently strong to reveal which, if any, might represent functional homology. Interestingly, multiple similarities to myosins and keratins did appear. The APC gene also was scanned for sequence motifs of known function; although multiple glycosylation, phosphorylation, and myristoylation sites were seen, their significance is uncertain.

Analysis of the APC peptide sequence did identify features important in considering potential protein structure. Hydropathy plots (Kyte and Doolittle, J. Mol. Biol. Vol. 157, pp. 105-132 (1982)) indicate that the APC protein is notably hydrophilic. No hydrophobic domains suggesting a signal peptide or a membrane-spanning domain were found. Analysis of the first 1000 residues indicates that α-helical rods may form (Cohen and Parry, Trends Biochem, Sci. Vol. 77, pp. 245-248 (1986); there is a scarcity of proline residues and, there are a number of regions containing heptad repeats (apolar-X-X-apolar-X-X-X). Interestingly, in exon 9A, the deleted form of exon 9, two heptad repeat regions are reconnected in the proper heptad repeat frame, deleting the intervening peptide region. After the first 1000 residues, the high proline content of the remainder of the peptide suggests a compact rather than a rod-like structure.

The most prominent feature of the second 1000 residues is a 20 amino acid repeat that is iterated seven times with semiregular spacing (Table 4). The intervening sequences between the seven repeat regions contained 114, 116, 151, 205, 107, and 58 amino acids, respectively. Finally, residues 2200-24000 contain a 200 amino acid basic domain.

### SEQUENCE LISTING

(1) GENERAL INFORMATION:
   (i) APPLICANT: ALBERTSEN, HANS
      ANAND, RAKESH
      CARLSON, MARY
      GRODEN, JOANNA
      HEDGE, PHILIP J.
      JOSLYN, GEOFF
      KINZLER, KENNETH
      MARKHAM, ALEXANDER F.
      NAKAMURA, YUSUKE
      THLIVERIS, ANDREW
   (ii) TITLE OF INVENTION: INHERITED AND SOMATIC MUTATIONS OF APC GENE IN COLORECTAL CANCER IN HUMANS
   (iii) NUMBER OF SEQUENCES: 94
   (iv) CORRESPONDENCE ADDRESS:
      (A) ADDRESSEE: Banner, Birch, McKie & Beckett
      (B) STREET: 1001 G Street, NW
      (C) CITY: Washington
      (D) STATE: D.C.
      (E) COUNTRY: USA
      (F) ZIP: 20001-4598
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US 07/741,940
      (B) FILING DATE: 08-AUG-1991
      (C) CLASSIFICATION:
   (viii) ATTORNEY/AGENT INFORMATION:
      (A) NAME: Kagan, Sarah A.
      (B) REGISTRATION NUMBER: 32,141
      (C) REFERENCE/DOCKET NUMBER: 1107.035574
   (ix) TELECOMMUNICATION INFORMATION:
      (A) TELEPHONE: 202-508-9100
      (B) TELEFAX: 202-508-9299
(2) INFORMATION FOR SEQ ID NO:1:
   (i) SEQUENCE CHARACTERISTICS:
      (A) LENGTH: 9606 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Homo sapiens
   (vii) IMMEDIATE SOURCE:
      (B) CLONE: DP2.5(APC)
   (ix) FEATURE:
      (A) NAME/KEY: CDS
      (B) LOCATION: 34..8562
   (xi) SEQUENCE DESCRIPTION: SEQ ID NO:1:
   GGACTCGGAA ATGAGGTCCA AGGGTAGCCA AGG ATG GCT GCA GCT TCA TAT GAT      54
                                        Met Ala Ala Ala Ser Tyr Asp
                                          1               5
   CAG TTG TTA AAG CAA GTT GAG GCA CTG AAG ATG GAG AAC TCA AAT CTT      102
   Gln Leu Leu Lys Gln Val Glu Ala Leu Lys Met Glu Asn Ser Asn Leu
           10                  15                  20
   CGA CAA GAG CTA GAA GAT AAT TCC AAT CAT CTT ACA AAA CTG GAA ACT      150
   Arg Gln Glu Leu Glu Asp Asn Ser Asn His Leu Thr Lys Leu Glu Thr
       25                  30                  35
   GAG GCA TCT AAT ATG AAG GAA GTA CTT AAA CAA CTA CAA GGA AGT ATT      198
   Glu Ala Ser Asn Met Lys Glu Val Leu Lys Gln Leu Gln Gly Ser Ile
    40                  45                  50                  55
   GAA GAT GAA GCT ATG GCT TCT TCT GGA CAG ATT GAT TTA TTA GAG CGT      246
   Glu Asp Glu Ala Met Ala Ser Ser Gly Gln Ile Asp Leu Leu Glu Arg
                   60                  65                  70
   CTT AAA GAG CTT AAC TTA GAT AGC AGT AAT TTC CCT GGA GTA AAA CTG      294
   Leu Lys Glu Leu Asn Leu Asp Ser Ser Asn Phe Pro Gly Val Lys Leu
               75                  80                  85
   CGG TCA AAA ATG TCC CTC CGT TCT TAT GGA AGC CGG GAA GGA TCT GTA      342
   Arg Ser Lys Met Ser Leu Arg Ser Tyr Gly Ser Arg Glu Gly Ser Val
           90                  95                 100
   TCA AGC CGT TCT GGA GAG TGC AGT CCT GTT CCT ATG GGT TCA TTT CCA      390
   Ser Ser Arg Ser Gly Glu Cys Ser Pro Val Pro Met Gly Ser Phe Pro
       105                 110                 115
   AGA AGA GGG TTT GTA AAT GGA AGC AGA GAA AGT ACT GGA TAT TTA GAA      438
   Arg Arg Gly Phe Val Asn Gly Ser Arg Glu Ser Thr Gly Tyr Leu Glu
   120                 125                 130                 135
   GAA CTT GAG AAA GAG AGG TCA TTG CTT CTT GCT GAT CTT GAC AAA GAA      486
   Glu Leu Glu Lys Glu Arg Ser Leu Leu Leu Ala Asp Leu Asp Lys Glu
                   140                 145                 150
   GAA AAG GAA AAA GAC TGG TAT TAC GCT CAA CTT CAG AAT CTC ACT AAA      534
   Glu Lys Glu Lys Asp Trp Tyr Tyr Ala Gln Leu Gln Asn Leu Thr Lys
               155                 160                 165
   AGA ATA GAT AGT CTT CCT TTA ACT GAA AAT TTT TCC TTA CAA ACA GAT      582
   Arg Ile Asp Ser Leu Pro Leu Thr Glu Asn Phe Ser Leu Gln Thr Asp
           170                 175                 180
   TTG ACC AGA AGG CAA TTG GAA TAT GAA GCA AGG CAA ATC AGA GTT GCG      630
   Leu Thr Arg Arg Gln Leu Glu Tyr Glu Ala Arg Gln Ile Arg Val Ala
       185                 190                 195
   ATG GAA GAA CAA CTA GGT ACC TGC CAG GAT ATG GAA AAA CGA GCA CAG      678
   Met Glu Glu Gln Leu Gly Thr Cys Gln Asp Met Glu Lys Arg Ala Gln
   200                 205                 210                 215
   CGA AGA ATA GCC AGA ATT CAG CAA ATC GAA AAG GAC ATA CTT CGT ATA      726
   Arg Arg Ile Ala Arg Ile Gln Gln Ile Glu Lys Asp Ile Leu Arg Ile
                   220                 225                 230
   CGA CAG CTT TTA CAG TCC CAA GCA ACA GAA GCA GAG AGG TCA TCT CAG      774
   Arg Gln Leu Leu Gln Ser Gln Ala Thr Glu Ala Glu Arg Ser Ser Gln
               235                 240                 245
   AAC AAG CAT GAA ACC GGC TCA CAT GAT GCT GAG CGG CAG AAT GAA GGT      822
   Asn Lys His Glu Thr Gly Ser His Asp Ala Glu Arg Gln Asn Glu Gly
           250                 255                 260
   CAA GGA GTG GGA GAA ATC AAC ATG GCA ACT TCT GGT AAT GGT CAG GGT      870
   Gln Gly Val Gly Glu Ile Asn Met Ala Thr Ser Gly Asn Gly Gln Gly
       265                 270                 275
   TCA ACT ACA CGA ATG GAC CAT GAA ACA GCC AGT GTT TTG AGT TCT AGT      918
   Ser Thr Thr Arg Met Asp His Glu Thr Ala Ser Val Leu Ser Ser Ser
   280                 285                 290                 295
   AGC ACA CAC TCT GCA CCT CGA AGG CTG ACA AGT CAT CTG GGA ACC AAG      966
   Ser Thr His Ser Ala Pro Arg Arg Leu Thr Ser His Leu Gly Thr Lys
                   300                 305                 310
   GTG GAA ATG GTG TAT TCA TTG TTG TCA ATG CTT GGT ACT CAT GAT AAG     1014
   Val Glu Met Val Tyr Ser Leu Leu Ser Met Leu Gly Thr His Asp Lys
               315                 320                 325
   GAT GAT ATG TCG CGA ACT TTG CTA GCT ATG TCT AGC TCC CAA GAC AGC     1062
   Asp Asp Met Ser Arg Thr Leu Leu Ala Met Ser Ser Ser Gln Asp Ser
           330                 335                 340
   TGT ATA TCC ATG CGA CAG TCT GGA TGT CTT CCT CTC CTC ATC CAG CTT     1110
   Cys Ile Ser Met Arg Gln Ser Gly Cys Leu Pro Leu Leu Ile Gln Leu
       345                 350                 355
   TTA CAT GGC AAT GAC AAA GAC TCT GTA TTG TTG GGA AAT TCC CGG GGC     1158
   Leu His Gly Asn Asp Lys Asp Ser Val Leu Leu Gly Asn Ser Arg Gly
   360                 365                 370                 375
   AGT AAA GAG GCT CGG GCC AGG GCC AGT GCA GCA CTC CAC AAC ATC ATT     1206
   Ser Lys Glu Ala Arg Ala Arg Ala Ser Ala Ala Leu His Asn Ile Ile
                   380                 385                 390
   CAC TCA CAG CCT GAT GAC AAG AGA GGC AGG CGT GAA ATC CGA GTC CTT     1254
   His Ser Gln Pro Asp Asp Lys Arg Gly Arg Arg Glu Ile Arg Val Leu
               395                 400                 405
   CAT CTT TTG GAA CAG ATA CGC GCT TAC TGT GAA ACC TGT TGG GAG TGG     1302
   His Leu Leu Glu Gln Ile Arg Ala Tyr Cys Glu Thr Cys Trp Glu Trp
           410                 415                 420
   CAG GAA GCT CAT GAA CCA GGC ATG GAC CAG GAC AAA AAT CCA ATG CCA     1350
   Gln Glu Ala His Glu Pro Gly Met Asp Gln Asp Lys Asn Pro Met Pro
       425                 430                 435
   GCT CCT GTT GAA CAT CAG ATC TGT CCT GCT GTG TGT GTT CTA ATG AAA      1398
   Ala Pro Val Glu His Gln Ile Cys Pro Ala Val Cys Val Leu Met Lys
   440                 445                 450                 455
   CTT TCA TTT GAT GAA GAG CAT AGA CAT GCA ATG AAT GAA CTA GGG GGA      1446
   Leu Ser Phe Asp Glu Glu His Arg His Ala Met Asn Glu Leu Gly Gly
                   460                 465                 470
   CTA CAG GCC ATT GCA GAA TTA TTG CAA GTG GAC TGT GAA ATG TAT GGG      1494
   Leu Gln Ala Ile Ala Glu Leu Leu Gln Val Asp Cys Glu Met Tyr Gly
               475                 480                 485
   CTT ACT AAT GAC CAC TAC AGT ATT ACA CTA AGA CGA TAT GCT GGA ATG      1542
   Leu Thr Asn Asp His Tyr Ser Ile Thr Leu Arg Arg Tyr Ala Gly Met
           490                 495                 500
   GCT TTG ACA AAC TTG ACT TTT GGA GAT GTA GCC AAC AAG GCT ACG CTA      1590
   Ala Leu Thr Asn Leu Thr Phe Gly Asp Val Ala Asn Lys Ala Thr Leu
       505                 510                 515
   TGC TCT ATG AAA GGC TGC ATG AGA GCA CTT GTG GCC CAA CTA AAA TCT      1638
   Cys Ser Met Lys Gly Cys Met Arg Ala Leu Val Ala Gln Leu Lys Ser
   520                 525                 530                 535
   GAA AGT GAA GAC TTA CAG CAG GTT ATT GCA AGT GTT TTG AGG AAT TTG      1686
   Glu Ser Glu Asp Leu Gln Gln Val Ile Ala Ser Val Leu Arg Asn Leu
                   540                 545                 550
   TCT TGG CGA GCA GAT GTA AAT AGT AAA AAG ACG TTG CGA GAA GTT GGA      1734
   Ser Trp Arg Ala Asp Val Asn Ser Lys Lys Thr Leu Arg Glu Val Gly
               555                 560                 565
   AGT GTG AAA GCA TTG ATG GAA TGT GCT TTA GAA GTT AAA AAG GAA TCA      1782
   Ser Val Lys Ala Leu Met Glu Cys Ala Leu Glu Val Lys Lys Glu Ser
           570                 575                 580
   ACC CTC AAA AGC GTA TTG AGT GCC TTA TGG AAT TTG TCA GCA CAT TGC      1830
   Thr Leu Lys Ser Val Leu Ser Ala Leu Trp Asn Leu Ser Ala His Cys
       585                 590                 595
   ACT GAG AAT AAA GCT GAT ATA TGT GCT GTA GAT GGT GCA CTT GCA TTT      1878
   Thr Glu Asn Lys Ala Asp Ile Cys Ala Val Asp Gly Ala Leu Ala Phe
   600                 605                 610                 615
   TTG GTT GGC ACT CTT ACT TAC CGG AGC CAG ACA AAC ACT TTA GCC ATT      1926
   Leu Val Gly Thr Leu Thr Tyr Arg Ser Gln Thr Asn Thr Leu Ala Ile
                   620                 625                 630
   ATT GAA AGT GGA GGT GGG ATA TTA CGG AAT GTG TCC AGC TTG ATA GCT      1974
   Ile Glu Ser Gly Gly Gly Ile Leu Arg Asn Val Ser Ser Leu Ile Ala
               635                 640                 645
   ACA AAT GAG GAC CAC AGG CAA ATC CTA AGA GAG AAC AAC TGT CTA CAA      2022
   Thr Asn Glu Asp His Arg Gln Ile Leu Arg Glu Asn Asn Cys Leu Gln
           650                 655                 660
   ACT TTA TTA CAA CAC TTA AAA TCT CAT AGT TTG ACA ATA GTC AGT AAT      2070
   Thr Leu Leu Gln His Leu Lys Ser His Ser Leu Thr Ile Val Ser Asn
       665                 670                 675
   GCA TGT GGA ACT TTG TGG AAT CTC TCA GCA AGA AAT CCT AAA GAC CAG        2118
   Ala Cys Gly Thr Leu Trp Asn Leu Ser Ala Arg Asn Pro Lys Asp Gln
   680                 685                 690                 695
   GAA GCA TTA TGG GAC ATG GGG GCA GTT AGC ATG CTC AAG AAC CTC ATT        2166
   Glu Ala Leu Trp Asp Met Gly Ala Val Ser Met Leu Lys Asn Leu Ile
                   700                 705                 710
   CAT TCA AAG CAC AAA ATG ATT GCT ATG GGA AGT GCT GCA GCT TTA AGG        2214
   His Ser Lys His Lys Met Ile Ala Met Gly Ser Ala Ala Ala Leu Arg
               715                 720                 725
   AAT CTC ATG GCA AAT AGG CCT GCG AAG TAC AAG GAT GCC AAT ATT ATG        2262
   Asn Leu Met Ala Asn Arg Pro Ala Lys Tyr Lys Asp Ala Asn Ile Met
           730                 735                 740
   TCT CCT GGC TCA AGC TTG CCA TCT CTT CAT GTT AGG AAA CAA AAA GCC        2310
   Ser Pro Gly Ser Ser Leu Pro Ser Leu His Val Arg Lys Gln Lys Ala
       745                 750                 755
   CTA GAA GCA GAA TTA GAT GCT CAG CAC TTA TCA GAA ACT TTT GAC AAT        2358
   Leu Glu Ala Glu Leu Asp Ala Gln His Leu Ser Glu Thr Phe Asp Asn
   760                 765                 770                 775
   ATA GAC AAT TTA AGT CCC AAG GCA TCT CAT CGT AGT AAG CAG AGA CAC        2406
   Ile Asp Asn Leu Ser Pro Lys Ala Ser His Arg Ser Lys Gln Arg His
                   780                 785                 790
   AAG CAA AGT CTC TAT GGT GAT TAT GTT TTT GAC ACC AAT CGA CAT GAT        2454
   Lys Gln Ser Leu Tyr Gly Asp Tyr Val Phe Asp Thr Asn Arg His Asp
               795                 800                 805
   GAT AAT AGG TCA GAC AAT TTT AAT ACT GGC AAC ATG ACT GTC CTT TCA        2502
   Asp Asn Arg Ser Asp Asn Phe Asn Thr Gly Asn Met Thr Val Leu Ser
           810                 815                 820
   CCA TAT TTG AAT ACT ACA GTG TTA CCC AGC TCC TCT TCA TCA AGA GGA        2550
   Pro Tyr Leu Asn Thr Thr Val Leu Pro Ser Ser Ser Ser Ser Arg Gly
       825                 830                 835
   AGC TTA GAT AGT TCT CGT TCT GAA AAA GAT AGA AGT TTG GAG AGA GAA        2598
   Ser Leu Asp Ser Ser Arg Ser Glu Lys Asp Arg Ser Leu Glu Arg Glu
   840                 845                 850                 855
   CGC GGA ATT GGT CTA GGC AAC TAC CAT CCA GCA ACA GAA AAT CCA GGA        2646
   Arg Gly Ile Gly Leu Gly Asn Tyr His Pro Ala Thr Glu Asn Pro Gly
                   860                 865                 870
   ACT TCT TCA AAG CGA GGT TTG CAG ATC TCC ACC ACT GCA GCC CAG ATT        2694
   Thr Ser Ser Lys Arg Gly Leu Gln Ile Ser Thr Thr Ala Ala Gln Ile
               875                 880                 885
   GCC AAA GTC ATG GAA GAA GTG TCA GCC ATT CAT ACC TCT CAG GAA GAC        2742
   Ala Lys Val Met Glu Glu Val Ser Ala Ile His Thr Ser Gln Glu Asp
           890                 895                 900
   AGA AGT TCT GGG TCT ACC ACT GAA TTA CAT TGT GTG ACA GAT GAG AGA        2790
   Arg Ser Ser Gly Ser Thr Thr Glu Leu His Cys Val Thr Asp Glu Arg
       905                 910                 915
   AAT GCA CTT AGA AGA AGC TCT GCT GCC CAT ACA CAT TCA AAC ACT TAC 2838
   Asn Ala Leu Arg Arg Ser Ser Ala Ala His Thr His Ser Asn Thr Tyr
   920 925 930 935
   AAT TTC ACT AAG TCG GAA AAT TCA AAT AGG ACA TGT TCT ATG CCT TAT 2886
   Asn Phe Thr Lys Ser Glu Asn Ser Asn Arg Thr Cys Ser Met Pro Tyr
   940 945 950
   GCC AAA TTA GAA TAC AAG AGA TCT TCA AAT GAT AGT TTA AAT AGT GTC 2934
   Ala Lys Leu Glu Tyr Lys Arg Ser Ser Asn Asp Ser Leu Asn Ser Val
   955 960 965
   AGT AGT AAT GAT GGT TAT GGT AAA AGA GGT CAA ATG AAA CCC TCG ATT 2982
   Ser Ser Asn Asp Gly Tyr Gly Lys Arg Gly Gln Met Lys Pro Ser Ile
   970 975 980
   GAA TCC TAT TCT GAA GAT GAT GAA AGT AAG TTT TGC AGT TAT GGT CAA 3030
   Glu Ser Tyr Ser Glu Asp Asp Glu Ser Lys Phe Cys Ser Tyr Gly Gln
   985 990 995
   TAC CCA GCC GAC CTA GCC CAT AAA ATA CAT AGT GCA AAT CAT ATG GAT 3078
   Tyr Pro Ala Asp Leu Ala His Lys Ile His Ser Ala Asn His Met Asp
   1000 1005 1010 1015
   GAT AAT GAT GGA GAA CTA GAT ACA CCA ATA AAT TAT AGT CTT AAA TAT 3126
   Asp Asn Asp Gly Glu Leu Asp Thr Pro Ile Asn Tyr Ser Leu Lys Tyr
   1020 1025 1030
   TCA GAT GAG CAG TTG AAC TCT GGA AGG CAA AGT CCT TCA CAG AAT GAA 3174
   Ser Asp Glu Gln Leu Asn Ser Gly Arg Gln Ser Pro Ser Gln Asn Glu
   1035 1040 1045
   AGA TGG GCA AGA CCC AAA CAC ATA ATA GAA GAT GAA ATA AAA CAA AGT 3222
   Arg Trp Ala Arg Pro Lys His Ile Ile Glu Asp Glu Ile Lys Gln Ser
   1050 1055 1060
   GAG CAA AGA CAA TCA AGG AAT CAA AGT ACA ACT TAT CCT GTT TAT ACT 3270
   Glu Gln Arg Gln Ser Arg Asn Gln Ser Thr Thr Tyr Pro Val Tyr Thr
   1065 1070 1075
   GAG AGC ACT GAT GAT AAA CAC CTC AAG TTC CAA CCA CAT TTT GGA CAG 3318
   Glu Ser Thr Asp Asp Lys His Leu Lys Phe Gln Pro His Phe Gly Gln
   1080 1085 1090 1095
   CAG GAA TGT GTT TCT CCA TAC AGG TCA CGG GGA GCC AAT GGT TCA GAA 3366
   Gln Glu Cys Val Ser Pro Tyr Arg Ser Arg Gly Ala Asn Gly Ser Glu
   1100 1105 1110
   ACA AAT CGA GTG GGT TCT AAT CAT GGA ATT AAT CAA AAT GTA AGC CAG 3414
   Thr Asn Arg Val Gly Ser Asn His Gly Ile Asn Gln Asn Val Ser Gln
   1115 1120 1125
   TCT TTG TGT CAA GAA GAT GAC TAT GAA GAT GAT AAG CCT ACC AAT TAT 3462
   Ser Leu Cys Gln Glu Asp Asp Tyr Glu Asp Asp Lys Pro Thr Asn Tyr
   1130 1135 1140
   AGT GAA CGT TAC TCT GAA GAA GAA CAG CAT GAA GAA GAA GAG AGA CCA 3510
   Ser Glu Arg Tyr Ser Glu Glu Glu Gln His Glu Glu Glu Glu Arg Pro
   1145 1150 1155
   ACA AAT TAT AGC ATA AAA TAT AAT GAA GAG AAA CGT CAT GTG GAT CAG       3558
   Thr Asn Tyr Ser Ile Lys Tyr Asn Glu Glu Lys Arg His Val Asp Gln
   1160                1165                1170                1175
   CCT ATT GAT TAT AGT TTA AAA TAT GCC ACA GAT ATT CCT TCA TCA CAG       3606
   Pro Ile Asp Tyr Ser Leu Lys Tyr Ala Thr Asp Ile Pro Ser Ser Gln
                   1180                1185                1190
   AAA CAG TCA TTT TCA TTC TCA AAG AGT TCA TCT GGA CAA AGC AGT AAA       3654
   Lys Gln Ser Phe Ser Phe Ser Lys Ser Ser Ser Gly Gln Ser Ser Lys
               1195                1200                1205
   ACC GAA CAT ATG TCT TCA AGC AGT GAG AAT ACG TCC ACA CCT TCA TCT       3702
   Thr Glu His Met Ser Ser Ser Ser Glu Asn Thr Ser Thr Pro Ser Ser
           1210                1215                1220
   AAT GCC AAG AGG CAG AAT CAG CTC CAT CCA AGT TCT GCA CAG AGT AGA       3750
   Asn Ala Lys Arg Gln Asn Gln Leu His Pro Ser Ser Ala Gln Ser Arg
       1225                1230                1235
   AGT GGT CAG CCT CAA AAG GCT GCC ACT TGC AAA GTT TCT TCT ATT AAC       3798
   Ser Gly Gln Pro Gln Lys Ala Ala Thr Cys Lys Val Ser Ser Ile Asn
   1240                1245                1250                1255
   CAA GAA ACA ATA CAG ACT TAT TGT GTA GAA GAT ACT CCA ATA TGT TTT       3846
   Gln Glu Thr Ile Gln Thr Tyr Cys Val Glu Asp Thr Pro Ile Cys Phe
                   1260                1265                1270
   TCA AGA TGT AGT TCA TTA TCA TCT TTG TCA TCA GCT GAA GAT GAA ATA       3894
   Ser Arg Cys Ser Ser Leu Ser Ser Leu Ser Ser Ala Glu Asp Glu Ile
               1275                1280                1285
   GGA TGT AAT CAG ACG ACA CAG GAA GCA GAT TCT GCT AAT ACC CTG CAA       3942
   Gly Cys Asn Gln Thr Thr Gln Glu Ala Asp Ser Ala Asn Thr Leu Gln
           1290                1295                1300
   ATA GCA GAA ATA AAA GGA AAG ATT GGA ACT AGG TCA GCT GAA GAT CCT       3990
   Ile Ala Glu Ile Lys Gly Lys Ile Gly Thr Arg Ser Ala Glu Asp Pro
       1305                1310                1315
   GTG AGC GAA GTT CCA GCA GTG TCA CAG CAC CCT AGA ACC AAA TCC AGC       4038
   Val Ser Glu Val Pro Ala Val Ser Gln His Pro Arg Thr Lys Ser Ser
   1320                1325                1330                1335
   AGA CTG CAG GGT TCT AGT TTA TCT TCA GAA TCA GCC AGG CAC AAA GCT       4086
   Arg Leu Gln Gly Ser Ser Leu Ser Ser Glu Ser Ala Arg His Lys Ala
                   1340                1345                1350
   GTT GAA TTT CCT TCA GGA GCG AAA TCT CCC TCC AAA AGT GGT GCT CAG       4134
   Val Glu Phe Pro Ser Gly Ala Lys Ser Pro Ser Lys Ser Gly Ala Gln
               1355                1360                1365
   ACA CCC AAA AGT CCA CCT GAA CAC TAT GTT CAG GAG ACC CCA CTC ATG       4182
   Thr Pro Lys Ser Pro Pro Glu His Tyr Val Gln Glu Thr Pro Leu Met
           1370                1375                1380
   TTT AGC AGA TGT ACT TCT GTC AGT TCA CTT GAT AGT TTT GAG AGT CGT       4230
   Phe Ser Arg Cys Thr Ser Val Ser Ser Leu Asp Ser Phe Glu Ser Arg
       1385                1390                1395
   TCG ATT GCC AGC TCC GTT CAG AGT GAA CCA TGC AGT GGA ATG GTA AGT      4278
   Ser Ile Ala Ser Ser Val Gln Ser Glu Pro Cys Ser Gly Met Val Ser
   1400                1405                1410                1415
   GGC ATT ATA AGC CCC AGT GAT CTT CCA GAT AGC CCT GGA CAA ACC ATG      4326
   Gly Ile Ile Ser Pro Ser Asp Leu Pro Asp Ser Pro Gly Gln Thr Met
                   1420                1425                1430
   CCA CCA AGC AGA AGT AAA ACA CCT CCA CCA CCT CCT CAA ACA GCT CAA      4374
   Pro Pro Ser Arg Ser Lys Thr Pro Pro Pro Pro Pro Gln Thr Ala Gln
               1435                1440                1445
   ACC AAG CGA GAA GTA CCT AAA AAT AAA GCA CCT ACT GCT GAA AAG AGA      4422
   Thr Lys Arg Glu Val Pro Lys Asn Lys Ala Pro Thr Ala Glu Lys Arg
           1450                1455                1460
   GAG AGT GGA CCT AAG CAA GCT GCA GTA AAT GCT GCA GTT CAG AGG GTC      4470
   Glu Ser Gly Pro Lys Gln Ala Ala Val Asn Ala Ala Val Gln Arg Val
       1465                1470                1475
   CAG GTT CTT CCA GAT GCT GAT ACT TTA TTA CAT TTT GCC ACA GAA AGT      4518
   Gln Val Leu Pro Asp Ala Asp Thr Leu Leu His Phe Ala Thr Glu Ser
   1480                1485                1490                1495
   ACT CCA GAT GGA TTT TCT TGT TCA TCC AGC CTG AGT GCT CTG AGC CTC      4566
   Thr Pro Asp Gly Phe Ser Cys Ser Ser Ser Leu Ser Ala Leu Ser Leu
                   1500                1505                1510
   GAT GAG CCA TTT ATA CAG AAA GAT GTG GAA TTA AGA ATA ATG CCT CCA      4614
   Asp Glu Pro Phe Ile Gln Lys Asp Val Glu Leu Arg Ile Met Pro Pro
               1515                1520                1525
   GTT CAG GAA AAT GAC AAT GGG AAT GAA ACA GAA TCA GAG CAG CCT AAA      4662
   Val Gln Glu Asn Asp Asn Gly Asn Glu Thr Glu Ser Glu Gln Pro Lys
           1530                1535                1540
   GAA TCA AAT GAA AAC CAA GAG AAA GAG GCA GAA AAA ACT ATT GAT TCT      4710
   Glu Ser Asn Glu Asn Gln Glu Lys Glu Ala Glu Lys Thr Ile Asp Ser
       1545                1550                1555
   GAA AAG GAC CTA TTA GAT GAT TCA GAT GAT GAT GAT ATT GAA ATA CTA      4758
   Glu Lys Asp Leu Leu Asp Asp Ser Asp Asp Asp Asp Ile Glu Ile Leu
   1560                1565                1570                1575
   GAA GAA TGT ATT ATT TCT GCC ATG CCA ACA AAG TCA TCA CGT AAA GGC      4806
   Glu Glu Cys Ile Ile Ser Ala Met Pro Thr Lys Ser Ser Arg Lys Gly
                   1580                1585                1590
   AAA AAG CCA GCC CAG ACT GCT TCA AAA TTA CCT CCA CCT GTG GCA AGG      4854
   Lys Lys Pro Ala Gln Thr Ala Ser Lys Leu Pro Pro Pro Val Ala Arg
               1595                1600                1605
   AAA CCA AGT CAG CTG CCT GTG TAC AAA CTT CTA CCA TCA CAA AAC AGG      4902
   Lys Pro Ser Gln Leu Pro Val Tyr Lys Leu Leu Pro Ser Gln Asn Arg
           1610                1615                1620
   TTG CAA CCC CAA AAG CAT GTT AGT TTT ACA CCG GGG GAT GAT ATG CCA      4950
   Leu Gln Pro Gln Lys His Val Ser Phe Thr Pro Gly Asp Asp Met Pro
       1625                1630                1635
   CGG GTG TAT TGT GTT GAA GGG ACA CCT ATA AAC TTT TCC ACA GCT ACA      4998
   Arg Val Tyr Cys Val Glu Gly Thr Pro Ile Asn Phe Ser Thr Ala Thr
   1640                1645                1650                1655
   TCT CTA AGT GAT CTA ACA ATC GAA TCC CCT CCA AAT GAG TTA GCT GCT      5046
   Ser Leu Ser Asp Leu Thr Ile Glu Ser Pro Pro Asn Glu Leu Ala Ala
                   1660                1665                1670
   GGA GAA GGA GTT AGA GGA GGA GCA CAG TCA GGT GAA TTT GAA AAA CGA      5094
   Gly Glu Gly Val Arg Gly Gly Ala Gln Ser Gly Glu Phe Glu Lys Arg
               1675                1680                1685
   GAT ACC ATT CCT ACA GAA GGC AGA AGT ACA GAT GAG GCT CAA GGA GGA      5142
   Asp Thr Ile Pro Thr Glu Gly Arg Ser Thr Asp Glu Ala Gln Gly Gly
           1690                1695                1700
   AAA ACC TCA TCT GTA ACC ATA CCT GAA TTG GAT GAC AAT AAA GCA GAG      5190
   Lys Thr Ser Ser Val Thr Ile Pro Glu Leu Asp Asp Asn Lys Ala Glu
       1705                1710                1715
   GAA GGT GAT ATT CTT GCA GAA TGC ATT AAT TCT GCT ATG CCC AAA GGG      5238
   Glu Gly Asp Ile Leu Ala Glu Cys Ile Asn Ser Ala Met Pro Lys Gly
   1720                1725                1730                1735
   AAA AGT CAC AAG CCT TTC CGT GTG AAA AAG ATA ATG GAC CAG GTC CAG      5286
   Lys Ser His Lys Pro Phe Arg Val Lys Lys Ile Met Asp Gln Val Gln
                   1740                1745                1750
   CAA GCA TCT GCG TCG TCT TCT GCA CCC AAC AAA AAT CAG TTA GAT GGT      5334
   Gln Ala Ser Ala Ser Ser Ser Ala Pro Asn Lys Asn Gln Leu Asp Gly
               1755                1760                1765
   AAG AAA AAG AAA CCA ACT TCA CCA GTA AAA CCT ATA CCA CAA AAT ACT      5382
   Lys Lys Lys Lys Pro Thr Ser Pro Val Lys Pro Ile Pro Gln Asn Thr
           1770                1775                1780
   GAA TAT AGG ACA CGT GTA AGA AAA AAT GCA GAC TCA AAA AAT AAT TTA      5430
   Glu Tyr Arg Thr Arg Val Arg Lys Asn Ala Asp Ser Lys Asn Asn Leu
       1785                1790                1795
   AAT GCT GAG AGA GTT TTC TCA GAC AAC AAA GAT TCA AAG AAA CAG AAT      5478
   Asn Ala Glu Arg Val Phe Ser Asp Asn Lys Asp Ser Lys Lys Gln Asn
   1800                1805                1810                1815
   TTG AAA AAT AAT TCC AAG GAC TTC AAT GAT AAG CTC CCA AAT AAT GAA      5526
   Leu Lys Asn Asn Ser Lys Asp Phe Asn Asp Lys Leu Pro Asn Asn Glu
                   1820                1825                1830
   GAT AGA GTC AGA GGA AGT TTT GCT TTT GAT TCA CCT CAT CAT TAC ACG      5574
   Asp Arg Val Arg Gly Ser Phe Ala Phe Asp Ser Pro His His Tyr Thr
               1835                1840                1845
   CCT ATT GAA GGA ACT CCT TAC TGT TTT TCA CGA AAT GAT TCT TTG AGT      5622
   Pro Ile Glu Gly Thr Pro Tyr Cys Phe Ser Arg Asn Asp Ser Leu Ser
           1850                1855                1860
   TCT CTA GAT TTT GAT GAT GAT GAT GTT GAC CTT TCC AGG GAA AAG GCT      5670
   Ser Leu Asp Phe Asp Asp Asp Asp Val Asp Leu Ser Arg Glu Lys Ala
       1865                1870                1875
   GAA TTA AGA AAG GCA AAA GAA AAT AAG GAA TCA GAG GCT AAA GTT ACC      5718
   Glu Leu Arg Lys Ala Lys Glu Asn Lys Glu Ser Glu Ala Lys Val Thr
   1880                1885                1890                1895
   AGC CAC ACA GAA CTA ACC TCC AAC CAA CAA TCA GCT AAT AAG ACA CAA      5766
   Ser His Thr Glu Leu Thr Ser Asn Gln Gln Ser Ala Asn Lys Thr Gln
                   1900                1905                1910
   GCT ATT GCA AAG CAG CCA ATA AAT CGA GGT CAG CCT AAA CCC ATA CTT      5814
   Ala Ile Ala Lys Gln Pro Ile Asn Arg Gly Gln Pro Lys Pro Ile Leu
               1915                1920                1925
   CAG AAA CAA TCC ACT TTT CCC CAG TCA TCC AAA GAC ATA CCA GAC AGA      5862
   Gln Lys Gln Ser Thr Phe Pro Gln Ser Ser Lys Asp Ile Pro Asp Arg
           1930                1935                1940
   GGG GCA GCA ACT GAT GAA AAG TTA CAG AAT TTT GCT ATT GAA AAT ACT      5910
   Gly Ala Ala Thr Asp Glu Lys Leu Gln Asn Phe Ala Ile Glu Asn Thr
       1945                1950                1955
   CCA GTT TGC TTT TCT CAT AAT TCC TCT CTG AGT TCT CTC AGT GAC ATT      5958
   Pro Val Cys Phe Ser His Asn Ser Ser Leu Ser Ser Leu Ser Asp Ile
   1960                1965                1970                1975
   GAC CAA GAA AAC AAC AAT AAA GAA AAT GAA CCT ATC AAA GAG ACT GAG      6006
   Asp Gln Glu Asn Asn Asn Lys Glu Asn Glu Pro Ile Lys Glu Thr Glu
                   1980                1985                1990
   CCC CCT GAC TCA CAG GGA GAA CCA AGT AAA CCT CAA GCA TCA GGC TAT      6054
   Pro Pro Asp Ser Gln Gly Glu Pro Ser Lys Pro Gln Ala Ser Gly Tyr
               1995                2000                2005
   GCT CCT AAA TCA TTT CAT GTT GAA GAT ACC CCA GTT TGT TTC TCA AGA      6102
   Ala Pro Lys Ser Phe His Val Glu Asp Thr Pro Val Cys Phe Ser Arg
           2010                2015                2020
   AAC AGT TCT CTC AGT TCT CTT AGT ATT GAC TCT GAA GAT GAC CTG TTG      6150
   Asn Ser Ser Leu Ser Ser Leu Ser Ile Asp Ser Glu Asp Asp Leu Leu
       2025                2030                2035
   CAG GAA TGT ATA AGC TCC GCA ATG CCA AAA AAG AAA AAG CCT TCA AGA      6198
   Gln Glu Cys Ile Ser Ser Ala Met Pro Lys Lys Lys Lys Pro Ser Arg
   2040                2045                2050                2055
   CTC AAG GGT GAT AAT GAA AAA CAT AGT CCC AGA AAT ATG GGT GGC ATA      6246
   Leu Lys Gly Asp Asn Glu Lys His Ser Pro Arg Asn Met Gly Gly Ile
                   2060                2065                2070
   TTA GGT GAA GAT CTG ACA CTT GAT TTG AAA GAT ATA CAG AGA CCA GAT      6294
   Leu Gly Glu Asp Leu Thr Leu Asp Leu Lys Asp Ile Gln Arg Pro Asp
               2075                2080                2085
   TCA GAA CAT GGT CTA TCC CCT GAT TCA GAA AAT TTT GAT TGG AAA GCT      6342
   Ser Glu His Gly Leu Ser Pro Asp Ser Glu Asn Phe Asp Trp Lys Ala
           2090                2095                2100
   ATT CAG GAA GGT GCA AAT TCC ATA GTA AGT AGT TTA CAT CAA GCT GCT      6390
   Ile Gln Glu Gly Ala Asn Ser Ile Val Ser Ser Leu His Gln Ala Ala
       2105                2110                2115
   GCT GCT GCA TGT TTA TCT AGA CAA GCT TCG TCT GAT TCA GAT TCC ATC      6438
   Ala Ala Ala Cys Leu Ser Arg Gln Ala Ser Ser Asp Ser Asp Ser Ile
   2120                2125                2130                2135
   CTT TCC CTG AAA TCA GGA ATC TCT CTG GGA TCA CCA TTT CAT CTT ACA      6486
   Leu Ser Leu Lys Ser Gly Ile Ser Leu Gly Ser Pro Phe His Leu Thr
                   2140                2145                2150
   CCT GAT CAA GAA GAA AAA CCC TTT ACA AGT AAT AAA GGC CCA CGA ATT      6534
   Pro Asp Gln Glu Glu Lys Pro Phe Thr Ser Asn Lys Gly Pro Arg Ile
               2155                2160                2165
   CTA AAA CCA GGG GAG AAA AGT ACA TTG GAA ACT AAA AAG ATA GAA TCT      6582
   Leu Lys Pro Gly Glu Lys Ser Thr Leu Glu Thr Lys Lys Ile Glu Ser
           2170                2175                2180
   GAA AGT AAA GGA ATC AAA GGA GGA AAA AAA GTT TAT AAA AGT TTG ATT      6630
   Glu Ser Lys Gly Ile Lys Gly Gly Lys Lys Val Tyr Lys Ser Leu Ile
       2185                2190                2195
   ACT GGA AAA GTT CGA TCT AAT TCA GAA ATT TCA GGC CAA ATG AAA CAG      6678
   Thr Gly Lys Val Arg Ser Asn Ser Glu Ile Ser Gly Gln Met Lys Gln
   2200                2205                2210                2215
   CCC CTT CAA GCA AAC ATG CCT TCA ATC TCT CGA GGC AGG ACA ATG ATT      6726
   Pro Leu Gln Ala Asn Met Pro Ser Ile Ser Arg Gly Arg Thr Met Ile
                   2220                2225                2230
   CAT ATT CCA GGA GTT CGA AAT AGC TCC TCA AGT ACA AGT CCT GTT TCT      6774
   His Ile Pro Gly Val Arg Asn Ser Ser Ser Ser Thr Ser Pro Val Ser
               2235                2240                2245
   AAA AAA GGC CCA CCC CTT AAG ACT CCA GCC TCC AAA AGC CCT AGT GAA      6822
   Lys Lys Gly Pro Pro Leu Lys Thr Pro Ala Ser Lys Ser Pro Ser Glu
           2250                2255                2260
   GGT CAA ACA GCC ACC ACT TCT CCT AGA GGA GCC AAG CCA TCT GTG AAA      6870
   Gly Gln Thr Ala Thr Thr Ser Pro Arg Gly Ala Lys Pro Ser Val Lys
       2265                2270                2275
   TCA GAA TTA AGC CCT GTT GCC AGG CAG ACA TCC CAA ATA GGT GGG TCA      6918
   Ser Glu Leu Ser Pro Val Ala Arg Gln Thr Ser Gln Ile Gly Gly Ser
   2280                2285                2290                2295
   AGT AAA GCA CCT TCT AGA TCA GGA TCT AGA GAT TCG ACC CCT TCA AGA      6966
   Ser Lys Ala Pro Ser Arg Ser Gly Ser Arg Asp Ser Thr Pro Ser Arg
                   2300                2305                2310
   CCT GCC CAG CAA CCA TTA AGT AGA CCT ATA CAG TCT CCT GGC CGA AAC      7014
   Pro Ala Gln Gln Pro Leu Ser Arg Pro Ile Gln Ser Pro Gly Arg Asn
               2315                2320                2325
   TCA ATT TCC CCT GGT AGA AAT GGA ATA AGT CCT CCT AAC AAA TTA TCT      7062
   Ser Ile Ser Pro Gly Arg Asn Gly Ile Ser Pro Pro Asn Lys Leu Ser
           2330                2335                2340
   CAA CTT CCA AGG ACA TCA TCC CCT AGT ACT GCT TCA ACT AAG TCC TCA      7110
   Gln Leu Pro Arg Thr Ser Ser Pro Ser Thr Ala Ser Thr Lys Ser Ser
       2345                2350                2355
   GGT TCT GGA AAA ATG TCA TAT ACA TCT CCA GGT AGA CAG ATG AGC CAA      7158
   Gly Ser Gly Lys Met Ser Tyr Thr Ser Pro Gly Arg Gln Met Ser Gln
   2360                2365                2370                2375
   CAG AAC CTT ACC AAA CAA ACA GGT TTA TCC AAG AAT GCC AGT AGT ATT      7206
   Gln Asn Leu Thr Lys Gln Thr Gly Leu Ser Lys Asn Ala Ser Ser Ile
                   2380                2385                2390
   CCA AGA AGT GAG TCT GCC TCC AAA GGA CTA AAT CAG ATG AAT AAT GGT      7254
   Pro Arg Ser Glu Ser Ala Ser Lys Gly Leu Asn Gln Met Asn Asn Gly
               2395                2400                2405
   AAT GGA GCC AAT AAA AAG GTA GAA CTT TCT AGA ATG TCT TCA ACT AAA      7302
   Asn Gly Ala Asn Lys Lys Val Glu Leu Ser Arg Met Ser Ser Thr Lys
           2410                2415                2420
   TCA AGT GGA AGT GAA TCT GAT AGA TCA GAA AGA CCT GTA TTA GTA CGC      7350
   Ser Ser Gly Ser Glu Ser Asp Arg Ser Glu Arg Pro Val Leu Val Arg
       2425                2430                2435
   CAG TCA ACT TTC ATC AAA GAA GCT CCA AGC CCA ACC TTA AGA AGA AAA      7398
   Gln Ser Thr Phe Ile Lys Glu Ala Pro Ser Pro Thr Leu Arg Arg Lys
   2440                2445                2450                2455
   TTG GAG GAA TCT GCT TCA TTT GAA TCT CTT TCT CCA TCA TCT AGA CCA      7446
   Leu Glu Glu Ser Ala Ser Phe Glu Ser Leu Ser Pro Ser Ser Arg Pro
                   2460                2465                2470
   GCT TCT CCC ACT AGG TCC CAG GCA CAA ACT CCA GTT TTA AGT CCT TCC      7494
   Ala Ser Pro Thr Arg Ser Gln Ala Gln Thr Pro Val Leu Ser Pro Ser
               2475                2480                2485
   CTT CCT GAT ATG TCT CTA TCC ACA CAT TCG TCT GTT CAG GCT GGT GGA      7542
   Leu Pro Asp Met Ser Leu Ser Thr His Ser Ser Val Gln Ala Gly Gly
           2490                2495                2500
   TGG CGA AAA CTC CCA CCT AAT CTC AGT CCC ACT ATA GAG TAT AAT GAT      7590
   Trp Arg Lys Leu Pro Pro Asn Leu Ser Pro Thr Ile Glu Tyr Asn Asp
       2505                2510                2515
   GGA AGA CCA GCA AAG CGC CAT GAT ATT GCA CGG TCT CAT TCT GAA AGT      7638
   Gly Arg Pro Ala Lys Arg His Asp Ile Ala Arg Ser His Ser Glu Ser
   2520                2525                2530                2535
   CCT TCT AGA CTT CCA ATC AAT AGG TCA GGA ACC TGG AAA CGT GAG CAC      7686
   Pro Ser Arg Leu Pro Ile Asn Arg Ser Gly Thr Trp Lys Arg Glu His
                   2540                2545                2550
   AGC AAA CAT TCA TCA TCC CTT CCT CGA GTA AGC ACT TGG AGA AGA ACT      7734
   Ser Lys His Ser Ser Ser Leu Pro Arg Val Ser Thr Trp Arg Arg Thr
               2555                2560                2565
   GGA AGT TCA TCT TCA ATT CTT TCT GCT TCA TCA GAA TCC AGT GAA AAA      7782
   Gly Ser Ser Ser Ser Ile Leu Ser Ala Ser Ser Glu Ser Ser Glu Lys
           2570                2575                2580
   GCA AAA AGT GAG GAT GAA AAA CAT GTG AAC TCT ATT TCA GGA ACC AAA      7830
   Ala Lys Ser Glu Asp Glu Lys His Val Asn Ser Ile Ser Gly Thr Lys
       2585                2590                2595
   CAA AGT AAA GAA AAC CAA GTA TCC GCA AAA GGA ACA TGG AGA AAA ATA        7878
   Gln Ser Lys Glu Asn Gln Val Ser Ala Lys Gly Thr Trp Arg Lys Ile
   2600                2605                2610                2615
   AAA GAA AAT GAA TTT TCT CCC ACA AAT AGT ACT TCT CAG ACC GTT TCC        7926
   Lys Glu Asn Glu Phe Ser Pro Thr Asn Ser Thr Ser Gln Thr Val Ser
                   2620                2625                2630
   TCA GGT GCT ACA AAT GGT GCT GAA TCA AAG ACT CTA ATT TAT CAA ATG        7974
   Ser Gly Ala Thr Asn Gly Ala Glu Ser Lys Thr Leu Ile Tyr Gln Met
               2635                2640                2645
   GCA CCT GCT GTT TCT AAA ACA GAG GAT GTT TGG GTG AGA ATT GAG GAC        8022
   Ala Pro Ala Val Ser Lys Thr Glu Asp Val Trp Val Arg Ile Glu Asp
           2650                2655                2660
   TGT CCC ATT AAC AAT CCT AGA TCT GGA AGA TCT CCC ACA GGT AAT ACT        8070
   Cys Pro Ile Asn Asn Pro Arg Ser Gly Arg Ser Pro Thr Gly Asn Thr
       2665                2670                2675
   CCC CCG GTG ATT GAC AGT GTT TCA GAA AAG GCA AAT CCA AAC ATT AAA        8118
   Pro Pro Val Ile Asp Ser Val Ser Glu Lys Ala Asn Pro Asn Ile Lys
   2680                2685                2690                2695
   GAT TCA AAA GAT AAT CAG GCA AAA CAA AAT GTG GGT AAT GGC AGT GTT        8166
   Asp Ser Lys Asp Asn Gln Ala Lys Gln Asn Val Gly Asn Gly Ser Val
                   2700                2705                2710
   CCC ATG CGT ACC GTG GGT TTG GAA AAT CGC CTG ACC TCC TTT ATT CAG        8214
   Pro Met Arg Thr Val Gly Leu Glu Asn Arg Leu Thr Ser Phe Ile Gln
               2715                2720                2725
   GTG GAT GCC CCT GAC CAA AAA GGA ACT GAG ATA AAA CCA GGA CAA AAT        8262
   Val Asp Ala Pro Asp Gln Lys Gly Thr Glu Ile Lys Pro Gly Gln Asn
           2730                2735                2740
   AAT CCT GTC CCT GTA TCA GAG ACT AAT GAA AGT CCT ATA GTG GAA CGT        8310
   Asn Pro Val Pro Val Ser Glu Thr Asn Glu Ser Pro Ile Val Glu Arg
       2745                2750                2755
   ACC CCA TTC AGT TCT AGC AGC TCA AGC AAA CAC AGT TCA CCT AGT GGG        8358
   Thr Pro Phe Ser Ser Ser Ser Ser Ser Lys His Ser Ser Pro Ser Gly
   2760                2765                2770                2775
   ACT GTT GCT GCC AGA GTG ACT CCT TTT AAT TAC AAC CCA AGC CCT AGG        8406
   Thr Val Ala Ala Arg Val Thr Pro Phe Asn Tyr Asn Pro Ser Pro Arg
                   2780                2785                2790
   AAA AGC AGC GCA GAT AGC ACT TCA GCT CGG CCA TCT CAG ATC CCA ACT        8454
   Lys Ser Ser Ala Asp Ser Thr Ser Ala Arg Pro Ser Gln Ile Pro Thr
               2795                2800                2805
   CCA GTG AAT AAC AAC ACA AAG AAG CGA GAT TCC AAA ACT GAC AGC ACA        8502
   Pro Val Asn Asn Asn Thr Lys Lys Arg Asp Ser Lys Thr Asp Ser Thr
           2810                2815                2820
   GAA TCC AGT GGA ACC CAA AGT CCT AAG CGC CAT TCT GGG TCT TAC CTT        8550
   Glu Ser Ser Gly Thr Gln Ser Pro Lys Arg His Ser Gly Ser Tyr Leu
       2825                2830                2835
   GTG ACA TCT GTT TAAAAGAGAG GAAGAATGAA ACTAAGAAAA TTCTATGTTA 8602
   Val Thr Ser Val
   2840
   ATTACAACTG CTATATAGAC ATTTTGTTTC AAATGAAACT TTAAAAGACT GAAAAATTTT 8662
   GTAAATAGGT TTGATTCTTG TTAGAGGGTT TTTGTTCTGG AAGCCATATT TGATAGTATA 8722
   CTTTGTCTTC ACTGGTCTTA TTTTGGGAGG CACTCTTGAT GGTTAGGAAA AAATAGAAAG 8782
   CCAAGTATGT TTGTACAGTA TGTTTTACAT GTATTTAAAG TAGCATCCCA TCCCAACTTC 8842
   CTTAATTATT GCTTGTCTAA AATAATGAAC ACTACAGATA GGAAATATGA TATATTGCTG 8902
   TTATCAATCA TTTCTAGATT ATAAACTGAC TAAACTTACA TCAGGGGAAA ATTGGTATTT 8962
   ATGCAAAAAA AAAATGTTTT TGTCCTTGTG AGTCCATCTA ACATCATAAT TAATCATGTG 9022
   GCTGTGAAAT TCACAGTAAT ATGGTTCCCG ATGAACAAGT TTACCCAGCC TGCTTTGCTT 9082
   ACTGCATGAA TGAAACTGAT GGTTCAATTT CAGAAGTAAT GATTAACAGT TATGTGGTCA 9142
   CATGATGTGC ATAGAGATAG CTACAGTGTA ATAATTTACA CTATTTTGTG CTCCAAACAA 9202
   AACAAAAATC TGTGTAACTG TAAAACATTG AATGAAACTA TTTTACCTGA ACTAGATTTT 9262
   ATCTGAAAGT AGGTAGAATT TTTGCTATGC TGTAATTTGT TGTATATTCT GGTATTTGAG 9322
   GTGAGATGGC TGCTCTTTAT TAATGAGACA TGAATTGTGT CTCAACAGAA ACTAAATGAA 9382
   CATTTCAGAA TAAATTATTG CTGTATGTAA ACTGTTACTG AAATTGGTAT TTGTTTGAAG 9442
   GGTTTGTTTC ACATTTGTAT TAATTAATTG TTTAAAATGC CTCTTTTAAA AGCTTATATA 9502
   AATTTTTTCT TCAGCTTCTA TGCATTAAGA GTAAAATTCC TCTTACTGTA ATAAAAACAT 9562
   TGAAGAAGAC TGTTGCCACT TAACCATTCC ATGCGTTGGC ACTT 9606
(2) INFORMATION FOR SEQ ID NO:2:
   (i) SEQUENCE CHARACTERISTICS:
      (A) LENGTH: 2843 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Ala Ala Ala Ser Tyr Asp Gln Leu Leu Lys Gln Val Glu Ala Leu
   1 5 10 15
   Lys Met Glu Asn Ser Asn Leu Arg Gln Glu Leu Glu Asp Asn Ser Asn
   20 25 30
   His Leu Thr Lys Leu Glu Thr Glu Ala Ser Asn Met Lys Glu Val Leu
   35 40 45
   Lys Gln Leu Gln Gly Ser Ile Glu Asp Glu Ala Met Ala Ser Ser Gly
   50 55 60
   Gln Ile Asp Leu Leu Glu Arg Leu Lys Glu Leu Asn Leu Asp Ser Ser
   65 70 75 80
   Asn Phe Pro Gly Val Lys Leu Arg Ser Lys Met Ser Leu Arg Ser Tyr
   85 90 95
   Gly Ser Arg Glu Gly Ser Val Ser Ser Arg Ser Gly Glu Cys Ser Pro
   100 105 110
   Val Pro Met Gly Ser Phe Pro Arg Arg Gly Phe Val Asn Gly Ser Arg
   115 120 125
   Glu Ser Thr Gly Tyr Leu Glu Glu Leu Glu Lys Glu Arg Ser Leu Leu
   130 135 140
   Leu Ala Asp Leu Asp Lys Glu Glu Lys Glu Lys Asp Trp Tyr Tyr Ala
   145 150 155 160
   Gln Leu Gln Asn Leu Thr Lys Arg Ile Asp Ser Leu Pro Leu Thr Glu
   165 170 175
   Asn Phe Ser Leu Gln Thr Asp Leu Thr Arg Arg Gln Leu Glu Tyr Glu
   180 185 190
   Ala Arg Gln Ile Arg Val Ala Met Glu Glu Gln Leu Gly Thr Cys Gln
   195 200 205
   Asp Met Glu Lys Arg Ala Gln Arg Arg Ile Ala Arg Ile Gln Gln Ile
   210 215 220
   Glu Lys Asp Ile Leu Arg Ile Arg Gln Leu Leu Gln Ser Gln Ala Thr
   225 230 235 240
   Glu Ala Glu Arg Ser Ser Gln Asn Lys His Glu Thr Gly Ser His Asp
   245 250 255
   Ala Glu Arg Gln Asn Glu Gly Gln Gly Val Gly Glu Ile Asn Met Ala
   260 265 270
   Thr Ser Gly Asn Gly Gln Gly Ser Thr Thr Arg Met Asp His Glu Thr
   275 280 285
   Ala Ser Val Leu Ser Ser Ser Ser Thr His Ser Ala Pro Arg Arg Leu
   290 295 300
   Thr Ser His Leu Gly Thr Lys Val Glu Met Val Tyr Ser Leu Leu Ser
   305 310 315 320
   Met Leu Gly Thr His Asp Lys Asp Asp Met Ser Arg Thr Leu Leu Ala
   325 330 335
   Met Ser Ser Ser Gln Asp Ser Cys Ile Ser Met Arg Gln Ser Gly Cys
   340 345 350
   Leu Pro Leu Leu Ile Gln Leu Leu His Gly Asn Asp Lys Asp Ser Val
   355 360 365
   Leu Leu Gly Asn Ser Arg Gly Ser Lys Glu Ala Arg Ala Arg Ala Ser
   370 375 380
   Ala Ala Leu His Asn Ile Ile His Ser Gln Pro Asp Asp Lys Arg Gly
   385 390 395 400
   Arg Arg Glu Ile Arg Val Leu His Leu Leu Glu Gln Ile Arg Ala Tyr
   405 410 415
   Cys Glu Thr Cys Trp Glu Trp Gln Glu Ala His Glu Pro Gly Met Asp
   420 425 430
   Gln Asp Lys Asn Pro Met Pro Ala Pro Val Glu His Gln Ile Cys Pro
   435 440 445
   Ala Val Cys Val Leu Met Lys Leu Ser Phe Asp Glu Glu His Arg His
   450 455 460
   Ala Met Asn Glu Leu Gly Gly Leu Gln Ala Ile Ala Glu Leu Leu Gln
   465 470 475 480
   Val Asp Cys Glu Met Tyr Gly Leu Thr Asn Asp His Tyr Ser Ile Thr
   485 490 495
   Leu Arg Arg Tyr Ala Gly Met Ala Leu Thr Asn Leu Thr Phe Gly Asp
   500 505 510
   Val Ala Asn Lys Ala Thr Leu Cys Ser Met Lys Gly Cys Met Arg Ala
   515 520 525
   Leu Val Ala Gln Leu Lys Ser Glu Ser Glu Asp Leu Gln Gln Val Ile
   530 535 540
   Ala Ser Val Leu Arg Asn Leu Ser Trp Arg Ala Asp Val Asn Ser Lys
   545 550 555 560
   Lys Thr Leu Arg Glu Val Gly Ser Val Lys Ala Leu Met Glu Cys Ala
   565 570 575
   Leu Glu Val Lys Lys Glu Ser Thr Leu Lys Ser Val Leu Ser Ala Leu
   580 585 590
   Trp Asn Leu Ser Ala His Cys Thr Glu Asn Lys Ala Asp Ile Cys Ala
   595 600 605
   Val Asp Gly Ala Leu Ala Phe Leu Val Gly Thr Leu Thr Tyr Arg Ser
   610 615 620
   Gln Thr Asn Thr Leu Ala Ile Ile Glu Ser Gly Gly Gly Ile Leu Arg
   625 630 635 640
   Asn Val Ser Ser Leu Ile Ala Thr Asn Glu Asp His Arg Gln Ile Leu
   645 650 655
   Arg Glu Asn Asn Cys Leu Gln Thr Leu Leu Gln His Leu Lys Ser His
   660 665 670
   Ser Leu Thr Ile Val Ser Asn Ala Cys Gly Thr Leu Trp Asn Leu Ser
   675 680 685
   Ala Arg Asn Pro Lys Asp Gln Glu Ala Leu Trp Asp Met Gly Ala Val
   690 695 700
   Ser Met Leu Lys Asn Leu Ile His Ser Lys His Lys Met Ile Ala Met
   705 710 715 720
   Gly Ser Ala Ala Ala Leu Arg Asn Leu Met Ala Asn Arg Pro Ala Lys
   725 730 735
   Tyr Lys Asp Ala Asn Ile Met Ser Pro Gly Ser Ser Leu Pro Ser Leu
   740 745 750
   His Val Arg Lys Gln Lys Ala Leu Glu Ala Glu Leu Asp Ala Gln His
   755 760 765
   Leu Ser Glu Thr Phe Asp Asn Ile Asp Asn Leu Ser Pro Lys Ala Ser
   770 775 780
   His Arg Ser Lys Gln Arg His Lys Gln Ser Leu Tyr Gly Asp Tyr Val
   785 790 795 800
   Phe Asp Thr Asn Arg His Asp Asp Asn Arg Ser Asp Asn Phe Asn Thr
   805 810 815
   Gly Asn Met Thr Val Leu Ser Pro Tyr Leu Asn Thr Thr Val Leu Pro
   820 825 830
   Ser Ser Ser Ser Ser Arg Gly Ser Leu Asp Ser Ser Arg Ser Glu Lys
   835 840 845
   Asp Arg Ser Leu Glu Arg Glu Arg Gly Ile Gly Leu Gly Asn Tyr His
   850 855 860
   Pro Ala Thr Glu Asn Pro Gly Thr Ser Ser Lys Arg Gly Leu Gln Ile
   865 870 875 880
   Ser Thr Thr Ala Ala Gln Ile Ala Lys Val Met Glu Glu Val Ser Ala
   885 890 895
   Ile His Thr Ser Gln Glu Asp Arg Ser Ser Gly Ser Thr Thr Glu Leu
   900 905 910
   His Cys Val Thr Asp Glu Arg Asn Ala Leu Arg Arg Ser Ser Ala Ala
   915 920 925
   His Thr His Ser Asn Thr Tyr Asn Phe Thr Lys Ser Glu Asn Ser Asn
   930 935 940
   Arg Thr Cys Ser Met Pro Tyr Ala Lys Leu Glu Tyr Lys Arg Ser Ser
   945 950 955 960
   Asn Asp Ser Leu Asn Ser Val Ser Ser Asn Asp Gly Tyr Gly Lys Arg
   965 970 975
   Gly Gln Met Lys Pro Ser Ile Glu Ser Tyr Ser Glu Asp Asp Glu Ser
   980 985 990
   Lys Phe Cys Ser Tyr Gly Gln Tyr Pro Ala Asp Leu Ala His Lys Ile
   995 1000 1005
   His Ser Ala Asn His Met Asp Asp Asn Asp Gly Glu Leu Asp Thr Pro
   1010 1015 1020
   Ile Asn Tyr Ser Leu Lys Tyr Ser Asp Glu Gln Leu Asn Ser Gly Arg
   1025 1030 1035 1040
   Gln Ser Pro Ser Gln Asn Glu Arg Trp Ala Arg Pro Lys His Ile Ile
   1045 1050 1055
   Glu Asp Glu Ile Lys Gln Ser Glu Gln Arg Gln Ser Arg Asn Gln Ser
   1060 1065 1070
   Thr Thr Tyr Pro Val Tyr Thr Glu Ser Thr Asp Asp Lys His Leu Lys
   1075 1080 1085
   Phe Gln Pro His Phe Gly Gln Gln Glu Cys Val Ser Pro Tyr Arg Ser
   1090 1095 1100
   Arg Gly Ala Asn Gly Ser Glu Thr Asn Arg Val Gly Ser Asn His Gly
   1105 1110 1115 1120
   Ile Asn Gln Asn Val Ser Gln Ser Leu Cys Gln Glu Asp Asp Tyr Glu
   1125 1130 1135
   Asp Asp Lys Pro Thr Asn Tyr Ser Glu Arg Tyr Ser Glu Glu Glu Gln
   1140 1145 1150
   His Glu Glu Glu Glu Arg Pro Thr Asn Tyr Ser Ile Lys Tyr Asn Glu
   1155 1160 1165
   Glu Lys Arg His Val Asp Gln Pro Ile Asp Tyr Ser Leu Lys Tyr Ala
   1170 1175 1180
   Thr Asp Ile Pro Ser Ser Gln Lys Gln Ser Phe Ser Phe Ser Lys Ser
   1185 1190 1195 1200
   Ser Ser Gly Gln Ser Ser Lys Thr Glu His Met Ser Ser Ser Ser Glu
   1205 1210 1215
   Asn Thr Ser Thr Pro Ser Ser Asn Ala Lys Arg Gln Asn Gln Leu His
   1220 1225 1230
   Pro Ser Ser Ala Gln Ser Arg Ser Gly Gln Pro Gln Lys Ala Ala Thr
   1235 1240 1245
   Cys Lys Val Ser Ser Ile Asn Gln Glu Thr Ile Gln Thr Tyr Cys Val
   1250 1255 1260
   Glu Asp Thr Pro Ile Cys Phe Ser Arg Cys Ser Ser Leu Ser Ser Leu
   1265 1270 1275 1280
   Ser Ser Ala Glu Asp Glu Ile Gly Cys Asn Gln Thr Thr Gln Glu Ala
   1285 1290 1295
   Asp Ser Ala Asn Thr Leu Gln Ile Ala Glu Ile Lys Gly Lys Ile Gly
   1300 1305 1310
   Thr Arg Ser Ala Glu Asp Pro Val Ser Glu Val Pro Ala Val Ser Gln
   1315 1320 1325
   His Pro Arg Thr Lys Ser Ser Arg Leu Gln Gly Ser Ser Leu Ser Ser
   1330 1335 1340
   Glu Ser Ala Arg His Lys Ala Val Glu Phe Pro Ser Gly Ala Lys Ser
   1345 1350 1355 1360
   Pro Ser Lys Ser Gly Ala Gln Thr Pro Lys Ser Pro Pro Glu His Tyr
   1365 1370 1375
   Val Gln Glu Thr Pro Leu Met Phe Ser Arg Cys Thr Ser Val Ser Ser
   1380 1385 1390
   Leu Asp Ser Phe Glu Ser Arg Ser Ile Ala Ser Ser Val Gln Ser Glu
   1395 1400 1405
   Pro Cys Ser Gly Met Val Ser Gly Ile Ile Ser Pro Ser Asp Leu Pro
   1410 1415 1420
   Asp Ser Pro Gly Gln Thr Met Pro Pro Ser Arg Ser Lys Thr Pro Pro
   1425 1430 1435 1440
   Pro Pro Pro Gln Thr Ala Gln Thr Lys Arg Glu Val Pro Lys Asn Lys
   1445 1450 1455
   Ala Pro Thr Ala Glu Lys Arg Glu Ser Gly Pro Lys Gln Ala Ala Val
   1460 1465 1470
   Asn Ala Ala Val Gln Arg Val Gln Val Leu Pro Asp Ala Asp Thr Leu
   1475 1480 1485
   Leu His Phe Ala Thr Glu Ser Thr Pro Asp Gly Phe Ser Cys Ser Ser
   1490 1495 1500
   Ser Leu Ser Ala Leu Ser Leu Asp Glu Pro Phe Ile Gln Lys Asp Val
   1505 1510 1515 1520
   Glu Leu Arg Ile Met Pro Pro Val Gln Glu Asn Asp Asn Gly Asn Glu
   1525 1530 1535
   Thr Glu Ser Glu Gln Pro Lys Glu Ser Asn Glu Asn Gln Glu Lys Glu
   1540 1545 1550
   Ala Glu Lys Thr Ile Asp Ser Glu Lys Asp Leu Leu Asp Asp Ser Asp
   1555 1560 1565
   Asp Asp Asp Ile Glu Ile Leu Glu Glu Cys Ile Ile Ser Ala Met Pro
   1570 1575 1580
   Thr Lys Ser Ser Arg Lys Gly Lys Lys Pro Ala Gln Thr Ala Ser Lys
   1585 1590 1595 1600
   Leu Pro Pro Pro Val Ala Arg Lys Pro Ser Gln Leu Pro Val Tyr Lys
   1605 1610 1615
   Leu Leu Pro Ser Gln Asn Arg Leu Gln Pro Gln Lys His Val Ser Phe
   1620 1625 1630
   Thr Pro Gly Asp Asp Met Pro Arg Val Tyr Cys Val Glu Gly Thr Pro
   1635 1640 1645
   Ile Asn Phe Ser Thr Ala Thr Ser Leu Ser Asp Leu Thr Ile Glu Ser
   1650 1655 1660
   Pro Pro Asn Glu Leu Ala Ala Gly Glu Gly Val Arg Gly Gly Ala Gln
   1665 1670 1675 1680
   Ser Gly Glu Phe Glu Lys Arg Asp Thr Ile Pro Thr Glu Gly Arg Ser
   1685 1690 1695
   Thr Asp Glu Ala Gln Gly Gly Lys Thr Ser Ser Val Thr Ile Pro Glu
   1700 1705 1710
   Leu Asp Asp Asn Lys Ala Glu Glu Gly Asp Ile Leu Ala Glu Cys Ile
   1715 1720 1725
   Asn Ser Ala Met Pro Lys Gly Lys Ser His Lys Pro Phe Arg Val Lys
   1730 1735 1740
   Lys Ile Met Asp Gln Val Gln Gln Ala Ser Ala Ser Ser Ser Ala Pro
   1745 1750 1755 1760
   Asn Lys Asn Gln Leu Asp Gly Lys Lys Lys Lys Pro Thr Ser Pro Val
   1765 1770 1775
   Lys Pro Ile Pro Gln Asn Thr Glu Tyr Arg Thr Arg Val Arg Lys Asn
   1780 1785 1790
   Ala Asp Ser Lys Asn Asn Leu Asn Ala Glu Arg Val Phe Ser Asp Asn
   1795 1800 1805
   Lys Asp Ser Lys Lys Gln Asn Leu Lys Asn Asn Ser Lys Asp Phe Asn
   1810 1815 1820
   Asp Lys Leu Pro Asn Asn Glu Asp Arg Val Arg Gly Ser Phe Ala Phe
   1825 1830 1835 1840
   Asp Ser Pro His His Tyr Thr Pro Ile Glu Gly Thr Pro Tyr Cys Phe
   1845 1850 1855
   Ser Arg Asn Asp Ser Leu Ser Ser Leu Asp Phe Asp Asp Asp Asp Val
   1860 1865 1870
   Asp Leu Ser Arg Glu Lys Ala Glu Leu Arg Lys Ala Lys Glu Asn Lys
   1875 1880 1885
   Glu Ser Glu Ala Lys Val Thr Ser His Thr Glu Leu Thr Ser Asn Gln
   1890 1895 1900
   Gln Ser Ala Asn Lys Thr Gln Ala Ile Ala Lys Gln Pro Ile Asn Arg
   1905 1910 1915 1920
   Gly Gln Pro Lys Pro Ile Leu Gln Lys Gln Ser Thr Phe Pro Gln Ser
   1925 1930 1935
   Ser Lys Asp Ile Pro Asp Arg Gly Ala Ala Thr Asp Glu Lys Leu Gln
   1940 1945 1950
   Asn Phe Ala Ile Glu Asn Thr Pro Val Cys Phe Ser His Asn Ser Ser
   1955 1960 1965
   Leu Ser Ser Leu Ser Asp Ile Asp Gln Glu Asn Asn Asn Lys Glu Asn
   1970 1975 1980
   Glu Pro Ile Lys Glu Thr Glu Pro Pro Asp Ser Gln Gly Glu Pro Ser
   1985 1990 1995 2000
   Lys Pro Gln Ala Ser Gly Tyr Ala Pro Lys Ser Phe His Val Glu Asp
   2005 2010 2015
   Thr Pro Val Cys Phe Ser Arg Asn Ser Ser Leu Ser Ser Leu Ser Ile
   2020 2025 2030
   Asp Ser Glu Asp Asp Leu Leu Gln Glu Cys Ile Ser Ser Ala Met Pro
   2035 2040 2045
   Lys Lys Lys Lys Pro Ser Arg Leu Lys Gly Asp Asn Glu Lys His Ser
   2050 2055 2060
   Pro Arg Asn Met Gly Gly Ile Leu Gly Glu Asp Leu Thr Leu Asp Leu
   2065 2070 2075 2080
   Lys Asp Ile Gln Arg Pro Asp Ser Glu His Gly Leu Ser Pro Asp Ser
   2085 2090 2095
   Glu Asn Phe Asp Trp Lys Ala Ile Gln Glu Gly Ala Asn Ser Ile Val
   2100 2105 2110
   Ser Ser Leu His Gln Ala Ala Ala Ala Ala Cys Leu Ser Arg Gln Ala
   2115 2120 2125
   Ser Ser Asp Ser Asp Ser Ile Leu Ser Leu Lys Ser Gly Ile Ser Leu
   2130 2135 2140
   Gly Ser Pro Phe His Leu Thr Pro Asp Gln Glu Glu Lys Pro Phe Thr
   2145 2150 2155 2160
   Ser Asn Lys Gly Pro Arg Ile Leu Lys Pro Gly Glu Lys Ser Thr Leu
   2165 2170 2175
   Glu Thr Lys Lys Ile Glu Ser Glu Ser Lys Gly Ile Lys Gly Gly Lys
   2180 2185 2190
   Lys Val Tyr Lys Ser Leu Ile Thr Gly Lys Val Arg Ser Asn Ser Glu
   2195 2200 2205
   Ile Ser Gly Gln Met Lys Gln Pro Leu Gln Ala Asn Met Pro Ser Ile
   2210 2215 2220
   Ser Arg Gly Arg Thr Met Ile His Ile Pro Gly Val Arg Asn Ser Ser
   2225 2230 2235 2240
   Ser Ser Thr Ser Pro Val Ser Lys Lys Gly Pro Pro Leu Lys Thr Pro
   2245 2250 2255
   Ala Ser Lys Ser Pro Ser Glu Gly Gln Thr Ala Thr Thr Ser Pro Arg
   2260 2265 2270
   Gly Ala Lys Pro Ser Val Lys Ser Glu Leu Ser Pro Val Ala Arg Gln
   2275 2280 2285
   Thr Ser Gln Ile Gly Gly Ser Ser Lys Ala Pro Ser Arg Ser Gly Ser
   2290 2295 2300
   Arg Asp Ser Thr Pro Ser Arg Pro Ala Gln Gln Pro Leu Ser Arg Pro
   2305 2310 2315 2320
   Ile Gln Ser Pro Gly Arg Asn Ser Ile Ser Pro Gly Arg Asn Gly Ile
   2325 2330 2335
   Ser Pro Pro Asn Lys Leu Ser Gln Leu Pro Arg Thr Ser Ser Pro Ser
   2340 2345 2350
   Thr Ala Ser Thr Lys Ser Ser Gly Ser Gly Lys Met Ser Tyr Thr Ser
   2355 2360 2365
   Pro Gly Arg Gln Met Ser Gln Gln Asn Leu Thr Lys Gln Thr Gly Leu
   2370 2375 2380
   Ser Lys Asn Ala Ser Ser Ile Pro Arg Ser Glu Ser Ala Ser Lys Gly
   2385 2390 2395 2400
   Leu Asn Gln Met Asn Asn Gly Asn Gly Ala Asn Lys Lys Val Glu Leu
   2405 2410 2415
   Ser Arg Met Ser Ser Thr Lys Ser Ser Gly Ser Glu Ser Asp Arg Ser
   2420 2425 2430
   Glu Arg Pro Val Leu Val Arg Gln Ser Thr Phe Ile Lys Glu Ala Pro
   2435 2440 2445
   Ser Pro Thr Leu Arg Arg Lys Leu Glu Glu Ser Ala Ser Phe Glu Ser
   2450 2455 2460
   Leu Ser Pro Ser Ser Arg Pro Ala Ser Pro Thr Arg Ser Gln Ala Gln
   2465 2470 2475 2480
   Thr Pro Val Leu Ser Pro Ser Leu Pro Asp Met Ser Leu Ser Thr His
   2485 2490 2495
   Ser Ser Val Gln Ala Gly Gly Trp Arg Lys Leu Pro Pro Asn Leu Ser
   2500 2505 2510
   Pro Thr Ile Glu Tyr Asn Asp Gly Arg Pro Ala Lys Arg His Asp Ile
   2515 2520 2525
   Ala Arg Ser His Ser Glu Ser Pro Ser Arg Leu Pro Ile Asn Arg Ser
   2530 2535 2540
   Gly Thr Trp Lys Arg Glu His Ser Lys His Ser Ser Ser Leu Pro Arg
   2545 2550 2555 2560
   Val Ser Thr Trp Arg Arg Thr Gly Ser Ser Ser Ser Ile Leu Ser Ala
   2565 2570 2575
   Ser Ser Glu Ser Ser Glu Lys Ala Lys Ser Glu Asp Glu Lys His Val
   2580 2585 2590
   Asn Ser Ile Ser Gly Thr Lys Gln Ser Lys Glu Asn Gln Val Ser Ala
   2595 2600 2605
   Lys Gly Thr Trp Arg Lys Ile Lys Glu Asn Glu Phe Ser Pro Thr Asn
   2610 2615 2620
   Ser Thr Ser Gln Thr Val Ser Ser Gly Ala Thr Asn Gly Ala Glu Ser
   2625 2630 2635 2640
   Lys Thr Leu Ile Tyr Gln Met Ala Pro Ala Val Ser Lys Thr Glu Asp
   2645 2650 2655
   Val Trp Val Arg Ile Glu Asp Cys Pro Ile Asn Asn Pro Arg Ser Gly
   2660 2665 2670
   Arg Ser Pro Thr Gly Asn Thr Pro Pro Val Ile Asp Ser Val Ser Glu
   2675 2680 2685
   Lys Ala Asn Pro Asn Ile Lys Asp Ser Lys Asp Asn Gln Ala Lys Gln
   2690 2695 2700
   Asn Val Gly Asn Gly Ser Val Pro Met Arg Thr Val Gly Leu Glu Asn
   2705 2710 2715 2720
   Arg Leu Thr Ser Phe Ile Gln Val Asp Ala Pro Asp Gln Lys Gly Thr
   2725 2730 2735
   Glu Ile Lys Pro Gly Gln Asn Asn Pro Val Pro Val Ser Glu Thr Asn
   2740 2745 2750
   Glu Ser Pro Ile Val Glu Arg Thr Pro Phe Ser Ser Ser Ser Ser Ser
           2755                    2760                    2765
   Lys His Ser Ser Pro Ser Gly Thr Val Ala Ala Arg Val Thr Pro Phe
       2770                2775                    2780
   Asn Tyr Asn Pro Ser Pro Arg Lys Ser Ser Ala Asp Ser Thr Ser Ala
   2785                    2790                    2795                2800
   Arg Pro Ser Gln Ile Pro Thr Pro Val Asn Asn Asn Thr Lys Lys Arg
                   2805                    2810                    2815
   Asp Ser Lys Thr Asp Ser Thr Glu Ser Ser Gly Thr Gln Ser Pro Lys
               2820                    2825                    2830
   Arg His Ser Gly Ser Tyr Leu Val Thr Ser Val
           2835                    2840
(2) INFORMATION FOR SEQ ID NO:3:
   (i) SEQUENCE CHARACTERISTICS:
      (A) LENGTH: 3172 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Homo sapiens
   (vii) IMMEDIATE SOURCE:
      (B) CLONE: DP1(TB2)
   (ix) FEATURE:
      (A) NAME/KEY: CDS
      (B) LOCATION: 1..630
   (xi) SEQUENCE DESCRIPTION: SEQ ID NO:3:
   GCA GTC GCC GCT CCA GTC TAT CCG GCA CTA GGA ACA GCC CCG GGN GGC      48
   Ala Val Ala Ala Pro Val Tyr Pro Ala Leu Gly Thr Ala Pro Gly Gly
     1               5                  10                  15
   GAG ACG GTC CCC GCC ATG TCT GCG GCC ATG AGG GAG AGG TTC GAC CGG      96
   Glu Thr Val Pro Ala Met Ser Ala Ala Met Arg Glu Arg Phe Asp Arg
               20                  25                  30
   TTC CTG CAC GAG AAG AAC TGC ATG ACT GAC CTT CTG GCC AAG CTC GAG     144
   Phe Leu His Glu Lys Asn Cys Met Thr Asp Leu Leu Ala Lys Leu Glu
           35                  40                  45
   GCC AAA ACC GGC GTG AAC AGG AGC TTC ATC GCT CTT GGT GTC ATC GGA     192
   Ala Lys Thr Gly Val Asn Arg Ser Phe Ile Ala Leu Gly Val Ile Gly
       50                  55                  60
   CTG GTG GCC TTG TAC CTG GTG TTC GGT TAT GGA GCC TCT CTC CTC TGC     240
   Leu Val Ala Leu Tyr Leu Val Phe Gly Tyr Gly Ala Ser Leu Leu Cys
    65                  70                  75                  80
   AAC CTG ATA GGA TTT GGC TAC CCA GCC TAC ATC TCA ATT AAA GCT ATA      288
   Asn Leu Ile Gly Phe Gly Tyr Pro Ala Tyr Ile Ser Ile Lys Ala Ile
                    85                  90                  95
   GAG AGT CCC AAC AAA GAA GAT GAT ACC CAG TGG CTG ACC TAC TGG GTA      336
   Glu Ser Pro Asn Lys Glu Asp Asp Thr Gln Trp Leu Thr Tyr Trp Val
               100                 105                 110
   GTG TAT GGT GTG TTC AGC ATT GCT GAA TTC TTC TCT GAT ATC TTC CTG      384
   Val Tyr Gly Val Phe Ser Ile Ala Glu Phe Phe Ser Asp Ile Phe Leu
           115                 120                 125
   TCA TGG TTC CCC TTC TAC TAC ATG CTG AAG TGT GGC TTC CTG TTG TGG      432
   Ser Trp Phe Pro Phe Tyr Tyr Met Leu Lys Cys Gly Phe Leu Leu Trp
       130                 135                 140
   TGC ATG GCC CCG AGC CCT TCT AAT GGG GCT GAA CTG CTC TAC AAG CGC      480
   Cys Met Ala Pro Ser Pro Ser Asn Gly Ala Glu Leu Leu Tyr Lys Arg
   145                 150                 155                 160
   ATC ATC CGT CCT TTC TTC CTG AAG CAC GAG TCC CAG ATG GAC AGT GTG      528
   Ile Ile Arg Pro Phe Phe Leu Lys His Glu Ser Gln Met Asp Ser Val
                   165                 170                 175
   GTC AAG GAC CTT AAA GAC AAG TCC AAA GAG ACT GCA GAT GCC ATC ACT      576
   Val Lys Asp Leu Lys Asp Lys Ser Lys Glu Thr Ala Asp Ala Ile Thr
               180                 185                 190
   AAA GAA GCG AAG AAA GCT ACC GTG AAT TTA CTG GGT GAA GAA AAG AAG      624
   Lys Glu Ala Lys Lys Ala Thr Val Asn Leu Leu Gly Glu Glu Lys Lys
           195                 200                 205
   AGC ACC TAAACCAGAC TAAACCAGAC TGGATGGAAA CTTCCTGCCC TCTCTGTACC      680
   Ser Thr
       210
   TTCCTACTGG AGCTTGATGT TATATTAGGG ACTGTGGTAT AATTATTTTA ATAATGTTGC    740
   CTTGGAAACA TTTTTGAGAT ATTAAAGATT GGAATGTGTT GTAAGTTTCT TTGCTTACTT    800
   TTACTGTCTA TATATATAGG GAGCACTTTA AACTTAATGC AGTGGGCAGT GTCCACGTTT    860
   TTGGAAAATG TATTTTGCCT CTGGGTAGGA AAAGATGTAT GTTGCTATCC TGCAGGAAAT    920
   ATAAACTTAA AATAAAATTA TATACCCCAC AGGCTGTGTA CTTTACTGGG CTCTCCCTGC    980
   ACGSATTTTC TCTGTAGTTA CATTTAGGRT AATCTTTATG GTTCTACTTC CTRTAATGTA   1040
   CAATTTTATA TAATTCNGRA ATGTTTTTAA TGTATTTGTG CACATGTACA TATGGAAATG   1100
   TTACTGTCTG ACTACANCAT GCATCATGCT CATGGGGAGG GAGCAGGGGA AGGTTGTATG   1160
   TGTCATTTAT AACTTCTGTA CAGTAAGACC ACCTGCCAAA AGCTGGAGGA ACCATTGTGC   1220
   TGGTGTGGTC TACTAAATAA TACTTTAGGA AATACGTGAT TAATATGCAA GTGAACAAAG   1280
   TGAGAAATGA AATCGAATGG AGATTGGCCT GGTTGTTTCC GTAGTATATG GCATATGAAT   1340
   ACCAGGATAG CTTTATAAAG CAGTTAGTTA GTTAGTTACT CACTCTAGTG ATAAATCGGG   1400
   AAATTTACAC ACACACACAC ACACACACAC ACACACACAC ACACACACAC ACACACACAG   1460
   AGTACCCTGT AACTCTCAAT TCCCTGAAAA ACTAGTAATA CTGTCTTATC TGCTATAAAC   1520
   TTTACATATT TGTCTATTGT CAAGATGCTA CANTGGAMNC CATTTCTGGT TTTATCTTCA   1580
   NAGSGGAGAN ACATGTTGAT TTAGTCTTCT TTCCCAATCT TCTTTTTTAA MCCAGTTTNA   1640
   GGMNCTTCTG RAGATTTGYC CACCTCTGAT TACATGTATG TTCTYGTTTG TATCATKAGC   1700
   AACAACATGC TAATGRCGAC ACCTAGCTCT RAGMGCAATT CTGGGAGANT GARAGGNWGT   1760
   ATARAGTMNC CCATAATCTG CTTGGCAATA GTTAAGTCAA TCTATCTTCA GTTTTTCTCT   1820
   GGCCTTTAAG GTCAAACACA AGAGGCTTCC CTAGTTTACA AGTCAGAGTC ACTTGTAGTC   1880
   CATTTAAATG CCCTCATCCG TATTCTTTGT GTTGATAAGC TGCACAKGAC TACATAGTAA   1940
   GTACAGANCA GTAAAGTTAA NNCGGATGTC TCCATTGATC TGCCAANTCG NTATAGAGAG   2000
   CAATTTGTCT GGACTAGAAA ATCTGAGTTT TACACCATAC TGTTAAGAGT CCTTTTGAAT   2060
   TAAACTAGAC TAAAACAAGT GTATAACTAA ACTAACAAGA TTAAATATCC AGCCAGTACA   2120
   GTATTTTTTA AGGCAAATAA AGATGATTAG CTCACCTTGA GNTAACAATC AGGTAAGATC   2180
   ATNACAATGT CTCATGATGT NAANAATATT AAAGATATCA ATACTAAGTG ACAGTATCAC   2240
   NNCTAATATA ATATGGATCA GAGCATTTAT TTTGGGGAGG AAAACAGTGG TGATTACCGG   2300
   CATTTTATTA AACTTAAAAC TTTGTAGAAA GCAAACAAAA TTGTTCTTGG GAGAAAATCA   2360
   ACTTTTAGAT TAAAAAAATT TTAAGTAWCT AGGAGTATTT AAATCCTTTT CCCATAAATA   2420
   AAAGTACAGT TTTCTTGGTG GCAGAATGAA AATCAGCAAC NTCTAGCATA TAGACTATAT   2480
   AATCAGATTG ACAGCATATA GAATATATTA TCAGACAAGA TGAGGAGGTA CAAAAGTTAC   2540
   TATTGCTCAT AATGACTTAC AGGCTAAAAN TAGNTNTAAA ATACTATATT AAATTCTGAA   2600
   TGCAATTTTT TTTTGTTCCC TTGAGACCAA AATTTAAGTT AACTGTTGCT GGCAGTCTAA   2660
   GTGTAAATGT TAACAGCAGG AGAAGTTAAG AATTGAGCAG TTCTGTTGCA TGATTTCCCA   2720
   AATGAAATAC TGCCTTGGCT AGAGTTTGAA AAACTAATTG AGCCTGTGCC TGGCTAGAAA   2780
   ACAAGCGTTT ATTTGAATGT GAATAGTGTT TCAAAGGTAT GTAGTTACAG AATTCCTACC   2840
   AAACAGCTTA AATTCTTCAA GAAAGAATTC CTGCAGCAGT TATTCCCTTA CCTGAAGGCT   2900
   TCAATCATTT GGATCAACAA CTGCTACTCT CGGGAAGACT CCTCTACTCA CAGCTGAAGA   2960
   AAATGAGCAC ACCCTTCACA CTGTTATCAC CTATCCTGAA GATGTGATAC ACTGAATGGA   3020
   AATAAATAGA TGTAAATAAA ATTGAGWTCT CATTTAAAAA AAACCATGTG CCCAATGGGA   3080
   AAATGACCTC ATGTTGTGGT TTAAACAGCA ACTGCACCCA CTAGCACAGC CCATTGAGCT   3140
   ANCCTATATA TACATCTCTG TCAGTGCCCC TC                                 3172
(2) INFORMATION FOR SEQ ID NO:4:
   (i) SEQUENCE CHARACTERISTICS:
      (A) LENGTH: 210 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Ala Val Ala Ala Pro Val Tyr Pro Ala Leu Gly Thr Ala Pro Gly Gly
    1               5                  10                  15
   Glu Thr Val Pro Ala Met Ser Ala Ala Met Arg Glu Arg Phe Asp Arg
               20                  25                  30
   Phe Leu His Glu Lys Asn Cys Met Thr Asp Leu Leu Ala Lys Leu Glu
           35                  40                  45
   Ala Lys Thr Gly Val Asn Arg Ser Phe Ile Ala Leu Gly Val Ile Gly
       50                  55                  60
   Leu Val Ala Leu Tyr Leu Val Phe Gly Tyr Gly Ala Ser Leu Leu Cys
   65                  70                  75                  80
   Asn Leu Ile Gly Phe Gly Tyr Pro Ala Tyr Ile Ser Ile Lys Ala Ile
                   85                  90                  95
   Glu Ser Pro Asn Lys Glu Asp Asp Thr Gln Trp Leu Thr Tyr Trp Val
               100                 105                 110
   Val Tyr Gly Val Phe Ser Ile Ala Glu Phe Phe Ser Asp Ile Phe Leu
           115                 120                 125
   Ser Trp Phe Pro Phe Tyr Tyr Met Leu Lys Cys Gly Phe Leu Leu Trp
       130                 135                 140
   Cys Met Ala Pro Ser Pro Ser Asn Gly Ala Glu Leu Leu Tyr Lys Arg
   145                 150                 155                 160
   Ile Ile Arg Pro Phe Phe Leu Lys His Glu Ser Gln Met Asp Ser Val
                   165                 170                 175
   Val Lys Asp Leu Lys Asp Lys Ser Lys Glu Thr Ala Asp Ala Ile Thr
               180                 185                 190
   Lys Glu Ala Lys Lys Ala Thr Val Asn Leu Leu Gly Glu Glu Lys Lys
           195                 200                 205
   Ser Thr
       210
(2) INFORMATION FOR SEQ ID NO:5:
   (i) SEQUENCE CHARACTERISTICS:
      (A) LENGTH: 434 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGANISM: Homo sapiens
   (vii) IMMEDIATE SOURCE:
      (B) CLONE: TB1
   (xi) SEQUENCE DESCRIPTION: SEQ ID NO:5:
   Val Ala Pro Val Val Val Gly Ser Gly Arg Ala Pro Arg His Pro Ala
   1               5                   10                  15
   Pro Ala Ala Met His Pro Arg Arg Pro Asp Gly Phe Asp Gly Leu Gly
               20                  25                  30
   Tyr Arg Gly Gly Ala Arg Asp Glu Gln Gly Phe Gly Gly Ala Phe Pro
           35                  40                  45
   Ala Arg Ser Phe Ser Thr Gly Ser Asp Leu Gly His Trp Val Thr Thr
       50                  55                  60
   Pro Pro Asp Ile Pro Gly Ser Arg Asn Leu His Trp Gly Glu Lys Ser
   65                  70                  75                  80
   Pro Pro Tyr Gly Val Pro Thr Thr Ser Thr Pro Tyr Glu Gly Pro Thr
                   85                  90                  95
   Glu Glu Pro Phe Ser Ser Gly Gly Gly Gly Ser Val Gln Gly Gln Ser
               100                 105                 110
   Ser Glu Gln Leu Asn Arg Phe Ala Gly Phe Gly Ile Gly Leu Ala Ser
           115                 120                 125
   Leu Phe Thr Glu Asn Val Leu Ala His Pro Cys Ile Val Leu Arg Arg
       130                 135                 140
   Gln Cys Gln Val Asn Tyr His Ala Gln His Tyr His Leu Thr Pro Phe
   145                 150                 155                 160
   Thr Val Ile Asn Ile Met Tyr Ser Phe Asn Lys Thr Gln Gly Pro Arg
                   165                 170                 175
   Ala Leu Trp Lys Gly Met Gly Ser Thr Phe Ile Val Gln Gly Val Thr
               180                 185                 190
   Leu Gly Ala Glu Gly Ile Ile Ser Glu Phe Thr Pro Leu Pro Arg Glu
           195                 200                 205
   Val Leu His Lys Trp Ser Pro Lys Gln Ile Gly Glu His Leu Leu Leu
       210                 215                 220
   Lys Ser Leu Thr Tyr Val Val Ala Met Pro Phe Tyr Ser Ala Ser Leu
   225                 230                 235                 240
   Ile Glu Thr Val Gln Ser Glu Ile Ile Arg Asp Asn Thr Gly Ile Leu
                   245                 250                 255
   Glu Cys Val Lys Glu Gly Ile Gly Arg Val Ile Gly Met Gly Val Pro
               260                 265                 270
   His Ser Lys Arg Leu Leu Pro Leu Leu Ser Leu Ile Phe Pro Thr Val
           275                 280                 285
   Leu His Gly Val Leu His Tyr Ile Ile Ser Ser Val Ile Gln Lys Phe
       290                 295                 300
   Val Leu Leu Ile Leu Lys Arg Lys Thr Tyr Asn Ser His Leu Ala Glu
   305                 310                 315                     320
   Ser Thr Ser Pro Val Gln Ser Met Leu Asp Ala Tyr Phe Pro Glu Leu
                   325                 330                 335
   Ile Ala Asn Phe Ala Ala Ser Leu Cys Ser Asp Val Ile Leu Tyr Pro
               340                 345                 350
   Leu Glu Thr Val Leu His Arg Leu His Ile Gln Gly Thr Arg Thr Ile
           355                 360                 365
   Ile Asp Asn Thr Asp Leu Gly Tyr Glu Val Leu Pro Ile Asn Thr Gln
       370                 375                 380
   Tyr Glu Gly Met Arg Asp Cys Ile Asn Thr Ile Arg Gln Glu Glu Gly
   385                 390                 395                     400
   Val Phe Gly Phe Tyr Lys Gly Phe Gly Ala Val Ile Ile Gln Tyr Thr
                   405                 410                 415
   Leu His Ala Ala Val Leu Gln Ile Thr Lys Ile Ile Tyr Ser Thr Leu
               420                 425                 430
   Leu Gln
(2) INFORMATION FOR SEQ ID NO:6:
   (i) SEQUENCE CHARACTERISTICS:
      (A) LENGTH: 185 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGANISM: Homo sapiens
   (vii) IMMEDIATE SOURCE:
      (B) CLONE: YS-39(TB2)
   (xi) SEQUENCE DESCRIPTION: SEQ ID NO:6:
   Glu Leu Arg Arg Phe Asp Arg Phe Leu His Glu Lys Asn Cys Met Thr
   1               5                   10                  15
   Asp Leu Leu Ala Lys Leu Glu Ala Lys Thr Gly Val Asn Arg Ser Phe
               20                  25                  30
   Ile Ala Leu Gly Val Ile Gly Leu Val Ala Leu Tyr Leu Val Phe Gly
           35                  40                  45
   Tyr Gly Ala Ser Leu Leu Cys Asn Leu Ile Gly Phe Gly Tyr Pro Ala
       50                  55                  60
   Tyr Ile Ser Ile Lys Ala Ile Glu Ser Pro Asn Lys Glu Asp Asp Thr
   65                  70                  75                  80
   Gln Trp Leu Thr Tyr Trp Val Val Tyr Gly Val Phe Ser Ile Ala Glu
                   85                  90                  95
   Phe Phe Ser Asp Ile Phe Leu Ser Trp Phe Pro Phe Tyr Tyr Ile Leu
               100                 105                 110
   Lys Cys Gly Phe Leu Leu Trp Cys Met Ala Pro Ser Pro Ser Asn Gly
           115                 120                 125
   Ala Glu Leu Leu Tyr Lys Arg Ile Ile Arg Pro Phe Phe Leu Lys His
       130                 135                 140
   Glu Ser Gln Met Asp Ser Val Val Lys Asp Leu Lys Asp Lys Ala Lys
   145                 150                 155                 160
   Glu Thr Ala Asp Ala Ile Thr Lys Glu Ala Lys Lys Ala Thr Val Asn
                   165                 170                 175
   Leu Leu Gly Glu Glu Lys Lys Ser Thr
               180                 185
(2) INFORMATION FOR SEQ ID NO:7:
   (i) SEQUENCE CHARACTERISTICS:
      (A) LENGTH: 2842 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGANISM: Homo sapiens
   (vii) IMMEDIATE SOURCE:
      (B) CLONE: APC
   (xi) SEQUENCE DESCRIPTION: SEQ ID NO:7:
(2) INFORMATION FOR SEQ ID NO:8:
   (i) SEQUENCE CHARACTERISTICS:
      (A) LENGTH: 31 amino acids
      (B) TYPE: amino acid
      (C) STRANDEDNESS: single
      (D) TOPOLOGY: linear
   (ii) MOLECULE TYPE: peptide
   (vii) IMMEDIATE SOURCE:
      (B) CLONE: ral2(yeast)
   (xi) SEQUENCE DESCRIPTION: SEQ ID NO:8:
   Leu Thr Gly Ala Lys Gly Leu Gln Leu Arg Ala Leu Arg Arg Ile Ala
   1               5                   10                  15
   Arg Ile Glu Gln Gly Gly Thr Ala Ile Ser Pro Thr Ser Pro Leu
               20                  25                  30
(2) INFORMATION FOR SEQ ID NO:9:
   (i) SEQUENCE CHARACTERISTICS:
      (A) LENGTH: 29 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Homo sapiens
   (vii) IMMEDIATE SOURCE:
      (B) CLONE: m3(mAChR)
   (xi) SEQUENCE DESCRIPTION: SEQ ID NO:9:
   Leu Tyr Trp Arg Ile Tyr Lys Glu Thr Glu Lys Arg Thr Lys Glu Leu
   1               5                   10                  15
   Ala Gly Leu Gln Ala Ser Gly Thr Glu Ala Glu Thr Glu
               20                  25
(2) INFORMATION FOR SEQ ID NO:10:
   (i) SEQUENCE CHARACTERISTICS:
      (A) LENGTH: 29 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Homo sapiens
   (vii) IMMEDIATE SOURCE:
      (B) CLONE: MCC
   (xi) SEQUENCE DESCRIPTION: SEQ ID NO:10:
   Leu Tyr Pro Asn Leu Ala Glu Glu Arg Ser Arg Trp Glu Lys Glu Leu
   1               5                   10                  15
   Ala Gly Leu Arg Glu Glu Asn Glu Ser Leu Thr Ala Met
               20                  25
(2) INFORMATION FOR SEQ ID NO:11:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:11:
   GTATCAAGAC TGTGACTTTT AATTGTAGTT TATCCATTTT                                     40
(2) INFORMATION FOR SEQ ID NO:12:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:12:
   TTTAGAATTT CATGTTAATA TATTGTGTTC TTTTTAACAG 40
(2) INFORMATION FOR SEQ ID NO:13:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:13:
   GTAGATTTTA AAAAGGTGTT TTAAAATAAT TTTTTAAGCT 40
(2) INFORMATION FOR SEQ ID NO:14:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:14:
   AAGCAATTGT TGTATAAAAA CTTGTTTCTA TTTTATTTAG 40
(2) INFORMATION FOR SEQ ID NO:15:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:15:
   GTAACTTTTC TTCATATAGT AAACATTGCC TTGTGTACTC 40
(2) INFORMATION FOR SEQ ID NO:16:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:16:
   NNNNNNNNNN NNNGTCCCTT TTTTTAAAAA AAAAAAATAG 40
(2) INFORMATION FOR SEQ ID NO:17:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:17:
   GTAAGTAACT TGGCAGTACA ACTTATTTGA AACTTTAATA 40
(2) INFORMATION FOR SEQ ID NO:18:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:18:
   ATACAAGATA TTGATACTTT TTTATTATTT GTGGTTTTAG 40
(2) INFORMATION FOR SEQ ID NO:19:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:19:
   GTAAGTTACT TGTTTCTAAG TGATAAAACA GYGAAGAGCT 40
(2) INFORMATION FOR SEQ ID NO:20:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:20:
   AATAAAAACA TAACTAATTA GGTTTCTTGT TTTATTTTAG 40
(2) INFORMATION FOR SEQ ID NO:21:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:21:
   GTTAGTAAAT TSCCTTTTTT GTTTGTGGGT ATAAAAATAG 40
(2) INFORMATION FOR SEQ ID NO:22:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:22:
   ACCATTTTTG CATGTACTGA TGTTAACTCC ATCTTAACAG 40
(2) INFORMATION FOR SEQ ID NO:23:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:23:
   GTAAATAAAT TATTTTATCA TATTTTTTAA AATTATTTAA 40
(2) INFORMATION FOR SEQ ID NO:24:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:24:
   CATGATGTTA TCTGTATTTA CCTATAGTCT AAATTATACC ATCTATAATG TGCTTAATTT    60
   TTAG                                                                  64
(2) INFORMATION FOR SEQ ID NO:25:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:25:
   GTAACAGAAG ATTACAAACC CTGGTCACTA ATGCCATGAC TACTTTGCTA AG            52
(2) INFORMATION FOR SEQ ID NO:26:
   (i) SEQUENCE CHARACTERISTICS:
      (A) LENGTH: 46 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:26:
   GGATATTAAA GTCGTAATTT TGTTTCTAAA CTCATTTGGC CCACAG                   46
(2) INFORMATION FOR SEQ ID NO:27:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:27:
   GTATGTTCTC TATAGTGTAC ATCGTAGTGC ATGTTTCAAA 40
(2) INFORMATION FOR SEQ ID NO:28:
   (i) SEQUENCE CHARACTERISTICS:
      (A) LENGTH: 56 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:28:
   CATCATTGCT CTTCAAATAA CAAAGCATTA TGGTTTATGT TGATTTTATT TTTCAG 56
(2) INFORMATION FOR SEQ ID NO:29:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:29:
   GTAAGACAAA AATGTTTTTT AATGACATAG ACAATTACTG GTG 43
(2) INFORMATION FOR SEQ ID NO:30:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:30:
   TTAGATGATT GTCTTTTTCC TCTTGCCCTT TTTAAATTAG 40
(2) INFORMATION FOR SEQ ID NO:31:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:31:
   GTATGTTTTT ATAACATGTA TTTCTTAAGA TAGCTCAGGT ATGA 44
(2) INFORMATION FOR SEQ ID NO:32:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:32:
   GCTTGGCTTC AAGTTGNCTT TTTAATGATC CTCTATTCTG TATTTAATTT ACAG 54
(2) INFORMATION FOR SEQ ID NO:33:
   (i) SEQUENCE CHARACTERISTICS:
      (A) LENGTH: 65 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:33:
   GTACTATTTA GAATTTCACC TGTTTTTCTT TTTTCTCTTT TTCTTTGAGG CAGGGTCTCA    60
   CTCTG                                                                 65
(2) INFORMATION FOR SEQ ID NO:34:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:34:
   GCAACTAGTA TGATTTTATG TATAAATTAA TCTAAAATTG ATTAATTTCC AG    52
(2) INFORMATION FOR SEQ ID NO:35:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:35:
   GTACCTTTGA AAACATTTAG TACTATAATA TGAATTTCAT GT    42
(2) INFORMATION FOR SEQ ID NO:36:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:36:
   CCAACTCNAA TTAGATGACC CATATTCAGA AACTTACTAG 40
(2) INFORMATION FOR SEQ ID NO:37:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:37:
   GTATATATAG AGTTTTATAT TACTTTTAAA GTACAGAATT CATACTCTCA AAAA 54
(2) INFORMATION FOR SEQ ID NO:38:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:38:
   ATTGTGACCT TAATTTTGTG ATCTCTTGAT TTTTATTTCA G 41
(2) INFORMATION FOR SEQ ID NO:39:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:39:
   TCCCCGCCTG CCGCTCTC 18
(2) INFORMATION FOR SEQ ID NO:40:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:40:
   GCAGCGGCGG CTCCCGTG 18
(2) INFORMATION FOR SEQ ID NO:41:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:41:
   GTGAACGGCT CTCATGCTGC 20
(2) INFORMATION FOR SEQ ID NO:42:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:42:
   ACGTGCGGGG AGGAATGGA 19
(2) INFORMATION FOR SEQ ID NO:43:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:43:
   ATGATATCTT ACCAAATGAT ATAC 24
(2) INFORMATION FOR SEQ ID NO:44:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:44:
   TTATTCCTAC TTCTTCTATA CAG 23
(2) INFORMATION FOR SEQ ID NO:45:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:45:
   TACCCATGCT GGCTCTTTTT C 21
(2) INFORMATION FOR SEQ ID NO:46:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:46:
   TGGGGCCATC TTGTTCCTGA 20
(2) INFORMATION FOR SEQ ID NO:47:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:47:
   ACATTAGGCA CAAAGCTTGC AA 22
(2) INFORMATION FOR SEQ ID NO:48:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:48:
   ATCAAGCTCC AGTAAGAAGG TA 22
(2) INFORMATION FOR SEQ ID NO:49:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:49:
   TGCGGCTCCT GGGTTGTTG 19
(2) INFORMATION FOR SEQ ID NO:50:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:50:
   GCCCCTTCCT TTCTGAGGAC 20
(2) INFORMATION FOR SEQ ID NO:51:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:51:
   TTTTCTCCTG CCTCTTACTG C 21
(2) INFORMATION FOR SEQ ID NO:52:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:52:
   ATGACACCCC CCATTCCCTC 20
(2) INFORMATION FOR SEQ ID NO:53:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:53:
   CCACTTAAAG CACATATATT TAGT 24
(2) INFORMATION FOR SEQ ID NO:54:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:54:
   GTATGGAAAA TAGTGAAGAA CC 22
(2) INFORMATION FOR SEQ ID NO:55:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:55:
   TTCTTAAGTC CTGTTTTTCT TTTG 24
(2) INFORMATION FOR SEQ ID NO:56:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:56:
   TTTAGAACCT TTTTTGTGTT GTG 23
(2) INFORMATION FOR SEQ ID NO:57:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:57:
   CTCAGATTAT ACACTAAGCC TAAC 24
(2) INFORMATION FOR SEQ ID NO:58:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: CDNA
   (vi) ORIGINAL SOURCE:
      (A) ORGANISM: Homo sapiens
   (xi) SEQUENCE DESCRIPTION: SEQ ID NO:58:
   CATGTCTCTT ACAGTAGTAC CA 22
(2) INFORMATION FOR SEQ ID NO:59:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:59:
   AGGTCCAAGG GTAGCCAAGG 20
(2) INFORMATION FOR SEQ ID NO:60:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:60:
   TAAAAATGGA TAAACTACAA TTAAAAG 27
(2) INFORMATION FOR SEQ ID NO:61:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:61:
   AAATACAGAA TCATGTCTTG AAGT 24
(2) INFORMATION FOR SEQ ID NO:62:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:62:
   ACACCTAAAG ATGACAATTT GAG 23
(2) INFORMATION FOR SEQ ID NO:63:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:63:
   TAACTTAGAT AGCAGTAATT TCCC 24
(2) INFORMATION FOR SEQ ID NO:64:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:64:
   ACAATAAACT GGAGTACACA AGG 23
(2) INFORMATION FOR SEQ ID NO:65:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:65:
   ATAGGTCATT GCTTCTTGCT GAT 23
(2) INFORMATION FOR SEQ ID NO:66:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:66:
   TGAATTTTAA TGGATTACCT AGGT 24
(2) INFORMATION FOR SEQ ID NO:67:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:67:
   CTTTTTTTGC TTTTACTGAT TAACG 25
(2) INFORMATION FOR SEQ ID NO:68:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:68:
   TGTAATTCAT TTTATTCCTA ATAGCTC 27
(2) INFORMATION FOR SEQ ID NO:69:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:69:
   GGTAGCCATA GTATGATTAT TTCT 24
(2) INFORMATION FOR SEQ ID NO:70:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:70:
   CTACCTATTT TTATACCCAC AAAC 24
(2) INFORMATION FOR SEQ ID NO:71:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:71:
   AAGAAAGCCT ACACCATTTT TGC 23
(2) INFORMATION FOR SEQ ID NO:72:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:72:
   GATCATTCTT AGAACCATCT TGC 23
(2) INFORMATION FOR SEQ ID NO:73:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:73:
   ACCTATAGTC TAAATTATAC CATC 24
(2) INFORMATION FOR SEQ ID NO:74:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:74:
   GTCATGGCAT TAGTGACCAG 20
(2) INFORMATION FOR SEQ ID NO:75:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:75:
   AGTCGTAATT TTGTTTCTAA ACTC 24
(2) INFORMATION FOR SEQ ID NO:76:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:76:
   TGAAGGACTC GGATTTCACG C 21
(2) INFORMATION FOR SEQ ID NO:77:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:77:
   TCATTCACTC ACAGCCTGAT GAC 23
(2) INFORMATION FOR SEQ ID NO:78:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:78:
   GCTTTGAAAC ATGCACTACG AT 22
(2) INFORMATION FOR SEQ ID NO:79:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:79:
   AAACATCATT GCTCTTCAAA TAAC 24
(2) INFORMATION FOR SEQ ID NO:80:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:80:
   TACCATGATT TAAAAATCCA CCAG 24
(2) INFORMATION FOR SEQ ID NO:81:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:81:
   GATGATTGTC TTTTTCCTCT TGC 23
(2) INFORMATION FOR SEQ ID NO:82:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:82:
   CTGAGCTATC TTAAGAAATA CATG
(2) INFORMATION FOR SEQ ID NO:83:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:83:
   TTTTAAATGA TCCTCTATTC TGTAT
(2) INFORMATION FOR SEQ ID NO:84:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:84:
   ACAGAGTCAG ACCCTGCCTC AAAG
(2) INFORMATION FOR SEQ ID NO:85:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:85:
   TTTCTATTCT TACTGCTAGC ATT
(2) INFORMATION FOR SEQ ID NO:86:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:86:
   ATACACAGGT AAGAAATTAG GA 22
(2) INFORMATION FOR SEQ ID NO:87:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:87:
   TAGATGACCC ATATTCTGTT TC 22
(2) INFORMATION FOR SEQ ID NO:88:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:88:
   CAATTAGGTC TTTTTGAGAG TA 22
(2) INFORMATION FOR SEQ ID NO:89:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEO ID NO:89:
   GTTACTGCAT ACACATTGTG AC 22
(2) INFORMATION FOR SEQ ID NO:90:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:90:
   GCTTTTTGTT TCCTAACATG AAG 23
(2) INFORMATION FOR SEQ ID NO:91:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:91:
   TCTCCCACAG GTAATACTCC C 21
(2) INFORMATION FOR SEQ ID NO:92:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:92:
   GCTAGAACTG AATGGGGTAC G 21
(2) INFORMATION FOR SEQ ID NO:93:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:93:
   CAGGACAAAA TAATCCTGTC CC 22
(2) INFORMATION FOR SEQ ID NO:94:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Homo sapiens
   (xi) SEQUENCE DESCRIPTION: SEQ ID NO:94:

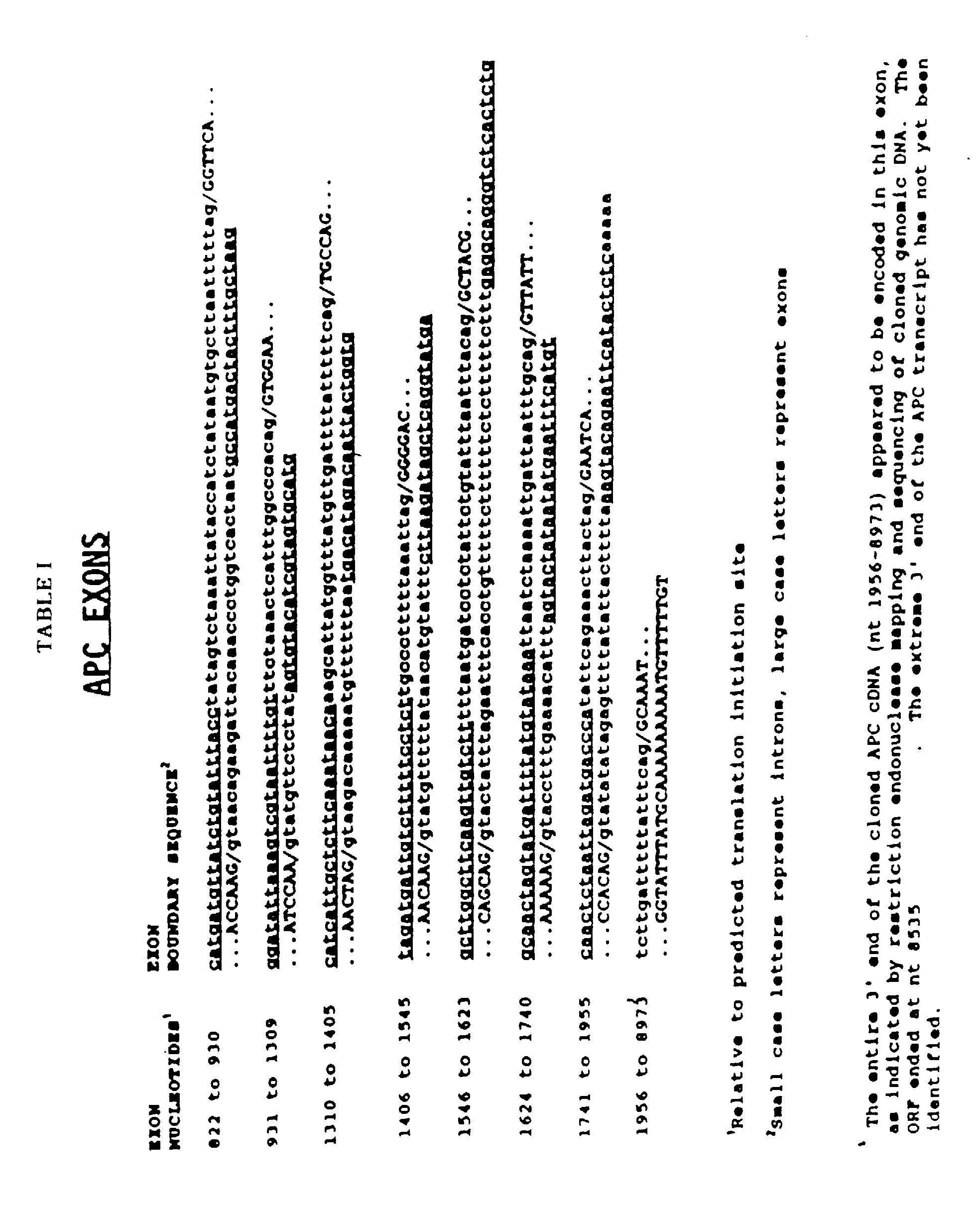

**TABLE IIA**

| Germline mutations of the APC gene in FAP and GS Patients | | | | | |
|---|---|---|---|---|---|
| EXTRA-COLONIC | | NUCLEOTIDE AMINO | | | ACID |
| PATIENT DISEASE | CODON | CHANGE | CHANGE | AGE | |
| 93 | 279 | TCA→TGA | Ser→Stop | 39 | Mandibular |
| | | | | | |
| Osteoma | | | | | |
| | | | | | |
| 24 | 301 | CGA→TGA | Arg→Stop | 46 | None |
| | | | | | |
| 34 | 301 | CGA→TGA | Arg→Stop | 27 | Desmoid |
| | | | | | |
| Tumor | | | | | |
| | | | | | |
| 21 | 413 | CGC→TGC | Arg→Cys | 24 | Mandibular |
| | | | | | |
| Osteoma | | | | | |
| | | | | | |
| 60 | 712 | TCA→TGA | Ser→Stop | 37 | Mandibular |
| | | | | | |
| Osteoma | | | | | |
| | | | | | |
| 3746 | 243 | CAGAG→CAG | splice-junction | | |
| | | | | | |
| 3460 | 301 | CGA→TGA | Arg->Stop | | |
| | | | | | |
| 3827 | 456 | CTTTCA->CTTCA | frameshift | | |
| | | | | | |
| 3712 | 500 | T→G | Tyr→Stop | | |
| * The mutated nucleotides are underlined. | | | | | |

**TABLE IIB**

| Somatic Mutations in Sporadic CRC Patients | | | |
|---|---|---|---|
| PATIENT | CODON¹ | NUCLEOTIDE CHANGE | AMINO ACID CHANGE |
| T35 | MCC 12 | GAG/gtaaga→ GAG/gtaaaa | (Splice Donor) |
| | | | |
| T16 | MCC 145 | ctcag/CGA→ atcag/CGA | (Splice Acceptor) |
| | | | |
| T47 | MCC 267 | CGG→GTG | Arg→Leu |
| | | | |
| T81 | MCC 490 | TCG→TTG | Ser→Leu |
| | | | |
| T35 | MCC 506 | CGG->CAG | Arg→Gln |
| | | | |
| T91 | MCC 698 | GCT->GTT | Ala→Val |
| | | | |
| T34 | APC 288 | CCAGT→CCCAGCCAGT | (Insertion) |
| | | | |
| T27 | APC 331 | CGA→TGA | Arg→Stop |
| | | | |
| T135 | APC 437 | CAA/gtaa→CAA/gcaa | (Splice Donor) |
| | | | |
| T201 | APC 1338 | CAG→TAG | Gln→Stop |

For splice site mutations, the codon nearest to the mutation is listed
The underlined nucleotides were mutant; small case letters represent introns, large case letters represent exons

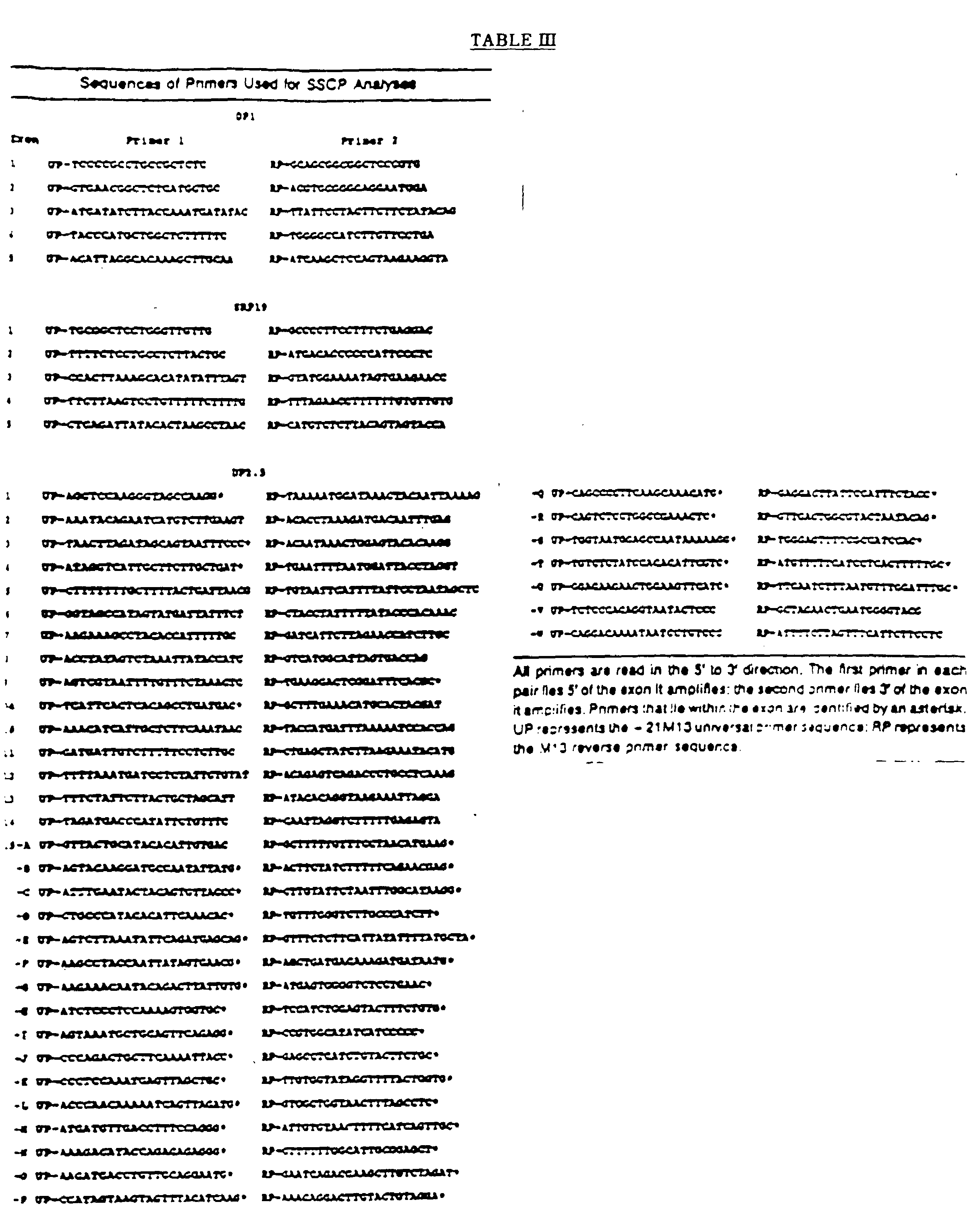

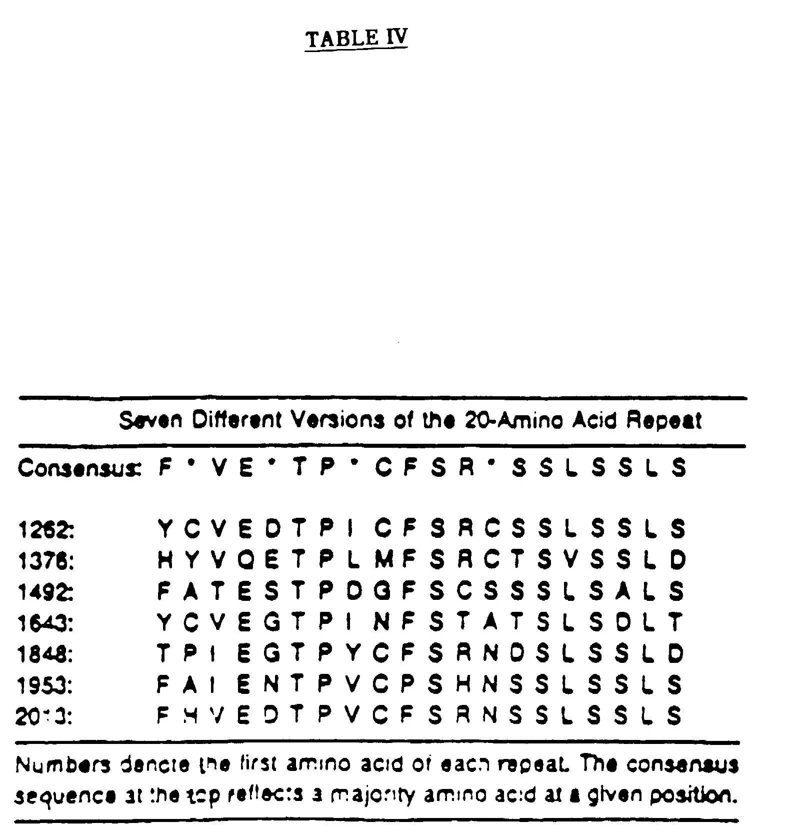

## Claims (Claims for the following Contracting State(s): AT, BE, CH, LI, DE, DK, FR, GB, IT, LU, MC, NL, SE)

1. A method of diagnosing or prognosing a neoplastic tissue of a human, comprising: detecting somatic alteration of wild-type APC gene sequences or their expression products in a tumor tissue isolated from a human, said alteration indicating neoplasia of the tissue; in particular wherein the expression products are mRNA molecules; in particular wherein the expression products are protein molecules.

2. The method of claim 1 wherein the alteration of wild-type APC mRNA is detected by hybridization of mRNA from said tissue to an APC gene probe.

3. The method of claim 1 wherein alteration of wild-type APC gene sequences is detected by hybridization of an APC gene sequence probe to genomic DNA isolated from said tissue; in particular wherein the method further comprises:
- subjecting genomic DNA isolated from a non-neoplastic tissue of the human to Southern hybridization with the APC gene sequence probe; and
- comparing the hybridizations of the APC gene probe to said tumor and non-neoplastic tissues; in particular wherein the APC gene probe detects a restriction fragment length polymorphism.

4. The method of claim 1 wherein the detecting of the alteration of wild-type APC gene sequences is selected from:
(a) observing shifts in electrophoretic mobility of single-stranded DNA on nondenaturing polyacrylamide gels;
(b) determining the sequence of all or part of an APC gene in said tissue, deviations in the APC sequence determined from that of the APC sequence shown in (i) Figure 7 (SEQ ID NO.: 1), or (ii) found in YAC 37HG4, suggesting neoplasia;
(c) identifying a mismatch between molecules (1) an APC gene or APC mRNA isolated from said tissue and (2) a nucleic acid probe complementary to the human wild-type APC gene sequence, when molecules (1) and (2) are hybridized to each other to form a duplex;
(d) amplifying APC gene sequences in said tissue and hybridizing the amplified APC sequences to nucleic acid probes which comprise APC sequences;
(e) molecular cloning of the APC genes in said tissue and sequencing all or part of the cloned APC gene; and
(f) screening for a deletion mutation, for a point mutation, for an inversion mutation or for an insertion mutation.

5. The method of claim 3 wherein the APC gene probe hybridizes to an exon selected from: (1) nucleotides 822 to 930; and (2) nucleotides 931 to 1309; (3) nucleotides 1406 to 1545; and (4) nucleotides 1956 to 2256.

6. The method of any of claims 1 to 5 wherein the neoplastic tissue is a colorectal tissue; in particular wherein the non-neoplastic tissue isolated from a human is from colonic mucosa.

7. The method of claim 1 wherein the detecting of the alteration of wild-type APC protein is selected from immunocytochemistry; immunoblotting; assaying for phospholipid metabolites; and assaying for binding interactions between APC protein of said tumor tissue and a second cellular protein, in particular wherein the second cellular protein is selected from MCC protein, wild-type APC protein, and G protein.

8. A method of supplying in vitro a wild-type APC gene function to a cell which has lost said function by virtue of a mutation in an APC gene, comprising as alternative steps:
- introducing a wild-type APC gene into a cell which has lost said gene function such that said wild-type APC gene is expressed in the cell, in particular wherein the wild-type APC gene introduced recombines with the endogenous mutant APC gene present in the cell by a double recombination event to correct the APC gene mutation;
- introducing a portion of a wild-type APC gene into a cell which has lost said gene function such that said portion is expressed in the cell, said portion encoding a part of the APC protein which is required for non-neoplastic growth of said cell;
- applying of human wild-type APC protein to a cell which has lost wild-type APC function.

9. A method of supplying in vitro wild-type APC gene function to a cell which has altered APC function by virtue of a mutation in an APC gene, comprising
- introducing into the cell a molecule which mimics the function of wild-type APC protein.

10. A pair of single stranded DNA primers for determination of a nucleotide sequence of an APC gene by polymerase chain reaction, the sequence of said primers being derived from chromosome 5q band 21, wherein the use of said primers in a polymerase chain reaction results in synthesis of DNA having all or part of the sequence shown in Figure 7; in particular wherein the primers have restriction enzyme sites at each 5' end; in particular wherein the primers have sequences corresponding to APC introns.

11. A nucleic acid probe complementary to human wild-type APC gene coding sequences; in particular wherein the nucleic acid probe hybridizes to an exon selected from: (1) nucleotides 822 to 930, and (2) nucleotides 931 to 1309; (3) nucleotides 1406 to 1545; (4) nucleotides 1956 to 2256.

12. A kit for detecting alteration of wild-type APC genes comprising a battery of nucleic acid probes which in the aggregate hybridize to all nucleotides of the APC gene coding sequences.

13. A method of detecting the presence of a neoplastic tissue in a human, comprising: detecting in a body sample isolated from a human an alteration of a wild-type APC gene sequence or wild-type APC expression product, said alteration indicating the presence of a neoplastic tissue in the human; in particular wherein said body sample is selected from serum, stool, urine and sputum.

14. A method for detecting genetic predisposition to cancer, including familial adenomatous polyposis (FAP) and Gardner's Syndrome (GS), in a human comprising:
detecting a germline alteration of wild-type APC gene sequences or their expression products in a human sample, in particular blood and fetal tissues, said alteration indicating predisposition to cancer.

15. The method of claim 14 wherein the expression products are mRNA molecules; in particular wherein the alteration of wild-type APC mRNA is detected by hybridization of mRNA from said tissue to an APC gene probe.

16. The method of claim 13 or 14 wherein the detecting of the alteration of wild-type APC gene sequences is selected from:
(a) observing shifts in electrophoretic mobility of single-stranded DNA on nondenaturing polyacrylamide gels;
(b) hybridizing an APC gene sequence probe to genomic DNA isolated from said tissue, in particular wherein the APC gene sequence probe detects a restriction fragment length polymorphism, in particular wherein the APC gene probe hybridizes to an exon selected from: (1) nucleotides 822 to 930; and (2) nucleotides 931 to 1309; (3) nucleotides 1406 to 1545 and (4) nucleotides 1956 to 2256;
(c) determining the sequence of all or part of an APC gene in said tissue, deviations in the APC gene sequence determined from the sequence of Figure 7, or the sequence found in YAC 37HG4, suggesting predisposition to cancer,
(d) identifying a mismatch between molecules (1) an APC gene or APC mRNA isolated from said tissue and (2) a nucleic acid probe complementary to the human wild-type APC gene sequence, when molecules (1) and (2) are hybridized to each other to form a duplex;
(e) amplifying of APC gene sequences in said tissue and hybridization of the amplified APC sequences to nucleic acid probes which comprise APC gene sequences;
(f) molecular cloning of the APC genes in said tissue and sequencing all or part of the cloned APC gene; and
(g) screening for a deletion mutation, for a point mutation, for an inversion mutation or screening for an insertion mutation.

17. The method of claim 14 wherein the expression products are protein molecules, in particular wherein the alteration of wild-type APC protein is detected by immunoblotting; by immunochemistry; or by assaying for binding interactions between APC protein isolated from said tissue and a second cellular protein, in particular wherein the second cellular protein is selected from MCC protein, wild-type APC protein and a G protein.

18. An in vitro method of screening for genetic predisposition to cancer, including familial adenomatous polyposis (FAP) and Gardner's Syndrome (GS), in a human comprising:
detecting among kindred persons the presence of a DNA polymorphism which is linked to a mutant APC allele in an individual having a genetic predisposition to cancer, said kindred being genetically related to the individual, the presence of said polymorphism suggesting a predisposition to cancer.

19. A preparation of the human APC protein substantially free of other human proteins, the amino acid sequence of said protein corresponding to that shown in Figure 3 or 7 (SEQ ID NO: 1).

20. A preparation of antibodies immunoreactive with a human APC protein and not substantially immunoreactive with other human proteins.

21. A method of testing therapeutic agents for the ability to suppress a neoplastically transformed phenotype, comprising:
- applying a test substance to a cultured epithelial cell which carries a mutation in an APC allele;
- determining whether said test substance suppresses the neoplastically transformed phenotype of the cell; in particular wherein the cultured epithelial cell has been genetically engineered to carry the mutation in the APC allele.

22. A method of testing therapeutic agents for the ability to suppress neoplastic growth, comprising:
- administering a test substance to an animal to the exclusion of humans which carries a mutant APC allele in its genome;
- determining whether said test substance prevents or suppresses the growth of tumors.

23. A transgenic animal to the exclusion of humans which carries a mutant APC allele from a second animal species in its genome.

24. An animal to the exclusion of humans which has been genetically engineered to contain an insertion or deletion mutation which disrupts an APC allele in its genome.

25. A cDNA molecule which encodes an APC protein.

26. A cDNA molecule which encodes a protein having the amino acid sequence shown in Figure 3 or 7 (SEQ ID NO: 7 or 1).

27. An isolated DNA molecule which encodes an APC protein.

28. An isolated DNA molecule which encodes a protein having the amino acid sequence shown in Figure 3 or 7 (SEQ ID NO: 7 or 1).

29. A yeast artificial chromosome from YAC clone 37HG4 with the NCIMB accession number 40 353.

30. A wild-type APC gene for use in a method of supplying wild-type APC gene function to a cell which has lost said function by virtue of a mutation in APC gene, wherein said wild-type APC gene after introduction into the cell is expressed in the cell.

31. The wild-type APC gene of claim 30, wherein said wild-type APC gene introduced recombines with the endogenous mutant APC gene present in the cell by a double recombination event to correct the APC gene mutation.

32. A portion of a wild-type APC gene for use in a method of supplying wild-type APC gene function to a cell which has altered APC function by virtue of a mutation in an APC gene, wherein said portion after introduction into the cell is expressed in the cell, said portion encoding a part of the APC protein which is required for non-neoplastic growth of said cell.

33. A human wild-type APC protein for use in a method of supplying wild-type APC gene function to a cell which has altered APC function by virtue of a mutation in an APC gene.

34. A molecule which mimics the function of wild-type APC protein for use in a method of supplying wild-type APC gene function to a cell which has altered APC gene function by virtue of a mutation in an APC gene.

35. The method of any of claims 1, 13, or 14, wherein the alteration is detected using the Amplification Refractory Mutation System (ARMS).

36. The method as claimed in any of claims 1, 2, 3, 4, 13, 14, 15, 16 or 18, wherein the alteration is selected from the group consisting of:
(a) C to T transition at codon 414 resulting in a change from Arg (CGC) to Cys (TGC);
(b) C to T transition at codon 302 resulting in a change from Arg (CGA) to Stop (TGA);
(c) C to G transition at codon 280 resulting in a change from Ser (TCA) to Stop (TGA);
(d) C to G transition at codon 713 resulting in a change from Ser (TCA) to Stop (TGA);
(e) C to T transition at codon 332 resulting in a change from Arg (CGA) to Stop (TGA);
(f) 5 base pair insertion (CAGCC) between codons 289-290, resulting in a Stop at codon 292 due to a frameshift;
(g) C to G transition at codon 1338 resulting in a change from Glu (CAG) to Stop (TAG);
(h) loss of a T around codon 456 causing a frameshift resulting in a downstream Stop;
(i) loss of AG around codon 243 resulting in a splice junction;
(j) T to C transition at codon 437 resulting in a splice donor site (CAA/gtaa → CAA/gcaa).

## Claims (Claims for the following Contracting State(s): ES)

1. A method of diagnosing or prognosing a neoplastic tissue of a human, comprising: detecting somatic alteration of wild-type APC gene sequences or their expression products in a tumor tissue isolated from a human, said alteration indicating neoplasia of the tissue; in particular wherein the expression products are mRNA molecules; in particular wherein the expression products are protein molecules.

2. The method of claim 1 wherein the alteration of wild-type APC mRNA is detected by hybridization of mRNA from said tissue to an APC gene probe.

3. The method of claim 1 wherein alteration of wild-type APC gene sequences is detected by hybridization of an APC gene sequence probe to genomic DNA isolated from said tissue; in particular wherein the method further comprises:
- subjecting genomic DNA isolated from a non-neoplastic tissue of the human to Southern hybridization with the APC gene sequence probe; and
- comparing the hybridizations of the APC gene probe to said tumor and non-neoplastic tissues; in particular wherein the APC gene probe detects a restriction fragment length polymorphism.

4. The method of claim 1 wherein the detecting of the alteration of wild-type APC gene sequences is selected from:
(a) observing shifts in electrophoretic mobility of single-stranded DNA on nondenaturing polyacrylamide gels;
(b) determining the sequence of all or part of an APC gene in said tissue, deviations in the APC sequence determined from that of the APC sequence shown in (i) Figure 7 (SEQ ID NO.: 1), or (ii) found in YAC 37HG4, suggesting neoplasia;
(c) identifying a mismatch between molecules (1) an APC gene or APC mRNA isolated from said tissue and (2) a nucleic acid probe complementary to the human wild-type APC gene sequence, when molecules (1) and (2) are hybridized to each other to form a duplex;
(d) amplifying APC gene sequences in said tissue and hybridizing the amplified APC sequences to nucleic acid probes which comprise APC sequences;
(e) molecular cloning of the APC genes in said tissue and sequencing all or part of the cloned APC gene; and
(f) screening for a deletion mutation, for a point mutation, for an inversion mutation or for an insertion mutation.

5. The method of claim 3 wherein the APC gene probe hybridizes to an exon selected from: (1) nucleotides 822 to 930; and (2) nucleotides 931 to 1309; (3) nucleotides 1406 to 1545; and (4) nucleotides 1956 to 2256.

6. The method of any of claims 1 to 5 wherein the neoplastic tissue is a colorectal tissue; in particular wherein the non-neoplastic tissue isolated from a human is from colonic mucosa.

7. The method of claim 1 wherein the detecting of the alteration of wild-type APC protein is selected from immunocytochemistry; immunoblotting; assaying for phospholipid metabolites; and assaying for binding interactions between APC protein of said tumor tissue and a second cellular protein, in particular wherein the second cellular protein is selected from MCC protein, wild-type APC protein, and G protein.

8. A method of supplying in vitro a wild-type APC gene function to a cell which has lost said function by virtue of a mutation in an APC gene, comprising as alternative steps:
- introducing a wild-type APC gene into a cell which has lost said gene function such that said wild-type APC gene is expressed in the cell, in particular wherein the wild-type APC gene introduced recombines with the endogenous mutant APC gene present in the cell by a double recombination event to correct the APC gene mutation;
- introducing a portion of a wild-type APC gene into a cell which has lost said gene function such that said portion is expressed in the cell, said portion encoding a part of the APC protein which is required for non-neoplastic growth of said cell;
- applying of human wild-type APC protein to a cell which has lost wild-type APC function.

9. A method of supplying in vitro a wild-type APC gene function to a cell which has altered APC gene function by virtue of a mutation in an APC gene, comprising the step of introducing into the cell a molecule which mimics the function of wild-type APC protein.

10. A process which comprises preparing a pair of single stranded DNA primers for determination of a nucleotide sequence of an APC gene by polymerase chain reaction, the sequence of said primers being derived from chromosome 5q band 21, wherein the use of said primers in a polymerase chain reaction results in synthesis of DNA having all or part of the sequence shown in Figure 7; in particular wherein the primers have restriction enzyme sites at each 5' end; in particular wherein the primers have sequences corresponding to APC introns.

11. A process which comprises preparing a nucleic acid probe complementary to human wild-type APC gene coding sequences; in particular wherein the nucleic acid probe hybridizes to an exon selected from: (1) nucleotides 822 to 930; and (2) nucleotides 931 to 1309; (3) nucleotides 1406 to 1545; (4) nucleotides 1956 to 2256.

12. A process which comprises preparing a kit for detecting alteration of wild-type APC genes comprising a battery of nucleic acid probes which in the aggregate hybridize to all nucleotides of the APC gene coding sequences.

13. A method of detecting the presence of a neoplastic tissue in a human, comprising: detecting in a body sample isolated from a human an alteration of a wild-type APC gene sequence or wild-type APC expression product, said alteration indicating the presence of a neoplastic tissue in the human; in particular wherein said body sample is selected from serum, stool, urine and sputum.

14. A method for detecting genetic predisposition to cancer, including familial adenomatous polyposis (FAP) and Gardner's Syndrome (GS), in a human comprising:
detecting a germline alteration of wild-type APC gene sequences or their expression products in a human sample, in particular blood and fetal tissues, said alteration indicating predisposition to cancer.

15. The method of claim 14 wherein the expression products are mRNA molecules; in particular wherein the alteration of wild-type APC mRNA is detected by hybridization of mRNA from said tissue to an APC gene probe.

16. The method of claim 13 or 14 wherein the detecting of the alteration of wild-type APC gene sequences is selected from:
(a) observing shifts in electrophoretic mobility of single-stranded DNA on nondenaturing polyacrylamide gels;
(b) hybridizing an APC gene sequence probe to genomic DNA isolated from said tissue, in particular wherein the APC gene sequence probe detects a restriction fragment length polymorphism, in particular wherein the APC gene probe hybridizes to an exon selected from: (1) nucleotides 822 to 930; and (2) nucleotides 931 to 1309; (3) nucleotides 1406 to 1545 and (4) nucleotides 1956 to 2256;
(c) determining the sequence of all or part of an APC gene in said tissue, deviations in the APC gene sequence determined from the sequence of Figure 7, or the sequence found in YAC 37HG4, suggesting predisposition to cancer;
(d) identifying a mismatch between molecules (1) an APC gene or APC mRNA isolated from said tissue and (2) a nucleic acid probe complementary to the human wild-type APC gene sequence, when molecules (1) and (2) are hybridized to each other to form a duplex;
(e) amplifying of APC gene sequences in said tissue and hybridization of the amplified APC sequences to nucleic acid probes which comprise APC gene sequences;
(f) molecular cloning of the APC genes in said tissue and sequencing all or part of the cloned APC gene; and
(g) screening for a deletion mutation, for a point mutation, for an inversion mutation or screening for an insertion mutation.

17. The method of claim 14 wherein the expression products are protein molecules, in particular wherein the alteration of wild-type APC protein is detected by immunoblotting; by immunochemistry; or by assaying for binding interactions between APC protein isolated from said tissue and a second cellular protein, in particular wherein the second cellular protein is selected from MCC protein, wild-type APC protein and a G protein.

18. An in vitro method of screening for genetic predisposition to cancer, including familial adenomatous polyposis (FAP) and Gardner's Syndrome (GS), in a human comprising:
detecting among kindred persons the presence of a DNA polymorphism which is linked to a mutant APC allele in an individual having a genetic predisposition to cancer, said kindred being genetically related to the individual, the presence of said polymorphism suggesting a predisposition to cancer.

19. A process which comprises preparing a preparation of the human APC protein substantially free of other human proteins, the amino acid sequence of said protein corresponding to that shown in Figure 3 or 7 (SEQ ID NO: 1).

20. A process which comprises preparing a preparation of antibodies immunoreactive with a human APC protein and not substantially immunoreactive with other human proteins.

21. A method of testing therapeutic agents for the ability to suppress a neoplastically transformed phenotype, comprising:
- applying a test substance to a cultured epithelial cell which carries a mutation in an APC allele;
- determining whether said test substance suppresses the neoplastically transformed phenotype of the cell; in particular wherein the cultured epithelial cell has been genetically engineered to carry the mutation in the APC allele.

22. A method of testing therapeutic agents for the ability to suppress neoplastic growth, comprising:
- administering a test substance to an animal to the exclusion of humans which carries a mutant APC allele in its genome;
- determining whether said test substance prevents or suppresses the growth of tumors.

23. A process which comprises preparing a transgenic animal to the exclusion of humans which carries a mutant APC allele from a second animal species in its genome.

24. A process which comprises preparing an animal to the exclusion of humans which has been genetically engineered to contain an insertion or deletion mutation which disrupts an APC allele in its genome.

25. A process which comprises preparing a cDNA molecule which encodes an APC protein.

26. A process which comprises preparing a cDNA molecule which encodes a protein having the amino acid sequence shown in Figure 3 or 7 (SEQ ID NO: 7 or 1).

27. A process which comprises preparing an isolated DNA molecule which encodes an APC protein.

28. A process which comprises preparing an isolated DNA molecule which encodes a protein having the amino acid sequence shown in Figure 3 or 7 (SEQ ID NO: 7 or 1).

29. A process which comprises preparing a yeast artificial chromosome from YAC clone 37HG4 with the NCIMB accession number 40 353.

30. Use of a wild-type APC gene for the manufacture of a medicament for supplying wild-type APC gene function to a cell which has lost said function by virtue of a mutation in APC gene, wherein said wild-type APC gene after introduction into the cell is expressed in the cell.

31. The use of claim 30, wherein said wild-type APC gene introduced recombines with the endogenous mutant APC gene present in the cell by a double recombination event to correct the APC gene mutation.

32. Use of a portion of a wild-type APC gene for the manufacture of a medicament for supplying wild-type APC gene function to a cell which has altered APC function by virtue of a mutation in an APC gene, wherein said portion after introduction into the cell is expressed in the cell, said portion encoding a part of the APC protein which is required for non-neoplastic growth of said cell.

33. Use of a human wild-type APC protein for the manufacture of a medicament for supplying wild-type APC gene function to a cell which has altered APC function by virtue of a mutation in an APC gene.

34. Use of a molecule which mimics the function of wild-type APC protein for the manufacture of a medicament for supplying wild-type APC gene function to a cell which has altered APC gene function by virtue of a mutation in an APC gene.

35. The method of any of claims 1, 13, or 14, wherein the alteration is detected using the Amplification Refractory Mutation System (ARMS).

36. The method as claimed in any of claims 1, 2, 3, 4, 13, 14, 15, 16 or 18, wherein the alteration is selected from the group consisting of:
(a) C to T transition at codon 414 resulting in a change from Arg (CGC) to Cys (TGC);
(b) C to T transition at codon 302 resulting in a change from Arg (CGA) to Stop (TGA);
(c) C to G transition at codon 280 resulting in a change from Ser (TCA) to Stop (TGA);
(d) C to G transition at codon 713 resulting in a change from Ser (TCA) to Stop (TGA);
(e) C to T transition at codon 332 resulting in a change from Arg (CGA) to Stop (TGA);
(f) 5 base pair insertion (CAGCC) between codons 289-290, resulting in a Stop at codon 292 due to a frameshift;
(g) C to G transition at codon 1338 resulting in a change from Glu (CAG) to Stop (TAG);
(h) loss of a T around codon 456 causing a frameshift resulting in a downstream Stop;
(i) loss of AG around codon 243 resulting in a splice junction;
(j) T to C transition at codon 437 resulting in a splice donor site (CAA/gtaa → CAA/gcaa).

## Claims (Claims for the following Contracting State(s): GR)

1. A method of diagnosing or prognosing a neoplastic tissue of a human, comprising: detecting somatic alteration of wild-type APC gene sequences or their expression products in a tumor tissue isolated from a human, said alteration indicating neoplasia of the tissue; in particular wherein the expression products are mRNA molecules; in particular wherein the expression products are protein molecules.

2. The method of claim 1 wherein the alteration of wild-type APC mRNA is detected by hybridization of mRNA from said tissue to an APC gene probe.

3. The method of claim 1 wherein alteration of wild-type APC gene sequences is detected by hybridization of an APC gene sequence probe to genomic DNA isolated from said tissue; in particular wherein the method further comprises:
- subjecting genomic DNA isolated from a non-neoplastic tissue of the human to Southern hybridization with the APC gene sequence probe; and
- comparing the hybridizations of the APC gene probe to said tumor and non-neoplastic tissues; in particular wherein the APC gene probe detects a restriction fragment length polymorphism.

4. The method of claim 1 wherein the detecting of the alteration of wild-type APC gene sequences is selected from:
(a) observing shifts in electrophoretic mobility of single-stranded DNA on nondenaturing polyacrylamide gels;
(b) determining the sequence of all or part of an APC gene in said tissue, deviations in the APC sequence determined from that of the APC sequence shown in (i) Figure 7 (SEQ ID NO.: 1), or (ii) found in YAC 37HG4, suggesting neoplasia;
(c) identifying a mismatch between molecules (1) an APC gene or APC mRNA isolated from said tissue and (2) a nucleic acid probe complementary to the human wild-type APC gene sequence, when molecules (1) and (2) are hybridized to each other to form a duplex;
(d) amplifying APC gene sequences in said tissue and hybridizing the amplified APC sequences to nucleic acid probes which comprise APC sequences;
(e) molecular cloning of the APC genes in said tissue and sequencing all or part of the cloned APC gene; and
(f) screening for a deletion mutation, for a point mutation, for an inversion mutation or for an insertion mutation.

5. The method of claim 3 wherein the APC gene probe hybridizes to an exon selected from: (1) nucleotides 822 to 930; and (2) nucleotides 931 to 1309; (3) nucleotides 1406 to 1545; and (4) nucleotides 1956 to 2256.

6. The method of any of claims 1 to 5 wherein the neoplastic tissue is a colorectal tissue; in particular wherein the non-neoplastic tissue isolated from a human is from colonic mucosa.

7. The method of claim 1 wherein the detecting of the alteration of wild-type APC protein is selected from immunocytochemistry; immunoblotting; assaying for phospholipid metabolites; and assaying for binding interactions between APC protein of said tumor tissue and a second cellular protein, in particular wherein the second cellular protein is selected from MCC protein, wild-type APC protein, and G protein.

8. A method of supplying in vitro a wild-type APC gene function to a cell which has lost said function by virtue of a mutation in an APC gene, comprising as alternative steps:
- introducing a wild-type APC gene into a cell which has lost said gene function such that said wild-type APC gene is expressed in the cell, in particular wherein the wild-type APC gene introduced recombines with the endogenous mutant APC gene present in the cell by a double recombination event to correct the APC gene mutation;
- introducing a portion of a wild-type APC gene into a cell which has lost said gene function such that said portion is expressed in the cell, said portion encoding a part of the APC protein which is required for non-neoplastic growth of said cell;
- applying of human wild-type APC protein to a cell which has lost wild-type APC function.

9. A method of supplying in vitro wild-type APC gene function to a all which has altered APC function by virtue of a mutation in an APC gene, comprising
- introducing into the cell a molecule which mimics the function of wild-type APC protein.

10. A pair of single stranded DNA primers for determination of a nucleotide sequence of an APC gene by polymerase chain reaction, the sequence of said primers being derived from chromosome 5q band 21, wherein the use of said primers in a polymerase chain reaction results in synthesis of DNA having all or part of the sequence shown in Figure 7; in particular wherein the primers have restriction enzyme sites at each 5' end; in particular wherein the primers have sequences corresponding to APC introns.

11. A nucleic acid probe complementary to human wild-type APC gene coding sequences; in particular wherein the nucleic acid probe hybridizes to an exon selected from: (1) nucleotides 822 to 930; and (2) nucleotides 931 to 1309; (3) nucleotides 1406 to 1545; (4) nucleotides 1956 to 2256.

12. A kit for detecting alteration of wild-type APC genes comprising a battery of nucleic acid probes which in the aggregate hybridize to all nucleotides of the APC gene coding sequences.

13. A method of detecting the presence of a neoplastic tissue in a human, comprising: detecting in a body sample isolated from a human an alteration of a wild-type APC gene sequence or wild-type APC expression product, said alteration indicating the presence of a neoplastic tissue in the human; in particular wherein said body sample is selected from serum, stool, urine and sputum.

14. A method for detecting genetic predisposition to cancer, including familial adenomatous polyposis (FAP) and Gardner's Syndrome (GS), in a human comprising:
detecting a germline alteration of wild-type APC gene sequences or their expression products in a human sample, in particular blood and fetal tissues, said alteration indicating predisposition to cancer.

15. The method of claim 14 wherein the expression products are mRNA molecules; in particular wherein the alteration of wild-type APC mRNA is detected by hybridization of mRNA from said tissue to an APC gene probe.

16. The method of claim 13 or 14 wherein the detecting of the alteration of wild-type APC gene sequences is selected from:
(a) observing shifts in electrophoretic mobility of single-stranded DNA on nondenaturing polyacrylamide gels;
(b) hybridizing an APC gene sequence probe to genomic DNA isolated from said tissue, in particular wherein the APC gene sequence probe detects a restriction fragment length polymorphism, in particular wherein the APC gene probe hybridizes to an exon selected from: (1) nucleotides 822 to 930; and (2) nucleotides 931 to 1309; (3) nucleotides 1406 to 1545 and (4) nucleotides 1956 to 2256;
(c) determining the sequence of all or part of an APC gene in said tissue, deviations in the APC gene sequence determined from the sequence of Figure 7, or the sequence found in YAC 37HG4, suggesting predisposition to cancer,
(d) identifying a mismatch between molecules (1) an APC gene or APC mRNA isolated from said tissue and (2) a nucleic acid probe complementary to the human wild-type APC gene sequence, when molecules (1) and (2) are hybridized to each other to form a duplex;
(e) amplifying of APC gene sequences in said tissue and hybridization of the amplified APC sequences to nucleic acid probes which comprise APC gene sequences;
(f) molecular cloning of the APC genes in said tissue and sequencing all or part of the cloned APC gene; and
(g) screening for a deletion mutation, for a point mutation, for an inversion mutation or screening for an insertion mutation.

17. The method of claim 14 wherein the expression products are protein molecules, in particular wherein the alteration of wild-type APC protein is detected by immunoblotting; by immunochemistry; or by assaying for binding interactions between APC protein isolated from said tissue and a second cellular protein, in particular wherein the second cellular protein is selected from MCC protein, wild-type APC protein and a G protein.

18. An in vitro method of screening for genetic predisposition to cancer, including familial adenomatous polyposis (FAP) and Gardner's Syndrome (GS), in a human comprising:
detecting among kindred persons the presence of a DNA polymorphism which is linked to a mutant APC allele in an individual having a genetic predisposition to cancer, said kindred being genetically related to the individual, the presence of said polymorphism suggesting a predisposition to cancer.

19. A preparation of the human APC protein substantially free of other human proteins, the amino acid sequence of said protein corresponding to that shown in Figure 3 or 7 (SEQ ID NO: 1).

20. A preparation of antibodies immunoreactive with a human APC protein and not substantially immunoreactive with other human proteins.

21. A method of testing therapeutic agents for the ability to suppress a neoplastically transformed phenotype, comprising:
- applying a test substance to a cultured epithelial cell which carries a mutation in an APC allele;
- determining whether said test substance suppresses the neoplastically transformed phenotype of the cell; in particular wherein the cultured epithelial cell has been genetically engineered to carry the mutation in the APC allele.

22. A method of testing therapeutic agents for the ability to suppress neoplastic growth, comprising:
- administering a test substance to an animal to the exclusion of humans which carries a mutant APC allele in its genome;
- determining whether said test substance prevents or suppresses the growth of tumors.

23. A transgenic animal to the exclusion of humans which carries a mutant APC allele from a second animal species in its genome.

24. An animal to the exclusion of humans which has been genetically engineered to contain an insertion or deletion mutation which disrupts an APC allele in its genome.

25. A cDNA molecule which encodes an APC protein.

26. A cDNA molecule which encodes a protein having the amino acid sequence shown in Figure 3 or 7 (SEQ ID NO: 7 or 1).

27. An isolated DNA molecule which encodes an APC protein.

28. An isolated DNA molecule which encodes a protein having the amino acid sequence shown in Figure 3 or 7 (SEQ ID NO: 7 or 1).

29. A yeast artificial chromosome from YAC clone 37HG4 with the NCIMB accession number 40 353.

30. Use of a wild-type APC gene for the manufacture of a medicament for supplying wild-type APC gene function to a cell which has lost said function by virtue of a mutation in APC gene, wherein said wild-type APC gene after introduction into the cell is expressed in the cell.

31. The use of claim 30 wherein said wild-type APC gene introduced recombines with the endogenous mutant APC gene present in the cell by a double recombination event to correct the APC gene mutation.

32. Use of a portion of a wild-type APC gene for the manufacture of a medicament for supplying wild-type APC gene function to a cell which has altered APC function by virtue of a mutation in an APC gene, wherein said portion after introduction into the cell is expressed in the cell, said portion encoding a part of the APC protein which is required for non-neoplastic growth of said cell.

33. Use of a human wild-type APC protein for the manufacture of a medicament for supplying wild-type APC gene function to a cell which has altered APC function by virtue of a mutation in an APC gene.

34. Use of a molecule which mimics the function of wild-type APC protein for the manufacture of a medicament for supplying wild-type APC gene function to a cell which has altered APC gene function by virtue of a mutation in an APC gene.

35. The method of any of claims 1, 13, or 14, wherein the alteration is detected using the Amplification Refractory Mutation System (ARMS).

36. The method as claimed in any of claims 1, 2, 3, 4, 13, 14, 15, 16 or 18, wherein the alteration is selected from the group consisting of:
(a) C to T transition at codon 414 resulting in a change from Arg (CGC) to Cys (TGC);
(b) C to T transition at codon 302 resulting in a change from Arg (CGA) to Stop (TGA);
(c) C to G transition at codon 280 resulting in a change from Ser (TCA) to Stop (TGA);
(d) C to G transition at codon 713 resulting in a change from Ser (TCA) to Stop (TGA);
(e) C to T transition at codon 332 resulting in a change from Arg (CGA) to Stop (TGA);
(f) 5 base pair insertion (CAGCC) between codons 289-290, resulting in a Stop at codon 292 due to a frameshift;
(g) C to G transition at codon 1338 resulting in a change from Glu (CAG) to Stop (TAG);
(h) loss of a T around codon 456 causing a frameshift resulting in a downstream Stop;
(i) loss of AG around codon 243 resulting in a splice junction;
(j) T to C transition at codon 437 resulting in a splice donor site (CAA/gtaa → CAA/gcaa).

37. A process which comprises preparing a preparation of the human APC protein substantially free of other human proteins, the amino acid sequence of said protein corresponding to that shown in Figure 3 or 7 (SEQ ID NO.: 7 or 1).

38. A process which comprises preparing a preparation of antibodies immunoreactive with a human APC protein and not substantially immunoreactive with other human proteins.

39. A process which comprises preparing a cDNA molecule which encodes an APC protein or a cDNA molecule which encodes a protein having the amino acid sequence shown in Figure 3 or 7 (SEQ ID NO.: 7 or 1).

40. A process which comprises preparing an isolated DNA molecule which encodes an APC protein or an isolated DNA molecule which encodes a protein having the amino acid sequence shown in Figure 3 or 7 (SEQ ID NO.: 7 or 1).

## Patentansprüche (Patentansprüche für folgende(n) Vertragsstaat(en): AT, BE, CH, LI, DE, DK, FR, GB, IT, LU, MC, NL, SE)

1. Verfahren zur Diagnose oder Prognose eines neoplastischen Gewebes eines Menschen, umfassend:
das Nachweisen einer somatischen Änderung von Wildtyp-APC-Gensequenzen oder deren Expressionsprodukten in einem von einem Menschen isolierten Tumorgewebe, wobei die Änderung eine Neoplasie des Gewebes anzeigt; insbesondere wobei die Expressionsprodukte mRNA-Moleküle sind; insbesondere wobei die Expressionsprodukte Protein-Moleküle sind.

2. Verfahren nach Anspruch 1, wobei die Änderung von Wildtyp-APC-mRNA durch Hybridisierung von mRNA aus dem Gewebe mit einer APC-Gensonde nachgewiesen wird.

3. Verfahren nach Anspruch 1, wobei die Änderung von Wildtyp-APC-Gensequenzen durch Hybridisierung einer APC-Gensequenz-Sonde mit genomischer DNA nachgewiesen wird, die aus dem Gewebe isoliert wurde; insbesondere wobei das Verfahren weiterhin die folgenden Schritte umfaßt:
- die genomische DNA, die aus einem nicht-neoplastischen Gewebe des Menschen isoliert wurde, wird einer Southern-Hybridisierung mit der APC-Gensequenz-Sonde unterworfen; und
- die Hybridisierungen der APC-Gensonde mit dem Tumor- und dem nicht-neoplastischen Gewebe werden verglichen; insbesondere wobei die APC-Gensonde einen Restriktionsfragment-Längenpolymorphismus nachweist.

4. Verfahren nach Anspruch 1, wobei das Nachweisen der Änderung von Wildtyp-APC-Gensequenzen ausgewählt wird aus:
(a) Beobachten von Veränderungen in der elektrophoretischen Mobilität einzelsträngiger DNA auf nicht-denaturierenden Polyacrylamidgelen;
(b) Bestimmen der gesamten oder eines Teils der Sequenz, eines APC-Gens in dem Gewebe, wobei Abweichungen in der APC-Sequenz, ermittelt von der APC-Sequenz, die in (i) Fig. 7 (SEQ ID NO: 1) gezeigt ist, oder (ii) die in YAC 37HG4 gefunden wird, eine Neoplasie nahelegen;
(c) Identifizieren einer Fehlpaarung zwischen Molekülen (1) eines APC-Gens oder einer APC-mRNA, isoliert aus dem Gewebe, und (2) einer Nukleinsäuresonde, die komplementär zu der humanen Wildtyp-APC-Gensequenz ist, wenn die Moleküle (1) und (2) unter Bildung einer Duplex miteinander hybridisiert werden;
(d) Amplifizieren von APC-Gensequenzen in dem Gewebe und Hybridisieren der amplifizierten APC-Sequenzen mit Nukleinsäuresonden, die APC-Sequenzen umfassen;
(e) Molekular-Klonieren der APC-Gene in dem Gewebe und Sequenzieren des gesamten oder eines Teils des klonierten APC-Gens; und
(f) Durchmustern auf eine Deletionsmutation, auf eine Punktmutation, auf eine Inversionsmutation oder auf eine Insertionsmutation.

5. Verfahren nach Anspruch 3, wobei die APC-Gensonde mit einem Exon hybridisiert, das ausgewählt ist aus: (1) Nukleotiden 822 bis 930; und (2) Nukleotiden 931 bis 1309; (3) Nukleotiden 1406 bis 1545; und (4) Nukleotiden 1956 bis 2256.

6. Verfahren nach einem der Ansprüche 1 bis 5, wobei das neoplastische Gewebe ein kolorektales Gewebe ist; insbesondere wobei das aus einem Menschen isolierte nicht-neoplastische Gewebe aus Kolonmucosa ist.

7. Verfahren nach Anspruch 1, wobei das Nachweisen der Änderung des Wildtyp-APC-Proteins ausgewählt wird aus Immuncytochemie; Immunblotting; dem Durchführen eines Assays auf Phospholipidmetabolite; und dem Durchführen eines Assays auf Bindungsinteraktionen zwischen dem APC-Protein des Tumorgewebes und einem zweiten zellulären Protein, insbesondere wobei das zweite zelluläre Protein ausgewählt wird aus einem MCC-Protein, einem Wildtyp-APC-Protein und einem G-Protein.

8. Verfahren zum Ausstatten einer Zelle *in vitro* mit einer Wildtyp-APC-Genfunktion, wobei die Zelle diese Funktion durch eine Mutation in einem APC-Gen verloren hat, wobei das Verfahren die folgenden alternativen Schritte umfaßt:
- Einführen eines Wildtyp-APC-Gens in eine Zelle, die diese Genfunktion verloren hat, derart, daß das Wildtyp-APC-Gen in der Zelle exprimiert wird, insbesondere wobei das eingeführte Wildtyp-APC-Gen mit dem endogenen mutanten APC-Gen, das in der Zelle vorliegt, durch ein doppeltes Rekombinationsereignis unter Korrektur der APC-Genmutation rekombiniert;
- Einführen eines Anteils eines Wildtyp-APC-Gens in eine Zelle, die diese Genfunktion verloren hat, derart, daß dieser Anteil in der Zelle exprimiert wird, wobei dieser Anteil einen Teil des APC-Proteins kodiert, der für nicht-neoplasmatisches Wachstum der Zelle erforderlich ist;
- Anwenden von humanem Wildtyp-APC-Protein auf eine Zelle, die die Wildtyp-APC-Funktion verloren hat.

9. Verfahren zum Ausstatten einer Zelle *in vitro* mit einer Wildtyp-APC-Genfunktion, wobei die Zelle durch eine Mutation in einem APC-Gen eine veränderte APC-Funktion besitzt, wobei das Verfahren den Schritt des Einführens eines Moleküls, das die Funktion des Wildtyp-APC-Proteins nachahmt, in die Zelle umfaßt.

10. Paar einzelsträngiger DNA-Primer zur Bestimmung einer Nukleotidsequenz eines APC-Gens durch Polymerase-Kettenreaktion, wobei die Sequenz der Primer aus Chromosom 5q, Bande 21, abgeleitet ist, wobei die Verwendung der Primer in einer Polymerase-Kettenreaktion in der Synthese von DNA resultiert, die die gesamte oder einen Teil der in Fig. 7 gezeigten Sequenz besitzt; insbesondere wobei die Primer Restriktionsenzym-Schnittstellen an jedem 5'-Ende besitzen; insbesondere wobei die Primer Sequenzen besitzen, die APC-Introns entsprechen.

11. Nukleinsäuresonde, die komplementär zu kodierenden Sequenzen eines humanen Wildtyp-APC-Gens ist; insbesondere wobei die Nukleinsäuresonde mit einem Exon hybridisiert, das ausgewählt ist aus: (1) Nukleotiden 822 bis 930; und (2) Nukleotiden 931 bis 1309; (3) Nukleotiden 1406 bis 1545; und (4) Nukleotiden 1956 bis 2256.

12. Kit zum Nachweis einer Änderung von Wildtyp-APC-Genen, umfassend eine Gruppe von Nukleinsäuresonden, die in dem Aggregat mit allen Nukleotiden der kodierenden Sequenzen des APC-Gens hybridisieren.

13. Verfahren zum Nachweis der Anwensenheit eines neoplastischen Gewebes in einem Menschen, wobei das Verfahren das Nachweisen einer Änderung einer Wildtyp-APC-Gensequenz oder eines Wildtyp-APC-Expressionsprodukts in einer aus einem Menschen isolierten Körperprobe umfaßt, wobei die Änderung die Anwesenheit eines neoplastischen Gewebes in einem Menschen anzeigt; insbesondere wobei die Körperprobe aus Serum, Stuhl, Urin und Sputum ausgewählt ist.

14. Verfahren zum Nachweis einer genetischen Veranlagung für Krebs, einschließlich der familiären adenomatösen Polyposis (familial adenomatous polyposis; FAP) und des Gardner-Syndroms (GS), bei einem Menschen, umfassend das Nachweisen einer Keimbahnänderung von Wildtyp-APC-Gensequenzen oder deren Expressionsprodukten in einer menschlichen Probe, insbesondere Blut oder fötalen Geweben, wobei die Änderung eine Veranlagung für Krebs anzeigt.

15. Verfahren nach Anspruch 14, wobei die Expressionsprodukte mRNA-Moleküle sind; insbesondere wobei die Änderung von Wildtyp-APC-mRNA durch Hybridisierung von mRNA aus dem Gewebe mit einer APC-Gensonde nachgewiesen wird.

16. Verfahren nach Anspruch 13 oder 14, wobei das Nachweisen der Änderung von Wildtyp-APC-Gensequenzen ausgewählt wird aus:
(a) Beobachten von Veränderungen in der elektrophoretischen Mobilität einzelsträngiger DNA auf nicht-denaturierenden Polyacrylamidgelen;
(b) Hybridisieren einer APC-Gensequenz mit aus dem Gewebe isolierter genomischer DNA, insbesondere wobei die APC-Gensequenz-Sonde einen Restriktionsfragment-Längenpolymorphismus nachweist, insbesondere wobei die APC-Gensonde mit einem Exon hybridisiert, das ausgewählt ist aus: (1) Nukleotiden 822 bis 930; und (2) Nukleotiden 931 bis 1309; (3) Nukleotiden 1406 bis 1545; und (4) Nukleotiden 1956 bis 2256;
(c) Bestimmen der Sequenz des gesamten oder eines Teils eines APC-Gens in dem Gewebe, wobei Abweichungen in der APC-Gensequenz, ermittelt von der Sequenz von Fig. 7 oder der in YAC 37HG4 gefundenen Sequenz, eine Veranlagung für Krebs nahelegen;
(d) Identifizieren einer Fehlpaarung zwischen Molekülen (1) eines APC-Gens oder einer APC-mRNA, isoliert aus dem Gewebe, und (2) einer Nukleinsäuresonde, die komplementär zu der humanen Wildtyp-APC-Gensequenz ist, wenn die Moleküle (1) und (2) miteinander unter Bildung einer Duplex hybridisiert werden;
(e) Amplifizieren von APC-Gensequenzen in dem Gewebe und Hybridisierung der amplifizierten APC-Sequenzen mit Nukleinsäuresonden, die APC-Gensequenzen umfassen;
(f) Molekular-Klonieren der APC-Gene in dem Gewebe und Sequenzieren des gesamten oder eines Teils des klonierten APC-Gens; und
(g) Durchmustern auf eine Deletionsmutation, auf eine Punktmutation, auf eine Inversionsmutation oder ein Durchmustern auf eine Insertionsmutation.

17. Verfahren nach Anspruch 14, wobei die Expressionsprodukte Proteinmoleküle sind, insbesondere wobei die Änderung von einem Wildtyp-APC-Protein durch Immunblotting nachgewiesen wird; durch Immunchemie; oder mittels Durchführens eines Assays auf Bindungsinteraktionen zwischen einem aus dem Gewebe isolierten APC-Protein und einem zweiten zellulären Protein, insbesondere wobei das zweite zelluläre Protein ausgewählt ist aus einem MCC-Protein, einem Wildtyp-APC-Protein und einem G-Protein.

18. *In vitro*-Verfahren zum Durchmustern auf eine genetische Veranlagung für Krebs, einschließlich der familiären adenomatösen Polyposis (familial adenomatous polyposis; FAP) und des Gardner-Syndroms (GS), bei einem Menschen, umfassend:
das Nachweisen des Auftretens eines DNA-Polymorphismus unter verwandten Personen, der mit einer mutanten APC-Allele in einem Individuum verknüpft ist, das eine genetische Veranlagung für Krebs besitzt, wobei die Verwandten mit dem Individuum genetisch verwandt sind, und wobei das Auftreten des Polymorphismus eine Veranlagung für Krebs nahelegt.

19. Zubereitung des humanen APC-Proteins, die im wesentlichen frei von anderen humanen Proteinen ist, wobei die Aminosäuresequenz des Proteins der in Fig. 3 oder 7 (SEQ ID NO: 1) gezeigten entspricht.

20. Zubereitung von Antikörpern, die mit einem humanen APC-Protein immunreaktiv sind und nicht im wesentlichen immunreaktiv mit anderen humanen Proteinen sind.

21. Verfahren zum Testen von therapeutischen Mitteln auf ihre Fähigkeit, einen neoplastisch transformierten Phänotyp zu unterdrücken, wobei das Verfahren die Schritte umfaßt:
- Anwenden einer Testsubstanz auf eine in Kultur gehaltene epitheliale Zelle, die eine Mutation in einem APC-Allel trägt;
- Bestimmen, ob die Testsubstanz den neoplastisch transformierten Phänotyp der Zelle unterdrückt, insbesondere wobei die in Kultur gehaltene epitheliale Zelle genetisch verändert worden ist, um die Mutation in dem APC-Allel zu tragen.

22. Verfahren zum Testen von therapeutischen Mitteln auf ihre Fähigkeit, neoplastisches Wachstum zu unterdrücken, wobei das Verfahren die folgenden Schritte umfaßt:
- Verabreichen einer Testsubstanz an ein Tier mit Ausnahme von Menschen, das ein mutantes APC-Allel in seinem Genom trägt;
- Bestimmen, ob die Testsubstanz das Wachstum von Tumoren verhindert oder unterdrückt.

23. Transgenes Tier mit Ausnahme von Menschen, das ein mutantes APC-Allel von einer zweiten Tierspezies in seinem Genom trägt.

24. Tier mit Ausnahme von Menschen, das genetisch verändert wurde, um eine Insertions- oder Deletions-Mutation zu enthalten, die ein APC-Allel in seinem Genom unterbricht.

25. cDNA-Molekül, das ein APC-Protein kodiert.

26. cDNA-Molekül, das ein Protein mit der Aminosäuresequenz kodiert, die in Fig. 3 oder 7 (SEQ ID NO: 7 oder 1) gezeigt ist.

27. Isoliertes DNA-Molekül, das ein APC-Protein kodiert.

28. Isoliertes DNA-Molekül, das ein Protein mit der Aminosäuresequenz kodiert, die in Fig. 3 oder 7 (SEQ ID NO: 7 oder 1) gezeigt ist.

29. Künstliches Hefechromosom aus YAC-Klon 37HG4 mit der NCIMB-Hinterlegungsnummer 40 353.

30. Wildtyp-APC-Gen zur Verwendung in einem Verfahren zum Ausstatten einer Zelle mit einer Wildtyp-APC-Genfunktion, wobei die Zelle diese Funktion durch eine Mutation in einem APC-Gen verloren hat, wobei das Wildtyp-APC-Gen nach Einführung in die Zelle in der Zelle exprimiert wird.

31. Wildtyp-APC-Gen nach Anspruch 30, wobei das eingeführte Wildtyp-APC-Gen mit dem in der Zelle vorkommenden endogenen mutanten APC-Gen durch ein doppeltes Rekombinationsereignis unter Korrektur der APC-Genmutation rekombiniert.

32. Anteil eines Wildtyp-APC-Gens zur Verwendung in einem Verfahren zur Ausstattung einer Zelle, die eine veränderte APC-Funktion mittels einer Mutation in einem APC-Gen besitzt, mit einer Wildtyp-APC-Genfunktion, wobei der Anteil nach Einführung in die Zelle in der Zelle exprimiert wird, und wobei der Anteil einen Teil des APC-Proteins kodiert, der für nicht-neoplastisches Wachstum der Zelle erforderlich ist.

33. Humanes Wildtyp-APC-Protein zur Verwendung in einem Verfahren zur Ausstattung einer Zelle, die eine veränderte APC-Funktion durch eine Mutation in einem APC-Gen besitzt, mit einer Wildtyp-APC-Genfunktion.

34. Molekül, das die Funktion eines Wildtyp-APC-Proteins nachahmt, zur Verwendung in einem Verfahren zur Ausstattung einer Zelle, die eine veränderte APC-Genfunktion durch eine Mutation in einem APC-Gen besitzt, mit einer Wildtyp-APC-Genfunktion.

35. Verfahren nach einem der Ansprüche 1, 13 oder 14, wobei die Änderung nachgewiesen wird unter Verwendung des Amplification Refractory Mutation System (ARMS).

36. Verfahren, wie in einem der Ansprüche 1, 2, 3, 4, 13, 14, 15, 16 oder 18 beansprucht, wobei die Änderung ausgewählt wird aus der Gruppe bestehend aus:
(a) Umwandlung von C zu T an Kodon 414, was zu einem Wechsel von Arg (CGC) zu Cys (TGC) führt;
(b) Umwandlung von C zu T an Kodon 302, was zu einem Wechsel von Arg (CGA) zu Stop (TGA) führt;
(c) Umwandlung von C zu G an Kodon 280, was zu einem Wechsel von Ser (TCA) zu Stop (TGA) führt;
(d) Umwandlung von C zu G an Kodon 713, was zu einem Wechsel von Ser (TCA) zu Stop (TGA) führt;
(e) Umwandlung von C zu T an Kodon 332, was zu einem Wechsel von Arg (CGA) zu Stop (TGA) führt;
(f) Insertion von 5 Basenpaaren (CAGCC) zwischen Kodons 289-290; was zu einem Stop bei Kodon 292 infolge einer Leserahmenverschiebung führt;
(g) Umwandlung von C zu G an Kodon 1338, was zu einem Wechsel von Glu (CAG) zu Stop (TAG) führt;
(h) Verlust eines T um Kodon 456 herum, was eine Leserahmenverschiebung verursacht, die zu einem stromabwärts gelegenen Stop führt;
(i) Verlust von AG um Kodon 243 herum, was zu einer Spleißschnittstelle führt;
(j) Umwandlung von T zu C an Kodon 437, was zu einer Spleiß-Donorstelle (CAA/gtaa → CAA/gcaa) führt.

## Patentansprüche (Patentansprüche für folgende(n) Vertragsstaat(en): ES)

1. Verfahren zur Diagnose oder Prognose eines neoplastischen Gewebes eines Menschen, umfassend:
das Nachweisen einer somatischen Änderung von Wildtyp-APC-Gensequenzen oder deren Expressionsprodukten in einem von einem Menschen isolierten Tumorgewebe, wobei die Änderung eine Neoplasie des Gewebes anzeigt; insbesondere wobei die Expressionsprodukte mRNA-Moleküle sind; insbesondere wobei die Expressionsprodukte Protein-Moleküle sind.

2. Verfahren nach Anspruch 1, wobei die Änderung von Wildtyp-APC-mRNA durch Hybridisierung von mRNA aus dem Gewebe mit einer APC-Gensonde nachgewiesen wird.

3. Verfahren nach Anspruch 1, wobei die Änderung von Wildtyp-APC-Gensequenzen durch Hybridisierung einer APC-Gensequenz-Sonde mit genomischer DNA nachgewiesen wird, die aus dem Gewebe isoliert wurde; insbesondere wobei das Verfahren weiterhin die folgenden Schritte umfaßt:
- die genomische DNA, die aus einem nicht-neoplastischen Gewebe des Menschen isoliert wurde, wird einer Southern-Hybridisierung mit der APC-Gensequenz-Sonde unterworfen; und
- die Hybridisierungen der APC-Gensonde mit dem Tumor- und dem nicht-neoplastischen Gewebe werden verglichen; insbesondere wobei die APC-Gensonde einen Restriktionsfragment-Längenpolymorphismus nachweist.

4. Verfahren nach Anspruch 1, wobei das Nachweisen der Änderung von Wildtyp-APC-Gensequenzen ausgewählt wird aus:
(a) Beobachten von Veränderungen in der elektrophoretischen Mobilität einzelsträngiger DNA auf nicht-denaturierenden Polyacrylamidgelen;
(b) Bestimmen der gesamten oder eines Teils der Sequenz eines APC-Gens in dem Gewebe, wobei Abweichungen in der APC-Sequenz, ermittelt von der APC-Sequenz, die in (i) Fig. 7 (SEQ ID NO: 1) gezeigt ist, oder (ii) die in YAC 37HG4 gefunden wird, eine Neoplasie nahelegen;
(c) Identifizieren einer Fehlpaarung zwischen Molekülen (1) eines APC-Gens oder einer APC-mRNA, isoliert aus dem Gewebe und (2) einer Nukleinsäuresonde, die komplementär zu der humanen Wildtyp-APC-Gensequenz ist, wenn die Moleküle (1) und (2) unter Bildung einer Duplex miteinander hybridisiert werden;
(d) Amplifizieren von APC-Gensequenzen in dem Gewebe und Hybridisieren der amplifizierten APC-Sequenzen mit Nukleinsäuresonden, die APC-Sequenzen umfassen;
(e) Molekular-Klonieren der APC-Gene in dem Gewebe und Sequenzieren des gesamten oder eines Teils des klonierten APC-Gens; und
(f) Durchmustern auf eine Deletionsmutation, auf eine Punktmutation, auf eine Inversionsmutation oder auf eine Insertionsmutation.

5. Verfahren nach Anspruch 3, wobei die APC-Gensonde mit einem Exon hybridisiert, das ausgewählt ist aus: (1) Nukleotiden 822 bis 930; und (2) Nukleotiden 931 bis 1309; (3) Nukleotiden 1406 bis 1545; und (4) Nukleotiden 1956 bis 2256.

6. Verfahren nach einem der Ansprüche 1 bis 5, wobei das neoplastische Gewebe ein kolorektales Gewebe ist; insbesondere wobei das aus einem Menschen isolierte nicht-neoplastische Gewebe aus Kolonmucosa ist.

7. Verfahren nach Anspruch 1, wobei das Nachweisen der Änderung des Wildtyp-APC-Proteins ausgewählt wird aus Immuncytochemie; Immunblotting; dem Durchführen eines Assays auf Phospholipidmetabolite; und dem Durchführen eines Assays auf Bindungsinteraktionen zwischen dem APC-Protein des Tumorgewebes und einem zweiten zellulären Protein, insbesondere wobei das zweite zelluläre Protein ausgewählt wird aus einem MCC-Protein, einem Wildtyp-APC-Protein und einem G-Protein.

8. Verfahren zum Ausstatten einer Zelle in *vitro* mit einer Wildtyp-APC-Genfunktion, wobei die Zelle diese Funktion durch eine Mutation in einem APC-Gen verloren hat, wobei das Verfahren die folgenden alternativen Schritte umfaßt:
- Einführen eines Wildtyp-APC-Gens in eine Zelle, die diese Genfunktion verloren hat, derart, daß das Wildtyp-APC-Gen in der Zelle exprimiert wird, insbesondere wobei das eingeführte Wildtyp-APC-Gen mit dem endogenen mutanten APC-Gen, das in der Zelle vorliegt, durch ein doppeltes Rekombinationsereignis unter Korrektur der APC-Genmutation rekombiniert;
- Einführen eines Anteils eines Wildtyp-APC-Gens in eine Zelle, die diese Genfunktion verloren hat, derart, daß dieser Anteil in der Zelle exprimiert wird, wobei dieser Anteil einen Teil des APC-Proteins kodiert, der für nicht-neoplasmatisches Wachstum der Zelle erforderlich ist;
- Anwenden von humanem Wildtyp-APC-Protein auf eine Zelle, die die Wildtyp-APC-Funktion verloren hat.

9. Verfahren zum Ausstatten einer Zelle *in vitro* mit einer Wildtyp-APC-Genfunktion, wobei die Zelle eine veränderte APC-Funktion besitzt durch eine Mutation in einem APC-Gen, wobei das Verfahren den Schritt des Einführens eines Moleküls, das die Funktion des Wildtyp-APC-Proteins nachahmt, in die Zelle umfaßt.

10. Verfahren, umfassend das Herstellen eines Paares einzelsträngiger DNA-Primer zur Bestimmung einer Nukleotidsequenz eines APC-Gens durch Polymerase-Kettenreaktion, wobei die Sequenz der Primer aus Chromosom 5q, Bande 21, abgeleitet ist, wobei die Verwendung der Primer in einer Polymerase-Kettenreaktion in der Synthese von DNA resultiert, die die gesamte oder einen Teil der in Fig. 7 gezeigten Sequenz besitzt; insbesondere wobei die Primer Restriktionsenzym-Schnittstellen an jedem 5'-Ende besitzen; insbesondere wobei die Primer Sequenzen besitzen, die APC-Introns entsprechen.

11. Verfahren, umfassend das Herstellen einer Nukleinsäuresonde, die komplementär zu kodierenden Sequenzen eines humanen Wildtyp-APC-Gens ist; insbesondere wobei die Nukleinsäuresonde mit einem Exon hybridisiert, das ausgewählt ist aus: (1) Nukleotiden 822 bis 930; und (2) Nukleotiden 931 bis 1309; (3) Nukleotiden 1406 bis 1545; und (4) Nukleotiden 1956 bis 2256.

12. Verfahren, umfassend das Herstellen eines Kits zum Nachweis einer Änderung von Wildtyp-APC-Genen, umfassend eine Gruppe von Nukleinsäuresonden, die in dem Aggregat mit allen Nukleotiden der kodierenden Sequenzen des APC-Gens hybridisieren.

13. Verfahren zum Nachweis der Anwesenheit eines neoplastischen Gewebes bei einem Menschen, wobei das Verfahren das Nachweisen einer Änderung einer Wildtyp-APC-Gensequenz oder eines Wildtyp-APC-Expressionsprodukts in einer aus einem Menschen isolierten Körperprobe umfaßt, wobei die Änderung die Anwesenheit eines neoplastischen Gewebes in einem Menschen anzeigt; insbesondere wobei die Körperprobe aus Serum, Stuhl, Urin und Sputum ausgewählt ist.

14. Verfahren zum Nachweis einer genetischen Veranlagung für Krebs, einschließlich der familiären adenomatösen Polyposis (familial adenomatous polyposis; FAP) und des Gardner-Syndroms (GS), bei einem Menschen, umfassend das Nachweisen einer Keimbahnänderung von Wildtyp-APC-Gensequenzen oder deren Expressionsprodukten in einer menschlichen Probe, insbesondere Blut oder fötalen Geweben, wobei die Änderung eine Veranlagung für Krebs anzeigt.

15. Verfahren nach Anspruch 14, wobei die Expressionsprodukte mRNA-Moleküle sind; insbesondere wobei die Änderung von Wildtyp-APC-mRNA durch Hybridisierung von mRNA aus dem Gewebe mit einer APC-Gensonde nachgewiesen wird.

16. Verfahren nach Anspruch 13 oder 14, wobei das Nachweisen der Änderung von Wildtyp-APC-Gensequenzen ausgewählt wird aus:
(a) Beobachten von Veränderungen in der elektrophoretischen Mobilität einzelsträngiger DNA auf nicht-denaturierenden Polyacrylamidgelen;
(b) Hybridisieren einer APC-Gensequenz mit aus dem Gewebe isolierter genomischer DNA, insbesondere wobei die APC-Gensequenz-Sonde einen Restriktionsfragment-Längenpolymorphismus nachweist, insbesondere wobei die APC-Gensonde mit einem Exon hybridisiert, das ausgewählt ist aus: (1) Nukleotiden 822 bis 930; und (2) Nukleotiden 931 bis 1309; (3) Nukleotiden 1406 bis 1545; und (4) Nukleotiden 1956 bis 2256;
(c) Bestimmen der Sequenz des gesamten oder eines Teils eines APC-Gens in dem Gewebe, wobei Abweichungen in der APC-Gensequenz, ermittelt von der Sequenz von Fig. 7 oder der in YAC 37HG4 gefundenen Sequenz, eine Veranlagung für Krebs nahelegen;
(d) Identifizieren einer Fehlpaarung zwischen Molekülen (1) eines APC-Gens oder einer APC-mRNA, isoliert aus dem Gewebe, und (2) einer Nukleinsäuresonde, die komplementär zu der humanen Wildtyp-APC-Gensequenz ist, wenn die Moleküle (1) und (2) miteinander unter Bildung einer Duplex hybridisiert werden;
(e) Amplifizieren von APC-Gensequenzen in dem Gewebe und Hybridisierung der amplifizierten APC-Sequenzen mit Nukleinsäuresonden, die APC-Gensequenzen umfassen;
(f) Molekular-Klonieren der APC-Gene in dem Gewebe und Sequenzieren des gesamten oder eines Teils des klonierten APC-Gens; und
(g) Durchmustern auf eine Deletionsmutation, auf eine Punktmutation, auf eine Inversionsmutation oder ein Durchmustern auf eine Insertionsmutation.

17. Verfahren nach Anspruch 14, wobei die Expressionsprodukte Proteinmoleküle sind, insbesondere wobei die Änderung von einem Wildtyp-APC-Protein durch Immunblotting nachgewiesen wird; durch Immunchemie; oder mittels Durchführens eines Assays auf Bindungsinteraktionen zwischen einem aus dem Gewebe isolierten APC-Protein und einem zweiten zellulären Protein, insbesondere wobei das zweite zelluläre Protein ausgewählt ist aus einem MCC-Protein, einem Wildtyp-APC-Protein und einem G-Protein.

18. *In vitro*-Verfahren zum Durchmustern auf eine genetische Veranlagung für Krebs, einschließlich der familiären adenomatösen Polyposis (familial adenomatous polyposis; FAP) und des Gardner-Syndroms (GS), bei einem Menschen, umfassend:
das Nachweisen des Auftretens eines DNA-Polymorphismus unter verwandten Personen, der mit einer mutanten APC-Allele in einem Individuum verknüpft ist, das eine genetische Veranlagung für Krebs besitzt, wobei die Verwandten mit dem Individuum genetisch verwandt sind, und wobei das Auftreten des Polymorphismus eine Veranlagung für Krebs nahelegt.

19. Verfahren, umfassend das Herstellen einer Zubereitung des humanen APC-Proteins, die im wesentlichen frei von anderen humanen Proteinen ist, wobei die Aminosäuresequenz des Proteins der in Fig. 3 oder 7 (SEQ ID NO: 1) gezeigten entspricht.

20. Verfahren, umfassend das Herstellen einer Zubereitung von Antikörpern, die mit einem humanen APC-Protein immunreaktiv sind und nicht im wesentlichen immunreaktiv mit anderen humanen Proteinen sind.

21. Verfahren zum Testen von therapeutischen Mitteln auf ihre Fähigkeit, einen neoplastisch transformierten Phänotyp zu unterdrücken, wobei das Verfahren die Schritte umfaßt:
- Anwenden einer Testsubstanz auf eine in Kultur gehaltene epitheliale Zelle, die eine Mutation in einem APC-Allel trägt;
- Bestimmen, ob die Testsubstanz den neoplastisch transformierten Phänotyp der Zelle unterdrückt, insbesondere wobei die in Kultur gehaltene epitheliale Zelle genetisch verändert worden ist, um die Mutation in dem APC-Allel zu tragen.

22. Verfahren zum Testen von therapeutischen Mitteln auf ihre Fähigkeit, neoplastisches Wachstum zu unterdrücken, wobei das Verfahren die folgenden Schritte umfaßt:
- Verabreichen einer Testsubstanz an ein Tier mit Ausnahme von Menschen, das ein mutantes APC-Allel in seinem Genom trägt;
- Bestimmen, ob die Testsubstanz das Wachstum von Tumoren verhindert oder unterdrückt.

23. Verfahren, umfassend das Herstellen eines transgenen Tiers mit Ausnahme von Menschen, das ein mutantes APC-Allel von einer zweiten Tierspezies in seinem Genom trägt.

24. Verfahren, umfassend das Herstellen eines Tiers mit Ausnahme von Menschen, das genetisch verändert wurde, um eine Insertions- oder Deletions-Mutation zu enthalten, die ein APC-Allel in seinem Genom unterbricht.

25. Verfahren, umfassend das Herstellen eines cDNA-Moleküls, das ein APC-Protein kodiert.

26. Verfahren, umfassend das Herstellen eines cDNA-Moleküls, das ein Protein mit der Aminosäuresequenz kodiert, die in Fig. 3 oder 7 (SEQ ID NO: 7 oder 1) gezeigt ist.

27. Verfahren, umfassend das Herstellen eines isolierten DNA-Moleküls, das ein APC-Protein kodiert.

28. Verfahren, umfassend das Herstellen eines isolierten DNA-Moleküls, das ein Protein mit der Aminosäuresequenz kodiert, die in Fig. 3 oder 7 (SEQ ID NO: 7 oder 1) gezeigt ist.

29. Verfahren, umfassend das Herstellen eines Künstlichen Hefechromosoms aus YAC-Klon 37HG4 mit der NCIMB-Hinterlegungsnummer 40 353.

30. Verwendung eines Wildtyp-APC-Gens zur Herstellung eines Arzneimittels zur Ausstattung einer Zelle mit Wildtyp-APC-Genfunktion, wobei die Zelle die Funktion durch eine Mutation in einem APC-Gen verloren hat, und wobei das Wildtyp-APC-Gen nach Einführung in die Zelle in der Zelle exprimiert wird.

31. Verwendung nach Anspruch 30, wobei das eingeführte Wildtyp-APC-Gen mit einem in der Zelle vorkommenden endogenen mutanten APC-Gen durch ein doppeltes Rekombinationsereignis unter Korrektur der APC-Genmutation rekombiniert.

32. Verwendung eines Anteils eines Wildtyp-APC-Gens zur Herstellung eines Arzneimittels zur Ausstattung einer Zelle, die eine veränderte APC-Funktion durch eine Mutation in einem APC-Gen besitzt, mit einer Wildtyp-APC-Genfunktion, wobei der Anteil nach Einführung in die Zelle in der Zelle exprimiert wird, und wobei der Anteil einen Teil des APC-Proteins kodiert, der für nicht-neoplastisches Wachstum der Zelle erforderlich ist.

33. Verwendung eines humanen Wildtyp-APC-Proteins zur Herstellung eines Arzneimittels zur Ausstattung einer Zelle, die eine veränderte APC-Funktion durch eine Mutation in einem APC-Gen besitzt, mit einer Wildtyp-APC-Genfunktion.

34. Verwendung eines Moleküls, das die Funktion eines Wildtyp-APC-Proteins nachahmt, zur Herstellung eines Arzneimittels zur Ausstattung einer Zelle, die eine veränderte APC-Genfunktion durch eine Mutation in einem APC-Gen besitzt, mit einer Wildtyp-APC-Genfunktion.

35. Verfahren nach einem der Ansprüche 1, 13 oder 14, wobei die Änderung nachgewiesen wird unter Verwendung des Amplification Refractory Mutation System (ARMS).

36. Verfahren, wie in einem der Ansprüche 1, 2, 3, 4, 13, 14, 15, 16 oder 18 beansprucht, wobei die Änderung ausgewählt wird aus der Gruppe bestehend aus:
(a) Umwandlung von C zu T an Kodon 414, was zu einem Wechsel von Arg (CGC) zu Cys (TGC) führt;
(b) Umwandlung von C zu T an Kodon 302, was zu einem Wechsel von Arg (CGA) zu Stop (TGA) führt;
(c) Umwandlung von C zu G an Kodon 280, was zu einem Wechsel von Ser (TCA) zu Stop (TGA) führt;
(d) Umwandlung von C zu G an Kodon 713, was zu einem Wechsel von Ser (TCA) zu Stop (TGA) führt;
(e) Umwandlung von C zu T an Kodon 332, was zu einem Wechsel von Arg (CGA) zu Stop (TGA) führt;
(f) Insertion von 5 Basenpaaren (CAGCC) zwischen Kodons 289-290; was zu einem Stop bei Kodon 292 infolge einer Leserahmenverschiebung führt;
(g) Umwandlung von C zu G an Kodon 1338, was zu einem Wechsel von Glu (CAG) zu Stop (TAG) führt;
(h) Verlust eines T um Kodon 456 herum, was eine Leserahmenverschiebung verursacht, die zu einem stromabwärts gelegenen Stop führt;
(i) Verlust von AG um Kodon 243 herum, was zu einer Spleißschnittstelle führt;
(j) Umwandlung von T zu C an Kodon 437, was zu einer Spleiß-Donorstelle (CAA/gtaa → CAA/gcaa) führt.

## Patentansprüche (Patentansprüche für folgende(n) Vertragsstaat(en): GR)

1. Verfahren zur Diagnose oder Prognose eines neoplastischen Gewebes eines Menschen, umfassend:
das Nachweisen einer somatischen Änderung von Wildtyp-APC-Gensequenzen oder deren Expressionsprodukten in einem von einem Menschen isolierten Tumorgewebe, wobei die Änderung eine Neoplasie des Gewebes anzeigt; insbesondere wobei die Expressionsprodukte mRNA-Moleküle sind; insbesondere wobei die Expressionsprodukte Protein-Moleküle sind.

2. Verfahren nach Anspruch 1, wobei die Änderung von Wildtyp-APC-mRNA durch Hybridisierung von mRNA aus dem Gewebe mit einer APC-Gensonde nachgewiesen wird.

3. Verfahren nach Anspruch 1, wobei die Änderung von Wildtyp-APC-Gensequenzen durch Hybridisierung einer APC-Gensequenz-Sonde mit genomischer DNA nachgewiesen wird, die aus dem Gewebe isoliert wurde; insbesondere wobei das Verfahren weiterhin die folgenden Schritte umfaßt:
- die genomische DNA, die aus einem nicht-neoplastischen Gewebe des Menschen isoliert wurde, wird einer Southern-Hybridisierung mit der APC-Gensequenz-Sonde unterworfen; und
- die Hybridisierungen der APC-Gensonde mit dem Tumor- und dem nicht-neoplastischen Gewebe werden verglichen; insbesondere wobei die APC-Gensonde einen Restriktionsfragment-Längenpolymorphismus nachweist.

4. Verfahren nach Anspruch 1, wobei das Nachweisen der Änderung von Wildtyp-APC-Gensequenzen ausgewählt wird aus:
(a) Beobachten von Veränderungen in der elektrophoretischen Mobilität einzelsträngiger DNA auf nicht-denaturierenden Polyacrylamidgelen;
(b) Bestimmen der gesamten oder eines Teils der Sequenz eines APC-Gens in dem Gewebe, wobei Abweichungen in der APC-Sequenz, ermittelt von der APC-Sequenz, die in (i) Fig. 7 (SEQ ID NO: 1) gezeigt ist, oder (ii) die in YAC 37HG4 gefunden wird, eine Neoplasie nahelegen;
(c) Identifizieren einer Fehlpaarung zwischen Molekülen (1) eines APC-Gens oder einer APC-mRNA, isoliert aus dem Gewebe, und (2) einer Nukleinsäuresonde, die komplementär zu der humanen Wildtyp-APC-Gensequenz ist, wenn die Moleküle (1) und (2) unter Bildung einer Duplex miteinander hybridisiert werden;
(d) Amplifizieren von APC-Gensequenzen in dem Gewebe und Hybridisieren der amplifizierten APC-Sequenzen mit Nukleinsäuresonden, die APC-Sequenzen umfassen;
(e) Molekular-Klonieren der APC-Gene in dem Gewebe und Sequenzieren des gesamten oder eines Teils des klonierten APC-Gens; und
(f) Durchmustern auf eine Deletionsmutation, auf eine Punktmutation, auf eine Inversionsmutation oder auf eine Insertionsmutation.

5. Verfahren nach Anspruch 3, wobei die APC-Gensonde mit einem Exon hybridisiert, das ausgewählt ist aus: (1) Nukleotiden 822 bis 930; und (2) Nukleotiden 931 bis 1309; (3) Nukleotiden 1406 bis 1545; und (4) Nukleotiden 1956 bis 2256.

6. Verfahren nach einem der Ansprüche 1 bis 5, wobei das neoplastische Gewebe ein kolorektales Gewebe ist; insbesondere wobei das aus einem Menschen isolierte nicht-neoplastische Gewebe aus Kolonmucosa ist.

7. Verfahren nach Anspruch 1, wobei das Nachweisen der Änderung des Wildtyp-APC-Proteins ausgewählt wird aus Immuncytochemie; Immunblotting; dem Durchführen eines Assays auf Phospholipidmetabolite; und dem Durchführen eines Assays auf Bindungsinteraktionen zwischen dem APC-Protein des Tumorgewebes und einem zweiten zellulären Protein, insbesondere wobei das zweite zelluläre Protein ausgewählt wird aus einem MCC-Protein, einem Wildtyp-APC-Protein und einem G-Protein.

8. Verfahren zum Ausstatten einer Zelle *in vitro* mit einer Wildtyp-APC-Genfunktion, wobei die Zelle diese Funktion durch eine Mutation in einem APC-Gen verloren hat, wobei das Verfahren die folgenden alternativen Schritte umfaßt:
- Einführen eines Wildtyp-APC-Gens in eine Zelle, die diese Genfunktion verloren hat, derart, daß das Wildtyp-APC-Gen in der Zelle exprimiert wird, insbesondere wobei das eingeführte Wildtyp-APC-Gen mit dem endogenen mutanten APC-Gen, das in der Zelle vorliegt, durch ein doppeltes Rekombinationsereignis unter Korrektur der APC-Genmutation rekombiniert;
- Einführen eines Anteils eines Wildtyp-APC-Gens in eine Zelle, die diese Genfunktion verloren hat, derart, daß dieser Anteil in der Zelle exprimiert wird, wobei dieser Anteil einen Teil des APC-Proteins kodiert, der für nicht-neoplasmatisches Wachstum der Zelle erforderlich ist;
- Anwenden von humanem Wildtyp-APC-Protein auf eine Zelle, die die Wildtyp-APC-Funktion verloren hat.

9. Verfahren zum Ausstatten einer Zelle *in vitro* mit einer Wildtyp-APC-Genfunktion, wobei die Zelle eine veränderte APC-Funktion besitzt durch eine Mutation in einem APC-Gen, wobei das Verfahren den Schritt des Einführens eines Moleküls, das die Funktion des Wildtyp-APC-Proteins nachahmt, in die Zelle umfaßt.

10. Paar einzelsträngiger DNA-Primer zur Bestimmung einer Nukleotidsequenz eines APC-Gens durch Polymerase-Kettenreaktion, wobei die Sequenz der Primer aus Chromosom 5q, Bande 21, abgeleitet ist, wobei die Verwendung der Primer in einer Polymerase-Kettenreaktion in der Synthese von DNA resultiert, die die gesamte oder einen Teil der in Fig. 7 gezeigten Sequenz besitzt; insbesondere wobei die Primer Restriktionsenzym-Schnittstellen an jedem 5'-Ende besitzen; insbesondere wobei die Primer Sequenzen besitzen, die APC-Introns entsprechen.

11. Nukleinsäuresonde, die komplementär zu kodierenden Sequenzen eines humanen Wildtyp-APC-Gens ist; insbesondere wobei die Nukleinsäuresonde mit einem Exon hybridisiert, das ausgewählt ist aus: (1) Nukleotiden 822 bis 930; und (2) Nukleotiden 931 bis 1309; (3) Nukleotiden 1406 bis 1545; und (4) Nukleotiden 1956 bis 2256.

12. Kit zum Nachweis einer Änderung von Wildtyp-APC-Genen, umfassend eine Gruppe von Nukleinsäuresonden, die in dem Aggregat mit allen Nukleotiden der kodierenden Sequenzen des APC-Gens hybridisieren.

13. Verfahren zum Nachweis der Anwesenheit eines neoplastischen Gewebes bei einem Menschen, wobei das Verfahren das Nachweisen einer Änderung einer Wildtyp-APC-Gensequenz oder eines Wildtyp-APC-Expressionsprodukts in einer aus einem Menschen isolierten Körperprobe umfaßt, wobei die Änderung die Anwesenheit eines neoplastischen Gewebes in einem Menschen anzeigt; insbesondere wobei die Körperprobe aus Serum, Stuhl, Urin und Sputum ausgewählt ist.

14. Verfahren zum Nachweis einer genetischen Veranlagung für Krebs, einschließlich der familiären adenomatösen Polyposis (familial adenomatous polyposis; FAP) und des Gardner-Syndroms (GS), bei einem Menschen, umfassend das Nachweisen einer Keimbahnänderung von Wildtyp-APC-Gensequenzen oder deren Expressionsprodukten in einer menschlichen Probe, insbesondere Blut oder fötalen Geweben, wobei die Änderung eine Veranlagung für Krebs anzeigt.

15. Verfahren nach Anspruch 14, wobei die Expressionsprodukte mRNA-Moleküle sind; insbesondere wobei die Änderung von Wildtyp-APC-mRNA durch Hybridisierung von mRNA aus dem Gewebe mit einer APC-Gensonde nachgewiesen wird.

16. Verfahren nach Anspruch 13 oder 14, wobei das Nachweisen der Änderung von Wildtyp-APC-Gensequenzen ausgewählt wird aus:
(a) Beobachten von Veränderungen in der elektrophoretischen Mobilität einzelsträngiger DNA auf nicht-denaturierenden Polyacrylamidgelen;
(b) Hybridisieren einer APC-Gensequenz mit aus dem Gewebe isolierter genomischer DNA, insbesondere wobei die APC-Gensequenz-Sonde einen Restriktionsfragment-Längenpolymorphismus nachweist, insbesondere wobei die APC-Gensonde mit einem Exon hybridisiert, das ausgewählt ist aus: (1) Nukleotiden 822 bis 930; und (2) Nukleotiden 931 bis 1309; (3) Nukleotiden 1406 bis 1545; und (4) Nukleotiden 1956 bis 2256;
(c) Bestimmen der Sequenz des gesamten oder eines Teils eines APC-Gens in dem Gewebe, wobei Abweichungen in der APC-Gensequenz, ermittelt von der Sequenz von Fig. 7 oder der in YAC 37HG4 gefundenen Sequenz, eine Veranlagung für Krebs nahelegen;
(d) Identifizieren einer Fehlpaarung zwischen Molekülen (1) eines APC-Gens oder einer APC-mRNA, isoliert aus dem Gewebe, und (2) einer Nukleinsäuresonde, die komplementär zu der humanen Wildtyp-APC-Gensequenz ist, wenn die Moleküle (1) und (2) miteinander unter Bildung einer Duplex hybridisiert werden;
(e) Amplifizieren von APC-Gensequenzen in dem Gewebe und Hybridisierung der amplifizierten APC-Sequenzen mit Nukleinsäuresonden, die APC-Gensequenzen umfassen;
(f) Molekular-Klonieren der APC-Gene in dem Gewebe und Sequenzieren des gesamten oder eines Teils des klonierten APC-Gens; und
(g) Durchmustern auf eine Deletionsmutation, auf eine Punktmutation, auf eine Inversionsmutation oder ein Durchmustern auf eine Insertionsmutation.

17. Verfahren nach Anspruch 14, wobei die Expressionsprodukte Proteinmoleküle sind, insbesondere wobei die Änderung von einem Wildtyp-APC-Protein durch Immunblotting nachgewiesen wird; durch Immunchemie; oder mittels Durchführens eines Assays auf Bindungsinteraktionen zwischen einem aus dem Gewebe isolierten APC-Protein und einem zweiten zellulären Protein, insbesondere wobei das zweite zelluläre Protein ausgewählt ist aus einem MCC-Protein, einem Wildtyp-APC-Protein und einem G-Protein.

18. *In vitro*-Verfahren zum Durchmustern auf eine genetische Veranlagung für Krebs, einschließlich der familiären adenomatösen Polyposis (familial adenomatous polyposis; FAP) und des Gardner-Syndroms (GS), bei einem Menschen, umfassend:
das Nachweisen des Auftretens eines DNA-Polymorphismus unter verwandten Personen, der mit einer mutanten APC-Allele in einem Individuum verknüpft ist, das eine genetische Veranlagung für Krebs besitzt, wobei die Verwandten mit dem Individuum genetisch verwandt sind, und wobei das Auftreten des Polymorphismus eine Veranlagung für Krebs nahelegt.

19. Zubereitung des humanen APC-Proteins, die im wesentlichen frei von anderen humanen Proteinen ist, wobei die Aminosäuresequenz des Proteins der in Fig. 3 oder 7 (SEQ ID NO: 1) gezeigten entspricht.

20. Zubereitung von Antikörpern, die mit einem humanen APC-Protein immunreaktiv sind und nicht im wesentlichen immunreaktiv mit anderen humanen Proteinen sind.

21. Verfahren zum Testen von therapeutischen Mitteln auf ihre Fähigkeit, einen neoplastisch transformierten Phänotyp zu unterdrücken, wobei das Verfahren die Schritte umfaßt:
- Anwenden einer Testsubstanz auf eine in Kultur gehaltene epitheliale Zelle, die eine Mutation in einem APC-Allel trägt;
- Bestimmen, ob die Testsubstanz den neoplastisch transformierten Phänotyp der Zelle unterdrückt, insbesondere wobei die in Kultur gehaltene epitheliale Zelle genetisch verändert worden ist, um die Mutation in dem APC-Allel zu tragen.

22. Verfahren zum Testen von therapeutischen Mitteln auf ihre Fähigkeit, neoplastisches Wachstum zu unterdrücken, wobei das Verfahren die folgenden Schritte umfaßt:
- Verabreichen einer Testsubstanz an ein Tier mit Ausnahme von Menschen, das ein mutantes APC-Allel in seinem Genom trägt;
- Bestimmen, ob die Testsubstanz das Wachstum von Tumoren verhindert oder unterdrückt.

23. Transgenes Tier mit Ausnahme von Menschen, das ein mutantes APC-Allel von einer zweiten Tierspezies in seinem Genom trägt.

24. Tier mit Ausnahme von Menschen, das genetisch verändert wurde, um eine Insertions- oder Deletions-Mutation zu enthalten, die ein APC-Allel in seinem Genom unterbricht.

25. cDNA-Molekül, das ein APC-Protein kodiert.

26. cDNA-Molekül, das ein Protein mit der Aminosäuresequenz kodiert, die in Fig. 3 oder 7 (SEQ ID NO: 7 oder 1) gezeigt ist.

27. Isoliertes DNA-Molekül, das ein APC-Protein kodiert.

28. Isoliertes DNA-Molekül, das ein Protein mit der Aminosäuresequenz kodiert, die in Fig. 3 oder 7 (SEQ ID NO: 7 oder 1) gezeigt ist.

29. Künstliches Hefechromosom aus YAC-Klon 37HG4 mit der NCIMB-Hinterlegungsnummer 40 353.

30. Verwendung eines Wildtyp-APC-Gens zur Herstellung eines Arzneimittels zur Ausstattung einer Zelle mit Wildtyp-APC-Genfunktion, wobei die Zelle die Funktion durch eine Mutation in einem APC-Gen verloren hat, und wobei das Wildtyp-APC-Gen nach Einführung in die Zelle in der Zelle exprimiert wird.

31. Verwendung nach Anspruch 30, wobei das eingeführte Wildtyp-APC-Gen mit einem in der Zelle vorkommenden endogenen mutanten APC-Gen durch ein doppeltes Rekombinationsereignis unter Korrektur der APC-Genmutation rekombiniert.

32. Verwendung eines Anteils eines Wildtyp-APC-Gens zur Herstellung eines Arzneimittels zur Ausstattung einer Zelle, die eine veränderte APC-Funktion durch eine Mutation in einem APC-Gen besitzt, mit einer Wildtyp-APC-Genfunktion, wobei der Anteil nach Einführung in die Zelle in der Zelle exprimiert wird, und wobei der Anteil einen Teil des APC-Proteins kodiert, der für nicht-neoplastisches Wachstum der Zelle erforderlich ist.

33. Verwendung eines humanen Wildtyp-APC-Proteins zur Herstellung eines Arzneimittels zur Ausstattung einer Zelle, die eine veränderte APC-Funktion durch eine Mutation in einem APC-Gen besitzt, mit einer Wildtyp-APC-Genfunktion.

34. Verwendung eines Moleküls, das die Funktion eines Wildtyp-APC-Proteins nachahmt, zur Herstellung eines Arzneimittels zur Ausstattung einer Zelle, die eine veränderte APC-Genfunktion durch eine Mutation in einem APC-Gen besitzt, mit einer Wildtyp-APC-Genfunktion.

35. Verfahren nach einem der Ansprüche 1, 13 oder 14, wobei die Änderung nachgewiesen wird unter Verwendung des Amplification Refractory Mutation System (ARMS).

36. Verfahren, wie in einem der Ansprüche 1, 2, 3, 4, 13, 14, 15, 16 oder 18 beansprucht, wobei die Änderung ausgewählt wird aus der Gruppe bestehend aus:
(a) Umwandlung von C zu T an Kodon 414, was zu einem Wechsel von Arg (CGC) zu Cys (TGC) führt;
(b) Umwandlung von C zu T an Kodon 302, was zu einem Wechsel von Arg (CGA) zu Stop (TGA) führt;
(c) Umwandlung von C zu G an Kodon 280, was zu einem Wechsel von Ser (TCA) zu Stop (TGA) führt;
(d) Umwandlung von C zu G an Kodon 713, was zu einem Wechsel von Ser (TCA) zu Stop (TGA) führt;
(e) Umwandlung von C zu T an Kodon 332, was zu einem Wechsel von Arg (CGA) zu Stop (TGA) führt;
(f) Insertion von 5 Basenpaaren (CAGCC) zwischen Kodons 289-290; was zu einem Stop bei Kodon 292 infolge einer Leserahmenverschiebung führt;
(g) Umwandlung von C zu G an Kodon 1338, was zu einem Wechsel von Glu (CAG) zu Stop (TAG) führt;
(h) Verlust eines T um Kodon 456 herum, was eine Leserahmenverschiebung verursacht, die zu einem stromabwärts gelegenen Stop führt;
(i) Verlust von AG um Kodon 243 herum, was zu einer Spleißschnittstelle führt;
(j) Umwandlung von T zu C an Kodon 437, was zu einer Spleiß-Donorstelle (CAA/gtaa → CAA/gcaa) führt.

37. Verfahren, umfassend das Herstellen einer Zubereitung des humanen APC-Proteins, die im wesentlichen frei von anderen humanen Proteinen ist, wobei die Aminosäuresequenz des Proteins der in Fig. 3 oder 7 (SEQ ID NO: 7 oder 1) gezeigten entspricht.

38. Verfahren, umfassend das Herstellen einer Zubereitung von Antikörpern, die mit einem humanen APC-Protein immunreaktiv sind und nicht im wesentlichen immunreaktiv mit anderen humanen Proteinen sind.

39. Verfahren, umfassend das Herstellen eines cDNA-Moleküls, das ein APC-Protein kodiert oder eines cDNA-Moleküls, das ein Protein mit einer Aminosäuresequenz kodiert, die in Fig. 3 oder 7 (SEQ ID NO: 7 oder 1) gezeigt ist.

40. Verfahren, umfassend das Herstellen eines isolierten DNA-Moleküls, das ein APC-Protein kodiert, oder eines isolierten DNA-Moleküls, das ein Protein mit der Aminosäuresequenz kodiert, die in Fig. 3 oder 7 (SEQ ID NO: 7 oder 1) gezeigt ist.

## Revendications (Revendications pour l'(les) Etat(s) contractant(s) suivant(s): AT, BE, CH, LI, DE, DK, FR, GB, IT, LU, MC, NL, SE)

1. Procédé pour diagnostiquer ou pronostiquer un tissu néoplasique d'un humain, comportant l'étape consistant à : détecter une altération somatique de séquences d'un gène APC du type sauvage ou de leurs produits d'expression dans un tissu tumoral isolé à partir d'un humain, ladite altération indiquant une néoplasie du tissu, dans lequel, en particulier, les produits d'expression sont des molécules d'ARNm, et dans lequel, en particulier, les produits d'expression sont des molécules protéiniques.

2. Procédé selon la revendication 1, dans lequel l'altération de l'ARNm d'un gène APC du type sauvage est détectée par hybridation de l'ARNm provenant dudit tissu à une sonde d'un gène APC.

3. Procédé selon la revendication 1, dans lequel l'altération des séquences d'un gène APC du type sauvage est détectée par une hybridation d'une sonde à séquence d'un gène APC à de l'ADN génomique isolé à partir dudit tissu, dans lequel le procédé comporte en particulier de plus les étapes consistant à :
- soumettre l'ADN génomique isolé à partir d'un tissu non-néoplasique de l'humain à une hybridation Southern avec la sonde à séquence d'un gène APC, et
- comparer les hybridations de la sonde d'un gène APC auxdits tissus tumoral et non-néoplasique ; dans lequel, en particulier, la sonde d'un gène APC détecte un polymorphisme de taille de fragments de restriction.

4. Procédé selon la revendication 1, dans lequel la détection de l'altération de séquences d'un gène APC du type sauvage est sélectionnée parmi les techniques suivantes :
(a) l'observation de différences de mobilité électrophorétique d'ADN simple-brin sur des gels de polyacrylamide non-dénaturants,
(b) la détermination de la séquence de la totalité d'un gène APC, ou d'une partie de celui-ci, dans ledit tissu, des écarts dans la séquence APC déterminés par rapport à la séquence APC représentée (i) sur la figure 7 (SEQ ID n° 1), ou (ii) trouvée dans YAC 37HG4, suggérant une néoplasie,
(c) l'identification d'une incompatibilité entre des molécules, (1) un gène APC ou un ARNm de APC isolé à partir du tissu et (2) une sonde d'acide nucléique complémentaire de la séquence d'un gène APC humain du type sauvage, lorsque les molécules (1) et (2) sont hybridées l'une à l'autre pour former un duplex,
(d) l'amplification de séquences d'un gène APC dans ledit tissu et l'hybridation des séquences APC amplifiées à des sondes d'acide nucléique qui comportent des séquences APC,
(e) le clonage moléculaire des gènes APC dans ledit tissu et le séquençage de la totalité du gène APC cloné, ou d'une partie de celui-ci, et
(f) le criblage vis-à-vis d'une mutation par délétion, d'une mutation ponctuelle, d'une mutation par inversion ou d'une mutation par insertion.

5. Procédé selon la revendication 3, dans lequel la sonde d'un gène APC s'hybride à un exon sélectionné parmi le groupe constitué de : (1) les nucléotides 822 à 930, et (2) les nucléotides 931 à 1309, (3) les nucléotides 1406 à 1545, et (4) les nucléotides 1956 à 2256.

6. Procédé selon l'une quelconque des revendications 1 à 5, dans lequel le tissu néoplasique est un tissu colorectal, et dans lequel en particulier le tissu non-néoplasique isolé à partir d'un humain provient de la muqueuse du colon.

7. Procédé selon la revendication 1, dans lequel la détection de l'altération de la protéine APC du type sauvage est sélectionnée parmi une immuno-cytochimie, une immuno-empreinte, une évaluation de métabolites de phospholipide, et une évaluation d'interactions de liaison entre la protéine APC dudit tissu tumoral et une seconde protéine cellulaire, dans lequel en particulier la seconde protéine cellulaire est sélectionnée parmi une protéine MCC, une protéine APC du type sauvage, et une protéine G.

8. Procédé pour affecter in vitro une fonction d'un gène APC du type sauvage à une cellule qui a perdu ladite fonction du fait d'une mutation dans un gène APC, comportant les étapes en variante consistant à :
- introduire un gène APC du type sauvage dans une cellule qui a perdu ladite fonction génique de sorte que ledit gène APC du type sauvage est exprimé dans la cellule, en particulier le gène APC du type sauvage introduit se recombinant avec un gène APC mutant endogène présent dans la cellule par un événement de double recombinaison pour corriger la mutation du gène APC,
- introduire une partie d'un gène APC du type sauvage dans une cellule qui a perdu ladite fonction génique de sorte que ladite partie est exprimée dans la cellule, ladite partie codant pour une partie de la protéine APC qui est nécessaire à la croissance non-néoplasique de ladite cellule,
- appliquer une protéine APC humaine du type sauvage à une cellule qui a perdu la fonction d'un gène APC du type sauvage.

9. Procédé pour affecter in vitro une fonction d'un gène APC du type sauvage à une cellule qui a une fonction d'un gène APC altérée du fait d'une mutation dans un gène APC, comportant l'introduction dans la cellule d'une molécule qui imite la fonction d'une protéine APC du type sauvage.

10. Paire d'amorces d'ALN simple-brin pour déterminer une séquence nucléotidique d'un gène APC par réaction de polymérisation en chaîne, la séquence desdites amorces étant dérivée de la bande 21 du chromosome 5q, dans laquelle l'utilisation desdites amorces dans une réaction de polymérisation en chaîne a pour résultat la synthèse de l'ADN ayant la totalité de la séquence représentée sur la figure 7, ou une partie de celle-ci, en particulier dans laquelle les amorces ont des sites d'enzyme de restriction au niveau de chaque extrémité 5', et dans laquelle en particulier les amorces ont des séquences correspondant à des introns APC.

11. Sonde d'acide nucléique complémentaire de séquences codant pour un gène APC humain du type sauvage, en particulier dans laquelle la sonde d'acide nucléique s'hybride à un exon sélectionné parmi le groupe constitué de : (1) les nucléotides 822 à 930, et (2) les nucléotides 931 à 1309, (3) les nucléotides 1406 à 1545, (4) les nucléotides 1956 à 2256.

12. Kit pour détecter une altération de gènes APC du type sauvage comportant un batterie de scndes d'acide nucléique qui, dans l'agrégat, s'hybrident à tous les nucléotides des séquences codant pour un gène APC.

13. Procédé pour détecter la présence d'un tissu néoplasique chez un humain, comportant l'étape consistant à :
détecter dans un échantillon corporel, isolé à partir d'un humain, une altération d'une séquence d'un gène APC du type sauvage ou d'un produit d'expression d'un gène APC du type sauvage, ladite altération indiquant la présence d'un tissu néoplasique chez l'humain, dans lequel en particulier ledit échantillon corporel est sélectionné parmi un sérum, des selles, de l'urine et un crachat.

14. Procédé pour détecter une prédisposition génétique à un cancer, incluant le polyadénome du gros intestin (FAP) et le Syndrome de Gardner (GS), chez un humain comportant l'étape consistant à :
détecter une altération germinale de séquences d'un gène APC du type sauvage ou de leurs produits d'expression dans un échantillon humain, en particulier du sang et des tissus foetaux, ladite altération indiquant une prédisposition à un cancer.

15. Procédé selon la revendication 14, dans lequel les produits d'expression sont des molécules d'ARNm, dans lequel en particulier l'altération de l'ARNm d'un gène APC du type sauvage est détectée par hybridation de l'ARNm provenant dudit tissu à une sonde d'un gène APC.

16. Procédé selon la revendication 13 ou 14, dans lequel la détection de l'altération de séquences d'un gène APC du type sauvage est sélectionnée parmi les techniques suivantes :
(a) l'observation de différences de mobilité électrophorétique d'ADN simple-brin sur des gels de polyacrylamide non-dénaturants,
(b) l'hybridation d'une sonde à séquence d'un gène APC à de l'ADN génomique isolé à partir dudit tissu, en particulier dans lequel la sonde à séquence d'un gène APC détecte un polymorphisme de taille de fragments de restriction, en particulier dans lequel la sonde d'un gène APC s'hybride à un exon sélectionné parmi : (1) les nucléotides 822 à 930, et (2) les nucléotides 931 à 1309, (3) les nucléotides 1406 à 1545 et (4) les nucléotides 1956 à 2256,
(c) la détermination de la séquence de la totalité d'un gène APC, ou d'une partie de celui-ci, dans ledit tissu, des écarts de séquence d'un gène APC déterminés par rapport à la séquence de la figure 7, ou la séquence trouvée dans YAC 37HG4, suggérant une prédisposition à un cancer,
(d) l'identification d'une incompatibilité entre des molécules, (1) un gène APC ou de l'ARNm d'un gène APC isolé à partir dudit tissu et (2) une sonde d'acide nucléique complémentaire de la séquence d'un gène APC humain du type sauvage, lorsque les molécules (1) et (2) sont hybridées l'une à l'autre pour former un duplex,
(e) l'amplification de séquences d'un gène APC dans ledit tissu et l'hybridation des séquences d'un gène APC amplifiées à des sondes d'acide nucléique qui comportent des séquences d'un gène APC,
(f) le clonage moléculaire des gènes APC dans ledit tissu et le séquençage de la totalité du gène APC cloné, ou d'une partie de celui-ci, et
(g) le criblage vis-à-vis d'une mutation par délétion, d'une mutation ponctuelle, d'une mutation par inversion ou un criblage vis-à-vis d'une mutation par insertion.

17. Procédé selon la revendication 14, dans lequel les produits d'expression sont des molécules protéines, en particulier dans lequel l'altération de la protéine APC du type sauvage est détectée par immuno-empreinte, par immuno-chimie, ou par évaluation d'interactions de liaison entre la protéine APC isolée à partir dudit tissu et une seconde protéine cellulaire, en particulier dans lequel la seconde protéine cellulaire est sélectionnée parmi une protéine MCC, une protéine APC du type sauvage, et une protéine G.

18. Procédé in vitro de criblage vis-à-vis d'une prédisposition génétique à un cancer, incluant le polyadénome du gros intestin (FAP) et le Syndrome de Gardner (GS), chez un humain comportant l'étape consistant à :
détecter parmi des personnes apparentées la présence d'un polymorphisme d'ADN qui est lié à un allèle d'un gène APC mutant chez un individu ayant une prédisposition génétique à un cancer, ledit lien de parenté étant génétiquement associé à l'individu, la présence dudit polymorphisme suggérant une prédisposition à un cancer.

19. Préparation de la protéine APC humaine essentiellement exempte d'autres protéines humaines, la séquence d'acides aminés de ladite protéine correspondant à celle représentée sur la figure 3 ou 7 (SEQ ID n° 1).

20. Préparation d'anticorps immunoréactifs avec une protéine APC humaine et pratiquement non-immunoréactifs avec d'autres protéines humaines.

21. Procédé pour tester des agents thérapeutiques vis-à-vis de la capacité à atténuer un phénotype transformé de manière néoplasique, comportant les étapes consistant à :
- appliquer une substance test à une cellule épithéliale cultivée qui porte une mutation dans un allèle APC,
- déterminer si ladite substance test atténue le phénotype transformé de manière néoplasique de la cellule, dans lequel en particulier la cellule épithéliale cultivée a été génétiquement manipulée pour porter la mutation dans l'allèle APC.

22. Procédé pour tester des agents thérapeutiques vis-à-vis de la capacité à atténuer une croissance néoplasique, comportant les étapes consistant à :
- administrer une substance test à un animal, à l'exception d'humains, qui porte un allèle APC mutant dans son génome,
- déterminer si ladite substance test empêche ou atténue la croissance de tumeurs.

23. Animal transgénique, à l'exception d'humains, qui porte dans son génome un allèle APC mutant provenant d'une seconde espèce animale.

24. Animal, à l'exception d'humains, qui a été génétiquement manipulé pour contenir une mutation par insertion ou délétion qui rompt un allèle APC dans son génome.

25. Molécule d'ADNc qui code pour une protéine APC.

26. Molécule d'ADNc qui code pour une protéine ayant la séquence d'acides aminés représentée sur la figure 3 ou 7 (SEQ ID n° 7 ou 1).

27. Molécule d'ADN isolée qui code pour une protéine APC.

28. Molécule d'ADN isolée qui code pour une protéine ayant la séquence d'acides aminés représentée sur la figure 3 ou 7 (SEQ ID n° 7 ou 1).

29. Chromosome artificiel de levure provenant du clone 37HG4 de YAC ayant le numéro de série NCIMB 40 353.

30. Gène APC du type sauvage destiné à être utilisé dans un procédé pour affecter une fonction d'un gène APC du type sauvage à une cellule qui a perdu ladite fonction du fait d'une mutation du gène APC, dans lequel ledit gène APC du type sauvage après introduction dans la cellule est exprimé dans la cellule.

31. Gène APC du type sauvage selon la revendication 30, dans lequel ledit gène APC du type sauvage introduit se recombine avec le gène APC mutant endogène présent dans la cellule par un événement de double recombinaison pour corriger la mutation du gène APC.

32. Partie d'un gène APC du type sauvage destinée à être utilisée dans un procédé pour affecter une fonction d'un gène APC du type sauvage à une cellule qui a une fonction d'un gène APC altérée du fait d'une mutation dans un gène APC, dans laquelle ladite partie, après introduction dans la cellule, est exprimée dans la cellule, ladite partie codant pour une partie de la protéine APC qui est nécessaire pour une croissance non-néoplasique de ladite cellule.

33. Protéine APC humaine du type sauvage destinée à être utilisée dans un procédé pour affecter une fonction d'un gène APC du type sauvage à une cellule qui a une fonction d'un gène APC altérée du fait d'une mutation dans un gène APC.

34. Molécule qui imite la fonction d'une protéine APC du type sauvage destinée à être utilisée dans un procédé pour affecter une fonction d'un gène APC du type sauvage à une cellule qui a une fonction d'un gène APC altérée du fait d'une mutation dans un gène APC.

35. Procédé selon l'une quelconque des revendications 1, 13 ou 14, dans lequel l'altération est détectée en utilisant le Système de Mutation Résistant à une Amplification (ARMS).

36. Procédé selon l'une quelconque des revendications 1, 2, 3, 4, 13, 14, 15, 16 ou 18, dans lequel l'altération est sélectionnée parmi le groupe constitué des altérations suivantes :
(a) transition de C en T au niveau du codon 414 ayant pour résultat un changement de Arg (CGC) en Cys (TGC),
(b) transition de C en T au niveau du codon 302 ayant pour résultat un changement de Arg (CGA) en codon d'arrêt (TGA),
(c) transition de C en G au niveau du codon 280 ayant pour résultat un changement de Ser (TCA) en codon d'arrêt (TGA),
(d) transition de C en G au niveau du codon 713 ayant pour résultat un changement de Ser (TCA) en codon d'arrêt (TGA),
(e) transition de C en T au niveau du codon 332 ayant pour résultat un changement de Arg (CGA) en codon d'arrêt (TGA),
(f) insertion de 5 paires de bases (CAGCC) entre les codons 289 et 290, ayant pour résultat un codon d'arrêt au niveau du codon 292 du fait d'un décalage du cadre de lecture,
(g) transition de C en G au niveau du codon 1338 ayant pour résultat un changement de Glu (CAG) en codon d'arrêt (TAG),
(h) perte d'un T autour du codon 456 entraînant un décalage du cadre de lecture ayant pour résultat un codon d'arrêt en aval,
(i) perte de AG autour du codon 243 ayant pour résultat une jonction d'épissure,
(j) transition de T en C au niveau du codon 437 ayant pour résultat un site donneur d'épissure (CAA/gtaa→CAA/gcaa).

## Revendications (Revendications pour l'(les) Etat(s) contractant(s) suivant(s): ES)

1. Procédé pour diagnostiquer ou pronostiquer un tissu néoplasique d'un humain, comportant l'étape consistant à : détecter une altération somatique de séquences d'un gène APC du type sauvage ou de leurs produits d'expression dans un tissu tumoral isolé à partir d'un humain, ladite altération indiquant une néoplasie du tissu, dans lequel, en particulier, les produits d'expression sont des molécules d'ARNm, et dans lequel, en particulier, les produits d'expression sont des molécules protéiniques.

2. Procédé selon la revendication 1, dans lequel l'altération de l'ARNm d'un gène APC du type sauvage est détectée par hybridation de l'ARNm provenant dudit tissu à une sonde d'un gène APC.

3. Procédé selon la revendication 1, dans lequel l'altération des séquences d'un gène APC du type sauvage est détectée par une hybridation d'une sonde à séquence d'un gène APC à de l'ADN génomique isolé à partir dudit tissu, dans lequel le procédé comporte en particulier de plus les étapes consistant à :
- soumettre l'ADN génomique isolé à partir d'un tissu non-néoplasique de l'humain à une hybridation Southern avec la sonde à séquence d'un gène APC, et
- comparer les hybridations de la sonde d'un gène APC auxdits tissus tumoral et non-néoplasique ; dans lequel, en particulier, la sonde d'un gène APC détecte un polymorphisme de taille de fragments de restriction.

4. Procédé selon la revendication 1, dans lequel la détection de l'altération de séquences d'un gène APC du type sauvage est sélectionnée parmi les techniques suivantes :
(a) l'observation de différences de mobilité électrophorétique d'ADN simple-brin sur des gels de polyacrylamide non-dénaturants,
(b) la détermination de la séquence de la totalité d'un gène APC, ou d'une partie de celui-ci, dans ledit tissu, des écarts dans la séquence APC déterminés par rapport à la séquence APC représentée (i) sur la figure 7 (SEQ ID n° 1), ou (ii) trouvée dans YAC 37HG4, suggérant une néoplasie,
(c) l'identification d'une incompatibilité entre des molécules, (1) un gène APC ou un ARNm de APC isolé à partir du tissu et (2) une sonde d'acide nucléique complémentaire de la séquence d'un gène APC humain du type sauvage, lorsque les molécules (1) et (2) sont hybridées l'une à l'autre pour former un duplex,
(d) l'amplification de séquences d'un gène APC dans ledit tissu et l'hybridation des séquences APC amplifiées à des sondes d'acide nucléique qui comportent des séquences APC,
(e) le clonage moléculaire des gènes APC dans ledit tissu et le séquençage de la totalité du gène APC cloné, ou d'une partie de celui-ci, et
(f) le criblage vis-à-vis d'une mutation par délétion, d'une mutation ponctuelle, d'une mutation par inversion ou d'une mutation par insertion.

5. Procédé selon la revendication 3, dans lequel la sonde d'un gène APC s'hybride à un exon sélectionné parmi le groupe constitué de : (1) les nucléotides 822 à 930, et (2) les nucléotides 931 à 1309, (3) les nucléotides 1406 à 1545, et (4) les nucléotides 1956 à 2256.

6. Procédé selon l'une quelconque des revendications 1 à 5, dans lequel le tissu néoplasique est un tissu colorectal, et dans lequel en particulier le tissu non-néoplasique isolé à partir d'un humain provient de la muqueuse du colon.

7. Procédé selon la revendication 1, dans lequel la détection de l'altération de la protéine APC du type sauvage est sélectionnée parmi une immuno-cytochimie, une immuno-empreinte, une évaluation de métabolites de phospholipide, et une évaluation d'interactions de liaison entre la protéine APC dudit tissu tumoral et une seconde protéine cellulaire, dans lequel en particulier la seconde protéine cellulaire est sélectionnée parmi une protéine MCC, une protéine APC du type sauvage, et une protéine G.

8. Procédé pour affecter in vitro une fonction d'un gène APC du type sauvage à une cellule qui a perdu ladite fonction du fait d'une mutation dans un gène APC, comportant les étapes en variante consistant à :
- introduire un gène APC du type sauvage dans une cellule qui a perdu ladite fonction génique de sorte que ledit gène APC du type sauvage est exprimé dans la cellule, en particulier le gène APC du type sauvage introduit se recombinant avec un gène APC mutant endogène présent dans la cellule par un événement de double recombinaison pour corriger la mutation du gène APC,
- introduire une partie d'un gène APC du type sauvage dans une cellule qui a perdu ladite fonction génique de sorte que ladite partie est exprimée dans la cellule, ladite partie codant pour une partie de la protéine APC qui est nécessaire à la croissance non-néoplasique de ladite cellule,
- appliquer une protéine APC humaine du type sauvage à une cellule qui a perdu la fonction d'un gène APC du type sauvage.

9. Procédé pour affecter in vitro une fonction d'un gène APC du type sauvage à une cellule qui a une fonction d'un gène APC altérée du fait d'une mutation dans un gène APC, comportant l'introduction dans la cellule d'une molécule qui imite la fonction d'une protéine APC du type sauvage.

10. Processus qui comporte la préparation d'une paire d'amorces d'ADN simple-brin pour déterminer une séquence nucléotidique d'un gène APC par réaction de polymérisation en chaîne, la séquence desdites amorces étant dérivée de la bande 21 du chromosome 5q, dans lequel l'utilisation desdites amorces dans une réaction de polymérisation en chaîne a pour résultat la synthèse de l'ADN ayant la totalité de la séquence représentée sur la figure 7, ou une partie de celle-ci, en particulier dans lequel les amorces ont des sites d'enzyme de restriction au niveau de chaque extrémité 5', et dans lequel en particulier les amorces ont des séquences correspondant à des introns APC.

11. Processus qui comporte la préparation d'une sonde d'acide nucléique complémentaire de séquences codant pour un gène APC humain du type sauvage, en particulier dans lequel la sonde d'acide nucléique s'hybride à un exon sélectionné parmi le groupe constitué de : (1) les nucléotides 822 à 930, et (2) les nucléotides 931 à 1309, (3) les nucléotides 1406 à 1545, (4) les nucléotides 1956 à 2256.

12. Processus qui comporte la préparation d'un kit pour détecter une altération de gènes APC du type sauvage comportant un batterie de sondes d'acide nucléique qui, dans l'agrégat, s'hybrident à tous les nucléotides des séquences codant pour un gène APC.

13. Procédé pour détecter la présence d'un tissu néoplasique chez un humain, comportant l'étape consistant à :
détecter dans un échantillon corporel, isolé à partir d'un humain, une altération d'une séquence d'un gène APC du type sauvage ou d'un produit d'expression d'un gène APC du type sauvage, ladite altération indiquant la présence d'un tissu néoplasique chez l'humain, dans lequel en particulier ledit échantillon corporel est sélectionné parmi un sérum, des selles, de l'urine et un crachat.

14. Procédé pour détecter une prédisposition génétique à un cancer, incluant le polyadénome du gros intestin (FAP) et le Syndrome de Gardner (GS), chez un humain comportant l'étape consistant à :
détecter une altération germinale de séquences d'un gène APC du type sauvage ou de leurs produits d'expression dans un échantillon humain, en particulier du sang et des tissus foetaux, ladite altération indiquant une prédisposition à un cancer.

15. Procédé selon la revendication 14, dans lequel les produits d'expression sont des molécules d'ARNm, dans lequel en particulier l'altération de l'ARNm d'un gène APC du type sauvage est détectée par hybridation de l'ARNm provenant dudit tissu à une sonde d'un gène APC.

16. Procédé selon la revendication 13 ou 14, dans lequel la détection de l'altération de séquences d'un gène APC du type sauvage est sélectionnée parmi les techniques suivantes :
(a) l'observation de différences de mobilité électrophorétique d'ADN simple-brin sur des gels de polyacrylamide non-dénaturants,
(b) l'hybridation d'une sonde à séquence d'un gène APC à de l'ADN génomique isolé à partir dudit tissu, en particulier dans lequel la sonde à séquence d'un gène APC détecte un polymorphisme de taille de fragments de restriction, en particulier dans lequel la sonde d'un gène APC s'hybride à un exon sélectionné parmi : (1) les nucléotides 822 à 930, et (2) les nucléotides 931 à 1309, (3) les nucléotides 1406 à 1545 et (4) les nucléotides 1956 à 2256,
(c) la détermination de la séquence de la totalité d'un gène APC, ou d'une partie de celui-ci, dans ledit tissu, des écarts de séquence d'un gène APC déterminés par rapport à la séquence de la figure 7, ou la séquence trouvée dans YAC 37HG4, suggérant une prédisposition à un cancer,
(d) l'identification d'une incompatibilité entre des molécules, (1) un gène APC ou de l'ARNm d'un gène APC isolé à partir dudit tissu et (2) une sonde d'acide nucléique complémentaire de la séquence d'un gène APC humain du type sauvage, lorsque les molécules (1) et (2) sont hybridées l'une à l'autre pour former un duplex,
(e) l'amplification de séquences d'un gène APC dans ledit tissu et l'hybridation des séquences d'un gène APC amplifiées à des sondes d'acide nucléique qui comportent des séquences d'un gène APC,
(f) le clonage moléculaire des gènes APC dans ledit tissu et le séquençage de la totalité du gène APC cloné, ou d'une partie de celui-ci, et
(g) le criblage vis-à-vis d'une mutation par délétion, d'une mutation ponctuelle, d'une mutation par inversion ou un criblage vis-à-vis d'une mutation par insertion.

17. Procédé selon la revendication 14, dans lequel les produits d'expression sont des molécules protéines, en particulier dans lequel l'altération de la protéine APC du type sauvage est détectée par immuno-empreinte, par immuno-chimie, ou par évaluation d'interactions de liaison entre la protéine APC isolée à partir dudit tissu et une seconde protéine cellulaire, en particulier dans lequel la seconde protéine cellulaire est sélectionnée parmi une protéine MCC, une protéine APC du type sauvage, et une protéine G.

18. Procédé in vitro de criblage vis-à-vis d'une prédisposition génétique à un cancer, incluant le polyadénome du gros intestin FAP et le Syndrome de Gardner (GS), chez un humain comportant l'étape consistant à :
détecter parmi des personnes apparentées la présence d'un polymorphisme d'ADN qui est lié à un allèle d'un gène APC mutant chez un individu ayant une prédisposition génétique à un cancer, ledit lien de parenté étant génétiquement associé à l'individu, la présence dudit polymorphisme suggérant une prédisposition à un cancer.

19. Processus qui comporte la préparation d'une préparation de la protéine APC humaine essentiellement exempte d'autres protéines humaines, la séquence d'acides aminés de ladite protéine correspondant à celle représentée sur la figure 3 ou 7 (SEQ ID n° 1).

20. Processus qui comporte la préparation d'une préparation d'anticorps immunoréactifs avec une protéine APC humaine et pratiquement non-immunoréactifs avec d'autres protéines humaines.

21. Procédé pour tester des agents thérapeutiques vis-à-vis de la capacité à atténuer un phénotype transformé de manière néoplasique, comportant les étapes consistant à :
- appliquer une substance test à une cellule épithéliale cultivée qui porte une mutation dans un allèle APC,
- déterminer si ladite substance test atténue le phénotype transformé de manière néoplasique de la cellule, dans lequel en particulier la cellule épithéliale cultivée a été génétiquement manipulée pour porter la mutation dans l'allèle APC.

22. Procédé pour tester des agents thérapeutiques vis-à-vis de la capacité à atténuer une croissance néoplasique, comportant les étapes consistant à :
- administrer une substance test à un animal, à l'exception d'humains, qui porte un allèle APC mutant dans son génome,
- déterminer si ladite substance test empêche ou atténue la croissance de tumeurs.

23. Processus qui comporte la préparation d'un animal transgénique, à l'exception d'humains, qui porte dans son génome un allèle APC mutant provenant d'une seconde espèce animale.

24. Processus qui comporte la préparation d'un animal, à l'exception d'humains, qui a été génétiquement manipulé pour contenir une mutation par insertion ou délétion qui rompt un allèle APC dans son génome.

25. Processus qui comporte la préparation d'une molécule d'ADNc qui code pour une protéine APC.

26. Processus qui comporte la préparation d'une molécule d'ADNc qui code pour une protéine ayant la séquence d'acides aminés représentée sur la figure 3 ou 7 (SEQ ID n° 7 ou 1).

27. Processus qui comporte la préparation d'une molécule d'ADN isolée qui code pour une protéine APC.

28. Processus qui comporte la préparation d'une molécule d'ADN isolée qui code pour une protéine ayant la séquence d'acides aminés représentée sur la figure 3 ou 7 (SEQ ID n° 7 ou 1).

29. Processus qui comporte la préparation d'un chromosome artificiel de levure provenant du clone 37HG4 de YAC ayant le numéro de série NCIMB 40 353.

30. Utilisation d'un gène APC du type sauvage pour la fabrication d'un médicament destiné à affecter une fonction d'un gène APC du type sauvage à une cellule qui a perdu ladite fonction du fait d'une mutation du gène APC, dans laquelle ledit gène APC du type sauvage après introduction dans la cellule est exprimé dans la cellule.

31. Utilisation selon la revendication 30, dans laquelle ledit gène APC du type sauvage introduit se recombine avec le gène APC mutant endogène présent dans la cellule par un événement de double recombinaison pour corriger la mutation du gène APC.

32. Utilisation d'une partie d'un gène APC du type sauvage pour la fabrication d'un médicament destiné à affecter une fonction d'un gène APC du type sauvage à une cellule qui a une fonction d'un gène APC altérée du fait d'une mutation dans un gène APC, dans laquelle ladite partie, après introduction dans la cellule, est exprimée dans la cellule, ladite partie codant pour une partie de la protéine APC qui est nécessaire pour une croissance non-néoplasique de ladite cellule.

33. Utilisation d'une protéine APC humaine du type sauvage pour la fabrication d'un médicament destiné à affecter une fonction d'un gène APC du type sauvage à une cellule qui a une fonction d'un gène APC altérée du fait d'une mutation dans un gène APC.

34. Utilisation d'une molécule qui imite la fonction d'une protéine APC du type sauvage pour la fabrication d'un médicament destiné à affecter une fonction d'un gène APC du type sauvage à une cellule qui a une fonction d'un gène APC altérée du fait d'une mutation dans un gène APC.

35. Procédé selon l'une quelconque des revendications 1, 13 ou 14, dans lequel l'altération est détectée en utilisant le Système de Mutation Résistant à une Amplification (ARMS).

36. Procédé selon l'une quelconque des revendications 1, 2, 3, 4, 13, 14, 15, 16 ou 18, dans lequel l'altération est sélectionnée parmi le groupe constitué des altérations suivantes :
(a) transition de C en T au niveau du codon 414 ayant pour résultat un changement de Arg (CGC) en Cys (TGC),
(b) transition de C en T au niveau du codon 302 ayant pour résultat un changement de Arg (CGA) en codon d'arrêt (TGA),
(c) transition de C en G au niveau du codon 280 ayant pour résultat un changement de Ser (TCA) en codon d'arrêt (TGA),
(d) transition de C en G au niveau du codon 713 ayant pour résultat un changement de Ser (TCA) en codon d'arrêt (TGA),
(e) transition de C en T au niveau du codon 332 ayant pour résultat un changement de Arg (CGA) en codon d'arrêt (TGA),
(f) insertion de 5 paires de bases (CAGCC) entre les codons 289 et 29C, ayant pour résultat un codon d'arrêt au niveau du codon 292 du fait d'un décalage du cadre de lecture,
(g) transition de C en G au niveau du codon 1338 ayant pour résultat un changement de Glu (CAG) en codon d'arrêt (TAG),
(h) perte d'un T autour du codon 456 entraînant un décalage du cadre de lecture ayant pour résultat un codon d'arrêt en aval,
(i) perte de AG autour du codon 243 ayant pour résultat une jonction d'épissure,
(j) transition de T en C au niveau du codon 437 ayant pour résultat un site donneur d'épissure (CAA/gtaa→CAA/gcaa).

## Revendications (Revendications pour l'(les) Etat(s) contractant(s) suivant(s): GR)

1. Procédé pour diagnostiquer ou pronostiquer un tissu néoplasique d'un humain, comportant l'étape consistant à : détecter une altération somatique de séquences d'un gène APC du type sauvage ou de leurs produits d'expression dans un tissu tumoral isolé à partir d'un humain, ladite altération indiquant une néoplasie du tissu, dans lequel, en particulier, les produits d'expression sont des molécules d'ARNm, et dans lequel, en particulier, les produits d'expression sont des molécules protéiniques.

2. Procédé selon la revendication 1, dans lequel l'altération de l'ARNm d'un gène APC du type sauvage est détectée par hybridation de l'ARNm provenant dudit tissu à une sonde d'un gène APC.

3. Procédé selon la revendication 1, dans lequel l'altération des séquences d'un gène APC du type sauvage est détectée par une hybridation d'une sonde à séquence d'un gène APC à de l'ADN génomique isolé à partir dudit tissu, dans lequel le procédé comporte en particulier de plus les étapes consistant à :
- soumettre l'ADN génomique isolé à partir d'un tissu non-néoplasique de l'humain à une hybridation Southern avec la sonde à séquence d'un gène APC, et
- comparer les hybridations de la sonde d'un gène APC auxdits tissus tumoral et non-néoplasique ; dans lequel, en particulier, la sonde d'un gène APC détecte un polymorphisme de taille de fragments de restriction.

4. Procédé selon la revendication 1, dans lequel la détection de l'altération de séquences d'un gène APC du type sauvage est sélectionnée parmi les techniques suivantes :
(a) l'observation de différences de mobilité électrophorétique d'ADN simple-brin sur des gels de polyacrylamide non-dénaturants,
(b) la détermination de la séquence de la totalité d'un gène APC, ou d'une partie de celui-ci, dans ledit tissu, des écarts dans la séquence APC déterminés par rapport à la séquence APC représentée (i) sur la figure 7 (SEQ ID n° 1), ou (ii) trouvée dans YAC 37HG4, suggérant une néoplasie,
(c) l'identification d'une incompatibilité entre des molécules, (1) un gène APC ou un ARNm de APC isolé à partir du tissu et (2) une sonde d'acide nucléique complémentaire de la séquence d'un gène APC humain du type sauvage, lorsque les molécules (1) et (2) sont hybridées l'une à l'autre pour former un duplex,
(d) l'amplification de séquences d'un gène APC dans ledit tissu et l'hybridation des séquences APC amplifiées à des sondes d'acide nucléique qui comportent des séquences APC,
(e) le clonage moléculaire des gènes APC dans ledit tissu et le séquençage de la totalité du gène APC cloné, ou d'une partie de celui-ci, et
(f) le criblage vis-à-vis d'une mutation par délétion, d'une mutation ponctuelle, d'une mutation par inversion ou d'une mutation par insertion.

5. Procédé selon la revendication 3, dans lequel la scnde d'un gène APC s'hybride à un exon sélectionné parmi le groupe constitué de : (1) les nucléotides 822 à 930, et (2) les nucléotides 931 à 1309, (3) les nucléotides 1406 à 1545, et (4) les nucléotides 1956 à 2256.

6. Procédé selon l'une quelconque des revendications 1 à 5, dans lequel le tissu néoplasique est un tissu colorectal, et dans lequel en particulier le tissu non-néoplasique isolé à partir d'un humain provient de la muqueuse du colon.

7. Procédé selon la revendication 1, dans lequel la détection de l'altération de la protéine APC du type sauvage est sélectionnée parmi une immuno-cytochimie, une immuno-empreinte, une évaluation de métabolites de phospholide, et une évaluation d'interactions de liaison entre la protéine APC dudit tissu tumoral et une seconde protéine cellulaire, dans lequel en particulier la seconde protéine cellulaire est sélectionnée parmi une protéine MCC, une protéine APC du type sauvage, et une protéine G.

8. Procédé pour affecter in vitro une fonction d'un gène APC du type sauvage à une cellule qui a perdu ladite fonction du fait d'une mutation dans un gène APC, comportant les étapes en variante consistant à :
- introduire un gène APC du type sauvage dans une cellule qui a perdu ladite fonction génique de sorte que ledit gène APC du type sauvage est exprimé dans la cellule, en particulier le gène APC du type sauvage introduit se recombinant avec un gène APC mutant endogène présent dans la cellule par un événement de double recombinaison pour corriger la mutation du gène APC,
- introduire une partie d'un gène APC du type sauvage dans une cellule qui a perdu ladite fonction génique de sorte que ladite partie est exprimée dans la cellule, ladite partie codant pour une partie de la protéine APC qui est nécessaire à la croissance non-néoplasique de ladite cellule,
- appliquer une protéine APC humaine du type sauvage à une cellule qui a perdu la fonction d'un gène APC du type sauvage.

9. Procédé pour affecter in vitro une fonction d'un gène APC du type sauvage à une cellule qui a une fonction d'un gène APC altérée du fait d'une mutation dans un gène APC, comportant l'introduction dans la cellule d'une molécule qui imite la fonction d'une protéine APC du type sauvage.

10. Paire d'amorces d'ADN simple-brin pour déterminer une séquence nucléotidique d'un gène APC par réaction de polymérisation en chaîne, la séquence desdites amorces étant dérivée de la bande 21 du chromosome 5q, dans laquelle l'utilisation desdites amorces dans une réaction de polymérisation en chaîne a pour résultat la synthèse de l'ADN ayant la totalité de la séquence représentée sur la figure 7, ou une partie de celle-ci, en particulier dans laquelle les amorces ont des sites d'enzyme de restriction au niveau de chaque extrémité 5', et dans laquelle en particulier les amorces ont des séquences correspondant à des introns APC.

11. Sonde d'acide nucléique complémentaire de séquences codant pour un gène APC humain du type sauvage, en particulier dans laquelle la sonde d'acide nucléique s'hybride à un exon sélectionné parmi le groupe constitué de : (1) les nucléotides 822 à 930, et (2) les nucléotides 931 à 1309, (3) les nucléotides 1406 à 1545, (4) les nucléotides 1956 à 2256.

12. Kit pour détecter une altération de gènes APC du type sauvage comportant un batterie de sondes d'acide nucléique qui, dans l'agrégat, s'hybrident à tous les nucléotides des séquences codant pour un gène APC.

13. Procédé pour détecter la présence d'un tissu néoplasique chez un humain, comportant l'étape consistant à :
détecter dans un échantillon corporel, isolé à partir d'un humain, une altération d'une séquence d'un gène APC du type sauvage ou d'un produit d'expression d'un gène APC du type sauvage, ladite altération indiquant la présence d'un tissu néoplasique chez l'humain, dans lequel en particulier ledit échantillon corporel est sélectionné parmi un sérum, des selles, de l'urine et un crachat.

14. Procédé pour détecter une prédisposition génétique à un cancer, incluant le polyadénome du gros intestin (FAP) et le Syndrome de Gardner (GS), chez un humain comportant l'étape consistant à :
détecter une altération germinale de séquences d'un gène APC du type sauvage ou de leurs produits d'expression dans un échantillon humain, en particulier du sang et des tissus foetaux, ladite altération indiquant une prédisposition à un cancer.

15. Procédé selon la revendication 14, dans lequel les produits d'expression sont des molécules d'ARNm, dans lequel en particulier l'altération de l'ARNm d'un gène APC du type sauvage est détectée par hybridation de l'ARNm provenant dudit tissu à une sonde d'un gène APC.

16. Procédé selon la revendication 13 ou 14, dans lequel la détection de l'altération de séquences d'un gène APC du type sauvage est sélectionnée parmi les techniques suivantes :
(a) l'observation de différences de mobilité électrophorétique d'ADN simple-brin sur des gels de polyacrylamide non-dénaturants,
(b) l'hybridation d'une sonde à séquence d'un gène APC à de l'ADN génomique isolé à partir dudit tissu, en particulier dans lequel la sonde à séquence d'un gène APC détecte un polymorphisme de taille de fragments de restriction, en particulier dans lequel la sonde d'un gène APC s'hybride à un exon sélectionné parmi : (1) les nucléotides 822 à 930, et (2) les nucléotides 931 à 1309, (3) les nucléotides 1406 à 1545 et (4) les nucléotides 1956 à 2256,
(c) la détermination de la séquence de la totalité d'un gène APC, ou d'une partie de celui-ci, dans ledit tissu, des écarts de séquence d'un gène APC déterminés par rapport à la séquence de la figure 7, ou la séquence trouvée dans YAC 37HG4, suggérant une prédisposition à un cancer,
(d) l'identification d'une incompatibilité entre des molécules, (1) un gène APC ou de l'ARNm d'un gène APC isolé à partir dudit tissu et (2) une sonde d'acide nucléique complémentaire de la séquence d'un gène APC humain du type sauvage, lorsque les molécules (1) et (2) sont hybridées l'une à l'autre pour former un duplex,
(e) l'amplification de séquences d'un gène APC dans ledit tissu et l'hybridation des séquences d'un gène APC amplifiées à des sondes d'acide nucléique qui comportent des séquences d'un gène APC,
(f) le clonage moléculaire des gènes APC dans ledit tissu et le séquençage de la totalité du gène APC cloné, ou d'une partie de celui-ci, et
(g) le criblage vis-à-vis d'une mutation par délétion, d'une mutation ponctuelle, d'une mutation par inversion ou un criblage vis-à-vis d'une mutation par insertion.

17. Procédé selon la revendication 14, dans lequel les produits d'expression sont des molécules protéines, en particulier dans lequel l'altération de la protéine APC du type sauvage est détectée par immuno-empreinte, par immuno-chimie, ou par évaluation d'interactions de liaison entre la protéine APC isolée à partir dudit tissu et une seconde protéine cellulaire, en particulier dans lequel la seconde protéine cellulaire est sélectionnée parmi une protéine MCC, une protéine APC du type sauvage, et une protéine G.

18. Procédé in vitro de criblage vis-à-vis d'une prédisposition génétique à un cancer, incluant le polyadénome du gros intestin (FAP) et le Syndrome de Gardner (GS), chez un humain comportant l'étape consistant à :
détecter parmi des personnes apparentées la présence d'un polymorphisme d'ADN qui est lié à un allèle d'un gène APC mutant chez un individu ayant une prédisposition génétique à un cancer, ledit lien de parenté étant génétiquement associé à l'individu, la présence dudit polymorphisme suggérant une prédisposition à un cancer.

19. Préparation de la protéine APC humaine essentiellement exempte d'autres protéines humaines, la séquence d'acides aminés de ladite protéine correspondant à celle représentée sur la figure 3 ou 7 (SEQ ID n° 1).

20. Préparation d'anticorps immunoréactifs avec une protéine APC humaine et pratiquement non-immunoréactifs avec d'autres protéines humaines.

21. Procédé pour tester des agents thérapeutiques vis-à-vis de la capacité à atténuer un phénotype transformé de manière néoplasique, comportant les étapes consistant à :
- appliquer une substance test à une cellule épithéliale cultivée qui porte une mutation dans un allèle APC,
- déterminer si ladite substance test atténue le phénotype transformé de manière néoplasique de la cellule, dans lequel en particulier la cellule épithéliale cultivée a été génétiquement manipulée pour porter la mutation dans l'allèle APC.

22. Procédé pour tester des agents thérapeutiques vis-à-vis de la capacité à atténuer une croissance néoplasique, comportant les étapes consistant à :
- administrer une substance test à un animal, à l'exception d'humains, qui porte un allèle APC mutant dans son génome,
- déterminer si ladite substance test empêche ou atténue la croissance de tumeurs.

23. Animal transgénique, à l'exception d'humains, qui porte dans son génome un allèle APC mutant provenant d'une seconde espèce animale.

24. Animal, à l'exception d'humains, qui a été génétiquement manipulé pour contenir une mutation par insertion ou délétion qui rompt un allèle APC dans son génome.

25. Molécule d'ADNc qui code pour une protéine APC.

26. Molécule d'ADNc qui code pour une protéine ayant la séquence d'acides aminés représentée sur la figure 3 ou 7 (SEQ ID n° 7 ou 1).

27. Molécule d'ADN isolée qui code pour une protéine APC.

28. Molécule d'ADN isolée qui code pour une protéine ayant la séquence d'acides aminés représentée sur la figure 3 ou 7 (SEQ ID n° 7 ou 1).

29. Chromosome artificiel de levure provenant du clone 37HG4 de YAC ayant le numéro de série NCIMB 40 353.

30. Utilisation d'un gène APC du type sauvage pour la fabrication d'un médicament destiné à affecter une fonction d'un gène APC du type sauvage à une cellule qui a perdu ladite fonction du fait d'une mutation du gène APC, dans laquelle ledit gène APC du type sauvage après introduction dans la cellule est exprimé dans la cellule.

31. Utilisation selon la revendication 3C, dans laquelle ledit gène APC du type sauvage introduit se recombine avec le gène APC mutant endogène présent dans la cellule par un événement de double recombinaison pour corriger la mutation du gène APC.

32. Utilisation d'une partie d'un gène APC du type sauvage pour la fabrication d'un médicament destiné à affecter une fonction d'un gène APC du type sauvage à une cellule qui a une fonction d'un gène APC altérée du fait d'une mutation dans un gène APC, dans laquelle ladite partie, après introduction dans la cellule, est exprimée dans la cellule, ladite partie codant pour une partie de la protéine APC qui est nécessaire pour une croissance non-néoplasique de ladite cellule.

33. Utilisation d'une protéine APC humaine du type sauvage pour la fabrication d'un médicament destiné à affecter une fonction d'un gène APC du type sauvage à une cellule qui a une fonction d'un gène APC altérée du fait d'une mutation dans un gène APC.

34. Utilisation d'une molécule qui imite la fonction d'une protéine APC du type sauvage pour la fabrication d'un médicament destiné à affecter une fonction d'un gène APC du type sauvage à une cellule qui a une fonction d'un gène APC altérée du fait d'une mutation dans un gène APC.

35. Procédé selon l'une quelconque des revendications 1, 13 ou 14, dans lequel l'altération est détectée en utilisant le Système de Mutation Résistant à une Amplification (ARMS).

36. Procédé selon l'une quelconque des revendications 1, 2, 3, 4, 13, 14, 15, 16 ou 18, dans lequel l'altération est sélectionnée parmi le groupe constitué des altérations suivantes :
(a) transition de C en T au niveau du codon 414 ayant pour résultat un changement de Arg (CGC) en Cys (TGC),
(b) transition de C en T au niveau du codon 3C2 ayant pour résultat un changement de Arg (CGA) en codon d'arrêt (TGA),
(c) transition de C en G au niveau du codon 280 ayant pour résultat un changement de Ser (TCA) en codon d'arrêt (TGA),
(d) transition de C en G au niveau du codon 713 ayant pour résultat un changement de Ser (TCA) en codon d'arrêt (TGA),
(e) transition de C en T au niveau du codon 332 ayant pour résultat un changement de Arg (CGA) en codon d'arrêt (TGA),
(f) insertion de 5 paires de bases (CAGCC; entre les codons 289 et 290, ayant pour résultat un codon d'arrêt au niveau du codon 292 du fait d'un décalage du cadre de lecture,
(g) transition de C en G au niveau du codon 1338 ayant pour résultat un changement de Glu (CAG) en codon d'arrêt (TAG),
(h) perte d'un T autour du codon 45c entraînant un décalage du cadre de lecture ayant pour résultat un codon d'arrêt en aval,
(i) perte de AG autour du codon 243 ayant pour résultat une jonction d'épissure,
(j) transition de T en C au niveau du ccdcn 437 ayant peur résultat un site donneur d'épissure (CAA/gtaa→CAA/gcaa).

37. Processus qui comporte la préparation d'une préparation de la protéine APC humaine pratiquement exempte d'autres protéines humaines, la séquence d'acides aminés de ladite protéine correspondant à celle représentée sur la figure 3 ou 7 (SEQ ID n° 7 ou 1).

38. Processus qui comporte la préparation d'une préparation d'anticorps immunoréactifs avec une protéine APC humaine et pratiquement non-immunoréactifs avec d'autres protéines humaines.

39. Processus qui comporte la préparation d'une molécule d'ADNc qui code pour une protéine APC ou d'une molécule d'ADNc qui code pour une protéine ayant la séquence d'acides aminés représentée sur la figure 3 ou 7 (SEQ ID n° 7 ou 1).

40. Processus qui comporte la préparation d'une molécule d'ADN isolée qui code pour une protéine APC ou d'une molécule d'ADN isolée qui code pour une protéine ayant la séquence d'acides aminés représentée sur la figure 3 ou 7 (SEQ ID n° 7 ou 1).
